(19) Europäisches Patentamt
European Patent Office
Office européen des brevets

(11) EP 2 647 645 B1

(12) **EUROPEAN PATENT SPECIFICATION**

(45) Date of publication and mention of the grant of the patent:
**27.07.2016 Bulletin 2016/30**

(51) Int Cl.:
***C07K 14/065*** *(2006.01)* ***C12N 15/863*** *(2006.01)*
***A61K 39/275*** *(2006.01)* ***A61P 31/20*** *(2006.01)*
***A61P 37/00*** *(2006.01)*

(21) Application number: **13175420.2**

(22) Date of filing: **17.12.2002**

(54) **Recombinant proteins of Parapoxvirus ovis and pharmaceutical compositions therefrom**

Rekombinante Proteine des Parapoxivirus Ovis und pharmazeutische Zusammensetzungen daraus

Protéines recombinées de Parapoxvirus ovis et compositions pharmaceutiques produites à partir de celles-ci

(84) Designated Contracting States:
**CH DE ES FR IT LI**

(43) Date of publication of application:
**09.10.2013 Bulletin 2013/41**

(62) Document number(s) of the earlier application(s) in accordance with Art. 76 EPC:
**10178276.1 / 2 351 769**
**02808241.0 / 1 575 614**

(73) Proprietor: **AiCuris GmbH & Co. KG**
**42117 Wuppertal (DE)**

(72) Inventors:
- **Weber, Olaf**
  **42489 Wülfrath (DE)**
- **Friederichs, Sonja-Maria**
  **53721 Siegburg (DE)**
- **Siegling, Angela**
  **1230 Wien (AT)**
- **Schlapp, Tobias**
  **47574 Goch (DE)**
- **Mercer, Andrew Allan**
  **9016 Dunedin (NZ)**
- **Fleming, Stephen Bruce**
  **Dunedin (NZ)**
- **Volk, Hans-Dieter**
  **10117 Berlin (DE)**

(74) Representative: **Kilger, Ute**
**Boehmert & Boehmert**
**Anwaltspartnerschaft mbB**
**Patentanwälte Rechtsanwälte**
**Pettenkoferstrasse 20-22**
**80336 München (DE)**

(56) References cited:
**WO-A1-00/25805**

- **MERCER A A ET AL: "A novel strategy for determining protective antigens of the parapoxvirus, Orf virus", VIROLOGY, ACADEMIC PRESS,ORLANDO, US, vol. 229, no. 1, 1 January 1997 (1997-01-01), pages 193-200, XP002249038, ISSN: 0042-6822, DOI: DOI:10.1006/VIRO.1996.8433**
- **ASTRID FRIEBE: "Immunmodulatorische Wirkung von inaktivierten Parapox-ovis-Viruspartikeln", DISSERTATION DER MEDIZINISCHEN FAKULTÄT CHARITE DER HUMBOLDT-UNIVERSITÄT ZU BERLIN,, 1 January 2002 (2002-01-01), page 94pp, XP009170360,**
- **MERCER A A ET AL: "THE ESTABLISHMENT OF A GENETIC MAP OF ORF VIRUS REVEALS A PATTERN OF GENOMIC ORGANIZATION THAT IS HIGHLY CONSERVED AMONG DIVERGENT POXVIRUSES", VIROLOGY, ACADEMIC PRESS,ORLANDO, US, vol. 212, no. 2, 1 October 1995 (1995-10-01), pages 698-704, XP002032169, ISSN: 0042-6822, DOI: DOI:10.1006/VIRO.1995.1527**
- **FLEMING S B ET AL: "SEQUENCE AND FUNCTIONAL ANALYSIS OF A HOMOLOG OF INTERLEUKIN-10 ENCODED BY THE PARAPOXVIRUS ORF VIRUS", VIRUS GENES, KLUWER ACADEMIC PUBLISHERS, BOSTON, US, vol. 21, no. 1/02, 1 August 2000 (2000-08-01), pages 85-95, XP009010497, ISSN: 0920-8569, DOI: DOI:10.1023/B:VIRU.0000018443.19040.99**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **HAIG D M ET AL: "ORF", VETERINARY RESEARCH, ELSEVIER, PARIS, NL, vol. 29, 1 January 1998 (1998-01-01), pages 311-326, XP002923564, ISSN: 0928-4249**
- **CASTRUCCI G ET AL: "Further investigations on the efficacy of a non-specific defence inducer evaluated in calves exposed to infectious bovine rhinotracheitis virus", COMPARATIVE IMMUNOLOGY MICROBIOLOGY AND INFECTIOUS DISEASES, vol. 21, no. 2, April 1998 (1998-04), pages 155-163, ISSN: 0147-9571**


Remarks:

The file contains technical information submitted after the application was filed and not included in this specification

## Description

Field of the invention

The present invention relates to polynucleotides of *Parapoxvirus ovis* (PPVO) and their use, alone or in combination with other substances, for the manufacture of pharmaceutical compositions.

Background of the invention

It is known that latent and chronically persistent viral infections can be activated or reactivated by immunosuppression, or conversely that the immune system suppresses acute diseases which may be caused by a latent virus (for example a latent herpes virus infection recurs as a result of immunosuppression in the form of lip vesicles in cases of stress or the administration of cortisone). It is also known that chronically persistent latent viral infections can only be treated with difficulty or not at all using conventional low-molecular-weight antiviral substances.

It was demonstrated that class I restricted cytotoxic T cells were capable of inhibiting hepatocellular HBV gene expression in HBV-transgenic mice, and that this process was caused by TNF-$\alpha$ and IFN-$\gamma$.

It is also known that in the case of chronically persistent viral infections a superinfection with another virus can produce antiviral effects against the chronically persistent virus. The dependence of this effect on interferons such as IFN-$\gamma$, as well as other cytokines and chemokines, such as TNF-$\alpha$, which are secreted by T cells, natural killer cells and macrophages, has been demonstrated.

BAYPAMUN®, a pharmaceutical product for inducing "paraspecific immunity", i.e., a pharmaceutical product for inducing the unspecific immune system, is used therapeutically, metaphylactically and prophylactically for the treatment of animals in need. BAYPAMUN® is manufactured from chemically inactivated PPVO strain D1701 (see German Patent DE3504940). The inactivated PPVO induces in animals non-specific protection against infections with the most diverse types of pathogens. It is assumed that this protection is mediated via various mechanisms in the body's own defense system. These mechanisms include the induction of interferons, the activation of natural killer cells, the induction of "colony-stimulating activity" (CSA) and the stimulation of lymphocyte proliferation. Earlier investigations of the mechanism of action demonstrated the stimulation of interleukin-2 and interferon-$\alpha$.

The processes for the production of the above-mentioned pharmaceutical compositions are based on the replication of the virus in cultures of suitable host cells.

One aspect of the invention relates to the use of particle-like structures comprising recombinant proteins of the invention. These particle-like structures can be, e.g., fusion proteins, protein-coated particles or virus-like particles.

Methods to produce fusion proteins, protein-coated particles or virus-like particles comprising recombinant proteins of the invention are well known to persons skilled in the art: Casal (Biotechnol. Genet. Eng. Rev. 2001, 18: 73-87) describes the use of *baculovirus* expression systems for the generation of virus-like particles. Ellis (Curr. Opin. Biotechnol. 1996, 7(6): 646-52) presents methods to produce virus-like particles and the application of suitable adjuvants. Roy (Intervirology 1996, 39(1-2): 62-71) presents genetically engineered particulate virus-like structures and their use as vaccine delivery systems. Methods to produce fusion proteins are also well known to the person skilled in the art (Gaudin et al., Gen. Virol. 1995, 76: 1541-56; Hughson, Curr. Biol. 1995, 5(3): 365-74; Uhlen et al., Curr. Opin. Biotechnol. 1992, 3(4): 363-369). Known to the person skilled in the art is also the preparation of protein-coated micro- and nanospheres (Arshady,

Biomaterials 1993, 14(1): 5-15). Proteins can be attached to biodegradable microspheres (Cleland, Pharm Biotechnol. 1997, 10: 1-43) or attached to other polymer microspheres (Hanes et al., Pharm. Biotechnol. 1995, 6:389-412) such as, e.g., polysaccharides (Janes et al., Adv. Drug Deliv. Rev. 2001, 47(1): 83-97).

PPVO NZ2 is another Parapoxvirus strain that exhibits immunostimulatory effects when administered in inactivated form to mammals.

The prior art describes the construction of an expression library representing about 95 % of the PPVO NZ2 genome using the *Vaccina lister* virus to create recombinant viruses comprising the complete *Vaccina lister* genome and various fragments of the PPVO genome (Mercer et al. 1997, Virology, 229: 193-200). For the construction of the library, 16 PPVO DNA fragments with an average size of 11,4 kb were inserted into the *Vaccinia lister* genome. Each fragment was mapped relative to the PPVO restriction endonuclease maps but was otherwise uncharacterized (Fig. 1). It was found that a major portion of the PPVO genes were expressed in cells infected by the recombinant virus. The authors also showed that the entirety of all PPVO proteins expressed by some of the recombinant viruses of the expression library was able to provide protection against challenge with virulent PPVO. Expression of PPVO genes of the individual recombinant viruses has been demonstrated by immunofluorescence and immune precipitation (Mercer et al. 1997, Virology, 229: 193-200).

The publication Mercer et al. 1995, Virology 212, 689 - 704 describes sequencing the ends of cloned restriction endonuclease fragments of the NZ2 strain of orf virus and its use of the translated sequences to search protein data bases. Said publication describes 32 genes of the fragments of the NZ2 strain of orf virus having homologs in *vaccinia virus* genes. Comparison of the orf virus gene map with that of the *vaccinia virus* genes showed that each orf virus gene and its *vaccinia virus* counterpart aligned in the same order and orientation on their respective genomes.

The doctoral thesis Friederichs 2001 (Untersuchungen zur Identifikation der immunstimulatorisch wirksamen Baypamun-Komponenten; Technische Universität Dresden, Fakultät Mathematik und Naturwissenschaften, Fachbereich Biologie; submitted by Dipl.-Biol. Sonja Maria Friederichs; public defense July 9, 2001) describes immunostimulatory components of *Parapox ovis* D1701. The immunostimulatory components were tested in an Aujeszky mouse model. Besides this, analysis of comparable *Parapox ovis* NZ2 and NZ7 strains showed immunostimulatory effects for such components as well. Specifically, 4 of 16 recombinant NZ2 candidate fragments (VVOV 215, 245, 285, and 330) - but not VVOV82 - were described as having immunostimulatory activity.

To identify components of PPVO responsible for the vaccinating activity of PPVO, the *Vaccinia lister*/ PPVO NZ2 expression library was applied.

Based on the above background it was desirable to develop PPVO based pharmaceutical compositions with antiviral and anti-tumor efficacy as well as with efficacy in paraimmunization and other desirable therapeutic effects. It was also desirable to obtain a pharmaceutical composition that exerts its full therapeutic effect while showing fewer side effects. It was furthermore desirable to find methods to produce PPVO based pharmaceutical compositions in large quantities and in economically advantageous manners.

These desirable effects have been achieved by the systematic use of selected recombinant proteins of PPVO alone or in combination with other recombinant proteins from PPVO for the preparation of pharmaceutical compositions for the treatment of objects in need.

## Summary of the invention

The invention relates to polynucleotides coding for the PPVO viral genome, to fragments of the polynucleotides coding for the PPVO genome and to polynucleotides coding for open reading frames (ORFs) of the PPVO viral genome.

"Fragments" of a polynucleotide shall be understood as polynucleotides that have the same nucleotide sequence as contiguous parts of the full length (the original) polynucleotide.

"Active fragments shall be those fragments of the PPVO genome the expression products of which have demonstrated to be pharmacologically active according to the invention, when inserted into the *Vaccina lister* genome and expressed in a suitable host.

Whereas the use of the complete PPVO virus for the manufacture of vaccines against PPVO challenge has been described, the present invention relates to the use of polynucleotides coding for the PPVO viral genome and selected fragments of the PPVO viral genome, alone or in combination with others, for the preparation of improved pharmaceutical compositions for the treatment of viral hepatitis.

The systematic use of selected genomic fragments of PPVO makes it possible to produce pharmaceutical compositions, which contain fewer (and may not contain any) inactive components (i.e., polynucleotides and proteins of PPVO) in addition to the active components.

These pharmaceutical compositions which contain less, or do not contain any additional inactive components are generally preferred by doctors and patients compared to the less well-defined biological preparations of inactivated virus material. Furthermore, the possibility of producing the recombinant product in fermentation processes allows an economically advantageous mode of production. It is well known to persons skilled in the art that an economically advantageous mode of production can be achieved, e.g., by using rapidly growing production organisms (host organisms), which might also place low demands on the culture medium employed. Microorganisms which can advantageously be used as hosts for the production of recombinant proteins include, e.g., but are not limited to, *Escherichia coli, Bacillus spec., Corynebacterium spec., Streptomyces spec.,* as well as yeasts, e.g., *Saccharomyces cerevisiae, Candida spec., Pichia spec., Hanselula spec.,* and filamentous fungi, e.g., *Aspergillus spec., Penicillium spec.,* and other suitable microorganisms.

Recombinant proteins can also be produced from cell lines expressing the proteins of interest. These cell lines can be recombinant mammalian cell lines, recombinant insect cell lines (e.g., using the *baculovirus* transfection system) or other suitable expression systems. Transfection can be achieved by various techniques known to the skilled person, one of which is the use or recombinant viruses such as the *Vaccinia virus*/PPVO recombinants (VVOVs) described in the examples.

## Description of the invention

The invention relates to a purified and isolated fragment of the PPVO genome with a sequence consisting of nucleotides 122616 to 136025 of SEQ ID NO: 01 and to the use thereof for the preparation of pharmaceutical compositions.

A protein is any polypeptide of at least five amino acids. A recombinant protein is any protein that is expressed in a cell, into which the coding polynucleotide was introduced using recombinant DNA technology.

A polynucleotide, within the meaning of the invention, is meant to comprise, polyribonucleotides and/or polydesoxyribonucleotides.

Pharmaceutical compositions can be used as immunotherapeutic or immunoprophylactic agents for the treatment of infectious immunodeficiencies. They can be used for the treatment of viral hepatitis. It is an object of the invention to use polynucleotides with a sequence consisting of nucleotides 122616 to 136025 of SEQ ID NO: 01 of PPVO for the production of pharmaceutical compositions for the treatment of the above mentioned condition and disease in humans and animals.

The viral strains disclosed herein are PPVO NZ2 and homologues, such as D1701, NZ7, NZ10 and orf-11 strains. It is also possible to use polynucleotides of the progeny of these strains obtained by passaging and/or adaptation using specific cells, such as e.g. WI-38, MRC-5 or Vero cells.

Recombinant proteins are effective for the treatment of viral diseases, cancer and other diseases or conditions in which a Th1 type immune response is of benefit. The results obtained also imply that PPVO gene products or parts thereof protect hepatitis virus-expressing hepatocytes (e.g. hepatitis B virus, HBV, or hepatitis C virus, HCV) from immune attack through HBV or HCV specific cytotoxic CD8+ T cells circulating in the blood because T cells will not leave the blood stream if their specific antigen is not presented by liver sinus endothelial cells (LSEC, that anatomically separate hepatocytes from T cells passing the liver with the blood). Recombinant protein that is derived from the ORFs 120-R3 (base pairs 122616 - 136025 Bp, recombinant virus VVOV82) may be able to down-modulate or prevent side effects such as necroinflammatory liver disease when immunostimulants, e.g. cytokines or any others including the proteins described above when administered to e.g. hepatitis patients, but is not part of the inventive subject-matter.

Considering the knowledge about the influence of a Thl type immune induction in conditions and diseases such as latent and or chronic viral infections, proliferative diseases such as cancer and the capability of recombinant proteins that contain gene products of PPVO or parts thereof to induce a Thl immune response or a local immune response selectively, we claim the use of an isolated and purified polynucleotide of PPVO as described above that contain gene products of PPVO for the manufacture of pharmaceutical compositions for use in humans and animals.

Recombinant proteins of PPVO shall be understood as proteins that derive from PPVO and are expressed in homologous or heterologous systems other than the systems in which PPVO is naturally produced. Examples for recombinant proteins of PPVO are proteins of PPVO, which are expressed using *Vaccinia virus* vectors and fibroblasts as host cells or *baculovirus* vectors and insect cells as host cells. Recombinant proteins could also be produced in bacterial cells (e.g., *Escherichia coli, Bacillus spec., Streptomyces spec.*) or in yeast (e.g. *Saccharomyces cerevisiae, Candida spec., Pichia pastoris*, *Hansenula spec.*) systems. In these cases, polynucleotides of the PPVO genome would typically be brought into the respective host genome so that PPVO genes are expressed by the host. Recombinant proteins of PPVO could also be expressed by the object in need in the sense of a gene therapy, but this is not part of the present invention.

Recombinant proteins can also be recombinant virus particles that contain PPVO derived proteins. Recombinant proteins could also be in form of viral-like particles that are formed or assembled from PPVO derived proteins. Recombinant proteins could also be chimeric proteins that contain PPVO gene products, but they are not part of the present invention.

Immunomodulating activity is defined as local or systemic suppression and/or stimulation and/or induction of any Th-1 or Th-2 type cytokine response or of any effector function of these cytokines, (e.g. cytolytic or antiviral activity or humoral response) or the modulation of MHC cross-presentation. Immunomodulating activity could also be the induction of apoptosis in antigen presenting cells or recruiting of antigen presenting cells.

Nucleotides of the invention can be administered at the same time or sequentially, administered with other agents and drugs, e.g. with drugs that treat the disease or are supportive.

The nucleotides can be administered systemically (e.g., intravenously, subcutaneously, intramuscularly, intracutaneously, intraperitoneally), locally or orally (per os). Pharmaceutical compositions of the invention can be administered, e.g., oral, nasal, anal, vaginal etc., as well as parenteral administration. Pharmaceutical compositions of the invention can be in the form of suspensions, solutions, syrups, elixirs or appropriate formulations in polymers as well as liposomes.

The invention relates to purified and isolated polynucleotides with the sequence of nucleotides 122616 to 136025 of SEQ ID 01.

The invention also relates to the use a purified and isolated polynucleotide which is an active fragment of the PPVO genome, with a sequence consisting of nucleotides 122616-136025 of SEQ ID 01 (PPVO insert of VVOV 82) that is comprised in a pharmaceutical composition as defined in the claim.

The disclosure also relates to purified and isolated polynucleotide which encode for the same amino acid sequence as the active fragments of the PPVO genome of the previous paragraph and to polynucleotides of at least 15 or 30 or 100 nucleotides binding under stringent conditions to the above mentioned active fragments of the PPVO genome or its complementary sequence.

Also disclosed herein are vectors containing polynucleotides and cells containing these vectors or polynucleotides.

The invention also relates to the use of the polynucleotide of the invention for the manufacture of pharmaceutical compositions.

The invention also relates to the use of the polynucleotide of the invention for the manufacture of pharmaceutical compositions for the treatment of viral hepatitis.

Brief description of the figures

Figure 1 shows the genomic locations of the DNA fragments constituting the insertion library. The position of each DNA fragment is shown against the KpnI map of PPVO NZ2 (Mercer et al. 1997, Virology, 229: 193-200).

## Examples

**Example 1:** Determination of the integrated PPVO Fragments in the active VVOVs.

DNA preparation from *Vaccinia lister*/PPVO recombinants was performed as follows:

BK-KL 3A cells were grown to confluency in 175 $cm^2$ flasks (Becton Dickson Labware, Heidelberg, Germany). Cells were infected with a recombinant *Vaccina lister*/PPVO virus (VVOV) of Mercer et al. (1997, Virology, 229: 193-200) at a MOI (multiplicity of infection) of 0.01-0.32 and incubated at 37°C until 100 % CPE (cytopathic effect) had been reached. The infected cells were frozen at -80°C, thawed and processed as follows, with modification to the RNA extraction method of Vilcek et al. (1994, J. Clin. Microbiol. 32: 2225-2231). Using 2 ml PLG Heavy Eppendorf tubes (Eppendorf, Hamburg, Germany) 0.5 ml aliquots of cellular suspension were incubated with 100 μg Proteinase K (Roche Molecular Biochemicals, Mannheim, Germany) and 50 μl SDS (Sigma-Aldrich, Chemie GmbH, Taufkirchen, Germany) at 56°C for 25 min. 0.5 ml Roti®-Phenol/Chloroform (Carl Roth GmbH, Karlsruhe, Germany) was added and the tubes were inverted for several times. After centrifugation at 12000 x g for 10 min, the upper phase was transferred into a fresh tube and two volumes of ethanol (Merck Eurolab GmbH, Darmstadt, Germany) and 1/10 volume of sodium acetate (Sigma-Aldrich, Chemie GmbH, Taufkirchen, Germany) was added. The reagents were mixed several times and stored at -80°C for 3 h. The tubes were centrifuged at 14000 x g for 30 min, the supernatant was decanted and the pellet was air-dried for 5-10 min. Finally the DNA pellet was resuspended in 30 μl nuclease free water and stored at -20°C until used.

DNA concentration was measured spectrophotometrically on a Bio-Photometer 6131 (Eppendorf, Hamburg, Germany) at 260/280 nm. The DNA yield of different sample preparations spanned from 100 ng/ml up to 1 μg/ml.

Polymerase chain reaction (PCR) of terminal flanking regions of the integrated fragments in the *Vaccinia lister*/*PPVO* recombinants was performed as follows:

Three different PCR amplification systems were used for amplifying the terminal flanking regions. Each reaction mixture of 50 μl contained 100 ng - 1 μg resuspended DNA and primers (Table 1) were added in a final concentration of 300 nM. Amplifications were carried out on a Mastercycler® gradient (Eppendorf, Hamburg, Germany).

The 3-prime flanking region of recombinant VVOV 285 had been analyzed using 2 x Ready-Mix™ PCR Master Mix (1.5 mM MgCl2) (AB Gene, Hamburg, Germany). 1 μl BSA (MBI Fermentas GmbH, St. Leon-Rot, Germany) was added to each reaction. Denaturation was performed at 94°C for 3 min, followed by 30 cycles (94°C for 30 s, 58.7°C - 65.3°C for 30 s, 72°C for 1 min) and 72°C for 5 min.

The 5-prime flanking region of the PPVO insert of recombinant VVOV 285, the 3-prime flanking region of VVOV 97, and both terminal flanking regions of VVOV 215, VVOV 243, WOV 245 were amplified using PfuTurbo® DNA Polymerase (Stratagene, Amsterdam, Netherlands). The reactions were setup with 2.5 U of enzyme, 1.5 mM MgCl2 and 200 μM of each dNTP. Denaturation was performed at 94°C for 3 min, followed by 30 cycles (94°C for 30 s, 58.7°C - 65.3°C for 30 s, 72°C for 1 min) and 72°C for 5 min.

The amplification of the 5-prime flanking region of VVOV 97 and VVOV 82, the 3-prime flanking region of VVOV 96 and VVOV 283 were performed with Piatinium® Pfx DNA Polymerase (Life Technologies GmbH, Karlsruhe, Germany). A reaction of 50 μl contained 1.25 U polymerase, 1-1.5 mM $MgCl_2$ and 300 μM of each dNTP. Additional use of PCRx Enhancer Solution was necessary for amplification of the 5-prime flanking regions of VVOV 96 (1x concentrated) and the 3-prime flanking regions of VVOV 82 (2x concentrated). Denaturation was performed at 94°C for 2 min, followed by 30 cycles (94°C for 15 s, 54.6°C - 60.7°C for 30 s, 68°C for 1-2 min) and 68°C for 5-7 min.

18 μl of each amplification product was analyzed by agarose gel electrophoresis on 1.5-2 % SeaKem LE agarose (Biozym, Hessisch Oldendorf, Germany). After staining in an ethidium bromide solution for 20 min the DNA fragments were visualized on an UV transilluminator UVT-20 M/W (Herolab, Wiesloch, Germany).

The sequence of the amplified DNA-fragments were determined by standard sequencing procedures and compared to the published *Vaccinia lister* thymidine kinase-sequence and the genome sequence of PPVO NZ2 to determine exactly the integrated PPVO NZ2 sequences.

**Table 1**

PCR-primers, amplification and sequencing of the terminal flanking regions of the integrated fragments in the *Vaccinia lister*/PPVO NZ2 recombinants

**Table 1**

| VVOV | Amplified terminal region of NZ2 insert | Primers used for amplification | | Length of amplification product [bp] |
|---|---|---|---|---|
| | | Primer name | Sequence 5' → 3' | |
| VVOV215 | 5' | VAC-P11-1 | ATTACAGTGATGCCTACATGCCG | 264 |
| | | PPVO 14r-1 | GCTGTAGTCGTGGTCCGGC | |
| | 3' | PPVO 4r-2 | CTTCCTAGGCTTCTACCGCACG | 402 |
| | | VAC-TK-1 | CGGTTTACGTTGAAATGTCCCAT | |
| VVOV245 | 5' | VAC-P11-1 | ATTACAGTGATGCCTACATGCCG | 553 |
| | | PPVO 57-1 | CTGGCCAACGACGCCTTC | |
| | 3' | PPVO 40-1 | TCTGGTACCCCTTGCCGG | 321 |
| | | VAC-TK-1 | CGGTTTACGTTGAAATGTCCCAT | |
| VVOV 285 | 5' | VAC-P11-1 | ATTACAGTGATGCCTACATGCCG | 241 |
| | | PPVO 78r-5 | GAACCCGCTCTCGCTCGA | |
| | 3' | PPVO 64r-1 | GCCGGGCAAGTGTCTGGTC | 320 |
| | | VAC-TK-1 | CGGTTTACGTTGAAATGTCCCAT | |
| VVOV 330 | 5' | VAC-P11-1 | ATTACAGTGATGCCTACATGCCG | 392 |
| | | PPVO 92-1 | CTCGAAGTAGCTGATGTCGCG | |
| | 3' | PPVO. 96r-1 | AGAGCTTTACGTAGACTCTCCAAGTGTC | 462 |
| | | VAC-TK-1 | CGGTTTACGTTGAAATGTCCCAT | |
| WOV 96 | 5' | VAC-TK-fwd | ATACGGAACGGGACTATGGACG | 239 |
| | | PPVO 22r-3 | GCGGTGGCCATGTACGTG | |
| | 3' | PPVO 22r-4 | GGTTGTGGCGATGGTCGG | 1055 |
| | | VAC-TK-1 | CGGTTTACGTTGAAATGTCCCAT | |
| VVOV 97 | 5' | VAC-TK-fwd | ATACGGAACGGGACTATGGACG | 309 |
| | | PPVO 18r-1 | CTTGATGAGCCGGACGCA | |
| | 3' | PPVO 25r-1 | CCGAGTTGGAGAGGAAGGAGC | 318 |
| | | VAC-TK-1 | CGGTTTACGTTGAAATGTCCCAT | |
| VVOV 243 | 5' | VAC-P11-1 | ATTACAGTGATGCCTACATGCCG | 478 |
| | | PPVO 79-1 | CTGTTGGAGGATGAGGTCAAGGA | |
| | 3' | PPVO 71r-1 | CGTGCTCATGCCTGTGGAC | 269 |
| | | VAC-TK-1 | CGGTTTACGTTGAAATGTCCCAT | |

(continued)

| VVOV | Amplified terminal region of NZ2 insert | Primers used for amplification | | Length of amplification product [bp] |
|---|---|---|---|---|
| | | Primer name | Sequence 5' → 3' | |
| VVOV283 | 5' | | | |
| | | | | |
| | 3' | PPVO 92-4 | CGACATCCTCACCTGCAAGAAG | 234 |
| | | VAC-TK-1 | CGGTTTACGTTGAAATGTCCCAT | |
| VVOV 82 | 5' | VAC-TK-fwd | ATACGGAACGGGACTATGGACG | 275 |
| | | PPVO 120-1 | TACAGGCAGCCCGTGACC | |
| | 3' | PPVO R3R4-3 | GCCGTGTGTCACGTTGATGC | 1960 |
| | | VAC-TK-1 | CGGTTTACGTTGAAATGTCCCAT | |

Example 2: Induction of interferon gamma and tumor necrosis factor alpha by PPVO gene products

The 16 recombinants were tested of their ability to induce tumor necrosis factor alpha (TNF-a) and interferon gamma (IFN-γ) in whole blood cultures.

Whole blood cultures containing blood and RPMI medium (Life Technologies GmbH, Karlsruhe, Germany) in the ratio of 1:5 were stimulated with the recombinant viruses. A pure *Vaccinia lister* and a whole PPVO preparation served as controls. All preparations were used at a final dilution of 1:10. The stimulation for the IFN-γ determination was done together with ConcanavalinA (SIGMA, St. Louis, MO), because the virus alone does not induce IFN-γ. Then the cells were incubated for 24 h (TNF-α) and or 72 h (IFN-γ). The cytokine concentration was then determined in the cell culture supernatants by TNF-α or IFN-γ specific ELISA. These time points were found to be optimal when the experimental conditions were determined using whole PPVO as a control.

It was possible to identify 5 active recombinant viruses (VVOV 96, VVOV 97, VVOV 243, VVOV 285, and (VVOV 330) that induced both TNF-α and IFN-γ secretion and, thus, could mimic the effect of the whole PPVO. The results are depicted in Table 2.

<u>**Table 2**</u>

TNF-α was determined after 24 h stimulation of blood cells with the recombinant virus or the controls, respectively. IFN-γ was determined after 72 h stimulation of blood cells with the recombinant virus or the controls. Stimulation was performed in the presence of the mitogen ConA. The relative induction in percent of the *Vaccinia virus* control is shown. Therefore, values greater than 100 % are due to the activity of the PPVO fragments. Active PPVO fragments are in bold. The data represent mean values of three different blood donors.

<u>**Table 2**</u>

| Recombinant Virus Clone or control | Interferon Induction (%) | TNF Induction (%) |
|---|---|---|
| Vaccinia virus control | 100 | 100 |
| NZ-2 control | 2224 | 264 |
| VVOV 80 | 200 | 66 |
| VVOV 82 | 173 | 65 |
| VVOV 85 | 209 | 94 |
| VVOV 86 | 138 | 73 |
| **VVOV 96** | <u>**1638**</u> | <u>**1016**</u> |

(continued)

| Recombinant Virus Clone or control | Interferon Induction (%) | TNF Induction (%) |
|---|---|---|
| **VVOV 97** | **1713** | **1285** |
| VVOV 212 | 94 | 62 |
| VVOV 213 | 192 | 38 |
| VVOV 215 | 97 | 82 |
| VVOV 216 | 197 | 71 |
| **VVOV 243** | **1446** | **933** |
| VVOV 245 | 98 | 45 |
| VVOV 247 | 85 | 74 |
| VVOV 283 | 115 | 78 |
| **VVOV 285** | **1128** | **1127** |
| **VVOV 330** | **1762** | **2135** |

**Table 3**

The recombinant *Vaccinia lister*/ PPVO viruses that induce both interferon gamma and TNF-$\alpha$ expression are listed in column 1, the corresponding PPVO sequence in column 2 and all open reading frames (ORFs) that are completely or partially contained in the recombinant are depicted in column 3.

**Table 3**

| Active recombinant PPVO *Vaccinia virus* | PPVO NZ2 Sequence [Bp] that is contained in the recombinant | PPVO NZ2ORFs that are contained in the recombinant |
|---|---|---|
| VVOV 97 | 24056 - 33789 | 18r - 25r |
| VVOV 96 | 31003 - 46845 | 22r - 39 |
| VVOV 285 | 74804 - 86576 | 64r - 76 |
| VVOV 243 | 82324 - 92502 | 71r - 79 |
| VVOV 330 | 102490- 108393 | 92 - 96r |

**Example 3:** Local immunomodulation by PPVO gene products in liver sinus endothelial cells (LSEC)

We have established a new cell-based assay system that allows testing of hepatoprotective properties of recombinant PPVO proteins expressed in different systems (e.g., *Vaccinia virus*). This assay system uses primary murine liver cells, which play the central role in deciding whether immunity or tolerance is induced in the liver, the LSEC. The unique ability of LSEC to present exogenous antigens to CD8+ T cells on MHC class I molecules allows immune surveillance of hepatocytes as viral antigens released by infected hepatocytes are likely to be taken by LSEC and presented to cells of the immune system. The new assay allows to measure the ability of LSEC to interact antigen-specifically with CD8+ T cells, that are responsible for tissue destruction in necroinflammatory hepatitis.

Pure populations of LSEC are isolated from murine liver by a stepwise procedure of portal-vein perfusion with collagenase A (0.05 %), mechanical dispersion and further enzymatic digestion in a rotatory waterbath for 40 min at 37°C (245 rpm), gradient centrifugation (metrizamide 1.089g/cm$^3$) and centrifugal elutriation using a Beckman Avanti J25I

centrifuge equipped with a JE-6B rotor and a standard elutriation chamber. LSEC cell populations isolated by this method are typically around 95-99 % pure as measured by uptake of endothelial cell specific substrate (acetylated low density lipoprotein). LSEC were seeded onto collagen type I coated 24 well tissue culture plates at a density of 100.000 cells

per well and were further cultured in Dulbecco's modified Eagle Medium supplemented with 5 % fetal calf serum (specially tested not to interfere with the assay system) and 2 % glutamine. Three days after isolation, when LSEC gained a postmitotic and quiescent state, we tested for the ability of LSEC to present soluble ovalbumin to (ovalbumin-specific) CD8+ T cells. LSEC were incubated with 1 $\mu$M of ovalbumin for three hours (antigen dose and time were previously shown to be optimal for testing of substances suspected to influence antigen-presentation), washed and incubated with a CD8+ T cell hybridoma (200.000 cells/well) that recognizes the peptide SIINFEKL. SIINFEKL is recognized in a H2b context and directly binds on the MHC-I molecules. Therefore, it has not to be processed by the cell. This allows to differentiate between accessory functions of LSEC (such as MHC-I expression) and antigen-processing function.

The extent of CD8+ T cell activation was measured by determining the extent of IL-2 release from T cells by specific sandwich ELISA.

Using *Vaccinia virus* expressed recombinant proteins derived from PPVO we have been able to attribute hepatoprotective activity to individual clones. To be able to compare different clones directly with respect to their ability to influence cross-presentation by LSEC, we used equal amounts of "infectious units".

We found that LSEC cross-present exogenous ovalbumin very efficiently on MHC class I molecules ($k^b$) to CD8+ T cells. To our surprise we found if LSEC were incubated with several recombinant PPVO proteins we observed subsequently a potent downregulation of cross-presentation by more than 60 % compared to the mock-treated control that includes all but the active ingredient. Several regions within the genome of PPVO have immunregulatory properties. Especially the region termed 82 (43 % reduction) which is

located at the 3' end of the genome appears to be responsible for the overall effect of PPVO on cross-presentation by LSEC. Further regions (VVOV 215, VVOV 212, VVOV 247 and VVOV 86) bear further immunregulatory potential, although to a lesser degree (around 30 % reduction in cross-presentation). It further appears that genes coding for proteins that downregulate cross-presentation are arranged in clusters. It is of interest to note that we identified two gene clusters coding for proteins that improved cross-presentation (VVOV 330, VVOV 283, VVOV 285, VVOV 97, and VVOV 96). However, for unknown reasons the downregulatory effect of the proteins mentioned above is dominant in the activity of PPVO on cross-presentation.

Our results strongly suggest that PPVO contains a mixture of different proteins that in a complementary way work to eliminate hepatocytes from hepatitis B virus while conserving hepatic integrity and avoiding long lasting damage secondary to hepatic fibrosis. As PPVO contains a gene with high homology to the anti-inflammatory agent IL-10 (located in the 5-prime region of the genome) we wondered whether the potent downregulatory effect of the clone 82 was due to expression of ovine IL-10. This assumes that there is cross-reactivity between murine and ovine IL-10 at the level of receptor recognition. We have been unable to demonstrate involvement of ovine IL-10 on the immunoregulatory potential of PPVO. Recombinant murine IL-10 did not show any influence on cross-presentation through LSEC and several monoclonal antibodies to murine and human IL-10 did not influence PPVO mediated downregulation of cross-presentation. We conclude that the immunoregulatory component of PPVO is probably not IL-10 but a new, so far not identified mediator. The data for the MHC-I cross-presentation - down-modulating recombinant virus are depicted in Table 4, those for the MHC-I cross-presentation - stimulating recombinant viruses in Table 5.

**Table 4**

The recombinant *Vaccinia lister*/ PPVO virus that down-modulates the MHC-I cross presentation is designated in column 1, the corresponding PPVO sequence in column 2 and all open reading frames (ORFs) that are completely or partially contained in the recombinant are depicted in column 3.

**Table 4**

| Active recombinant PPVO Vaccinia virus | PPVO NZ2 Sequence [Bp] that is contained in the recombinant | PPVO NZ2ORFs that are contained in the recombinant |
|---|---|---|
| VVOV 82 | 122616-136025 | 120-R3 |

**Table 5**

The recombinant *Vaccinia lister*/ PPVO viruses that stimulate the MHC-I cross presentation are designated in column 1, the corresponding PPVO sequence in column 2 and all open reading frames (ORFs) that are completely or partially contained in the recombinant are depicted in column 3.

**Table 5**

| Active recombinant PPVO Vaccinia virus | PPVO NZ2-Sequence [Bp] that is contained in the recombinant | PPVO NZ2-ORFs that are contained in the recombinant |
|---|---|---|
| (VVOV 97 | 24056 - 33789 | 18r - 25r |
| WOV 96 | 31003 - 46845 | 22r - 39 |
| VVOV 285 | 74804 - 88576 | 64r - 76 |
| (VVOV 283 | 89,4 -103483 | 78r - 92 |
| VVOV 330 | 102490 - 108393 | 92 - 96r |

**Example 4:** Determination of the immunostimulatory activity of the Vaccinia lister / PPVO recombinants in the Aujeszky mouse model

We also tested the activity of recombinant *Vaccinia lister*/PPVO NZ2-viruses in the Aujeszky mouse model, a lethal challenge model of acute Suid Herpesvirus 1 disease for determining the activity of various immunostimulators (e.g. Baypamun®, CpG oligonucleotides).

a) Conditions employed for the mice

The NMRI mice (outbreed strain HdsWin:NMRI; female; weight: 18-20 g; obtained via Harlan/Winkelmann, Borchen, Germany) were kept in autoclavable polycarbonate crates lined with sawdust in an S2 isolation stall at 20-22°C (atmospheric humidity: 50-60 %) and subjected to an artificial day/night rhythm (illumination from 6:30 h to 18:30 h and darkness from 18:30 h to 6:30 h). They had free access to feed and water.

b) Challenge model

Groups of mice consisting of 10 mice per group were used for the tests. All of the animals in one group were given the same test substance.

After the mice were supplied they were kept in the animal stall for 2-3 days. Then the *Vaccinia lister*/PPVO NZ2 recombinants were diluted with PBS (Life Technologies GmbH, Karlsruhe, Germany) to a titer equivalent of approx. $10^8$ TCID50/ml and thermally inactivated (twice for one hour at 58°C). Of these solutions 0.2 ml was administered per mouse intraperitoneally,

24 hours after the treatment the mice were infected with the pseudorabies virus of the Hannover H2 strain by intraperitoneal administration. For this purpose the virus was diluted in PBS to a test titer of approx. 104 TCID50/ml and 0.2 ml of this suspension was administered.

As a negative control one group of mice was treated with PBS and then infected. The mice in this group died 3-8 days after infection. A large proportion of the mice treated the *Vaccinia lister*/PPVO NZ2 recombinants VVOV 215, VVOV 245, VVOV 285 or VVOV 330 survived infection with the pseudorabies virus. 10 days after the infection with the virus the test was ended.

The level of induced immunostimulation was determined by comparing the number of dead mice in the PBS control group with the number of dead mice in the test groups and was quantified by the efficacy index (EI). This index indicates the percentage proportion of mice protected against the lethal effects of the Aujeszky virus infection through immune stimulation by the substance to be tested. It is calculated by means of the following formula:

$$\mathrm{EI} = (b\text{-a})/b \times 100,$$

where *b* is the percentage proportion of the dead mice in the control group and a the percentage proportion of the dead mice in the test group.

A chi-square test was used for the statistical evaluation. This test reveals the minimum activity indices indicating a significant difference between the mortality rate of those mice treated with the test substance and those treated with PBS. Activity indices of >= 60 % are significant where at least 5 of the mice used in tests with n=6 mice per group in the PBS control group and at least 7 of the mice used in tests with n=10 in the PBS control group do not survive the infection with the Aujeszky virus.

Altogether 3 separate tests were carried out in each case. The testing of *Vaccinia lister*/*PPVO* NZ2 recombinants in the Aujeszky mouse model shows the following:

Surprisingly, after the treatment of the mice with the *Vaccinia lister*/PPVO NZ2 recombinants VVOV 215, VVOV 245, VVOV 285 or WOV 330 the average activity indices of higher than 60 % demonstrated immunostimulation. By contrast all of the other *Vaccinia lister*/*PPVO* NZ2 recombinants were ineffective. The data is summarized in Table 6.

**Table 6**

The recombinant *Vaccinia listerl* PPVO viruses that protected mice from herpesvirus induced death are designated in column 1, the corresponding PPVO sequence in column 2 and all open reading frames (ORFs) that are completely or partially contained in the recombinant are depicted in column 3.

**Table 6**

| Active recombinant PPVO Vaccinia virus | PPVO NZ2-Sequence [Bp] that is contained in the recombinant | PPVO NZ2 ORFs that are contained in the recombinant |
|---|---|---|
| VVOV215 | 10264 - 20003 | 4r - 14r |
| VVOV 245 | 47952 - 66263 | 40r - 57 |
| VVOV 285 | 74804 - 88576 | 64r - 76 |
| (VVOV 330 | 102490 - 108393 | 92 - 96r |

**Table 7**

Sequences of the Parapox ovis open reading frames. ORFs the names of which end with "r" are encoded on the complementary DNA strand. Base pair positions in the "from" and "to" column are relative to SEQ ID 01.

**Table 7**

| ORF | from | to | N-term | C-term | Comment |
|---|---|---|---|---|---|
| L1 | 3 | 539 | IRGFAG | PQKVFRL | long termal repeat (LTR)-protein, retroviral pseudoprotease |
| L2r | 781 | 449 | MSEGGRL | LLGLLFP | LTR-protein, retroviral pseudopro-tease |
| L3r | 1933 | 1664 | MTVHPPK | VLPPNSL | LTR-protein, retroviral pseudopro-tease |
| L4r | 3269 | 2790 | MHPSPRR | PVSHPFL | LTR-protein, retroviral pseudopro-tease |
| L5 | 2799 | 3851 | MGDREGE | FEDGVKC | LTR-protein, retroviral pseudopro-tease |
| L6 | 2962 | 3753 | MCTVATF | GAPRAGW | LTR-protein, similar to 134r, retroviral pseudoprotease |
| L7r | 3784 | 3122 | MTPTSRE | ARTAPPR | LTR-protein, retroviral pseudopro-tease |
| L8r | 4341 | 4129 | MPGEGQY | NGGLGKI | LTR-protein, retroviral pseudopro-tease |
| 1ar | 4904 | 4428 | MEFCHTE | DTAWYIS | dUTPase |
| 1r | 6517 | 4970 | MLSRESV | RAMLTRP | homolog of G1L in NZ2, Ankyrin-repeats |
| 2r | 8042 | 6684 | MFFWFWC | SGEGVPV | |
| 3r | 9989 | 8070 | MLGFWGK | VLPSVSR | involved in maturation of EEV (Extracellular Enveloped Virions) |
| 4r | 11195 | 10062 | MWPFSSI | EFCKPIN | Phospholipase D-type enzyme |
| 5r | 11493 | 11227 | MLIYGPR | RLLKDFP | homolog of B3L in NZ2 |
| 6 | 11802 | 12038 | MGVVMCG | APAGVTE | |
| 7r | 12358 | 12080 | MPVKVKQ | ASREFIV | ubiquitination protein with RING-finger-motiv (related to yeast proteins APC11 and HRT1) |
| 8r | 13980 | 12364 | MEEELTR | SPMVVFN | no Vaccinia virus homolog |

(continued)

| ORF | from | to | N-term | C-term | Comment |
|---|---|---|---|---|---|
| 9ar | 14826 | 14053 | MIRIGGG | DNMRVDD | |
| 10 | 15080 | 15394 | MDGGVHK | EQMCRRQ | virion core DNA-binding phos-phoprotein |
| 11r | 16838 | 15423 | MAPPVIE | AKNVITH | polyA polymerase |
| 12r | 19021 | 16847 | MLQLLKR | NNRGFRK | |
| 13r | 19704 | 19156 | MACECAS | NNCGISF | interferon resistance protein, homology to mammalian PACT (protein activator of the inter-feron-induced protein kinase) also called PRKRA (dsRNA dependent activator of Interferon-induced proteinkinase),13r-protein con-tains a dsRBDmotiv (double-stranded RNA binding domain) and a 'DRADA'-domain that is typical for RNA-edifing enzymes) |
| 14r | 20314 | 19736 | MDEDRLR | KKGKPKS | RNA polymerase |
| 15 | 20401 | 22101 | MDFVRRK | VVLQGRA | |
| 16 | 22125 | 22940 | MVDSGTH | PENVVLL | |
| 17 | 23003 | 23866 | MASYISG | RTHTVYV | |
| 18r | 26908 | 23873 | MLFEMEL | SKPVFTG | DNA polymerase |
| 19 | 26926 | 27213 | MEPRFWG | AKVRPLV | distant homolog of the ERV1/ALR-protein-family (ERV1: yeast pro-tein, Essential for Respiration and Vegatative growth, ALR: mammal-ian protein, Augmenter of Liver Regeneration) |
| 20r | 27626 | 27216 | MEAINVF | RAYEGML | |
| 21r | 29754 | 27616 | MLLYPKK | LLGDGGD | related to 12r |
| 22r | 32217 | 29800 | MLIRTTD | EAQNMQN | |
| 23r | 33380 | 32418 | MEDERLI | PSPCGGE | |
| 24r | 33602 | 33393 | MDKLYTG | FHYLKLV | |
| 25r | 34466 | 33612 | MKRAVSK | LEAPFNI | DNA binding phosphoprotein |
| 26r | 34735 | 34502 | MESRDLG | LNARRQN | |
| 27r | 35905 | 34739 | MNHFFKQ | RSLYTVL | |
| 28r | 37194 | 35905 | MDKYTDL | PEKPAAP | core protein |
| 29 | 37200 | 39248 | MENHLPD | IEAEPPF | RNA helicase |
| 30r | 41037 | 39229 | MIVLENG | RMGARPR | Zn-protease, involved in virion morphogenesis |
| 31 | 41374 | 42066 | MTFRELI | DSMASRS | late transcription factor |
| 32r | 42336 | 41731 | MRGHPAH | VAPREEL | |
| 33r | 42407 | 41997 | MASDASP | QPSSSRR | Glutaredoxin-like enzyme |
| 34 | 42410 | 43765 | MGIKNLK | PRLLKLR | |
| 35 | 43770 | 43958 | MVFPIVC | LPMLDIS | RNA polymerase |
| 36 | 43980 | 44534 | MREFGLA | AEPPWLV | |
| 37r | 45727 | 44537 | MESSKQA | TRAPPLF | core virion protein precursor |
| 38 | 45760 | 46557 | MTLRIKL | DRSLSCD | late transcription factor |
| 39 | 46567 | 47568 | MGGSVSL | YLLIVWL | |

(continued)

| ORF | from | to | N-term | C-term | Comment |
|---|---|---|---|---|---|
| 40 | 47572 | 48303 | MGAAASI | TEFPPSV | virion protein, related to vaccinia F9L |
| 41 | 46352 | 48621 | MVRRVLL | LCLFSMD | |
| 42r | 49887 | 48634 | MEEKRGR | ARAMVCL | |
| 43 | 49917 | 50693 | MTNLLSL | TGAEAAP | core protein, DNA binding domain |
| 44 | 50719 | 51102 | MAAPTTP | VDVLGGR | |
| 44a | 51059 | 51511 | MDHEKYV | ATLSPGL | |
| 45 | 51584 | 52591 | MEGVEMD | RPLRGGK | polyA polymerase |
| 46 | 52509 | 53066 | MNRHNTR | SVSWLD | RNA polymerase |
| 47r | 53523 | 53023 | MFFRRRA | GRRPPRP | |
| 48 | 53607 | 57473 | MSVVARV | EAAEEEF | RNA polymerase chain 1 |
| 49r | 58070 | 57528 | MGDKSEW | FVCDSPS | tyrosine phosphatase |
| 50 | 57700 | 58662 | MAAAPLR | ATSGVLT | |
| 51r | 59674 | 58673 | MDPPEIT | LLVTAIV | immunodominant envelope protein |
| 52r | 62089 | 59678 | MDSRESI | YMINFNN | RNA polymerase-associated tran-scription specificity factor (also called RAP94) |
| 53 | 62198 | 62881 | MSSWRLK | KAAACKK | late transcription factor |
| 55 | 62909 | 63862 | MRALHLS | NSEQVNG | topoisomerase I |
| 56 | 63858 | 64271 | MDEALRV | FIRAAVA | |
| 67 | 64309 | 66831 | MDAPSLD | LYVFSKR | mRNA capping enzyme |
| 58r | 67266 | 66799 | MEPSAMR | DVQHVDL | virion protein |
| 58ar | 67803 | 67273 | MAGFSQS | TTCVPPQ | |
| 59 | 67915 | 68607 | MATPANA | FSFYSEN | Uracil DNA glycosylase |
| 60 | 68624 | 70984 | MAAPICD | IEDVENK | ATPase, involved in DNA replica-tion |
| 61 | 70994 | 72898 | MNSDVIK | EVSVVNI | early transcription factor |
| 62 | 72938 | 73507 | MSTFRQT | ASPAAKN | RNA polymerase |
| 63 | 73540 | 74211 | MRTYTSL | WGAAVTR | NTP pyrophosphohydrolase |
| 64r | 76120 | 74207 | MTSAHAA | VDPASIA | virion NTPase |
| 65r | 76749 | 76186 | MEGRARF | RFCNYCP | |
| 66r | 77698 | 76799 | MKTDCAS | KLKLLLQ | mRNA capping enzyme |
| 67r | 79343 | 77709 | MNNSWS | AEKVTAQ | rifampicin resistance, virion membrane |
| 68r | 79816 | 79367 | MKRIALS | MALKSLI | late transactivator protein |
| 69r | 80529 | 79858 | MNLRMCG | AACSLDL | late transactivator protein |
| 70r | 80774 | 80529 | MGDNVWF | VLGLEQA | thioredoxin-like protein |
| 71r | 82815 | 80788 | MESPACA | NMCDVLC | major core protein |
| 72r | 83835 | 82834 | MDLRRRF | VDNTGTS | core protein |
| 73 | 83874 | 85583 | MEESVAV | LLNYGCG | RNA-polymerase |
| 74r | 85535 | 84402 | MDRLRTC | AEAAESA | |
| 75r | 88096 | 85574 | MVSVMRK | QEFYPQP | early transcription factor |

(continued)

| ORF | from | to | N-term | C-term | Comment |
|---|---|---|---|---|---|
| 76 | 87759 | 88667 | MFQPVPD | SACRASP | |
| 77r | 88920 | 88642 | MRPCYVT | TRGTQTG | |
| 78r | 91652 | 88938 | MTAPNVH | AVSFDSE | major core protein |
| 79 | 91667 | 92674 | MTAVPVT | VRKLNLI | |
| 80r | 93466 | 92681 | MASEKMA | DLDGGMC | virion protein |
| 81r | 93761 | 93486 | MGLLDAL | RFSAASS | virion membrane protein |
| 82r | 94060 | 93788 | MDIFETL | DIELTAR | virion membrane protein |
| 83r | 94238 | 94080 | MVSDYDP | HFVHSVI | |
| 84r | 94508 | 94242 | MFLDSDT | DMPFSW | |
| 85r | 95571 | 94498 | MGDTVSK | KTINVSR | |
| 86r | 96187 | 95600 | MESYFSY | EDLFFAE | virion membrane protein |
| 87 | 96202 | 97665 | MFGGVQV | GRDLAAV | RNA helicase |
| 88r | 97915 | 97643 | MSAVKAK | PLRDLAR | Zn-finger protein |
| 89 | 98251 | 99537 | MTSESDL | AIARAQP | DNA polymerise processivity factor |
| 90 | 99537 | 99974 | MIVAAFD | NYVLRTN | |
| 91 | 100001 | 101140 | MLALFEF | LKELLGP | intermediate transcription factor |
| 92 | 101168 | 104650 | MEQALGY | SLFSPED | RNA polymerase b-chain |
| 93r | 106354 | 104795 | MESDNAL | GQHAAIW | A-type inclusion bady/Fusion peptide |
| 94r | 107947 | 106400 | MEKLVSD | GRSGAIW | A-type inclusion body/Fusion peptide |
| 95r | 108256 | 107990 | MDENDGE | QTGYSRY | viral fusion protein |
| 96r | 108719 | 108300 | MDAVSAL | LFLKSIL | |
| 97r | 109679 | 108738 | MADAPLV | RELRANE | RNA polymerase subunit |
| 98r | 109861 | 109682 | MEEDLNE | MGQASSA | |
| 99r | 110830 | 110033 | MDVVQEV | ADSDGGN | ATPase |
| 100 | 110208 | 110417 | MRSWFWQ | PLTGMCL | |
| 100a | 110469 | 110651 | MRPKSVG | SGHTKPS | |
| 101 | 110915 | 111397 | MAHNTFE | KYFCVSD | enveloped virion glycoprotein |
| 102 | 111419 | 111913 | MGCCKVP | CMKEMHG | enveloped virion glycoprotein |
| 103 | 111949 | 112485 | MSRLQIL | RKLDVPI | |
| 104 | 112593 | 113450 | MKAVLLL | LNLNPGN | GM-CSF/IL-2 inhibition factor |
| 105r | 113323 | 112967 | MHASLSS | DETLTYR | |
| 106 | 113526 | 114152 | MEVLVII | GEFFYDE | |
| 107 | 114199 | 115236 | MPLFRKL | RDALDGL | |
| 108 | 115353 | 115787 | MACFIEL | TTFSSSE | |
| 109 | 115859 | 116551 | MSSSSSETT | TTGTSTS | |
| 110 | 116729 | 117523 | MACLRVF | CSMQTAR | GM-CSF/IL-2 inhibition factor |
| 111r | 117572 | 117114 | MAIAHTT | FRFRTPG | |
| 112 | 117423 | 118085 | MAATIQI | KRDGYSR | |

(continued)

| ORF | from | to | N-term | C-term | Comment |
|---|---|---|---|---|---|
| 114r | 118968 | 118375 | MEGLMPK | RPISVQK | |
| 115 | 118508 | 119119 | MDSRRLA | LGDSDSD | |
| 116 | 119588 | 120202 | MRLILAL | PQMMRIG | |
| 117 | 120314 | 121231 | MAGFLGA | CKVEEVL | |
| 118 | 121380 | 123920 | MHLHKDP | LAFPSLA | |
| 119 | 121288 | 122256 | MANRLVF | RPMEIDG | |
| 120 | 122350 | 123924 | MENNDGN | RFLPSHK | related to 1r/G1L with Ankyrin-repeats |
| 121 | 123962 | 125566 | MDPAGQR | CSETDRW | |
| 122r | 125193 | 124591 | MSSSAAA | IAPDSRM | |
| 123r | 125689 | 123935 | MTAEASI | DPVYHKK | |
| 123ar | 123839 | 123297 | MPRTTSG | REQTEGL | |
| 124 | 125652 | 126170 | MANREEI | VRVLRRT | |
| 125r | 126121 | 125699 | MTAPTPR | AAYSLAR | |
| 126 | 126279 | 127769 | MADEREA | LACAMRK | related to 1r/G1L with Ankyrin-repeats |
| 127 | 127851 | 128408 | MSKNKIL | SYMTTKM | sheep-like Interleukín 10 |
| 128 | 128520 | 130076 | MLTRCYI | RASGLAE | related to 1r/G1L wih Ankyrin-repeats |
| 129 | 130105 | 131700 | MVGFDRR | CGRRAPE | related to 1r/G1L, with Ankyrin-repeats (NT slightly changed) |
| 130 | 131790 | 133283 | MILARAG | PDAAALS | Kinase |
| 131 | 133246 | 133920 | MPPRTPP | RPAALRA | |
| 132 | 133972 | 134370 | MKLLVGI | RPPRRRR | homolog to the sheep VEGF (Vascular Endothelial Growth Factor) |
| 133a | 14418 | 134693 | MRKKAPR | ARTAPPR | corresponds to L7r |
| R1 | 134402 | 134992 | MMRSGHA | RMHRSEL | LTR-protein (corresponds to L4r), retroviral pseudoprotease |
| R2r | 134853 | 134419 | MCTVATF | SVAPSSA | LTR-protein (corresponds to L6, 134r), retroviral pseudoprotease |
| R3 | 135628 | 135897 | MTVHPPK | VLPPNSL | LTR-protein (corresponds to L3r), retroviral pseudoprotease |
| R4 | 136780 | 137112 | MSEGGRL | LLGLLFP | LTR-protein (corresponds to L2r), retroviral pseudoprotease |
| R5r | 137558 | 137022 | IRGFAGG | PQKWRL | LTR-protein (corresponds to L1r), retroviral pseudoprotease |

SEQUENCE LISTING

<110> Bayer AG, Leverkusen, Germany

<120> Recombinant proteins of Parapoxvirus ovis and pharmaceutical compositions therefrom.

<130> LeA 35228

<160> 1
<170> PatentIn version 3.0

<210> 1
<211> 137560
<212> DNA
<213> Parapoxvirus ovis NZ2

<220>
<221> CDS
<222> (3)...(539)
<223> ORF:L1

<220>
<221> CDS
<222> (781)...(449)
<223> ORF: L2r

<220>
<221> CDS
<222> (1933)...(1664)
<223> ORF: L3r

<220>
<221> CDS
<222> (3269)...(2790)
<223> ORF: L4r

<220>
<221> CDS
<222> (2799)...(3851)
<223> ORF: L5

<220>
<221> CDS
<222> (2962)...(3753)
<223> ORF: L6

<220>
<221> CDS
<222> (3784)...(3122)
<223> ORF: L7r

<220>
<221> CDS
<222> (4341)...(4129)
<223> ORF: L8r

<220>
<221> CDS
<222> (4904)...(4428)
<223> ORF: 1ar

<220>
<221> CDS
<222> (6517)...(4970)
<223> ORF: 1r

<220>
<221> CDS
<222> (8042)...(6684)
<223> ORF:2r

<220>
<221> CDS
<222> (9989)...(8070)
<223> ORF:3r

<220>
<221> CDS
<222> (11195)...(10062)
<223> ORF:4r

<220>
<221> CDS
<222> (11493)...(11227)
<223> ORF:5r

<220>
<221> CDS
<222> (11802)...(12038)
<223> ORF:6

<220>
<221> CDS
<222> (12358)...(12080)
<223> ORF:7r

<220>
<221> CDS
<222> (13980)...(12364)
<223> ORF: 8r

<220>
<221> CDS
<222> (14826)...(14053)
<223> ORF:9ar

<220>
<221> CDS
<222> (15080)...(15394)
<223> ORF: 10

<220>
<221> CDS
<222> (16838)...(15423)
<223> ORF:11r

<220>
<221> CDS
<222> (19021)...(16847)
<223> ORF:12r

<220>
<221> CDS
<222> (19704)...(19156)

<223> ORF: 13r

<220>
<221> CDS
<222> (20314)...(19736)
<223> ORF: 14r

<220>
<221> CDS
<222> (20401)...(22101)
<223> ORF: 15

<220>
<221> CDS
<222> (22125)...(22940)
<223> ORF:16

<220>
<221> CDS
<222> (23003)...(23866)
<223> ORF: 17

<220>
<221> CDS
<222> (26908)...(23873)
<223> ORF: 18r

<220>
<221> CDS
<222> (26926)...(27213)
<223> ORF: 19

<220>
<221> CDS
<222> (27626)...(27216)
<223> ORF: 20r

<220>
<221> CDS
<222> (29754)...(27616)
<223> ORF: 21r

<220>
<221> CDS
<222> (32217)...(29800)
<223> ORF: 22r

<220>
<221> CDS
<222> (33380)...(32418)
<223> ORF: 23r

<220>
<221> CDS
<222> (33602)...(33393)
<223> ORF: 24r

<220>

<221> CDS
<222> (34466)...(33612)
<223> ORF: 25r

<220>
<221> CDS
<222> (34735)...(34502)
<223> ORF: 26r

<220>
<221> CDS
<222> (35905)...(34739)
<223> ORF: 27r

<220>
<221> CDS
<222> (37194)...(35905)
<223> ORF: 28r

<220>
<221> CDS
<222> (37200)...(39248)
<223> ORF: 29

<220>
<221> CDS
<222> (41037)...(39229)
<223> ORF: 30r

<220>
<221> CDS
<222> (41374)...(42066)
<223> ORF: 31

<220>
<221> CDS
<222> (42336)...(41731)
<223> ORF: 32r

<220>
<221> CDS
<222> (42407)...(41997)
<223> ORF: 33r

<220>
<221> CDS
<222> (42410)...(43765)
<223> ORF: 34

<220>
<221> CDS
<222> (43770)...(43958)
<223> ORF: 35

<220>
<221> CDS
<222> (43980)...(44534)
<223> ORF:36

<220>
<221> CDS
<222> (45727)...(44537)
<223> ORF:37r

<220>
<221> CDS
<222> (45760)...(46557)
<223> ORF: 38

<220>
<221> CDS
<222> (46567)...(47568)
<223> DRF:39

<220>
<221> CDS
<222> (47572)...(48303)
<223> ORF:40

<220>
<221> CDS
<222> (48352)...(48621)
<223> ORF:41

<220>
<221> CDS
<222> (49887)...(48634)
<223> ORF: 42r

<220>
<221> CDS
<222> (49917)...(50693)
<223> ORF: 43

<220>
<221> CDS
<222> (50719)...(51102)
<223> ORF:44

<220>
<221> CDS
<222> (51059)...(51511)
<223> ORF:44a

<220>
<221> CDS
<222> (51584)...(52591)
<223> ORF:45

<220>
<221> CDS
<222> (52509)...(53066)
<223> ORF:46

<220>
<221> CDS
<222> (53523)...(53023)

<223> ORF: 47r

<220>
<221> CDS
<222> (53607)...(57473)
<223> ORF:48

<220>
<221> CDS
<222> (58070)...(57528)
<223> ORF: 49r

<220>
<221> CDS
<222> (57700)...(58662)
<223> ORF:50

<220>
<221> CDS
<222> (59674)...(58673)
<223> ORF:51r

<220>
<221> CDS
<222> (62089)...(59678)
<223> ORF: 52r

<220>
<221> CDS
<222> (62198)...(62881)
<223> ORF: 53

<220>
<221> CDS
<222> (62909)...(63862)
<223> ORF: 55

<220>
<221> CDS
<222> (63858)...(64271)
<223> ORF:56

<220>
<221> CDS
<222> (64309)...(66831)
<223> ORF: 57

<220>
<221> CDS
<222> (67266)...(66799)
<223> ORF:58r

<220>
<221> CDS
<222> (67803)...(67273)
<223> ORF:58ar

<220>

<221> CDS
<222> (67915)...(68607)
<223> ORF:59

<220>
<221> CDS
<222> (68624)...(70984)
<223> ORF:60

<220>
<221> CDS
<222> (70994)...(72898)
<223> ORF: 61

<220>
<221> CDS
<222> (72938)...(73507)
<223> ORF: 62

<220>
<221> CDS
<222> (73540)...(74211)
<223> ORF:63

<220>
<221> CDS
<222> (76120)...(74207)
<223> ORF: 64r

<220>
<221> CDS
<222> (76749)...(76186)
<223> ORF: 65r

<220>
<221> CDS
<222> (77698)...(76799)
<223> ORF: 66r

<220>
<221> CDS
<222> (79343)...(77709)
<223> ORF: 67r

<220>
<221> CDS
<222> (79816)...(79367)
<223> ORF: 68r

<220>
<221> CDS
<222> (80529)...(79858)
<223> ORF: 69r

<220>
<221> CDS
<222> (80774)...(80529)
<223> ORF:70r

<220>
<221> CDS
<222> (82815)...(80788)
<223> ORF: 71r

<220>
<221> CDS
<222> (83835)...(82834)
<223> ORF: 72r

<220>
<221> CDS
<222> (83874)...(85583)
<223> ORF:73

<220>
<221> CDS
<222> (85535)...(84402)
<223> ORF: 74r

<220>
<221> CDS
<222> (88096)...(85574)
<223> ORF: 75r

<220>
<221> CDS
<222> (87759)...(88667)
<223> ORF:76

<220>
<221> CDS
<222> (88920)...(88642)
<223> ORF: 77r

<220>
<221> CDS
<222> (91652)...(88938)
<223> ORF: 78r

<220>
<221> CDS
<222> (91667)...(92674)
<223> ORF: 79

<220>
<221> CDS
<222> (93466)...(92681)
<223> ORF:80r

<220>
<221> CDS
<222> (93761)...(93486)
<223> ORF:81r

<220>
<221> CDS
<222> (94060)...(93788)

<223> ORF: 82r

<220>
<221> CDS
<222> (94238)...(94080)
<223> ORF: 83r

<220>
<221> CDS
<222> (94508)...(94242)
<223> ORF: 84r

<220>
<221> CDS
<222> (95571)...(94498)
<223> ORF: 85r

<220>
<221> CDS
<222> (96187)...(95600)
<223> ORF: 86r

<220>
<221> CDS
<222> (96202)...(97665)
<223> ORF: 87

<220>
<221> CDS
<222> (97915)...(97643)
<223> ORF: 88r

<220>
<221> CDS
<222> (98251)...(99537)
<223> ORF:89

<220>
<221> CDS
<222> (99537)...(99974)
<223> ORF:90

<220>
<221> CDS
<222> (100001)...(101140)
<223> ORF: 91

<220>
<221> CDS
<222> (101168)...(104650)
<223> ORF:92

<220>
<221> CDS
<222> (106354)...(104795)
<223> ORF: 93r

<220>

<221> CDS
<222> (107947)...(106400)
<223> ORF: 94r

<220>
<221> CDS
<222> (108256)...(107990)
<223> ORF:95r

<220>
<221> CDS
<222> (108719)...(108300)
<223> ORF: 96r

<220>
<221> CDS
<222> (109679)...(108738)
<223> ORF: 97r

<220>
<221> CDS
<222> (109861)...(109682)
<223> ORF: 98r

<220>
<221> CDS
<222> (110830)...(110033)
<223> ORF: 99r

<220>
<221> CDS
<222> (110208)...(110417)
<223> ORF:100

<220>
<221> CDS
<222> (110469)...(110651)
<223> ORF:100a

<220>
<221> CDS
<222> (110915)...(111397)
<223> ORF:101

<220>
<221> CDS
<222> (111419)...(111913)
<223> ORF:102

<220>
<221> CDS
<222> (111949)...(112485)
<223> ORF:103

<220>
<221> CDS
<222> (112593)...(113450)
<223> ORF: 104

<220>
<221> CDS
<222> (113323)...(112967)
<223> ORF:105r

<220>
<221> CDS
<222> (113526)...(114152)
<223> ORF: 106

<220>
<221> CDS
<222> (114199)...(115236)
<223> ORF:107

<220>
<221> CDS
<222> (115353)...(115787)
<223> ORF:108

<220>
<221> CDS
<222> (115859)...(116551)
<223> ORF: 109

<220>
<221> CDS
<222> (116729)...(117523)
<223> ORF:110

<220>
<221> CDS
<222> (117572)...(117114)
<223> ORF:111r

<220>
<221> CDS
<222> (117423)...(118085)
<223> ORF: 112

<220>
<221> CDS
<222> (118968)...(118375)
<223> ORF: 114r

<220>
<221> CDS
<222> (118508)...(119119)
<223> ORF: 115

<220>
<221> CDS
<222> (119588)...(120202)
<223> ORF: 116

<220>
<221> CDS
<222> (120314)...(121231)

<223> ORF: 117

<220>
<221> CDS
<222> (121380)...(123920)
<223> ORF: 118

<220>
<221> CDS
<222> (121288)...(122256)
<223> ORF: 119

<220>
<221> CDS
<222> (122350)...(123924)
<223> ORF:120

<220>
<221> CDS
<222> (123962)...(125566)
<223> ORF: 121

<220>
<221> CDS
<222> (125193)...(124591)
<223> ORF:122r

<220>
<221> CDS
<222> (125689)...(123935)
<223> ORF:123r

<220>
<221> CDS
<222> (123839)...(123297)
<223> ORF:123ar

<220>
<221> CDS
<222> (125652)...(126170)
<223> ORF: 124

<220>
<221> CDS
<222> (126121)...(125699)
<223> ORF:125r

<220>
<221> CDS
<222> (126279)...(127769)
<223> ORF:126

<220>
<221> CDS
<222> (127851)...(128408)
<223> ORF:127

<220>

<221> CDS
<222> (128520)...(130076)
<223> ORF: 128

<220>
<221> CDS
<222> (130105)...(131700)
<223> ORF: 129

<220>
<221> CDS
<222> (131790)...(133283)
<223> ORF:130

<220>
<221> CDS
<222> (133246)...(133920)
<223> ORF: 131

<220>
<221> CDS
<222> (133972)...(134370)
<223> ORF:132

<220>
<221> CDS
<222> (134418)...(134693)
<223> ORF: 133a

<220>
<221> CDS
<222> (134402)...(134992)
<223> ORF:R1

<220>
<221> CDS
<222> (134853)...(134419)
<223> ORF: R2r

<220>
<221> CDS
<222> (135628)...(135897)
<223> ORF: R3

<220>
<221> CDS
<222> (136780)...(137112)
<223> ORF: R4

<220>
<221> CDS
<222> (137558)...(137022)
<223> ORF:R5r

<400> 1

ggatccgcgg cttcgcgggc ggcggccggc tcccgcggcg gctgagccgc ggtgccgcga 60

cgaacgcgga ccaggagttc ctgcgggagg agctacggca gaggctggaa ctgctgaatg 120

ctttcgagga cgggcgtccg cgggaacgcg actccgcgga ggcggcaccc cgcagccgcg 180
agacctcgct ctggagtcag ggagtcaggg agtcgggagt cagggagtcg ggagtcaggg 240
agtcgggagt cagggagtcg ggagtcaggg agtcgggagt cagggagtcg ggagtcaggg 300
agtcgggagt cagggagtcg ggagtcaggg agtcgggagt cagggagtcg ggagtcaggg 360
agtcgggagt cagggagtcg ggagtcaggg agtcgggagt cagggagtcg ggagtcaggg 420
agtcgggagg tcagggagtc gggagtcagg gaaacagaag tccaagtagt acgggtgaga 480
caggagtcag gtggtcgggt gaccgcgccg tccgagtccc gcaaaaagtt tttagactct 540
gagagaggcc gacctgcctc caacggttcc gcggaaaagt ttttataaaa agttttcggg 600
agaggccgac tgccttccaa cggttcctcc tctcgcgtgc cgcgggcggc cgctctcccg 660
cgacggtccc gccaacgcgt ttacaaagtt ttaaaagttt tcgagagagg ccgacctgtc 720
ttccaacggt tgcgcgaaaa ggttctgcgg aggtttggaa gagccgcccg ccctccgaca 780
tcctccgaaa agttttcgca aaaagttttt aaaggtttcg cgaggagttt tcgagggagg 840
cgacctgcct ccgaggttcc gcgtaaacgt ttttacaaag cgtcggaggt gggggcgacc 900
tgcccttcct ctcctccgaa aagctcttga gagtgtggga gaggcggccg gccttcgcgg 960
tcgcgcgaaa aggttctgcg ggagttcgcg ggagctgacc cgccctccgc gcccctccga 1020
aaagtttttg cacgaagtct tttggcgttc tcgagaggac gcctaccccg acggtaacgc 1080
ggaacgtctc gggaggtcgg cctctccgct cccgcggtcg cggcggcggc ccgtctccgg 1140
aggttccgcg aaaagtttta taaaaagttt tgagggaggt accgacctcc taaagtttta 1200
cagagagttc tcgcaaaagt tttgggaggt cgacctgacc tcctaaagtt atcgaagagt 1260
tctcgcgaag agttctcgca aagagtttga gaggccgacc tgcctccaac ggttccgcgg 1320
aaaagtttta taagagtttt gagagaggtc gacctacctt ccaacggttc cgcggaaaag 1380
ttttataaga gttttgagag aggtcgacct accttccaac ggttccgcgg aaaagtttta 1440
taagagtttt gagagaggtc gacctactct ccgaggttcc gcggaaagtt ttataaaaag 1500
tttttacaaa gtttttgagg gaggtcgaca cctcggatcc tccgcctcgc gtgccgcggg 1560
cggcctccaa gagttttacg gaacgttctg gagaggagag gccgacctcc caaagatttt 1620
gcggaacgtt ttggagagga ggactggcct cgaaaagatt ttacaaagag tttggaggga 1680
ggactggcct ccaacggttc cgcgaaaaag ttttgcaaga gtttggagag aggtcgacca 1740
ccctccgagg ttccgcgaaa gtgtttgcgg agagtccgag agaggcagac actgcctgcg 1800

aaaagttttc gcggacggtt gagcgggtca ccgacctccg acagtttaca aacgttttac 1860

ggagagttag agaggcagca gaccgacctc cgaacagttt agttttacaa agttttggag 1920

ggtgaactgt catcgggaaa gttttacgaa gagttttggg agaggccgac ggttagccga 1980

gcgagcgcgc gagcgagttt acgttctctc gctcgtgtgc gcgttaactc gcactggttc 2040

cctctctaac tcggagtggg cgagcgagtg gttgactcgt cctccgctct cactctgagt 2100

ggtgataact gttaaccatt aactcgtcct ccactctctc actctctcac tctgagtgag 2160

gattgacttg ttaactcgtc ctccactctc tcgctctctc gctctgagtg agtgaggatt 2220

aactgttcaa ctcgtcaact cgtcctctgc ctctcatcca agactgagtg ggtagttgac 2280

tgctcttgtt ctcttactcc gaggggtgat tgagttagca acagctactc gttctcttgt 2340

tctctcacac cgagtgagcg agtgagcaac gttacttgtt ctctcacacc gagtgagcga 2400

gtgagcaacg gttaactcgt tctctcgttc tctcgttcta ttattccgag gagtggcgag 2460

tgaggtaacg gttactgtta cttgttctct gttcccttcc tcggtgagcg gtgcgtcaac 2520

ttgttctcgt gagtgagtac ggtcacttgt tctctcgttc tctacctcga ggagtgagtc 2580

aacttgttct cgtgagtgag ttgaccgtaa ccgttccctt acctcaagtg agtggcggtc 2640

gcttgttctc gtgagtgggt taaccgcgtt cccttaccgg agtgagcggg cgggcgataa 2700

aaataatcaa ttgactgatt cgctcgtgag cgagcgaagg gcggcggaca agggcgcggg 2760

atgctggtct aatctactaa ggccgattac aaaaacggat gggagaccgg gagggagagg 2820

gtcacagctc cgagcggtgc atccgcgcca gctggcggcg ccactcggcc gcgggccgcg 2880

gccgcccagg ccgcgttgta gccgcccgcc accgcgacgc aggtcagctc gaactccggc 2940

ccgagcgcgc gcacgtcgta gatgtgcacg gtcgcgacgt tcagcagcag cgcgcccttg 3000

cgcagcgagg cgaaggtcgc gtgcatgccg gcggcgagcg ggtacaccgg cgagagcgtc 3060

ccgccgccgt gcgcgaccac cgccatgtgc cgcccgtcgc cgccgacggt caggcaggtc 3120

accgaggcgg agccgttcgc gcggacgggg cccgcctcca cgagggcggc cggcagcggc 3180

ggcgccgcgg ggcggaagag cccgccgagg aggaagccca gcgccaccag cgcgagcgcg 3240

ccgagcagcc tgcgcggcga ggggtgcatg cttgttggct gcggtgttgg cgtctggcgg 3300

gtggaaggcg ggtgtggggt gtccgcggct gcggcgggag ggtctcgact gctaggcggt 3360

cctttttcac tttgctccgt ggcgcctggt ccggggcaag ggctgcggcg ggcgcccggg 3420

cgggcgggcc gctaccccgc cgcgcggccc gcggccgcca gcgccgcggc cagccgccgc 3480

caccgcgggc ccgccgcgcg cagcagcccc gccgccgagc gccccgcgcc gccgcgggcg 3540

cgcgccgagg ccgcggcccc gcgccgcgcc agcagcagcg gcagccgcgc gtccagcggg 3600

ccgccgcggc gcagcgccgc gcgcagcagc gccgccagcg gcagccgccc cgccgcgtcc 3660

gggcccgccg cgcgcgcgcc cgccgccagc agcgcgcgca ccagcgccgg cgagggcgcc 3720

gcgcgcggac caggtgctcc acgagcaggg tggtgagcaa ggattctcga gaagtaggag 3780

tcatgtgtga cgacaaggag agacgttata ttaggcgcgt cctacttcac tttgaagatg 3840

gtgtaaagtg ttaaaacttg aacaccgttc actccaccac tgccgttacc gtgtcctgcc 3900

ccaaaagcaa ccacagtgct ttttccacca cctgttccaa atccgttcca aaagctccca 3960

tccattgttg ttagaacttt cagatgtttc tctaggttgt ttagttccac tgcaagtttc 4020

gaccattatc gttactggac atgctgttgg taatgagttt aataaccaat cataaaaata 4080

gttataattt gttataaagc taataaagta gcaaacactt taatgttata ttttgcctaa 4140

ccctccgtta acaccaccat taacaccacc acttaagctt ttactaccac cactaccacc 4200

tccaacacac attcttttct ctaaaggtcc ccaaattcca cctcctgaac ttggacgttt 4260

tacagcacct ccgggtgtac ttgcgtaccc tttagaagtt ccactgtgac tgtagatatg 4320

atactgtcct tctccaggca tgattaaagt gtgttgtaat tagtgttatc tacgcaactg 4380

tgcgagactc tcgaataaaa agaagctaca ttttacaatt ttgattagct gatgtaccac 4440

gctgtatcgc ggccaccaca agcacccgat ccagtagaac caaatccaga gtcgccgcgg 4500

tcagtgttgt ccaagcagtt aacctcttga actgctgggc acgatatgcg ttcgcatatt 4560

agctgagcta tcctgtctcc cttcttaacc tcaaagtcac tgtttccaaa gttaaacagc 4620

accactccga cgttgcctcg gtagtcttcg tcgatcacgc cagcgcccac gtcgataaag 4680

tgtttgactg caaggccaga acgtggtgct atgcgtccgt agcaaccaga agggggcttt 4740

atcagaaggt cagtaaatac tacgcgactg caatgcgaag ggatgacaca gtcgtatgca 4800

ctacataggt ctaatcctgc ggcaccagga gatcctctgg ctggtatagt ggcgttttgg 4860

ctgaggcgaa caacctgaag agtttccgtg tggcagaact ccatggctag ggtggcgagc 4920

ggccgatcga ctacggggtg tacaatttac actttctcca gaaaaatcag gggcgggtca 4980

gcatggcgcg gcgcaagtcc agcagcgagt cgtacgacag gaagcacagg atggaggtca 5040

cgatctccgg cggcagggcg cacgggcaca tgaggccggc gatctgctcg gccagcgaga 5100

cgcgcagccg catcatgcag atcttgccga agagcgccgt cccgtagatg gggaactcgg 5160

ccgcgcgctc caaaaaggcg ttctgcacga agagcgcctt cgcgtcgtcc gacgcgcgca 5220

gcacgtccaa cagcgtcgcg tccgtgtggc agcgcaccgc gcgcatgctt gcgatctcct 5280

gctcgcacgc gcggatcacg gtcgcgtagt ccgccagcgc gcgctccgcc agcatgcgcg 5340

cgccctcgcc gcgcagcgcc agctcctgca cgcacagcag cgcggcctcc gagcgtcgga 5400

acacgtggcc ccattgctcg gatgtgatca gcgcgcgcgc gagcagctcc gtcggcggcc 5460

ggcgcgcgag cacggccgcc gtcgcgcgca cgttgttgcg gcgcagcatc tccgaaaccg 5520

cgcataggcc cgaggccgac atgtgctcga gctccgcgcc catgcgcacc agccggcagc 5580

aggcgccgtg gctgaacacc gccgcgcggt gcagcgcggt ctgcaggttg ttgttgcgca 5640

ggttcaggtc cagcccgcgc tcgagcacga agtccacgac gccgcgctcg cagctcccgt 5700

aggtcgccat gtagtgcagc atggtgttcc cacacgcgtc tacggcggcc gggtccacgc 5760

ctaggcccgt gagcgtgcgc accatgccct cggagatctt ggccgtgcgc gcgaggtggt 5820

gcagcgttgt gcgcccgtac gcgtccacga cgcacgcgtc cgcgcccgcg cgcagcatca 5880

tgtccacgag cgcggcggag acgccgccgg agcacagcag cgccgccagc ggcgtcaagc 5940

cgttgcagtc gcaggcgttt gggttcgcgc cgcgctcgag cagcagccgc agcacgtcct 6000

cgcggatcca ctggttcttg gcgtacacgt gcagcggcgt tacgccgtag gtgttgccct 6060

cgttcacgcg cgcgcccgcg tccagcagca gccgcgcgac ctcgagctcg gcgccgtcgg 6120

ggccgcagaa agccaggaag gaggagagca cgctgtcgca gacaacgacg ctggcgtcgc 6180

agaccacgtc cgcgcccgcc tccagcatga gcgcgaccac ctccggccgc acgccgtcgt 6240

actgcacgta ggcgtgcagc ggcgtgcggc cgcaggagtc cttggctttc acgtccgcac 6300

cggcctccag cagcacgcgc acgatctccg cgcactgctc gtgccgcgcg aagtgcacgc 6360

agaggtgcag cggcgtgcgc ccgtgctcgc cgcggaagtt cacgtctgcg tcggtggcta 6420

cgagcgcgcg gaccgtttcg aggtccacct gcccggactc caggtagcgg aagagcaggt 6480

ccgcgtgcgg gaccacgacg gactcccgcg agagcatggc ggcgtttaca aatattgaaa 6540

tctttttca ctcatcttta tggcgctgaa cgcgcaataa gggtgagagt aaaaaacttc 6600

tacaaaaagc gtacaaaagg tacaaaaggt aaaaaaggcg gggcggggac gggctggggt 6660

gctgcgagct gaattggcct ctacacaggg acgccctcgc cggagccggt gagccggtag 6720

ccggcgccgg cgatcatggt caagcgctgc acgagctcgt tgcgcttgac gccggcctct 6780

gaaacgcaca ccatgtggtg gatgtaccgc tcgatgcact cgcagcgcgg gagagtggag 6840

tcaagatcgg atgcgagttg cagaatgtca tcccagagct cggagaactt gctgtacagt 6900

tctcggaggt ctctctccat tcgagccgta agagagtcag gatgcggtgt tccttcggga 6960

gtctgagcga acaccgcgaa caggctggtt atgccgtgtt ctagaataga gtggttccgc 7020

gttaatgccg cagacaaggg tcgtcgtccg cgcaacgact ggcggcagag cgctgtttgt 7080

gccgcaccgc ccattcctct ggcgatcgca tccaccgacg cagtgatcat ctgcgcgccg 7140

acgtcattgt agcgcgcgtt aaactcagta atcatgatta cgagattgca gatttcatag 7200

tagcactttt ccaagtcgac gcgcagtttc acgatctggt tgacaatctt gcacgccttt 7260

cgccgcgtct ccgccacgtt ggcgactcgg acttgcgctt cctggtcgat ggacggcgga 7320

aacacttcaa acccaaggtc gcacagttca gcggtgggga ctagcgtcac gatgatgtac 7380

tccgcatcgc cacccacttg cggcaggaag aacaccgacc gcgcggcggg aacgaccaga 7440

acgtcgcctt cctgcatgtt ggtttttaga aacttagtgt tgttcacgga gatgccggcc 7500

atgccctcgt ttttgacaca tattatggtg acgtacgcgg cgaccgtggg ggccatgtgg 7560

tggcgcatgt accactcgtc gtgcttgagt ttcagaccgt gagattcgcc aacctcgaag 7620

tgcatgttgg cgtctctgac gtagcgcgag aactcgctgc gacagattcg cgcgggcgcc 7680

cggtggaacg tcgactcgaa gagactgatg gctgtccatt cgcccacatg agtgaccacc 7740

gaagaagtgt tttcgatccg agtctcgaac accgagtcca cgagcaccgg acagttggtt 7800

ccgggcaccg tcagcaccaa gggccgcgcc tccacggggg cgacggacga agccacggag 7860

tcggtgtccc cgtacccgta gtcgtcgtcg gagtcgccgc ctccgtcggc cccgtcgcgc 7920

ggcctccgca gcggcatgca gccggcggtg ggaacgcact ggtttcggcc acggccgaag 7980

cggccaaaca gtctcgccag ggctgacatc cttggacggc cacaccaaaa ccaaaaaaac 8040

atattttatc agttatttgt cgattttcac cggctcaccg agggcaggac ctcctggatc 8100

ccggacaccc ccgccaggca gcgggccgcg cgctcgcgca cccaaaagcg gtcgtagccg 8160

tgccggagca cgaaggccgc cgtggcgtgg cagtccacgc gctcgatgaa gccgtggacg 8220

gcgcggcgcg cgtagctcgc cgcgaaggcg cggaccaccg ccgagcagcg ccccgagggc 8280

gagtcgtccg tctccagcgc cagcggcatg ctcgcgatgc gcgacatcag gttggaggtc 8340

tgcgggatgt tgagctcgcg cgtggcggtc atctgcgcct cgagcccggc cttgagcacc 8400

tcgtcgcagc ggccccactc cagcgcgcag accacgcgga tctcgtaccc cttgagccgc 8460

agcgcggtct cgatgtccac ggaggtgagc accgcgctga agcgcaggcg ctcttcgtcc 8520

gcggggtcga agagcacggg gatcttaacc tccgcgctgc gcgtgacctc gcagagcgcg 8580

atcgcgagca gcccgcgcgt gagcttgctc accatgcgcg gcttgcccac ggggtacagc 8640

tggctcgcga cctcgcgcag cgggtacgcc agtcggaagc agcgcgcgtc cgcgggcacc 8700

gggctcgcgc ccgtctcctc gaggaagagc gcggcctcaa ccatgttcag cgcggagaag 8760

tgcaccgggc aggcggcgca gccgcgcgcg gcgttcgcga gcaccatctc gcgcagcccg 8820

cggaaggccg ccatgtcgca ggaggggaag atgcgcgcga gcgcggcctg gtgcgcgagc 8880

gccgcgtccg agagcgcctg cgcgccgcgg cggcgctcct cggcggcggc cgcgctctcg 8940

tccgcggaga ccacgtcttc gggcacgtcc acgcagacgc cgccccagaa ctcgcagtac 9000

tcggagaaga gcgtcgcggg cgcaaagcgc gcgaggtcca cgaaggcgac gcggttgccg 9060

agcctggaga gcagcgtgtt ctccgagatg cgcgtccagc ccttgccggc gagctccatg 9120

acctgccgcg tgtcgaagag ggagctgtag aagccgtaca cggtgatgtt ttccttgcac 9180

gtcgtcagcc acatgaggaa gtcgcgcacc accagcttcg cgcagtctcc ggagaacacg 9240

gggccggcgt tcgtcgcgat ggagttcagg cgcacggtgc cgtcactgcc gaagcggtac 9300

acgaaccagg cggccacgct gttgccagag ggcgtgtgaa cgtgtggctg cgcccaggag 9360

tcggcgctcg cggcggtgcg cacgtcgtgc gagagcacct cggtgtcggg gcgcgagtag 9420

gtgctggggt ctttgatcca gatggcgtag ctgcccacgc agcacacgtt catgaggtcg 9480

agcagcgtct gccggcgcag cggcgtgccg agccggcgca cggcgtcgtg cgagaccatg 9540

cgcaggtcgt agaggcccac gtccgagagc cactggttga gctcgtccat ggacagggcg 9600

tcgcgggggg gcgggctgtc ttcgaaggcg gcgcggagct cgggctccgt ctccgcgcgc 9660

tgccgcagga tgtccaggaa ggggctggag gagtcgggga tgtagcagtc ggggtcgtgc 9720

ctggacacta tagcgaaccg ctgcgtcgcg ggcggcgggg ctagcgcgtc ggcgcgtgcg 9780

tcgatgaagg tgcacgatat acgcacggac ttgagcgagg ggaggacgac tgcggcggcg 9840

cgcgcgccct ccgcgtcgaa gatcatcgtc tttccgtccc tcgcctttgc gagcgcgtat 9900

tctccaggca cgaggtccgt cggcggcggc tcgtcccagg cctgccggtc agggacgccg 9960

ccgcacacct ttccccagaa ccccagcatc ctccaaaata cctataagga cggccaatag 10020

cggggcttgc gggcgttcgg accttccgcg ctttaatttt aatttattgg cttgcagaac 10080

tccgagcgcc agtcccgctc gaagaccgcg gacaggtcct tgacgatgtc gcccttctcg 10140

gcgttcacgc tcacgaaggc gtggtagcgg tagtgcgtgc cgtcgaggtt ggcgaccgtg 10200

aggtgcgcga aggtgtcgtc cacgatgagc agcttagtgt tgttcgcggc gtcgtcccgg 10260
ccgggtacca cgaacttgcg cacggacatg tccacgctgc cgacgccaaa gtcgtcgagg 10320
ctgcgcgcgg ccgagaccga aagcgggtcc gcgttcttcc actcggtaat gatcacgcgc 10380
acgcgcacgc cgcggttgat ggccgcgcgc agcagcgcgt caatgatctg cggccagtac 10440
tccacggcgc tggcgtgctt gatcaccggc accatcgaga gcagcgagag gtcgatgctg 10500
ttcttggcgt tctcgatgcg gtgcagcacg aggtcctcgt cgagcgtgcg gtagaagcct 10560
aggaagcgct ccggcgagtc cgagaagaat acgccgcccc cggagtggtc gaggtggaag 10620
ttcgtggccg tgggcgtgac gatggcgcag cagagccgcg tgaacggcac cttcggctcc 10680
acgatcatgg agtagaaggt gttgtagcgg ttcatgaggt cccaggccag gtgcttgttg 10740
gtggagtaga gcccgaggtt cttgatggtg gacacggacc cgcccgtgag cgaggcgctt 10800
cccacgtacc agtgcccggc gtccgagagc cagaagctgc cgagaaggtt gccgacgccc 10860
tccttggtgg acaccttgac cttgtagtag ttgacgcccg cctcgcgcag ctcgtccgcg 10920
tccttgtcct tgctctgcac gtccacgagc agcgtgacgt ctacgccctc cttggcgagc 10980
gtgcagagct tgtccttgac gtcgacgccc tccttggtgg agctcaggtt gcagcagaag 11040
ctgcagatgt acaagaactt cttcgcggac tcggcgatag cggtgaagca gtcgagggtg 11100
ctcatgttgc cctgcgccaa agacgccacc tctgcgggca gcgtctccac gacgcggcag 11160
tcggcgccca gggggatgga ggagaacggc cacatttatt tatctcacaa aaataatagg 11220
gcttcaggga aagtctttta gcaggcgggc gagttcttcg agttcgctta ggagttcttc 11280
catttcttcg gaagtcagca actggagctc ggacttgatt tgaatatctt cgaggaaacc 11340
gtctagcatg ttcgccatgt cttccgggga gcactgcgcc acatcttcgg ggacaggatc 11400
gggtgtgggc attaggtctc cgcttacttg aacgtcgtcc atcatcctgt cgatgaggtc 11460
ttcgacttct agacggggtc cgtagatcag catatttggt gatggaggta gtttaaggtg 11520
cgagagttag tgttatacga ccgccaacgt gtgtttatcg cgcgtacatt ttcaataatt 11580
aacaaactcc ccttcctgcg cctgctcgag aagcagctcg tccagctcct cctgtcggcg 11640
cgcggccacg cgtctttccg cgaagagtac catcagctcc agccccacgc cgcacagacc 11700
caggacgccg aacaccaccg ccgccgagat cgacagaccc agcagcaccg acatcctcac 11760
gcgggcatcc ggctatttaa tcgttctgga aacgtattaa tatgggcgtc gtcatgtgcg 11820
ggtgtctgtt tgtgtgggcg ggctggatcg cgcgccgcgt gcgcggcctc tgcgcggcgc 11880

tgcgccagag ggtgtcgcgc gacaagggct acgtggccgt catccagacc tgcgacgacg 11940
actacttcac agaggaggag ttcgacgacg gcaagcaggt ggtcgcgctc ctgcgcgacg 12000
tctcgcgcgt ggttgccgcg cccgcgggcg tgacggaata agttaggata aggagtcgag 12060
gggagaaaaa cagcggtcac actataaact cgcgcgaggc cgattttgac gtgctcatgt 12120
ctggaagctc cgctttctgc agcgcggagc ggcacacgaa gcacacttcc gtgttggtgg 12180
gagttatgca gtggacgtgg tagccgtgcc cgcacaccat gacttggaac ggacacgcgc 12240
cgggacaggc cgcgtttatg catccttccg gcgagcgctt gttgcagatg tagcacacgt 12300
ctgagcacgc cagcgtgcag gacacggcca gcttccactg cttaacctta acgggcatgg 12360
ctagttgaac acgaccatgg gcgagtcgcg agcctcgagt cgggggttca gggcaaaccg 12420
tttcacgccg tcaacggttc ttctctttgc aattttctct ctgcacaggc tcgtcagcgt 12480
catctcggcc aggcgcgcgt cgttgcctag gtgccgcgcg gcgtcctcga ccgtcacgcc 12540
cgtcttgccg gcctcgtcca tgagcacaat gcataccagg tgcgcgctag agcatatgac 12600
ctcctgctcg cgcccgccgg cagcggggat ggttagctcc gcgcgcccga aggccgccag 12660
cggcgccacg tcgtaggcag tgtctgctcg ggcgagcgcc gactccacgg caccgcggag 12720
cgactccggc ggcgtcagcg cggccagcgg caccggcgtg ggcacggtgt acacgttcac 12780
gggcatgagc accatctccg ggtcgtggtg gccgctctct tcgccgtcgt gctccatggg 12840
ctgcggcggc ggcagcagcg ggagcagcag ccgtccggac atgagccggc gcacaaggtc 12900
gttgagcgcg gacgaggcca tcggcgggta cagctccatg gccagcttca gcgataggtg 12960
cttctcgagg ttgacgccgg tgtagacgct cttcacgatg cgcgcgaagg ccacgcgcgc 13020
gaaggccgcc agctcctcgc gcggcaggcg ctcgatgtag gagagcagca tgtcggtgtc 13080
gcacggcggc gccgcgacca ccgcgccgta gagcgccttg cccgagagct tttccagcgc 13140
ccttgcgtgc aggccgtggg tcttgagcac gtccacgtag ttcacgtaca ggcagagcgc 13200
gcgatcgagg ttgctctccg cgacgtgcgt ctcgatgcac tccacgatga gcgggcccat 13260
gcggtccttg atgaggtcta tgagcccgcc gtaggcgact cgcgcgctca tgaggcacgt 13320
gcggcagtac gccatcaggc cctcgaggtc cgcggctatc acgtcctcga ccacgttcgc 13380
cacgacgcgc tgccagagcc gcaccttgct cacgttctgg tgccgcacca tgtccacgag 13440
ctcgtcgtac gagccgccgg gctcgtgcgc gcgatcgacg atgcacctcg ccatggtgcg 13500
gctctggcgc atgagctcgt tcgtgaagcg cacgcacgcg tcctcggaga agagcgcgct 13560

caggcaggag tagcagcggt ccgcgacgag gtgcgggaag cggcactcca cgacgccgcg 13620
gccgatccgc agcacgcact cgccgtacat ctcgtccatg gcctcgcgca gacagtcgtc 13680
cagcacgtcc gcgttgtgcg cccactggat cacgcagagg tagggctcga tgttctcgcg 13740
cgcgttttcc acctcctgca ccatgtactc gagcacggtc atgtcctcgt ggatgtcggt 13800
gcccagcatg cgcccgggcg gcagccagct cttgcgcgcg atcgcctctc gcaggcacgc 13860
caccgccgtg aaggtgttga cgcggagctt ggtcagcagc cgccgcagtc gggagatgtg 13920
tgccacggag aggtccatct ccatggcctg ggcgatgagg cgcgtgagtt cctcctccat 13980
ggcggcggct ccgcgggcag atatacgcga acaacggtaa gccgtgctat ttcatttttg 14040
gacaaaaagc tagtcgtcga cgcgcatgtt gtcgaggttc cggcacagcg agagcacgtc 14100
gtcgcgcgcg cgcctccggc gcagttgatt gttcgcgcgc cgcgcgtccg cgagcgcctg 14160
tctgtacatc gcggagtccg cgtacccgtg cagcggcgag cgccgagcgc cgggcctcgg 14220
gctcgcgcgg cgcgggagcg gcgttggcgc gcgcctcgag cgccgcgcga agtgcgcctg 14280
catggccagc aggcaaccga acggcaccat gtatcggtcc atgaggcact ggctggccgc 14340
ggacggctcg cgcgggtgca tcacgccgcc gcccacgtcc tccatgacgt cgcgcagcac 14400
gcagcgcagc atggtctcca tgctgcgctg cgtgaaccgc accggggagg cgtcgacgta 14460
gaagccgtcg gccacgaagt agagcgcgtc cagcccgccg agtttctcgc cgagaccgac 14520
gaagagctcg tccacgtgcc agtccaccac cgaggccttg aagagcacca cgtgccggat 14580
gtcgtgcgag cgcgcgagct cccaggtgtc ctcgccgatg ttgctggcgt cgatgcggcc 14640
tgtcatgcgc acgctcacgc acggcgtcat cccgttcttg tagcagaact ggcgcgcgag 14700
ctcctcctgg cgtacgatgt cgaccatgct ctccatgaag gaggtggaga gcagcatcgc 14760
gccgcgcgcg gcgcgggtcg cgttttcgtc cacctccact tccatcccgc cgccgatcct 14820
aatcatctat cgtatttaaa ttttcggcgg agcagacacg cggctgctcg ctgcgcgatc 14880
gcttcagccg cggcggcgtc acgcacgcgt tgcggcggcc ggcacgcacg gacgaccgcc 14940
ggggctgttc gctgagcgag cgccgcggcc gcgtgacgcg acagtcgcag gtgggttgcc 15000
gggagtcgct cgcgcgcctt cttcgcattt cgcccggaacg ccgcgtttat gtaggggatt 15060
atattttcaa cgtaactaaa tggacggggg cgtgcacaaa cggcctttca tcgtgaacgt 15120
ggatggcatg ggcaaggtgc tcgtgctccg gtacttgcgg atgtgcgagg tgcccgaggc 15180
caagtgcgag gggtcgcgcg cgtcctgcgt gctcaagatg gaccctcccc gctcacccag 15240

ctgcgagcgc aggccgtctc tcccgccgtc ccccccatgc cccatgcgca cgcctcccgg 15300

gtcgccgctc caggctccct tgatgcgcac gcagatgctc caggggctgt tcgacgctgc 15360

caaaaacaac ggcgagcaga tgtgccgccg ccagtaacct aggctgcgca gtacgaaagt 15420

tagtgcgtga tcacgttttt tgcaatgtcg atcacgccgt gcgtgcccgt cttgcgctcg 15480

cgctccacca cgctagtcac gggccgcgcg tccgagacta gcgaccccag catcgagcgc 15540

acggcgccct ccgcggcggg gtggcgcgtc agcagcagga acatcacgat gtgcgcggag 15600

acgccgcgcc ggctcagatc gtgcacggcg tcgccgtcca tgagcacggt gttcgagaag 15660

tacgtgaaca gagtgttgtc tcgcaccagg aaggccgagt tcgagacgct ctcgaagtcc 15720

acgatctcgt cgtcctgcac gcccatgtcc aacagcgtct gcacgagcgc gggctcgtcc 15780

aggaacacca ctgcgcgcgc gaacccgcag tccagcgcgc gcgcgtccgc ctccaacacg 15840

cgcgaggcgc cgccctccgg cggcaggaag gcgcagggca gcggcgtgcg tccgtccgcc 15900

ggcgcctccc cgagctcctc gagcgcgaag gccagcagcg tctccatgcg cgcgcgcgcc 15960

ttgtcgaagt tgtccgcgag gtcgcggatg cggtctgtct gcgagaacat cttcagcatc 16020

gccatgagct gcacgaaggg gtgcagcacg tatatgttgt ccacgagcag cgtgggcagc 16080

gcgcgcagcg tggcctgccg cacgttgaag ctgtccagga tgtgcccgcc ctcctcgtcc 16140

tgcagcacca cgtagttctt caggtagggc acgcgcagca gcacggtctg ccgccccgtg 16200

acgaagtata tcaggaaggc gaggttgatc aggaacgggc gcgcgttcgt ctgcaccatg 16260

tcgatgtcgc cgtactctat ctcggggttc agcaggtgca gggcgtacga gccgtagcac 16320

acgcaccgct tgttgtgtcg gcgcaggtgc tccttcacga gccgcttgac cacctccacc 16380

aggtccgagt gcttgtgccg cgccatcggc gcggcctcct cgggcggcgg cagcactgcg 16440

tacgagttga gcgcgcggct ggcgagcgcg cgcgcgcggg gcgcgtccac gcgccgcacc 16500

gccgcgttga ttgcaggcgt cggcgtcgtg agagagccca gcgtgcgcgt gaactcgctc 16560

acgatcacgc tctgcagctc cagcaccgtc aggatctggc ccagcttctc caggcgccgc 16620

tgcctcgaga agtactcctc gatgcgcgcg gcgatctcct tctcggagcc gcctagcttc 16680

ttgaagaagc gacgacgact ctttacaaca agagagagaa aaagcttcct atcgaagttg 16740

aggacgcggg tcatgttgcg gcgctgcgcg cgcaggagca cgcagcgctc catggagggg 16800

cgcgagccga ggtactcttc gatcacgggt ggagccatga cagctatttt ctgaacccgc 16860

gattattgta cagcgcaagc cgcgcgcaga cctgctggca cagcagcgtc gtgtttcgca 16920

tgcacacgcg cgaggactcg atcgtgcgcg cgtccggtgc ccaggcgcgc agctccatca 16980

gttcctgctc gacgaagtcc acgggctcca cgaagcgctc tgcgcagagt ccgtccgtga 17040

acgcgttgac gatctgccgc acgagcacta ccacgtccac ctgctccacg aggcgcacgc 17100

ccatggcgac gtgcacgaag aggcagcgga agagcgcgtc catggccatc tggtggtccg 17160

agcagggccc gaccgcggtc ttgcagccca gcgcgaagcg cccgatgccg cggtactgca 17220

ccatctccga gggcgagaag gagagccgct ccatcttgag cacgggcggc gggcccgccg 17280

gcagtccgcg cgcgaggtcc agcaccggcg tccacccggg cgtgaacatg tccgggatca 17340

ggaagagccc gtagctggcc atgcgcgcga tgtcgaaggc gtggtccacg accttgttca 17400

cggcgctgtc cgcgcggttt acgcgcagcg cctgcaggat cacgtttccg gaagcgtgcc 17460

gcgtgatcgc gaggtccgcg gtcgcgtacc cgcgcaggcc cggcaccgcg tacgcggtca 17520

gacacacggc caggcgcgcg ctgtgctccg aggagaagat ctcctgctcg tagccctcct 17580

cgggctcctc gcactcgcga gggcgccgca cgtcctcgac cgagcgcagc cgcatctctc 17640

cctccgacac cagacagcca agcgactccc tcaccgccgg cgcgagcacc tccgtggcgc 17700

agagcgcgtc gtgcacgcgc ttgagtgtgt tcggcttcag cgcgtagccg aagagcagcc 17760

gcgtcatccg cgagcccgag aacgcgaagc ggcgcacgta ctcctccgcg agctcggggc 17820

ggtcgttgat ccacgaggta gagaagacgt ggtcggaggc gaaggggtct gcaccaaccg 17880

cgagcagtgt ggacagaggt acggtgtcga ggaagtccac gacgtcgggg aagttctggc 17940

gcacgcaggc ctcggcgacg cgtctggtgt gcacgcacat gtcggtgacg ggcacccggt 18000

ggccggactc cacgacggac acgcagacgt cctcggtgac ggcgtccacg ggcatggtcc 18060

gcagcagctt gccgagcacg tcgccgaacc cgccctcgag cgccttgcgc cacacgaacc 18120

ccgcgtcgaa cttcccgggg aagtccgcga tcaccgaaag ctccgcgtgc gagaggttgt 18180

ccacgttgag gtaggtggcg gcgtccacga agatgggccc gaaggcgccg gtgtcggaga 18240

cgcggtctct gaggtagtcc ctggcgtagt ggaggtactc ccgcacctgg ccggcgcgga 18300

tgcgctcgag cgcgaaggcc ttcatggtct cggagcagag cacggagtgc cgcagcccgt 18360

ccagtgtgcg ccgcacgtcg tagacgccgc gcatctgcgc gagcatctcg acggcgtccg 18420

ccggcgtcgc cgccgcgagc cccgggttca ctggaggtat cctgtgttct gcgagcatgc 18480

gcttgaggaa acagaggtcc agcggccgcg tggtgtacag cgcggaggcc atctcggggc 18540

gcgtctcgac gatgtcctcg atcatctcgt ccgtgaatgc cgcgttgatg ttgtgcacgc 18600

tgcgcgcgtt cacgtgcagg aggatgtcgc ccacgttgtc ggggaatcgc tcctcgatga 18660
gccggacgtc gtcctccgtg atgttcatgt agggaataca gcggcagagc agcgcgtagt 18720
ccgcgaactg cgcgatgtag ggcgtgtgga actcgatgtg tctggcgaag agcgcgccgc 18780
agcgccgccg cgagagctct tcgagcaggt cctcgggcgt gacgtgctgc gggcggaaga 18840
ggtgcaggtg cgtggggtgc tcggcggcca cgcgcgcgta cagccgccgc gggaggtgtc 18900
tggggtgcac gccggcgagc acgagatcca tggcctctga ggtagacagt gcggcgaacg 18960
cgcgctcggt gccgcccgcg gcgacagcgg cgccgacaaa tctcttgagc agctgcagca 19020
tcgcgtgttt gggctttcgc ggaaggcgct tattttaatg ttattggcgg tggccggtgc 19080
gagataaaaa ttagaactga tgccgcagtt gttgatgata tgatgattgc gctggccggc 19140
gcgagataaa aattagaagc tgatgccgca gttgttgatg aggatggtga gtgcgctgga 19200
gcaagcggtg tggcgcgcta gcttcttgct ggccccgtcg gccacggcaa cgacctttcc 19260
ggatatcgtg atggtgcagg tgaagcgcgg acagtgatcc tctccgccag cacgcgtctc 19320
gcagaactcc agaggtctgt gtgtcatcat gcagaactcg ttgaccgcgc tgaccgggtt 19380
aagacttttg aggcgtatca cggcagactg agtcatgatg tcgatgtcgc cgccgaaaag 19440
cgtatcgcac ccagcctcgg tctccatggg ctcggtgtcg gagttttcgt cctcctcggt 19500
gggcgcggag ggcgcgcact ctacgaacca gcggggcggg tttccgtcct cacagcaaac 19560
ctcgtccgag tccagcaggc ggtacagctg gcggtttgcc tcgtgtttgg atatgccgag 19620
ctccttcgcg atttgcttgg ccggcagctt gtcgtcggat tttctgagaa gctcgaggat 19680
cagagacgcg cactcgcagg ccattgtggc gatttacggg gcgtgcgttt ttttaggatt 19740
ttggcttgcc tttcttttcg cagaacttgg gaggattgaa actcttttgg caatttttgc 19800
aggcgtactt gatcaagggc ggctcgtccg ccgagcgcgt ctggatcatc atcggcatgg 19860
tgttcttgct ctggcacgag gggcagggca ggttgaactt ctcgtcgagc acgttgaagt 19920
acccgctgta gtcgtggtcc ggcacctcct cgatgtcgta gggcacgcgc gcggccacgc 19980
acttgaccgc gaagagcagg taccgcagcg cgtcgtgctc cgcgccgctg gtcgcgcgga 20040
tctgcgcgca caggtccgcg tagtcctcgt tcgcgtccac ctccaggctg cgcttgttct 20100
tgtacgagag ccggttcttg gcgtccttcg agtactcgat gccgatgttg tgcgcggggt 20160
caaagttggt ctcgtcggtg ttcgaggtct tggtgttcac gatgttcttc agcgcgaagc 20220
gctgcgcgca gtccagcgcc catcgcgcga tgcgcgcggc ctccgccgcg tcggtgtgct 20280

tcgccgcgag gtcgcgcagc cggtcttcgt ccatcgcccg attttaggtt gggtatatta 20340

tctcaattcc gctcttccgc gggccgcggg cgcgcgcccg cggcaaatta ggcgttacaa 20400

atggacttcg tgcggcggaa gtacatgata cacgccatcg accgcaacct cgacttcatg 20460

aaggccgagg tccagcagaa ggtctccatc ttctccctcg ggcacgtgct cgcgctccac 20520

tacctggtca ccgcctttcc gcaggcggtc atcaccaagg acgtgctcgc gagcacaaac 20580

ttcttcgtgt tcgtgcacat gtcgcagcgg cacgaggtct tcgacgccgt gctcaaggcg 20640

gccttcgacg cgcctcagct ctttgtgcgg gcgctctcgc ggcacttcga ggccttcgtt 20700

gccgccatcc gggcctaccg cgcgacctgc gcggagctgc tggccgacgc gcgcttcatg 20760

gaggtggctg cgcgcgcggc cgagctcgcg gaggtcattg gcgtgaacca cgacatcgcc 20820

gcgaacccgc tcttcgcgga cggcgagccc gtgcgcgacg cggagctcat tttcgcaaag 20880

accttccgca agaccgagtt ccgcgccgtc aagcgcctcg ccgtgctgcg gctgctggtc 20940

tgggccttcc tcgtgaagaa ggaccttggc ggcgagtacg cggacaacga ccgccaggac 21000

ctgtttacgc tgctgcagaa ggccgcgggg cccgtgcgcc acagcgcgct cacagagagc 21060

atccgcgagt acctcttccc cggagacagg cccagccact gggtctggct gaacgcgcgc 21120

gtggccgacg acgcagaggt gtaccgcgac cggcccgcgc gcacgctcta cgagcgcgtg 21180

ctcagctacg cgtactcaga ggtcaagcag gggcgcgtga acgccaacac gctcaagctc 21240

gtgtaccggc tcgaggacga ccccgacatc aagggtctgc tgctgcagct catctacgac 21300

gtgcccgcgg acatcgtcgg cgtcgtggac tccgcgaacg aggagtggcg gagctacttc 21360

gtgagtctgt accgcgagaa cttcgtcgac ggacgcacct tcacctcgga cgcgcgcttc 21420

cgcgacgacc tcttccgcgt ggtcgccgcc gtcgatcccg acttcttcga gcccgagcgc 21480

atccgcgagg ccttcagcgc agacgcgcgg ctgcgagagc gcttcacgga catggacctc 21540

aacaacgcct tcatgtcgca cctcatctac gactccgtgg accccgacgt cgccgccgcc 21600

gagcgcgggc tcgcactgcg cgtgcacaac gaggactccg actacttcat ccgggagtac 21660

aacacctacc tcttcctcag cgagaaggac ccgctggtgc tggaccgcgg ggcgctcacg 21720

cggctctcgg acgtccccgc cgagcgcttc cgcgacctct tcagcgacag tgtgctgcgg 21780

tacttcctgg acgcgaagct gggcacgctc gggctggtgc tcgaggacta ccgcgaggac 21840

gtggtcgccg ccatgcttcg gcacctgcgc cgcgtcgagg acgtgtcttc cttcgtgacg 21900

tacgccgcgc gcaagaaccc cgcctgcgtt cccggcgtcg tgcgcgcggt cgtgagcaac 21960

ttcaaccccg cggtggtcgc ggccatgcgc cccttcctgc gcgagcacat gacgcgcgtg 22020

gacgcgctgc tggacggaat gccgcacctc tcggaggccg accgtcggta catccgccgc 22080

gtggtgctgc agggccgcgc ctgattcgcc gtcaataaat cgcgatggtg gacagcggca 22140

cgcacgacgt ggactccgcc gcgcaggagc gcacgcccaa ccagcagacc ttcttcacca 22200

aggggctcag tccgctgatg cgccacacct acatctacaa caactacgcc tacggctgga 22260

ttcccgagac cgcgctctgg agcagccgtc tgggcgacta ccgcgtcacg gacttctacc 22320

cgatctcgct gggcatgctc aagaagttcg agttcatgtt ctcgctgctg gcggaccccg 22380

gcggcgcctg ccccgcgtac gagcccaagc tcaacaccga gttcctgaac cgcggctcct 22440

tctcgggacg gtacgtgaac cccttccacc gcttcgcggc gctgcccgag cgcgagtaca 22500

tatccttcct gctgctgagc tcggtgccca tcttcaacat cctcttctgg tttaagggcg 22560

agaccttcga cactgccaag cacagcctgc tcggcgccgt gtacaccacg cccgagcggc 22620

acatcgagct cgcgcggtac ctgcggcgca cgggcgacta caagccgctg ttcagccgcc 22680

tgggcaacga cgacacctac tcgaagccct tctctgggtt cacgcgcatc agcaacccca 22740

cgcccatcgg gcggctgccg ccctcggact tcgagacgct ggccaacctg agcaccattc 22800

tctactacac gcgctacgac ccggtgctct gtttcctggt cttctacgtg ccggggctct 22860

ccgcgaccac gaagatcacg cccggcgtgg agttcctcat ggagaagctc tcgctcgcgc 22920

ccgagaacgt ggtgctgctg tagcctcaaa cataaaatat aggcgccttt gatcgcactg 22980

cttcagttca gacagagcta agatggcttc ctacatcagc ggcgctagcg ccagcgcgaa 23040

caccgcccag ggcggcgatt ctcagtaccc acagtactat tatcacacac gcacctccca 23100

aggcgacatc cgcgacgaaa gcgaaggttg cttccacacc acggacgacg agcacttgga 23160

tctgtccgac gactacctcg gcgatggcgc accacactgc ggacacagcc acaaccacag 23220

tcgcagagat ggagatcggc accgccagcg cgcaccgcgg ctctatgagg acccggtgcc 23280

cgcgaacatc atggtgccca cgctcagtct agagcagctg ctggaggaaa cctcggtcgc 23340

ggggggcctt ctcggcggca ggacagagag ggacgtggaa cagctcctgg aggagttctc 23400

cgcactctgt cccggggacc agatcaccgc gctgcgctgc atggcggcct ccttttaccg 23460

cgacgcgctg ttcgcgccgt acgcctgcat gcacctcatc gccagtcgga tgcgcgtgca 23520

ctacgcgcgc gaggtcgtgc acgtggccga ggacctcgcg gacgcgatgt ctgcgaacag 23580

cggcgtctgc ttccggcggt accgaaagcg cgtgctagag gacatgctcg cggaggagat 23640

gggcgtgtac aattacctcg cgcgcgccaa cgcggacatc tgcgaggaca acctgctatc 23700

ggccgtggag acgctgctgc ggcgcttccg tcggatgggc tgctaccgct ctctgtgcat 23760

gctcaagatc ctcgcgctgc agcacgagga cctggccggc ttcatccgcc gcagcataag 23820

aaaaacctgc aacttggcac acgcgcgcac gcacacggtt tacgtgtagt taccctgtaa 23880

agacgggctt gctcccgaac aagcgctcga agaagagcgt gcacatagcc ttattgtcca 23940

gcaagttgac tatctctgta cacagcctct tgaagtacac ctcgtacatg atccgctcgt 24000

ttttatccag tctgaaggtc ttgtcgacca cgcgctcgta ggacttcacg ttcgcgatcc 24060

ggcgccgcca ggggccctcc tcgcacacgt acgcgaagaa gtagcgctcg ccgatctcga 24120

tggcctccgc gttcgccgcg ttgtaccgcg tcactagcgc cacgttgggg ttgtcggggg 24180

acttgaagtt cttgtggtgc gttcggctca gcaggaacca gtccagcggc atgctgcgcg 24240

cctcgaactc gaaggtgagc tcgtcctcca gcgagcgcag gatctccacg cccacgttcc 24300

cggagccctc ctccgccagc gcgcggcaga gcatgtcctt gtacttgcgg atcatgagct 24360

tgtggaaggg cgccacgtcg cggcgcgtct cgctggtgcc cttgctcacg cgctcgctgc 24420

cgccgccgtc gctcaccgca aacttgatcg tggtgtactt cttcttggac tgcatgatca 24480

ggttgcagta caccgcttcg aactccacct tgaagttcgc gaagagcacg tgctcgttga 24540

tcacgcgctc cagacagcgc cccacgcgcc gcgagaacgc gatgtcggag gcgcccacct 24600

ccaggaacac ggagtcggtg tcgccgtaca cgctgcggaa gcccacgcgc tccgtgcgct 24660

cgccggccac cgccgcgtcg atctctagct ccgcggcgcg gcccgcgaag gcctcgtcgc 24720

gcagcagcgg gttgtccggc gccgccgcca gcgacagccg cgtgccgcac accgacgcgc 24780

cgtctagcgt gcgctccagg tacgcgatca tggtgcgccc gatggccgtg cagctcttgg 24840

ccgaggcgta cgagaagagc gcgctgttgc ggaagcccat gagcccgtac acggagttgg 24900

ccgtgatctt gtacgtgtac tgcatcgagt tgtagatctc gcggtccacc gcggtctccg 24960

cggccttcat cagcttcttg tacttggcgc gcgcgtccag gaaggagcgc agcagcatcg 25020

ggatgatgcc cttggcctcg cggtcgaaga tggccacctc ggcgacgagc tccggcgagc 25080

gcggctcgca gggcaccgcg atgtaccgcg gcgccgggaa catccgccgg acgtccacgg 25140

ccgcgacctc cgcgtcgagc cggttgtccg agacgaccac gccgacaagc gtctccggcg 25200

acaggttcgc gtagatgcac acgttcgggt acaggctgtt gtagtcgaag atgagcacgt 25260

gcttgttgtg catcttctgc ttgggcgcca tcacgcggcc gccctcgtag aagaacttgg 25320

acttcgtgtc cgcgcgcacc atcaccgtgc ggttctccag cagcagcttc atcagcgggc 25380

ccttgatgca ggtgctcgcg cggtactcga agaccacgct ctgcggcagc aggtacgtgg 25440

acgcggcggc cgcgatcttg gtctccacgc cgtagtgcga ccagaggtag aggcagaggc 25500

aggcgtcgtg caggcagtac cgcgccatgt ccagacacac gtccagcgag tagttcgcgt 25560

acatgtccgc gaggctgacg tcgtccttgc cgaaggccag cgtgacgcgg tcgccgggcg 25620

cgcgcgccgc ggggtccgcg aggtccacgg tgaagccgtc ctcgtcgacg cgtttgtgca 25680

gcacgcggca cacgcgctcg tccacggtca cgtagttgcc ggtgctgagc acgcgagcga 25740

acacggccgc gttcccgtcg gcgtcggtgc tgcggtcgcc gcgaaagcgg accgcgtccg 25800

gccgcgcgtc ctccacgacc gcggtgcagt ggaaggcgtt cttggatatg gcgtccagct 25860

tgtaggagtc cagcttctcg gtgcgctgga tgaaggcgta caggtcgaag tagatggtcc 25920

cgttgttgtt gttgatgtgg aaggtggtgc tcgagacgcc gccgacgccc ttgtggctgg 25980

acttcgtgcg ctcgtacacg cagaagttga ctgtctcggt cccgtccggc agccggaagc 26040

ggatgtgctc gcccgtgagc agcgacagcc gcgagtccag gtaccgcagg tcgaagttgt 26100

ggccgttgaa ggtgaccacg aagtccagcg gcatctcgag caggcgcttg gccacgcgca 26160

gcagcgtcac ctcgggacac agcgtgacct ccgcgtcgaa cttcacgtcc gccgggtcca 26220

ggcagaccgg gatctcgcgc cgtgccgcct cctcgaggtc cgcgtcggag agcatatcgg 26280

agttcgtaag cgtgaatcgc cgctccgcgc cgtccttgtc caccacgcag aagctgatgt 26340

gcgagacggc gttcttgaag acggaaggaa actttttctc gaagtggcac tctatgtcga 26400

ggaagagccc cgagcgcgtc acgttgaagc gcgggatctt ctccgcgaag cacgcgccgg 26460

ggtcgtcgca gtggaagcag ttgctcccca ggtcgcgcag cagcgcgggg tccacgcggt 26520

agcacccgtc agggtcgatg tcgtgcgcca cgaagaacca ggacacgttc agaaagtcgg 26580

acatgaagac ctctggcggc gcgagcttgc gctcgctggc caccagacac agctctatct 26640

ccgagcgctg gcgctccgga atcttcgccg agcgcgctac gatctcgtcg atgctgacca 26700

cggacatggg cccgagcgcg cgcgtccacg ccagcggctg ggcgatgtcg gccaccgcgt 26760

ccgcgcgcac cacgtagtaa aagtgctgca cgaagcgcag gtacacgacg gcgttgtcgg 26820

cgcggcgcgc cttgaggaag aggaaccggc tgtcattgcc gcggttctcg aaccagttca 26880

aacatttcag ctccatttca aagagcataa taacatttca tttaaatgga gcctcgcttc 26940

tggggccgcg ccatgtgggc ggtgatcttc atcgtgctgc ggcgcttcga ggagcaccgc 27000

gacctcgagc gctgcaagcg gcagctgtac gtgatctgct ccacgctgcc ctgcatcgcg 27060

tgccgacgac acgccaccgc cgccatcgag aaaaacaacg tcctctccag cgaggacccc 27120

aactacgtgc tcttcttctt catcaagctc ttcaacaacc tcgccttcga cgacagatac 27180

aagatcgacc ccgcgaaggt gcgcccgctc gtctagagca tgccctcgta cgcgcgcgag 27240

ttgtccgagt acacggtcac cgcgatgccc tcgcgcggcg tcacgtgcac gtgcatggag 27300

tccgtgatca cgaagcccac gcccgtgacc ggctcctcgc ccgcgtcgtc cgtgaagagc 27360

agcccgtggt tgaagaggta gaactcgttc tctgcgagcg aaagccgccc gcggtcgcag 27420

aggtagtaga agatgtcgta gtcgcgcacg gccagcgtga acgtggactc gctcaccacg 27480

atgtacttgt ccagctcctc cagcacggac gcggccgcgc ggcgcgcggt ggcgcggcac 27540

tgcgtcgggc actcgcacgt gtctgcagag tacaggatgc gcgttcgccc cgaggcggtc 27600

tcgagaaaaa cgttaatcgc ctccatcgcc cagaagcgac tcgaggatcg cgagcaccgt 27660

gcgcagcacg agcccgatgg tgcagaaggg aacctctccc gactccgagc actcgcggat 27720

ctccgtctcc acgcggtcgt gcacttttat ggagggagcc gtcgttccag tggcctccat 27780

cgcgacggac accaccttgg ccacgaactc gcggatcttg ctcatgcgcc ggagcacggt 27840

cacgcggaag aagacggccg cgagcaggta ctcggccacg cagctcacgg cgatgagcgc 27900

ctgcgcgtgc ctggtgttga gcacgtgcgc gtcgggcacg aagtccgaga tcttcaggtg 27960

cgagagccgc acgagcgcgt tggtgtcctt gacgccgtcg caggaaatgt tcgcgaagat 28020

gagcttctcg tagtcgagcg cctcgaccac gcgctgcgcg tccatgtgcc gctcgcccga 28080

gagcgcgcgg ctcaaagggc cccagcacac cgagcccgcg aagcaggggt ccaccacgcc 28140

gtgcatcgcc agcagcgtca cgtcggccac cgccgcgagg atggccgcgt cgtcgagctc 28200

gttcgcgggc gtcgcgcccg tcagggacgc gctgcgcagc gcggacccgc ccggcgccgc 28260

gtgccgcgcg cagaactcgc acccgcagcc cggcggcagc ggcggcttcg agagcagact 28320

tatgagcccg cccacgtgcc cgtgctcggt gatcagaagc gccaggatgc tgtcggtgct 28380

gccctctatg gcggctgtgg ccgcgctaga aggctctccc gtgacctgcc atccgcagac 28440

aaccttgagc atcttgcgct tgagctcgtt gggcgcctcc gaggccagca tcgtgcgcgg 28500

gaacgtcgcg ttgaggcgga agtcctgcag cagcttttcg agcgtcgcgg tcttggcgtg 28560

ccgccctttg cgccactcct cccccaggtg ccacatgagc tcctcggccg tgtccagcgt 28620

cggcgagacg cgggccttgg gcacgcgcgc cgcgctgcgc atcagcggct ccgaggcgcg 28680

gaagctgccg cgcgcgtgca ggcgcatgga cgcagacgag gaccgcatcg agggtctctg 28740

gtggaagctc gtcatcgtga agcgccgcgt gagcggacct acctcgtcgc gcgactcgtc 28800

gaagtccggg tcagggtccg tcgtgtcggt ctcggcgctg tgcgtgctgg gcgcgctgct 28860

gcgggcgctg cgcgtgctgc gcgtgctgcg ggtgctctgc gtgctgaagc tgcgcgagca 28920

ggagtccgcc gtgtccgctt cctcgtagtg gaagtccacg tgctcctccg gcagccggcg 28980

cgagcgcgac ttggagatgc cgaccatctt gtccgcgccg accgggcgca cgcagagcgc 29040

catcgcgccc tcgcgcaccg cctgcgcgaa ctcgcggctg gcaaccatgc tggggttgag 29100

gaacttgatg atgttgaagt atggccactc gcagacgagc cgcgcgcagg tcgcgacgtc 29160

gtcgacgctt ctgagggccc tgttcagcgg ggggatgttg ctgccgtcgg ccagtgcgac 29220

gaggtcctcg ggggagatct cgagcttggg aatcatctcg ttcacgagcg cggggtcgat 29280

ggcgtggcag acggtctcta cctcggtgcg cgagagcttg agctgcgcgc aggccgccca 29340

cggcgcgcac gtgagcgcga acaccgcgga gactcggccc ttgccgacca tcgccacaac 29400

ctcgggctcg tagaccaggc cggccttgag cagagcctcg agcacctcta tgtcgtcggc 29460

ggcgagcagc gcgcgcctgt ggaagcacac cgcgggcatc atcttcttgc agatgccgcg 29520

ggtgctcttg agggccgtga taaggtcggg gagcctgacg tgctgcggct ggaagaacat 29580

gacgttggcg gggctcgcgg ccaccgcctc gtcgtacacc gacatcggca ggtcgccgtg 29640

gagcccgcac ggcagcaggc tcagcagctg cgcgcgcgag agcttctcgg cgcagaagtt 29700

cttcttggcc agggcggccg ccgcgtcgcg ggccttcttg gggtataaca acatggcggg 29760

ctttaaacac gaaacaaaaa tccgggttgt aacatttcaa ttttgcatgt tctgggcctc 29820

ctcgcagagt ttctccaggc cgccggccac gatggcgtcg acgaagaggt cggcctcggt 29880

gaagcggtgg ttgccgcgca ccgccaccag cgcgttctcg gtgaccacca ccgacagctg 29940

ctgcgcagcc gtgcgcaggt cgaagtgtcg gcacgcgagc ccgtggccca gagtctggtc 30000

cagcgccgcg agcacctcct ccaggctctc ctcgcggtgg ttgtagaacc acatcagcac 30060

gaagtaggcc acgtaggtgt agaggtagtg cgcgaccgcg cgggcgcgca ccagcggctg 30120

gttgcagcgc gcgaaggcca ttccgctggc gatgaggtcg tcgtccagcg tggcgtagtc 30180

gccccagttg agcgcgctga actgcacgct gtagaccgcg cgcacgaacc cggactcgag 30240

gatgtcgatc tgcgacggcg cgccccaggt caccagccgg tacacgatgc cgcgctgcat 30300

cagccgcatg aggtcgccgc cgacctcgcg caggcggtgc gtcagcgagg cgaaggccag 30360

cttccgctgc gtgtactgca ggcccacggc cacgcacccg tccgactcca ccagcacgag 30420
cttgtggtcc gtgcagccgt cgctgcgcag gccgccctcg cgcgccatca tgtcggtgac 30480
gaacatgtac ttgcgcgcgc gcggcccgac caggatgcgc ttcttgcctt ccatgcgcag 30540
caccacgtcc tctagcagcc gcgtgctgtc cacgcgctcc acctgcgggt ggatggtggg 30600
ctggtagttg tacagcagcc gcggggacca gttgtaggcg aacgcgaaga ggtgcgtgtc 30660
caggagagag atggggaaga cgccgccttc ggtggcgcgc cggaggatgg cgagcttggt 30720
ctccgcgggc agcaggctcc cggtcaccgc gccgaagaac atggggtggt tgcggaactt 30780
gagcgcgtac gcgctcagca cctcctctcg cgagaatatc gagtccgcgg ggttggagct 30840
cgcgggcagg agcacgtcct ccacgctcag cacctcgtcg atgaagggca gcaccatgtc 30900
cacgttggag gcggtgaacc actccatgtc cacggcgttg cgctccacga tcacccggat 30960
cgtctcctcg gatatgttct cggcgaaggc gctctgcagc tcgatcaggt tctcggtggc 31020
gtcgatgctg agcccgaggc gcatgccctg ttgcagcacg tcgttgatgg ggttcacgta 31080
catggccacc gccatgcacc cggccggctg cacgcgccag aggttggagt acgcggacac 31140
gtccgtgatg cgcagcgtct ccagcacgtt gtacatcgcc gcgcgcattt ccagcgtgtc 31200
cacgtacacc tccgcgtgct cgaagatgat gtcgagctcg aacgcggaca gcggcagact 31260
cacgtagatc tggcaggcgt caaagagttc ctgcgcgtac tcagagaagc acgcgtacgc 31320
gatcacgccc gcgcggttga cgatgtagtc cgccttgaac atcttcgtga ggtaggcgta 31380
cagcgaccgg caggccacgt gcggccttag ctcgtcgagc acggcgtcca gcacctcgcg 31440
gtgctgcagc acggaggggt gcaggaactg cgtgaagtcc accgcggtct catccatgaa 31500
gtcggggatg gtctcgacca ccaagcggtg gttccgcacc gcgcggaagc cggtgttcat 31560
cggcgcgatg ctgtgctggt cgtggacctc cagcaggatc tcgtagccga tctctcggca 31620
gctcagcgcc gcgcgcgcct ccgtcacgct cgcgttccgg aagaactgcc gcaggtgctc 31680
gtccgtgcgg cagagcgtga cggattgggg gatgcgggaa aggtcgcaaa gagggctgtg 31740
gacggagatg cgccgcagcg cgtggtctac gaactcaaaa ccgcgcctcg acatcgtgaa 31800
gtcgcggagg gcgtacatgt tgtaggaaca gaggcggaag aggcagtcta tgtctagcat 31860
gttggaaacg cagtacgcgt gtctgtggag gtgcgggagc atggcgggca cgacctcggt 31920
cgtgttctgg agcatggtgc atacgaggtc gggcgctgtg aggtcggggg tgacgatgag 31980
gatgcaggag ctggagaact tgctgagctc ggaggccagc cggtgaaggt tgtagtcgtg 32040

gcgctggctg aagttgctgt ttatcgtgcc cgtgaagccc atgagcgccg ccagcgtcag 32100

gtcctcgcgc tcgatggccc agatgtctag gccggaggct atcatgcagc gcaccagcgc 32160

ggcggcgcgg tcgctcgtgg ggtagctcat ggtgctgtcg gtcgtgcgaa tcagcatggg 32220

ataatgcttc atttttacgg tcgggggttg cggactgtgg ggcgcacagg gcctgcgggc 32280

ggctcgtgcc ggtccgcggc gttcgccgaa cgcaggaacg ggcccatgcg cgcccaggcc 32340

atccacagcc ccgccgtcag cgccagcagc cagacgaata ccacgatcat cttttatgta 32400

gctggaactc gcgctcactc cccgccgcac ggcgacgggg agagcccaga gccgagctcc 32460

atgcgcgtgc tctgcacggt gagcgactcc acgagcttgg acacgttcat gcgcgtgttg 32520

tcgggcacca ggtgcgcgag gcgcgcgtac acgtcatcgt gcatgcgctt gctgcagcgg 32580

tccaggctcg cggagagcgc ggagactagc gcggtgtgct tcgcgtacac gaagtcgccg 32640

agacacacgg tctcgagccc gagcacgtcg gcgctctccg cgtccttgag cgccacgaat 32700

atcttctgct cctcgcgcgt catcgagcgc atgaggtagt cgtgcagccg cgagcgcgag 32760

atgagcccct gagagatctg cgggctgcgc atgaagcgcc ggcgcatcgc gcacagcagc 32820

tcctcgtcga cgacgtacat gctgtccttg atggagctct tctcgtcgat cacgagcaga 32880

ccgtcgttgg cgaccacgtt gatgaaatcg tccacgtgcc gcgcgtctat gtcgtagcgc 32940

gtgccgccgc actcgatgtg cgagggcggc gacttgagcc ggttcgcgag cgccttcacg 33000

tcggagacgt cgatgtacag cgaggactcg cgcgggacgc agccgaggat gcgcgtctcg 33060

agcggcgtga ggatgagcac gcgctcggcg ccgtcgacga gcttgccgtc gtcgggggaa 33120

aagaagttgt tctccacgat gctcgagacg aggctggcga gcacgccgtc gcggtaggcg 33180

ccgagcgcaa actcctgcac aaagggcgcg tgcagcaagt ccacgggaat gcgcatctcc 33240

acgcggcgcg cgaccgactt cttcttctgc aggtgccgcc ggtccaccat ctcgtccacg 33300

atgtccgata tgcgggagca gaggtacgcc ttgaggacgt tggcgtttac cttgttgaag 33360

atgagccgtt cgtcctccat ttaagctgct cagacgagct ttaaatagtg gaaacacagc 33420

agcacgccga tcgccgccgc tatcaggccg attagaaaaa cggtggtcca ggggacgccc 33480

ttgggcctat cgcacgccgg cttttcggtc attacggtgc gcacgatgtt taggaactcc 33540

tcgaagtcct cgtccgagtt ggagaggaag gagccgaaga cgccggtgta cagtttgtcc 33600

atttactact agatattaaa cggcgcttcc aactcctcgt cctcgaagcc cgcgccaggc 33660

tcgacgacgc ccaggccgcg cacgtcctgc tcctcgttga acgtggtctg agtctcgctc 33720

atgcgcacac acgtctgctc gccctcgaga ccgagcacgg tcagcgagca ctcgcgcggc 33780

atggtgatct tcttaaccgc gaaggtgact ttgccctcgc cgcccgagcg gtagaacacc 33840

accggcgcca ggatgagcgt cgccatctgc gcgtcgcggg ttgcgaggtt ttctatctct 33900

cgcgtcagcg gacatatctg cggctgggtg tcgtcgtcgc tggtgaactc caggagagcg 33960

ccggtcaggc ggttgagata cagacatccg gacttaaagg tgttgtcgat ggccgtttcg 34020

gtgttgaagt tgcgcagcga cggcggaacg cgagcgccgg ttttgatgtt gtcgtatatg 34080

ttctccagca gctggtagag cagcggactg gcgctcacgg gcttgaggcg accgaagtac 34140

ccgctgtcgt tctgccgctg catgtcggtc ttcttgctcg ggtagatctt aaactcgccc 34200

ttcacgacga tgagcggcga gacgagcttg gatgctagac tttcgacgag acacacgttg 34260

atgttggagc agctcgggta ctgcgacgga gttagagtca cggcctcgat gaccttggtt 34320

tggctcgacg agagcgactt agcgaagttg atcgcgtcgg tgcacgacat cgcctggttc 34380

tcgccgaacc gcctggcgga cgctgcatcc tcctgctgag gagcgcggtt agacgcgacg 34440

gtggttttgg atacagcgcg tttcattatt gcggcgattt taaagtacgt gtatactttc 34500

agttttgtcg ccgagcgttc agcgcctgca tgcagaggaa gtacaggatg atggtgcacg 34560

ggatcgtggt cagcagcgat acgaagtcca tcactgtgag gacgcgcagc gccccgcgcg 34620

agcggatgcc cagcgagggc gcgccgcggc gcgcgatggt ggccccgttc gtcaccacta 34680

ccagcagcat taggatggtc gcgcccacgg cgacgcccag gtcccgcgac tccatttata 34740

gtacagtata gagcgaccgc gtcacgaact ctcggctggc caacacgcgt ccgtcgggcg 34800

ggtgtccgcc ggccttcccg cggaactccg ggacctcgaa gctggacttc gtcacgcggt 34860

acgtgtactt gccgcgccag accaggtttt ccttctggaa gacgccgtcc atggtcacgc 34920

ccgccatgaa ggcgtccttg acgatgacca gcaccgcgtc tagcttgcgc ccgttgatgt 34980

gcgtgacgaa gtccgtgccg ctgcggctcg cgcagcggat gtccacgccc gagggcaggt 35040

ccaccacgaa cacgaagcgc ttcggcgcgt agagcaccag gtccgaggac ggcgacgccg 35100

aaggcgccga ggggaactgc cggtggtcaa aagggtgcac cacgcccacg atggacgtga 35160

cgcggtcgtc cgggaactgc gtcgcggcgc cgccgccgcg gtgccgcgtg accgtgcttc 35220

tgcccacgtc gtcgcagacc acgtgcagct ccgacacgat cggcagcagc gtggccagca 35280

tgcggtcggt ctctgtgcgc gtcgcgcagc ggtacgcgat cccgcagtgc gcgtcctgcg 35340

tgcgcccgaa gaagagcacc agcacgctcg cgtcctggtc gaagggacac acggccatca 35400

cgcccaccgg cggcggcccg tggcctgcgt acgcggagga gaactcctgc acctcgacca 35460
cggcgtcctc gcgcgcctcg ccgggcacca tcgccgccgc cggccgcagc gcccgcacgg 35520
tctgcttaac cgcacgcgcg gcggaggccg cgctcggcgc gactacgcgc acggccgcgt 35580
gcgcgcccgg cggcggcgcg gttccggcca tccagcccac cggcgagaag aacacgtcgc 35640
agacgtgcac gcccgcggcc tgcagcgcgc gcgcgagcgc gcgcacggcc tcccactcct 35700
cgcgaaaggc gctcgcgacc gcgagcgcct tcagcaccgt gtccacggag ttgacgggct 35760
tctggaagag gttctcgttg ttgtagatga actcggggag ctccacgggc actgtgaaca 35820
gcctaatctc gtgcgcgccg ctgggcgtga gccgcgtcgc gggcttgcgc acgccggcgc 35880
cgatctgctt gaagaagtgg ttcatggcgc cgccggcttc tcgggctccg gcgggagcag 35940
actatttatt cgggaggtta tcctttccga aagcacctgc acggacttcc gcgtccagcg 36000
ctccatcttc atgtactcct tcatgccgtc gctgagcacc tcgacggcct ccagcttggg 36060
cgctgtcggg tcgaagagga tgctcttgag cagcgtcatc ttcttgtccg cgaggaagcg 36120
gaagtaagtg tagatgcagc gcagcgcgcg gaagttctcc gggtgcttga tggtgcacag 36180
gatcatgaag atgcaggtga acatgccgca ctcggactcc atgagctggt tgacctcgag 36240
gttgatgcag ccgcgccgcg ccttgaagtt gtccacgaag aagcgcatga gcacgtccac 36300
gtcgcagttg cggttgtcca ggtccgcggt ctcggcgttc acgttgaagc cgtccgagaa 36360
ggagtagaag tagaagtact tgcaggggtg gaactccgag gggctgttgc cgccggagtc 36420
gtagaaggac acgagccgcg agacggtgtc gaagatgcag cacttccagt ggaacatgta 36480
gcagaagccg aacatcacgt agcgccgccc ggcgcgctcg atcttgtcct tgagcgtgag 36540
gctgaccatg ttgcagcgga agcggtccgc cttttcgtgg atggccgcgc cgttgaggaa 36600
gttcaggttg aactggccca ggtacgcgac ctcggtgccg aacgcgaagg gcgccaccag 36660
actctggatg ctcacgttgc tcatccaggc gctgcggtcg ggctttatgg cgatgggcac 36720
taccttggtg ttcacgcccg tgctgacgcc cgcgcgcgcg aggtcgtcca cgttcagcgg 36780
catctgcgag aagtccacgg cctcggacac cttctcgcgc aacgagggct tgaagaagaa 36840
gcccagcggg accttccact ccagcgcgat cgcctcgcgg aagccgtagc gacccttgag 36900
gctggccagc aacgcggtct tctgcgcgac ctcgtccttc tcggtgtccg gcggcgcggc 36960
gtcgatgagc ccgcgcttcg cgaagtccag cagcgccgcc agcgggatgc acgagacgcg 37020
accggccgtc gcggattcgt cgaagcgccg caccacgtac ccgttgcagt tggtcttgaa 37080

gttggacacg tccaggtgcg cgctgagccc caccaccgag tagatgtggc acagaaggtt 37140

ggtgaacccc agctccggga ttttgctcac cactaaatcc gtgtacttgt ccatttatca 37200

tggagaatca tctgccggac atgctgatgt ttcccaactg cgtttctgtg tttccctttg 37260

agtactcgct ggaggacgtg ttccgcctcc ccgaggagcg acggcgcgcg ttcgccatgg 37320

ccgtgttccc gctctccaag caccgctgga ggggcgcgcg gctccagcgc gacgagcgaa 37380

gcgtgtggct cagcgtcgag gaggaccgcg ggcgcgcgct ggacgagcgg aactgctcct 37440

ggctctcgga cgtggccgcg cgcatggtcg acgacgaggg ccgcgcggtc acgcccgagg 37500

cgtacgcctt catgcgcgcc gcgcccggcg cgcgcgtcgc cgagctcgcc gcggacgcgg 37560

gcgtgctagc gggccttgtc gccggcggca acgcgctgcg cgtcttctcc tcggagtcca 37620

cgcaggcgcg cgagggctgg aaggcgcgca gcgtgggcgt gctcggcaac gcggcgccgc 37680

tggcgcccgt gccgctggca tcgctgcgtc cggaagtgca gcgcgagctc ttcgccgcct 37740

ggatcggccg ccgcccccgtg gtgctcacgg gcggcacggg cgtggggaag acctcgcagg 37800

ttcccaagct gctgatgtgg ttcaactacc tcttcggcgg cttcgagcgc ctggacgccg 37860

tccgcgagtt cgcggagcgc ccgctcgtgc tctcgctgcc gcgcgtcacg ctggtgcgcg 37920

cgcacaccgc gacctacctc gcctcgctgg gcttcggctc ggccgacggc tccccggtct 37980

cgccgcggta cggcgccatc ccggacgccg agcggaacac ggccccgcgc gcctacgggc 38040

tcgtggtggc cactcaccgg ctcacactga ctgccatccg ccgctacgac acggtcgtag 38100

tggacgagat ccacgagcac gaccagatgg gcgacatcgt ggtcgcggtc gcgcggaaac 38160

tgggctcgaa catgcgatcg ctggtgctta tgacggccac gctcgaggac gaccgcgcgc 38220

gcctggagga gttcctggac cggcccgcct ttgtgcacat agagggcgac acgctcttcc 38280

ccatccgcga ggtctacgtg aagaacacgc aacagccgcc gctctcgcgc aagtacgcgg 38340

aggcggagct gcagaacgtg gcgcaggcgc tgggcacctt cgtccccgag cagggaaagt 38400

gcggcatcct cttcgtagcc acggtggcgc agtgcgcgct cttcgcggag accatcgagg 38460

ccaagcaccc cgggctgctg gtgcgcgtgg tgcacggaaa ggtgccctcc gtggccgcgg 38520

tgctcgagga ggtatacgcc gcggaccggc ccgcggtgct ggtttccacg ccgtacctgg 38580

agtccagcgt gaccgtgcgc accgccacgc acgtctacga cactgggcgc gtgtacgtgc 38640

ccgagccctt cggcggccgc gagaccttcg tctccaagtc catgtacacg cagcgcaagg 38700

gccgcgtggg ccgcgtggcg cccggcacct acgtgcgctt cttcgacacg cggctcgcgc 38760

tgccgctgaa gcgcatcgac tccgagttcc tgcacccgta cgtgctttac gcgcgcatct 38820

tcgggctaac gctgcccgac gacctgctcg tgcagcccag cgacctcgcg ctgctgcgcc 38880

gcaccgagga gtacgtcgac ggcttcggca tcagcctctc gcgctggacg cagctgctgg 38940

accggcacta catgcacatg gtcgagtacg cgaaggtgta cgtgcgcggc gggcgcctcg 39000

ccgccgcgct ggacgccttc gagcgcaccg gcgtgatgac gcacgaggcc accgaggcca 39060

tccgcgccgt ggacatgctc gcggccgtcc taaacgtgcg caagtccaag gaccgctacc 39120

gcgcggagtg caaggtgctc ttcgggccct tcgcgggcaa aaagttcgtg gtcgccgggc 39180

ggcgtccgcc cggctcgcac gtgctcatgg tcacagaccg cgtcttcatc gaggccgagc 39240

ccccattctg aggaccacct tcttggagac gcccgagaag tcgtcggcga cgccgcggcg 39300

cgccaccaca aggcagtacg aggtcacgtg cgggcagcgc gcgatgcagc ggaaggcttc 39360

ctcctgcgac agcgagaagg cgaacacgta gaaggtgtgc ggggacttca gcggcgtgtg 39420

gtccatcgag tagatgacac cgagcttctt catgcgccac ataagcgcgt tgatgtggtc 39480

ggcgcgcagc gcgcggccct tgagcacgcc gcagacgaag ctcgagcagg ccacgacgtc 39540

gtagcgcgtg ttcctgccga agaccaggtg cggcgcgccg gcggcgcgcc gcgcggccgc 39600

gcgattctcc acgatgtcct ctatggagcg ctcgctcgca aagaagtcca ggaacatgta 39660

ctggtaggcc acggccgggc gcgacttgct gaacttcatg aaggcgtccg agtccatgat 39720

ggcgtccatg tcctcagcgg cgagccggtg ctgcagccgg atgccctcga aggtgtggaa 39780

gagccgcgcg tccgcgtgca tggacagcgc gagagtgacg aagttgagaa ggtccgcgtc 39840

gccaaagcgc acgagcacgt taccgggcgt gcgcgtcttg cgcatgagcc gcgcgggcgc 39900

gccgtcgttg tggctgcggc ggcgcatttt gtcgccgggg gactcgggcg gcaggtcgat 39960

catgaccagc cggtgccgct gcgcgtcctc ggcgttgaag atcgaggacg tgaagcccgg 40020

gtacagcacc acgcagtcgc gctccgagat ggcgtgcagc acgtcgcgct tgagcccggc 40080

caccagccgc tccgcgttct cgacgaagta gttctcgtag tccaggatgt cgtgcgccat 40140

ccaggggaag ttcaggtacg cgttcatggc gtagtcctcg gcgtcgaagc agatgcgcgt 40200

gtctggcgtc gccgcgatcg gaaggtcctt gatgccgcgg agcagcccgt cgtagtcgga 40260

ctcgtccacg aaggagagca ccacaaagag gtcctcgccc acggtttcgt agtcgaagag 40320

gtggtaaagc tctcttagcg ccagcacggc gagcgcgttg tccagcgagg cgtgcacgcg 40380

cgccaggatg ctgtagaagg gcgtggccat catcacggcc ttgccgccct cgcaggcaac 40440

ggcgcgcggg aaaatgacct ccggcgtgcg cggcagccgc ccgaacgtcg cgttcagcag 40500
cgcgaccgtg gccgcgtcgc tctggcgcag gaacactacc accgaggggc ccgagatgct 40560
gagcatgcgc tcgcgcatgc gcgctggcag gtccggcgtg gtcacgaggt ccgcgaagcg 40620
gccgccgttg tagaggtcgc cgccgccgag gaaggtgagc acgtcgaagc agtgcagcac 40680
ctcgttgcgg aagtagtact cgttctcgag ctccttggcg tacgcgcgta tgtccacgtt 40740
ctcgaagttt gttcgcagac cgccgccgtc gaagaaccag gacgcgagct cgcggacggc 40800
gtccgcgggc ctgttgcggc ggctcttgca ccagaagctc atgtagttgc gcgaggtgga 40860
ggcgttcgcc aggaagaagc ggtggtcgaa ggagatgagc acatgctcga gcaggtgcgc 40920
gagccccagg accgcgccca cgtcgcgccc aaaaccgaag tttgatatcc ccaggtagac 40980
gtcccgtttc atagacggcc tcaggaacac cctgacgccg ttttccaaca ctatcattct 41040
ccggtattta cttacccaaa agtagtatgg ggagaagtgt ttgaacgtcc cctcgccttt 41100
ttaaatcaaa agtagacttc tcgcgcccgt gcgccaccgt cacgcgcgcg cggcgcgagt 41160
ccataccggc gatcaccgcg ctgctctgcg gtgcgtccgg ccgcgggaag agcacggtct 41220
cggagatccc gtccagctgc gcgtcggtgc gctgccgcca cgcgtgcgcg tccgcgagct 41280
cgcgcacggc cagctgcatc ttgttcgtcg gcaggaacgt gaacacgtac gccgccgcca 41340
ggaaggctgc gaagagcacg aactcaaccg cccatgacat ttagggagct gattttgttc 41400
cacgcggcga cgcacgtcgt gacgggcgac cccgaggcgc cgcggcgcgc ggcctcgctg 41460
tgccgcggct tcggcgtgga cttccgcgcg attcacgcgg agttcgcgcg gcggtacccg 41520
cgcaccgcgg ccgccgtgga gcgcgcgcag ccgctgcccg aagtcgatgc cgcctttccg 41580
ccggacgcgc gccggcaagt cgtgcggctg cgcctcgagg ctgcggcgct ggtcgtcaag 41640
gagtcgcgtg cgctatcggc ctccatgcgc ggcgtggcgg tggtcgacgg ctgctgcgtg 41700
cgcgtgtgcc gcgccaacga cgagctgcta gagttcctcg cgcggcgcta cgaccccgcg 41760
gtctaccgct acgcggaggt gccctcgccg agcgtgcgcc cgggctcgaa agtcttcgcg 41820
tgcgcgggcc gcagcgtcac ctttgcggcc gcgcaccgga gccgcatcac ggccaaccgc 41880
ccgctgcgcg tggtcgtgac cgaggcctgt gtggacggcg tgctcgcgcg cggcgccgcg 41940
gaggtcttcg accgcggctc cggcgtgctg ccccgcgcgc tgcgcgagat cttctaccgc 42000
ctcgacgagg acggctgtcc cacgggccag acgccaggct tcgcggacag tatggcgtcg 42060
cgcagctgat ctatgtccac ctttttctcg tcgatctgcg ccacgaccac gaaactgcga 42120

atgtccacag cggccatggt cttggccacc gggtcgtact tgaggagcag cacgtactcg 42180

ttgccgaagt gctcggtgac ctcggtgatg agccggtaca cgcccatgcc gagcacgttc 42240

accgcaccgt ccttggcgaa gagcgagagg atgttcacgc acttcagctc catctcgccc 42300

tcgaggcgcg cgagcatgcg ccgggtgacc tcgcatactg aacaaagagg cttacctagt 42360

aagataagcg ttagcttagc cgcggtcggt gacgcgtcgg aggccattta tggggatcaa 42420

aaacttaaag gcgttgctgc tcagccacgg cgcgctgacc ccgcacgagc cgggcggcga 42480

cgagcgcttc cctgccgtgt tcgtggacgg cttcagcgtc atgatgacca tggcgtactc 42540

gtgcgcggac gaagacgagt tccgcgcggc cgtcgaggag cgcgtgcagc actggatgag 42600

cgtgtccgag agcgggcgga tcgtggtctt cctcgaccgc ggcgagattc cgatcaagca 42660

gccgctgcgc gaccagcgcc gcaaagccac gcgcgaccgc gccgcgcgcc accgcgagtt 42720

catcgccgcc gcggaggcag acgcggcggc agaggccgtt ggcgcccgcg aggacaaaca 42780

ggaggacgag cacgcggagt tcgccgagga gatccgcgct gagaagcagc taaagctgca 42840

gcgcatccgc ttccagctca gcatcgccaa ccacgaggtc gttaagtcgc tgatagagtc 42900

cacgctcgcg cgcgctggcg atgccgtgga gatcgtcttc tgcgacggcg tcgacgcgga 42960

gatggtcatg tgcgcgcgcg gacgcgccga ggccgagcgt cgcgggcgct ggccgctgct 43020

cgtgaccacg gaccaggacg cgcttttgtt cacgtccacc gatcgcgacg agaagatagt 43080

gagcaccgtc tccgcctgct acgcgttcag gcccaccgag acgaccgagt acctgtgcaa 43140

acttgcggcg ctggccaacg gctgcgactt cttccccggg ctcggcggca tatgcgtgag 43200

tgtggagtcg ctgcgccgcg ccacgctttt cccggaattc tccgtgcgca acgccgccgt 43260

gagtctgtgc acgcggccca tgcggctgtc cacgcaggac gcgctggagc cagaggccgc 43320

cgccgaggtc gtggaattca tcaggcggta cgccgccggc gacgagcgca tctaccgcga 43380

ggtgccgccc ggcgcgtgct gcggacgcgc gtttgtgcgc ggagcgctcg cggccgagtg 43440

ggccgacgcg ctgccggcgg ccacgggtct gagcgtggtc gcggacatga tcgcgtgtct 43500

gcccgcgcgg cgggaccccg cgcccgagga ggtagagcgg ctgctggcgc tggaggcgcg 43560

cgcgcgaggc gcgcgcgtca cggatgcgat gctcgcgcag actgcgcagc tgctgggtta 43620

cggcgcgagt gcgggcgccg acggcgcctc cgccttcgcg gtctcgggcg ccaagggcct 43680

gatgtgtcgc ctgcgcggca cggccatgtt cttcaacgcg gagtacgtgg aaattgaaag 43740

cgaacccaga ctgttaaagc tgcggtagca tggtgttccc gatcgtgtgc tcaacgtgcg 43800

gccgcgacct gtcgcacgag cggtttctgc tcatcgtgcg acagcggccg ctaaaggttg 43860

ttttgcggac ggtgcgcaac gtctgctgcc gtataaagtt gtctacacaa atagagccgc 43920

accggaacct gacggtgctg cccatgctcg acataagctg atttttcttt tccgctcgta 43980

tgcgcgagtt cggactcgcg gcgcgcatgg cccgcgccat cgaggacgtg tgtccgcgcg 44040

gcgcggtgat attcgtatcc agcgccgcgt ccatgaccga ctgcctgaac ccgtcggtgt 44100

tcaagcacgc ggcgatatac gcggggcgcg tggaccgcgc gccgctgccg ccgccctcgc 44160

cggtcccggc ggaggccgtg acggagccct gtgcgataga cgccatagcg ccttacggcg 44220

cgcgcgtggt cctgctctcg gagctgctgc ggagctgcgt ggccgttcag gcctaccgcc 44280

tggcagtccc cggcgccctc gcgctcatga acctcgcggc cgacgcggcc ttcgagctcg 44340

tgggcacgcc ctacggcttt aacagcgacc gaacgtactg cttcaagctc gttgccgact 44400

gctttgctag cgtgggcgtg acaacgaaga ccaggcgcat catgggtcgc gacgtcgtgc 44460

tcagccagga cttcctggag agcggcatgt ggaccaaggt gctggactcc gccgcggagc 44520

cgccgtggct ggtctagaac agcggcggcg cgcgggtccc gagcacgggc cgcgccacct 44580

gcagccgctg ctgcagcgcg cggcactgcg cctcggcgtc ggccgtctcg gcggggtcga 44640

cgggcgtcgg agttgcggag gtggtcctga acggctgcgt gttcaccgag acgcggatgc 44700

gctccttgca ggagcgctgc tcgatgcagt tggccagcat cttcatcacg tgcaggtact 44760

ccagcaacac gaacttttcg agggtgatgc cgtcgaaggg cgacgacccc accacgccca 44820

gcgggctgga caccgcgccg tcgagcacct cgccgcggga ctccttgcgc gcgcgctcga 44880

gcaggtcctc tgtccgagcc accacactgc cgaagtcggc ggccgcgggg gcgggaacag 44940

gcgcagcagc gctgtccgcg tccgccggca tctcctcgat cttgagaccg gccgcgaact 45000

ccgaggccgc gtgcacgggc gaggcgccgc gccgcaccat gaagtcgcac agacgcgata 45060

gcgcggagga gcgcaccggc atgtcgagca ggcgctcggc ctccatctcg gcgaccgagt 45120

cggcgcacgc gtccggcgcg cccgcccgca cgagctcgtc gcagcacccc gcctccttca 45180

tgagcgcggg catgagcttg tactgcgcca tgttcaccag cccgtacttg agctcgagca 45240

ggtccgcgag ctcggaggcc atgggtcggt ttttggtgta gatgacgcgc tccacggcct 45300

ccgccatgtc cacggcctgc atgagctcgc cgacgagcac gctggccacg agcgtggcca 45360

gcgtgacgcg cacggtgggc acgcagaccg cgaagaagga ggtggagtgg gtgaagcgca 45420

tgagcgcgcc gtgcagacgc gcgaggtccg cgctgttgcc cgcgtgcacg aagcgccggc 45480

gcagccgcgc cagcgcctcc acaaggtcct ctcgcgtggt cacgcgcacg ttcgcgatgc 45540

acaggtcgtg gatcgcgttg gcgatctgcg cgcggcgctg cggcgagctg ccgggcagca 45600

gccgcgcctt ggcctcgacg tcgacggtgc tcgagagaca gccgcaggcg gcgccgcgga 45660

cgacgaactt caacaacgac tcgaacacgc gcgcgcccgc gcggggcgct tgcttggacg 45720

actccattta ctttaaataa tttacgagat caaaataaaa tgactctgcg catcaaactc 45780

gagaagctca agcagatcgt aacttacttc tcggagttca gcgaggaggt ctcggtgaac 45840

gtggacgtcg gcgatggcct catgtacata ttcgcggcgc tgggcgggtc cgtgaacatc 45900

tggaccatcg tgccgctcag cgcgagcgtg gtatacgacg gcgatgtcag ccgcgtgttc 45960

aacctgcccg tgctcaaggt gaaggcctgt ctgtgcagct tccaccccga ctcggtggtg 46020

agcctggagc ccgacctcga ggacaacgtg gtgcggctct cgagccacca cgtggtcagc 46080

gtggactgcg acaacgagcc cgtggcgcac cgcacgaaca ccgccatctg cctgggcatt 46140

aaccagcgca agtcctacgt gttcaacttc cggcgctacg aggagaagtg ctgcggccgc 46200

accatcgtca acctggacct gctgctgggg ttcatcaagt gcatccacca gtaccagtac 46260

atcacggtct gcttccgcga caagaagatg gtgctgcaca cgcccgggaa ggtggacaac 46320

ttcttccgcg agtactccat gaccgagtgg gcgcccgacc tcgagcgctt ctcgttcaag 46380

atccccatct cctccgtgaa caaactccgc ggcttcaaga agcgcgtggt catgttcgag 46440

tcgcgcgtgg tcatggacgc cgacgacaac atcatcggca tgctcttcac cgaccgcgtg 46500

ggcatgtacc gcgtgaacgt gttcatgtcc tttcaggacc ggtctctttc atgcgactaa 46560

atactcatgg gcgggtcggt gagcctgccc tcgcgggacc tgccgccgcc ggtgcgcacg 46620

ccggagatga acatcgtgcc cgagcgcgac ctcgcggaca cgatggcgcg cctctccacc 46680

gcagacccgc cgcagccgct gggcgtcggc gacgacgcgc gcatggccgt gctgaagacg 46740

accttccccg agttcgcgat atcgcggccc gcgacgggca tgctcgccgc gcagcgaatc 46800

aggtacgacg gcgacccgcg cgtctgctgc ggcgggttcg ggatctcgca ttactgggag 46860

aagggggcgc gccgatcgaa cgtcgcgttc gagggcgcgg cgctgcgcac ctgcgacccc 46920

acgcgcttcg acgcgggcgc gtgcgacgcg ctgctcttcc gcgagtgcgc cgccggcggc 46980

gtcgacgcgg acttctgcgc gcactggatc aacgcggccg tgacgcggcg cacggaccga 47040

cagtcgcgcg cgcggctgaa cgacatgttc gtgcgcgatt gccaaaacga cgccgcccgg 47100

cctcactgcg tggcctggat ccgcgcgatg cgaagcgcgc gcgcgacggc ggacgacggt 47160

ctaatagacg ccgtgctctc ggtgcagagt cccgagttca agggcaagca catgcgctgc 47220

agctacccct cgccggccac tctcgccatg gccgcgaacg tggacgagcc gcgcgagtgc 47280

tgggaccccg agtgcgtggc cgggaacgtg gacttcatgc taagcgataa ctacacgaac 47340

ctgggcttgt gtcggctctc gcgctgctcc atcggcgtca cacacctgcg gattgacgcg 47400

cgttcgcggc tgcgcatgcg gtgcgccggc gcgcttgccg ggctcacgaa ggcgcccgtg 47460

aaccagactg tcgtcgtcgg cgacaacctc gcgcgcgcct tcgagccgcg cgtggaaacg 47520

ctcagcgtgt tggcgctgtg cgtggtgtat ctgctaattg tctggctcta aatgggggcc 47580

gccgccagca ttcagaccac cgtgaccacc gtcagcgagc gcatccgcaa cgagctcgag 47640

cagagcgcga gcgctagcgc gaccgccgac tgcgacgtca ccatcgggag tctgattatc 47700

cgcaagaacc taggatgcag cgtttccgtc cggaacatgt gctcggccaa cgccggcgcg 47760

cagctggacg ccgtcatgaa ggccgtgagc agcaccttca acgacctctc gtcggaccag 47820

aaggcctacg tgcccgggct gctcacggcc gcgctcaaca tccagaccac ggtgaacacc 47880

gccgtcaagg acttcgagac gtacatgaag cagacctgca cggcggacgc ggtcgttcac 47940

aacaaaatca agatccaaaa catcgtcatg gaagagtgcg cctctctgcc agggagtccg 48000

gccacgcacc tggatttcgt gaacaccggc acggccgtgg gcaactgcgg cgtgaaggcc 48060

gtgatggacg tgctcgcgaa ggccagcacc accgtgcgca acgaccagga ggccggcaag 48120

ggctaccaga ccatcatcat cgcgatcgtg gtcgccatcc tggcggccat cttcgcctgg 48180

tacgcgcggc acatgctatt catgtccacc tccgacaaaa tcaagctcga gctcgccaag 48240

aagcccgtgg tgcactggac cacctacctg gacaccttct ttacggaatt tccgccgtcc 48300

gtctagatac gcgcaacatt gaaacattat atccacctct caaacggcgg tatggtccga 48360

cgcgtcctcc tcgagcgcgt ggacggcatc gtcgagcact cgcgcgcaga ccgacgctac 48420

ttggaggcca ttcagcgaca cctcgagggg tctacgcccg ggctgcggca gatgtggcgc 48480

ttcctctacg acctgctgct gacggtgttc gtcgtcatgt acatcgtctt ccgcctaatc 48540

gtgcgcaacc ccggcatctg cgccatcctc gcgctcgcgg ccgcggtgta ctacctgttt 48600

ttgtgtctct ttagcatgga ctgatggcga tcacagacag accatcgccc gcgcgcgcgt 48660

gaccagctcc ggcgccgcga agacgtcctg caccgggaag tcgtcgatct cgaacacgga 48720

gccgtccgcg gaccagatca cgcgcacgtt gtcgctcacc gagacctcgg tcagcgtcac 48780

gcccagcaca accgcgtcgt tggtgctcac cagcaccagc gcgccgggct ccgcgcgccg 48840

gtgcagcggc ggccccgaga ctgagcgccg ctgcacgcgg aacatgtccg cgaactgctt 48900

cgagagcaag tccaggtggt tgcggatgat ccactcgaag aagtacgcgc aaccgccgcc 48960

gccgcacagg aagcgcgaac ccgcgggcat cagcagccgc acaacgtcca tgtagcaggc 49020

ctgcggcagg ctcgcgcggt acagccgcgt cttcggcgag agcaccacca ggctggaggt 49080

gctcatctgg aagaccagct ggctaacgga gacggtgagc gtgcacgcgg gcacggaaac 49140

cacgtccagg cagatgtcgt ccagaaagat gctccgctgg tagaggtggt acaggatggc 49200

cacgatctga aaggccgtgg cgtcgctgat ggcgcagggg cggtcggcgc agcgcatctg 49260

cgcgcaggac cagcccccga aggactcgaa gcagacggtg agcatgcccg tgctcggaca 49320

gtgtggcgag cgccgacaca ccggaaagcc cacggccttg cggcagcgca ccatggtcga 49380

gagctctatc cagcagcctg cctcctcctc gcccatgccc atggctaccg gcgtgaaggc 49440

cgtgacgtcg tcgcagatgc gccgctccag aaaccccacg cccgaggagg ggtgcgcggc 49500

cggcggcgag gtgatgcgcg ccgggacgcg gctcggagcg ggctcgggag gcgagctgcg 49560

ctcgacccgg gcagtcgccg ccggccgcga tgccctgcgc gcgggcgcgc gctcgcgcaa 49620

cttgtttgac ttgctggcct cgtcgctagc gtcatcgaag cggtcgttcc tgtcgccgcg 49680

gacgtccgcc tcgtcgcccg tcggctgcgc ggcgggcgac gtgccgtccc gcgtacggcc 49740

cgcgttcggc gcgaatgtca cgcgccggtg cacgtacggc tccgtagagc ccgtgggggc 49800

gccgcgcccg cgcccgcggc ggaaggcctg ccgggacgcg ccgaagcggg cgaactcccc 49860

cttcgcccgg cccctttttt cttccatgat atttatcaca aaaaaaactt ctctaaatga 49920

ccaatctgct ttcgttggtc gacccggagg acctggcctt ctgcgccggg ttcccgtcct 49980

tcgacgagac catgctcgtg atcgcggggg cgcgagtgcg cttcccacgc tcgctgctct 50040

cgctcttcaa cgtggtgccg cgcaccatga cgcgctacga aaccgagctc gtgggcaccg 50100

agatggtggt gggcgccgtg ttcaccaccg cgtacaacgt ccgccgcaac ctaggcctcg 50160

gcgaggagcc cgtgaccatg cgcgacatcg agaagtactt cctggactcc gagaacgagg 50220

tgctcacgct catcgtgcac aacaccgact tttccgccat gagcggcgtg cgccggcgcg 50280

gcggccggcg catcgccaac cccgtcatct tccgcagcgg gtccacgccg ctgctcatcg 50340

tgatggagtc gcgcaagaag accaacatct accgcgagcg caccgcggag caggccaacg 50400

cctcctacag ggaggtcggc tcctcgctcg cgctggtcac tcggtacgcg ggtctgcagc 50460

tggttgacgt gcacacgccc agctccgtgc taacggtctc cgccgtctac ggcttcaccg 50520

aggacaaggg gctcaagaag ctgggctccg acaaggagct cgcggactac cagtccacgc 50580
cgctcaccga ccccatccgg ctcagcgact tctccaatat attcgacggc gtcaagaaga 50640
gcatccagct cacgaacgtg cccgtgccct ccaccggcgc cgaggccgcg ccgtaggctt 50700
tcatgcgcga taaatcggat ggcggcgccg acgacgcccg cggtgcacct cacgccggtg 50760
ttcgtggagc ctacgatcgc gcactcgctg ctgcgcgcag agtcctacct cgcgatcgcg 50820
gtccttgagc tcgtgctcgc gctcgcgctc gcgctcgtct tcttccgcga cgagctaggc 50880
tcgctattcc gctgcgcgcc gcgagcgcct tcgccgctgg acgcgtacct gcaggcgagc 50940
ctcgtctgcg acggcgacgc gctgctgatc gagctgcccg agggccgggt gccggcgctc 51000
gcgctggacg ggcggcccgt cgcgttcccg gggtgcgaga gccttttgta ccgcataaat 51060
ggaccacgaa aagtacgtct tgtcgatgtt cttggaggaa gataactcct tcttctcgtt 51120
cgtcgccgcg ctgtccgatg acgaggcgct cggcgccgtg cagtccgctg ccgccctcct 51180
ggacttcctg ctctccgtgg tggtccgcgg caaggagaag ctcgccgccg cggggcacca 51240
ctacgactcc atcgcggacg gacgcgcgcg cgccgcgttc gagttccgag acctgcgcga 51300
gctggcgcag ctcttcgacc ggcggccctg cggcgtccag gaccgcgtgc gtgtgcgcga 51360
cgggcccgcg cgcgccttcg tggacgcggc actggggctc atgcgcgagc gaggcttcga 51420
cggcacgcag gccgcggagc gcgcgcgcta catcgcgccg aacgatctgc ccgcgctggg 51480
ggcaatatcg gccacgctct cgccgggtct ataacgtaaa aaatattagt aaaattctga 51540
aggtccgtgt gtttcgcggg cggccaacaa accagtcgct taaatggagg gggtggaaat 51600
ggacaagccg ctcctctact tcgacgagat cgcgggcgcg cgcgactacg acgcggcctt 51660
cgcggagaag cacgagccgc ccaagatccc cggccgcgga cagatgaagc tgctggtctg 51720
cgagctcgtg tttctcaacc ggctgcacct gcacggcatg ctcgacggca gcgtcatcgt 51780
gtacgtgggc tccgcgcccg gacggcacat ctgctgcctg cactcgcact tccaggagct 51840
cggcgtctcg cttaagtggg tgctcattga cgggcgcaag cacgacccct gtctctcggg 51900
gctgcggaac gtgaccacgg tgacgcgatt cgcggacgag gcctacctcc gcgagctgcg 51960
cggcgagctg cggcgcgcca agatcgtgct catttcggac atccgctcca accgcgtgga 52020
cacagagccc accaccgcgg acctgctgcg cgactacgcg ctccagaaca ccatggtgag 52080
cgtgctcaag cccgtggcct ccagcctgaa gtggcgctgc cccttcccgg actcctggga 52140
gaaggacttc tacgtgccct gcggcaagga gatgctgcag ccgttcgcgc cgccgttctc 52200

cgcggagatg cggctgctca ccgtgtactc ggagacgcgc ccgaagctgc gtctgatcac 52260

gctcagcgac gcggtcaact atgaaaagag gatgttctac ctcaatagcg tggtccgcca 52320

gcgcgtaatt ctgaactttg actatcccaa ccaggagtac gacttctttc acatgttctg 52380

tctgctctcg tcggtggtgt gctcgtgcga atttaaatcg cccaaagaga aggtgctgag 52440

cctgcagaac cgcttcttcc gcttcctgcg catcccgccc tccatcacgc tcgggctgcg 52500

ccggcacgat gaaccgccac aacacgcggt acctggccaa gatcctctgc ctaaaggccg 52560

cggtaagaag cgaccccttc gcggtggtaa gtagggacac cgtgcgcatg tacgacatcg 52620

aggtcgagta cggcgacctc gtgacggtgg tcaccgtcac gcacaaactc gagaccagcc 52680

gcaccgtctt ccaggtcttc aacgagacct ctgtcgcgta ctcgccgctg ccggacgact 52740

acggcgagcc catcgtgctc accacgtaca tgcagcgcga gcacaccaag ttcccgctct 52800

ccatgctcta catcgacgtg gtcgcctcgg acatgttccc cacgtttaag cgccccaccg 52860

aggaggaggc cgcggtggtc gcggccatgc agcgcgtggg cgggcgccgc gagcccgtgc 52920

tcaagctccc gcgcatgctg gacaccgagc tcgtgtgcaa gatactgcac ctgcccgagc 52980

acccgctgcg cgtggtgcgc ttcctgcgcc gaaacatgtt cacgggcgtg gaggtcgccg 53040

accgctcggt gtccgtggtc ctcgactgac gaagggcagc acggtcagcg aggccgccgc 53100

caccaagcac agcggcagcc acgcgcgcgg gtccgccacg ggcacgaaga cgtgctggtt 53160

caggtacttc gcctggaagc gctccgcggt ggagtccacc ttggacccgc aggcgttggt 53220

gaggcgcacg accgcgtccg cgacgcgcac gtccccgagc gatatcacgc agtcagagac 53280

gttgcacccg gcgatgtttt tcttcagcgc gcgcggcagc agcgcgtccg cgcgcttgca 53340

gggcgcgtac cagcagtagt agggcaggcg cgtgtcgcgg ccggtgtcga ccacggcctg 53400

gctgggcttg aggcacgcgc agcgctcgtc gtccgggtgc gcgtcgcaga aggcgtaaat 53460

ctcctcgtcg ggcgcgtccg gcccgggcgc ggtcggcggc gccgcgcgac ggcggaagaa 53520

catctctgaa aaaatacttc gaccagaaaa cgaccaccga tcttatttca aagataaaaa 53580

tactattaat acgcactcgg agaatcatgt cggtggtggc gcgcgtgtcg tacagcctgt 53640

actcgcagag cgagataagc gccacggacg tggtcatcag ccagttgaag aacgacgagg 53700

acctgggcac ggtgaaggac ccgcgcctgg gcgcctcgga cgggtccata tgccgcacct 53760

gcgggctcac ggagatggag tgtttcgggc actggggcaa ggtgcgcatc tacgagtcct 53820

acatcgtgcg ccccgagtac atccccgagg tggtgcggct gctcaaccac ctctgcgtgc 53880

gctgcgggct gctgcgctcg cgcgacccgt acacgacgga cttggccgcg ctcagcgtgc 53940
acgagatgcg caagatgaag gaccggatga tgtccaagaa gaaggcctgc tggaacagca 54000
agtgtctgca gccgtaccag aagatcgtct tctccaagaa gaagatctgc ttcgtgaaca 54060
aggtggacga gatacccgtc cccaacgcgc tcatctacca gaagctgacc tccatccacc 54120
gcaagttctg gccgctgctg gaggtgttcc aggaccccgc gaacctgttc tacaaggagt 54180
acatgcccgt cccgccgctg ctcatccggc cggcgatcag cttctggata gacaacatcc 54240
ccaaggagac caacgagctc acctacctgc tgggcatgat cgtgaagtac tgctccatga 54300
acgccgagga gcaggtcatc cagcgcgccg tgatcgagta cgacaacatc aagatcatct 54360
cctcgaactc gagcagcatc aacctctcct acatcatcgc gggcaagagc aacatgctgc 54420
gcagcttcgt ggtcgcgcgg cgcaaggacc agaccgcgcg ctcggtcatc gggcccgact 54480
ccgcgctctc ggtgtgcgag gtcggcatcc ccgactacat ccggaacacg ctcacgcaga 54540
aggtgttcgt gaactacctc accagcaagc gcgtgcgcgc gctgttcgag gaccgcgcgg 54600
tcaagttcta cttcaacaag cggctgcgcc agctcacgcg catcaaggag ggcaagttca 54660
tcaaggacaa gatccacctg ctgcccggcg actgggtgga gatccccatg tccgagggca 54720
cgaacgtgat attcggccgc cagccctcgc tgcaccgaca caacgtcata tcctcgaccg 54780
cgcgcgcctc gcccggctac accatcaaga tcccgcccgg gatcgcgaac tcgcagaacg 54840
cggacttcga cggcgacgag gagtgggccg tgctcgagca gaaccccaag tccgtgatcg 54900
agcagagcgt gctcatgtac ccggtgacta tcttcaagca cgacgcgcac ggcgcgccgg 54960
tgtacgggtc catccaggac gagatcgtgg ccgcgttctc gctgttccgg caccagaacc 55020
tctcgctgga cgaggtgctg aacctgctcg ggcgctacgg gcgagacttc gcgccggagc 55080
ctggccagaa gaccttctcg ggcgccgacg tcttccgatt catgataggc gcggacataa 55140
acttcaaggg cgtgctcgag aacgggcgcg tggtggcgcc gaacgtcgac agcgacctcg 55200
tggtggccat gcgcgcaacc tcgctagcgg ggctgatcgc ggactacgcc acgaacgtgg 55260
agggcgtgcg cttcgtggac atggcctcct acgtgtacaa gcggtacctg gccatctacg 55320
gcttcggcgt gaccttccgc gacctgcgcc cggacccgag tttggttcgc cggctgcacg 55380
cgctgaacac cgagaagata gagcagatca aggacgcgta ctcgcggtac ctgcaggacg 55440
tcgcggacgg gaagctggtg ccgatggcgc ccgcggacga ggccgacgcg ctggactcgc 55500
tgctggccaa cctgaccaac ctcaacgtgc gcgagatcaa cgagtacatg cgcgagacgc 55560

tggagcgcaa ccccgataac agcctgctaa agatggcgcg cgccgggtac aaggtcaacc 55620
ccacagagct catgtacctg ctgggcacct acgggcagca gcgcgtgaac ggcgccgtcg 55680
ccgagaccaa gatatacggg cgcgtgctcc cgtacgcgtt ccccgactcc gcggacccgg 55740
aggcgcgcgg ctacatcatc aactcgctca tgaacggtct ctccggctcg cagttctact 55800
tcgcgatgct ggtggcgcgc tcgcagtcca cggacatcgt ctgcgagacc tcgcgcacgg 55860
gcacgctcgc gcgcaaggtc atcaagaaga tggaggacac ggtcgtggac gggtacggac 55920
agatcgtgag cggctcggta ctgctcaagt acgcggccaa ctacgcgaag atcccggggt 55980
ccaccaccaa gcccgtggag ctgctcttcc cgcacgagag catgacctgg ttcctggaga 56040
taagcgcgct ctggacgaag atccggcacg ggttcgtgcg catgcaccgg cagcgcctgg 56100
ccaccaagat cctggcgccg ttcaacttcc tggtcttcgt gaaaccggcg ccctcggagg 56160
cggaggcgct ctccgcgcgg gacctgtacc acatgatcca gcgcgtgatg aacgacgtgc 56220
gcgagaagta cttcttctcg ctggcgaacg tggacttcat ggagtacgtc ttcctcacgc 56280
acctgaaccc ctcgcgcgtg cgcatcacgc gcgcgaccgc cgagctcatc ttccgcaagc 56340
tgtaccagaa gctgaacgcg ctgctcggcg gcggcacgcc cgtgggcatc atgtccgcgc 56400
aggtgctctg cgagaagttc acgcagcagg cgctctcgag cttccacacc accgagaaga 56460
gcggcgccgc gaaggtgaag ctgggcttca acgagttcag caacctcatc agcatgagcc 56520
gcaaccacac cgagatagtg gcgctgaccg cgccgagcgc ggacaagctg atgccgctga 56580
aggtaaactt cgagttcgtg tgtctgggcg agctcgtgcc cgagatcgag acccggccct 56640
cgggacggcc ctccgtgcac cgcgtggaca tcacggtgca ccgcctgcgc atcaagcgcg 56700
cgcacctgac cgaggtcctg gtggacacca tcatcgagcg cttcgtgtcc ttcaacgtgc 56760
tcgtgaagga gtggggcagc gacatgaccg tggagggcga ccgcgtcacg tacacgctgc 56820
tgctgcgctt cgtggagccg gagcagctca acttccacaa gttcatgctg gtgctgcccg 56880
gcgccgcgaa caagggcaag gtgagcaggt tcaagatccc gatcaccgag accacggtct 56940
acgacgactt cgacgccgcg cgcaaggcct accgcatgaa catcgagctc atgagtctga 57000
aggagctggg gatattcgac ctcgaggacg tgaacgtggt ccccggcatg tggaacacct 57060
tcgacatatt cggcatcgag gccgcgcgcg ggcacctctg cgagagcatg ctggacacct 57120
acggcacggg cttcgactac ctgtttccct cctgcgacct gctcgcgagc ctgctctgct 57180
ccgggtacga gcccgagtcc gtaaacaagt tcaagttctg gaacgcgagc gcgctgaaga 57240

aggccacctt cggcgacggc cgcgcgctgc tgaacgcggc gctgcacaac cgcaccgacg 57300

cggtcgcgga caacagcagc tgccacttct tcagcaagac gccctgcgtg ggcacgggct 57360

actacaagta cttcgtgaac gtggagatgt tcatgcgcat ggagcgcgag atccaggcgc 57420

gcgtggcggc gcgcaagatg gaggagatcg aggaggccgc cgaggaggag ttctaggcgc 57480

gacggcgcct tactttgcga ccgtgtcacg acgacacgac acggttagga cggcgagtcg 57540

cagacgaaca tttttatgag ctggtagcgg aagttggcgt tttccaggaa ggcgccgcgg 57600

aggtcccgga tctcgtagta ggttttgagg aagtacacga agcgcgcggg ctgcgtcata 57660

gtcgggttct ccgcaagccg cttgtgcatc acgtacccca tggcggcggc gccgctgcgg 57720

ttgacgccgg ccacgcagtg cacgagcgtg ggcttctgct cggcctcgag gcgcgccagc 57780

agcttcacga gcgcgggcat gatggaagcg atgttcgtcg tgtcgtcgtc tctcagcgga 57840

atgtggtacg ccgttatccc cgcgggcgtc gagtacttgg acatggtcat gttaaccaga 57900

cacttgaagt cgacgccgga gtccccccgc agcacggcgc gcgcgtcctc ggcgctgccc 57960

aagtacacgt ggtccgtgag ccgcgtcatg cccgagggca gggccagcgg cggccccgcg 58020

cgcgtgcacc gcagcaggag cctggcgtac cactcgctct tatcgcccat atttatttat 58080

atgatacaaa tggcagacgt cacaacactg acggccaacg gtctgaccct ggagttcgcg 58140

cgcgagcgcg ctctgcgcag tctgcgcgcc gcgcgcacct ccacgctggt gttcttcacg 58200

ctcacgctcg cggcctcgct gttcgtgctc tggctgcagc taaccgagtt tcccgtcttc 58260

gaggagctcg gcaagtacgc gcgcatcaag agcgcggtgc ggtcctggcg cccgctggtg 58320

gaggctaaga cagagatcga gtccgacctc ggccggcaga agaccgccga ccggcccgag 58380

ctcttcgagt tcaggtgcgt ggacttcggc aagttctacc tgccggtgag gtacagcccc 58440

acgaccttcc tgccgcaagc cgtgcgccgc ggcgcgggcg atggctggat ggtgcacaag 58500

gcggcggccg tggacctcgc cgcgcagcag ttctgcgagt ccgtgctgcg gcaccgcgcc 58560

aacaacgtca tcacatgcgg gtcagagatg atgcggctgg tgggctacag cggctacttc 58620

gaggacgacc actggtgcgc cgcgacgtcc ggcgtgctga cgtgaacgat cacacgatgg 58680

ccgtgaccag cagcccggcg atgaaccaca gcagccgcga gttcggcagc agcagcacga 58740

gcaccagcag gtacgccagg atgaagatgt cgaccacgtc cacgtcgaag agccccatga 58800

aggagaagag cggcgtggtg aggaagtaga tggcgccggg ccagaagcgc gccagccacg 58860

tggcgagcag cgaccacagg gagggcgcgc cgctgagccg cgtcttcacc tgtatgtagt 58920

actcggggta gaccacctgc tcggcgccgg agagcaccac gcgcgccaga gagagccgct 58980

tctccagcgt gaacacctcg gtgagcaggc cgctgcgcag ccctccctcc ttgatgatcg 59040

cgtcgtagag cttcttcatg ccgccgacgc tgatgatgta ggcgtctagc gagacgtcgt 59100

acccgccggg gtagaccatg agctcggggt cgccggtgcc ggggacgttg gtggccagcg 59160

cgccggtcat gtaggtctcc ttgagctgcg tcatgtacca gccgttcgcc ttcatcgcct 59220

cgatgagcgg cttcaccatc tcgggcttgc ggaaggtcat gtcgttgtcg accaccagga 59280

tgaagtcatc gtcggagtac ttggtgggga cagtgccggc cgatatgctc tcccagaggt 59340

tgaggtggtg cgctgcgcgg cgctgcatct ccttcggaca cgtggacttg cacatgtccg 59400

tgaagaagtg cgggtagtct ttggagtcca cgtctttcca ttccaccgcc ttgagcacgt 59460

ggtcgccctt ggggtgcggc gcgggaggag atggcttggg tgccggcgcg ggggccggtg 59520

cgggggctgg ggcaggagag ggagcaggcg cgggttgagg cttgggcggg tcgtcggcga 59580

ggcccaccag gtacggcagc gtggggaaca cctccttggt cccgcggcct tcggcaaccc 59640

cgattatgta ggccgtgatt tcgggtggat ccatttagtt attaaaatta atcatataca 59700

actcttttat ggcggctatg gattcggcta tccagtcctt gaccgagccc acgatgcccg 59760

ccaggaacag gaagaaggcg aactccaggt ccacgcggtt cagagagtcg ctgaagtaca 59820

cgaagacgtc gctgtccggg aagaagctgc gccggaacat gttgtacccg ttgaccttgt 59880

gcgcgacgtg ctccgcgctc agcagcgtct cgtcgaaggg gtacgggtcg ctgaagcgga 59940

acacgtacat ggccgggttt gcgtagtagt acttcatggt gtttgtgacg aagaggctcg 60000

ccagcgagat gatgattttt ttcttctcga tctcgatctt gatgtggtcc tcgaagcgct 60060

tcatgttgta ggcgttggtg tcgtgcacgc ggatgagcac gcgcgagtcc gacatgatgt 60120

cctggaactc cgcgcgcgcg tcggggctct cggcgggcgt ctccgcgggc cgcgccacct 60180

ccgcgcacac cgtcggccta gcgcgcggcg gcgtgcgcat gggccgcgcc cccacgcgct 60240

gcgaagcgaa aaactccacg gcgcgagcct cgcccgcgtc cgcgtacgac tccaccaggt 60300

agttgcggct gcgcgtggtg cggccgatgg tgttcagccg gtgcagctcc gcgaccagcc 60360

ggcggtagtg cgcctccagc tcctcgggca tgatggaggt gtacacctcg gtgagcagca 60420

tcacggtgtc gaagtcctcc ttgccgcaga cgcgcgtctt cacgaggaag tggtgcacag 60480

ccgtcgcgat agagagccgc agcgtggact cggtgacctc gacgctggcg tccttggtct 60540

tcttcgcgct ccgcgaggcc atgaacgaga cgaggaagtc cgcgctgctg ttgagcacga 60600

tgaccagcgc gacgatgaag ttgaggttca gcgtcttcgc ggactggaac agctcggtgg 60660

ccgacgcgtg cacgtcgagc aggttcgcgg agagccgcag gaagaacacg ccgcgcttga 60720

tctcggccgc gaagcgacgt tcgtactcct gccggcgcgc gttgatcgcg atgaggaagt 60780

tcaggatgag ccggttgatg ttgtacttca cggcccaggt ctgcgtcttc atgatggtgt 60840

cgaaggacat cacgatgttg aagatgaagc gctggctgtg cgagaagtag ctgtagggct 60900

cgctgaggaa gatggacttg ttggtcgcgg gcaccaccac gcccgcgcgc gcgccggacg 60960

cgtcggtgtt caggtccggg atgttcatgc cgcagatgcg gcagtaggcc atgccgtcct 61020

caaagtacac gaactcctcc acgaactcgt tgatcttggc gaagtagtcc acgtccacgc 61080

gcatcgcgac cgcgagccgg atctggtgct cgcagggcgg cgactcgaag cgcacccccct 61140

cgccccagcc cggcggctcg cgcacgacca gcgcggtgcg cgaggccggg cggaacttgg 61200

cgtcgcgcgc gttgagcagc gccgggaaga ggtcgcagag gtgccggctc gagaggaaca 61260

cgtacttgta cagcagccgg cgcgcgtccg cggccatggc gtccacgaag gcgcggcccc 61320

actccgcgac cgcgggctgc tcctccgcaa agttgttcgg gtagaccttg tccgtggccg 61380

cgaggaacac cttcttcacg tcgaggaagt cgcggatcac gatggggacg cgcgcgccgt 61440

cgagctcgta catgaacacg tagcgcaggt tgagcttgcg ccgcgagacc gggatgccga 61500

tgtgccgaca caggtacgcg aactcgaggt acttcttcga gaagcggatg cggtccaggt 61560

tcttggagac gtactgcagc atgttgcgca tgttgaaggg gatctcgcgc acggcgggct 61620

ccgcggcgtc gtcgaaggcg gtgcgcagat cgctggtgcg ctgtacgacc acggcttcgc 61680

cggtggcgtc gtcgtgcacc agcacgttaa cgcgccgctg ccggatgacc atgtcgaagg 61740

tgttgaagaa catctcgtac atgctgtgcc gagtgtcgtc cgcgatgcgc tcgcccaccg 61800

agaggctcgc ggtggcgtcg tcacgcacct gcttctcgaa cttgtacccg atgtaggaga 61860

atatcgagat cagcgtggcg tcgtcggcgt cggggttctg ctccatggtc gcgaagagca 61920

ggcggatgtc gtcctccgtg atcgcgtcca cgttgtacag gttgaccacg aagatggact 61980

tgttctcggc gatgaagtcc gtgtaggact tggtggccgt gttcgggtcg cgcatgtacg 62040

cgcggatctt cggcacgatg ctcgcgagga tggactccct ggaatccatt taaggacggc 62100

aagggcgcgc gagaccgtct caaaactgaa atcgtataaa ctcttaaaaa atcggtattg 62160

aaagtacgca ccaccaaata aagcgtcgag gtcgggcatg tcttcgtggc gactcaaaat 62220

gagcaagtgt tcaggttcca gcagcgtcca gactctcgag gatctgcgta atcgtcttcg 62280

ctccgaggcc ttgggcaacg attgccaaga gccccgcgac gacctcttcc ccagcggcga 62340
ggagtgtctg gacatcgacg ggccctgccc ttgcgatgag gcggagcagg agatcgacca 62400
ggagcagttg cccgtgcccg aaaccgtgcc cgaaccgccg gccaagactc ctaagcgccg 62460
accagtgaag aaggataagg cagataaggc agataaggac aagtcgacca gaggcgcaaa 62520
gaaaccgtgc ccttcggacg acaaggatga cgagctcaag agcaacgacg tcgacaacaa 62580
cgaagagtcc ggcgacacag acggcggcgc gagcgcccga agccccagcg acatcgacaa 62640
cgtggacgaa atggacgact ccgacctcat ggtggcgttc tccaccatcc tcgcagactt 62700
caaggacctt acccaacgag tgaaagctct ttcgtccgtg ctcacggacg tgcaggcggc 62760
cggcatacgc aggagcttct cgacgctcgg caaggctctg acggaggcgg cccacatcgc 62820
caacaccgga tctaagccag tcactgcgcc tcgcaagaag aaggccgccg cctgcaagaa 62880
gtaggcgcac taaatagcga ggctcggtat gcgggcgctg cacctgtcag acggcaaact 62940
ttttttgac aaggagctga cgcagccggt ccccgacgac aaccccgcgt acgctgtcct 63000
tgcgaagatc cggatcccac cgcacctctc ggatgtggtc gtgtacgagc aggacctcga 63060
gtctgcgcag cagggcctca tcttcgtcgg gcgcgacgcc aagggccgaa agcagtactt 63120
ctacgggcgc ggacacgtgg agcggcgcac ggccgtccgc aacgccgtgt tcgtgcgcgt 63180
gcaccgcgtc atgaacaaga taaacgcctt catcgacgac cacctcgcct ccggcagcga 63240
ggccgaggcg cagatggccg ccttcctgct catggagacg agcttcttca tccgcgtcgg 63300
caagacgcgc tacgagcgcg agagcggcac cgtgggcatg ctcacgctgc gcaacaagca 63360
cctcgccgag gccgagggcg gtgaggagat ccgcgtcgcc ttcgtgggca aggaccgagt 63420
cgcgcacgag tttgccgtgc gcgaggggca gcggctcttc gcggcgctgc gtcggctctg 63480
ggacccgggc gcgcccgaca ggctgctgtt cgaccggctg agcgagcgcc gcgtgtacac 63540
cttcatgcga cgcttcggca tccgcgtcaa ggacctgcgc acctacggcg tgaactacac 63600
cttcctgtac aacttctggt ccaacgtgcg ctcgctggag ccgcgtccct ccgtgaagtc 63660
gctcatctgc acctccgtgc ggcagaccgc cgagacggtg gggcacacgc cctcgatctc 63720
gcgcagcgcc tacatggcca ccgcggtgct cgagctcgtc agggacggcg cgttcctgga 63780
cagagtcgcc gccaccgaca cgctcgacga cttcgtggac atcgtcgtgg actatgtaaa 63840
taactctgag caggtaaatg gatgaggcgc tgcgcgtggc ggcgcgcgtc gtggacgggc 63900
tccggccgct ggacgtggcc gtgtgtctcg cgcagctgcg cggagccgcg cccgagcgcc 63960

gcttcccggc gctcgacgag tgctccggcg aggccttcct ggactttgag ttcgccggcg 64020

gggacgtggc gtcgcggtac ctctccgcgc acacgcgcga gctccgtgcg gcggagcggc 64080

gcgagcacat ggccgcgatc gcgcgctgcg tcaccgaggc cgacctggcg ctcgcagacc 64140

gcccccgggg caaggcgcgc gcggcgctgc gcgtgtgccg caaccgcgag aaagtcgcgc 64200

gcttggcgag gctgctgcgc gacgccgaga gcagcggcgc ggacttcgcc ttcatacgcg 64260

cggccgtggc gtagcaaaac gtaaaaacaa cacattccct aaatcgccat ggacgcgcca 64320

agtctcgact gcatgctcgc cgcactcgcg gcgaaggcgg cctcggtgga ccgaggcgct 64380

cccgaggacg aggtgcacca cgaagtggag ctcgtgctcg tggacccgcc gctgtccacc 64440

ctggccgcca cgctgcgcct ggcctcggag acggagtcct tcatcctctt cacggtgacc 64500

gcgctcgcca aggaggaggg caagctgcgc gcgcgcgtgc ccatgtcgcg cgtcgtcggc 64560

ctggacgtga agaacgtgca gctggtcaac gccatcgaca gcatcgtctg ggagcgcaag 64620

gcgctcgtgg aggagaccgc gctgcaggaa ggctgtctgc tgcgccactc caccgagcgg 64680

cggcacctct tcgtggacta caagaagtac ctctcggcca tccgcgtgga gctggtaaac 64740

cgcgtgcgcg tgcgctccaa agaagtcgtc gcggacttca agttcaagta ctttctgggg 64800

tccggcgcgc aggccaagag ctcgctgctg cacgcactca accaccccaa ggtgcggccc 64860

tcgcccacgc tggagttcga ggtcgtcccc gcgggcgagg ccgtggacga ggccgccgtg 64920

ctcgcggagc tgcgcgccgt ggcgaaggcg ctcttcatgg cgcccaccga cgccgtcttc 64980

ctggcgccgc cggccgagat gccggtgcgc acgctcatgc tgcagaagca ggagatcccc 65040

gcgctagacc tcgacggcct tttcgcggtc tccaagacgg acggcgtctc cgcgagcgtg 65100

tgcgtggacg aggacggcgt cttctgcgcg ttctcgcacc tcgcgtacac catccggtac 65160

ccgctcgcgc gcgaagtgca gggccggtac cggctctggt gcgaggccgt gcggcccgtg 65220

ggcgagcgcg tgtggtccat gttcgtgctg gtcgtggagg agcctgcggg cgatgaccgc 65280

gtcgcggccg tggccggcgc cgtggaggcg ctgcgcggcg tgtgtgcacg cgtcgagttc 65340

aaacctaagc gcgtggacgg gcccttctcg gcgacctccg agctggtgga gcacatcaag 65400

agcgcgctgc agacggagcc agagggcgtg gtgctcttct acgcgcgcgg agagaagtcc 65460

aagcgcgacc tcaaggtcaa gcgcgacaac acggtggacc agaccacgaa cgtgatgttc 65520

cggtacatgt ccagcgagcc catcgtcttc ggcgagggct ccaccttcct ggagttcaag 65580

cggtacagca acgaccgcgg gttccccaag gagtacggcg cggggcgcat cttcctgcgc 65640

gaggacgtgg tctaccacaa caacatctac tgcatcgagt tcacgaagac gcacctggag 65700

gtgggcctcc gcagcgtggt cgtgcccgtg aagttcatcg gcgagttctc gcaggagggg 65760

tacctgctgc ggccgcgcct ggccaaaacg gagtgctact tccgcaaccc ctcattctac 65820

gggaaccagc actcggtggt gctcgagcac actcgcgacc agctgctctc ggtgggggac 65880

gtgttcgacg agagccgcat ggccgccgtc gggcagacgc tggccaacga cgccttccgc 65940

ctgaacccgg acacgcccta cttcaccaac cgacgcacgc gcgggccgct gggcgtgctc 66000

tccaactacg tgaagacgct catgatatcg ctgtactgct cgaagacctt cctgaacaac 66060

gccgagcgac gcaaggtgct ggccgtggac ttcggcaacg gcgcggacct ggagaagtac 66120

ttcttcggcg agatcgcgtc catggtggcc acggacccgg acgcgcgcgc gatcgagcgc 66180

gccatggagc gctacaaccg cctcaacgcg gggctgaagt cgcgctacta caagtttaac 66240

tacatccagg agaccatccg atccgagacc tacgtggaga gcatccgcca ggtcatgtac 66300

ttcgggcgct tcaacatcgt ggactggcag atggccatcc actactcctt ccacccgcgg 66360

cacttcgcca cggtgatgcg caacctgcgc gagctcaccg cgcccggctg caaggtgctc 66420

atcaccacca tggacgggga cttcctgtcg acgctctccg agaagaccag cttcgtgatc 66480

aaccgcaacc tgcaggagag cgaaaacttc atgtcgatcg agcgcgtggc cgatgaccag 66540

gtcatggtct acgcgccctc gaccatggcg cagcccatga cggagtacat cgtgcgccgc 66600

gcggacatcg tcaagctctt cgcggacaac ggcttcgacc tcgtggacca cgcgaacttc 66660

gagaccgtga tccggcgcag ccgccgcttc gtcgagggcg tctcgcggct ggagacgcgg 66720

ccctccacca agaacttctt cgagctcaac cgcaacgcgc tcacggagat ggacagcacc 66780

gacgtggccg cgctgctaaa gatctacgtg ctgtacgtct tcagcaagcg gtaggcagaa 66840

ccagggcgtc gattccgcgc ccgcgccggc gcggaaggcg ttgaacagct ccgccagcca 66900

ggctgcggtc tcgcgcgcgt cgatcgggcc gccgtcgtcc ggcggcggct cgcgcgccgc 66960

gcgcaacacc agcgtctccg cgggcggcag aggctccaga gcctcgaaga ccgcgcggct 67020

cgggaacagc gcgcgcatca tgcgcgcgcg gtggccgaac accgccttga ccgcgcgcag 67080

tgccgagcgg ttgtccagcc gcagcgctcg gtcaaaacga tgcacgcgcg cgggcgcgcc 67140

gcggtggtcg cgctccacga gcacgtgccg ccacgccagc gccgcgccga cgcggtccag 67200

gctgggcgcg agcgccacca ggcttttcag cgcatgtaaa tctccgcgca tggccgacgg 67260

ctccatttac tactgcggag gaacgcacgt ggtcgcggcc gcgccgggcg ccgcgcttgt 67320

ggtgctggac gcgcccggtg cggcggcggc ggccgcgccc gcggggcagc gcgtcttctt 67380

cgccgagtac ggcctcgaga agcgggccgg cggcccgatc acggcgcggc tgcgccgctc 67440

cgggttccgc ggcgccgcga acgcctgggc ctccgtggcg gacttcgagg ccggcggccg 67500

tccctccgcg tggacgctgc gcgcggagga ggcttcgcgc gtgccgctgc cgacggacgc 67560

ggcgctggtc ctggcctggg gcgcgcgcga ggagccgctg cgggcgtgcg tgctggcgcg 67620

cgcggcagac gcggaggcgc cggtgggcgc cgcgctcaaa gaagccgcct tcgacgcgcg 67680

ggcgccggcg gccgcgctgt tcgcggcgct gggcgcgccc gcgctcgcgc ccccgctgcg 67740

ggcgcggcta gtggcgccgc cgggcgcgcc gccgcggacg cggctctgcg agaacccggc 67800

catgctgcgc gcgttcgcgg tgggctggtt cggcgcgcag ctgggcgagg cctccgaaaa 67860

tgaaaaggta tttgccgcct ttgataaggc gaggtcgtgt ttggacgacc gctgatggcg 67920

acgcccgcga acgcgcccgc gctgctcgtc gcggcgctgc gacaccgccc gtaccgcgtg 67980

gagtaccacc cggactggga gccggtcatc gagacgctgg tggacgagta cgacgcggtc 68040

gcgccctggc tgctgcgcga cgcgacgagc cccgagcccg agcgcttctt cgcgcagctg 68100

gcgaagccgc tggcggacaa gcgagtgtgc gtgtgcggca tcgacccgta cccgcgcggc 68160

ggcaccggcg tgcccttcca gtccccggac ttcagcaaga agaccatccg cgcgatcgcg 68220

agctcggtcg cgcgcacgac cggcacgcag ggctacgcga actacgacct ggacgcggtt 68280

ccgggcgtgc tgccctggaa ctactacctc tcctgccgcg agggcgagac caagagccac 68340

gcgatgtact gggagcgcat ctcgcggctg ctgctgcagc acgtggccaa gcacgtgagc 68400

gtgctctact gcatggggcg cacggacttc cagaacgtgc gcgcgcgcct ggacgtgccg 68460

gtgacgctgg tggtgggctt ccaccccgcg gcgcgcgacg ggcagttcgc gcgcgagcgg 68520

gccttcgagg tcatcaacgc cttattggag ctcaacggga agtctcaagt ggactgggcg 68580

cgaggatttt ctttttatag tgaaaattaa tccgtggtcc taaatggcgg cgcccatatg 68640

cgataactct cacgtgttcc tcctcaagcg cctgggcgtg ccgtcttcct gccggcgctc 68700

ggaggacccg cgcttcgtgg agatcctgac tcccttcgag ctctcaaact acatcgagcg 68760

gcacccggga tgctgcctct tcgagacgct gcgcgacgag gaggactgct ccgtcgtgcg 68820

cgtcttcgcg gacgtggaca tggacagcgt gctcgaggag gaggacttcg tcgcggcgct 68880

ggaggacctc atcgtggagc tcgcggcctt cttcgaccgc ttcgcgagcg gctcctgcgg 68940

caccgtgccc ggcgaggtca agcgcgccat gctcgcgaac ttctcggtca cgcgatccac 69000

ggccgagcac aagaccagct tccacctgat cttcacggag acgtacacca cgctggacac 69060
gctggtggcg gcgaagcgcc cgctgctgga cctgtgccgg cgctcggaca acgtgctgct 69120
gcgcgcgctg gacacggccg tgtaccgccg cggcgcgacg ctgcgcgtgg tgggcacgcg 69180
caagacgccg gagtcgagcg cggtccaccg catgcagtcg cccgacgacg acatcaagga 69240
ctacctgttc acgttcgtgg agctctcgga cgcgagcgtg tacttcgagc tcgcggagcg 69300
cgagcagcac acgctgagca ccgtctgctg ggagacctcc tacatcccct tcggcgacgc 69360
gatgcggcgc gtgtgccagg cggtggtcaa cgacatcgtg aacctccgcg acatcaccga 69420
ggacaacttc ctcgacacgc cgctggtcat cgactacgcg acgcgctgcg cgctgtgcaa 69480
gaagcccaag cacaagcacg cgcaccacat caccatgggc aacggctgcc tgcgcctggt 69540
caagggcggg aacgcgcaca gctgcaaggt caagatcatc cagctcgagg gcaaccggct 69600
cttcacggcc gcgcagatca tcatcgcgtc cgaggtcgtg aagctcaccg agcgcaacga 69660
ctacatcgtg tggctgaaca actcctggcg cttcagcgcg gaggagtcgc tcatcaccaa 69720
gctcatcctg gacgtgcggc actcgctgcc cgcggactac gccaacgaca tgctgtgtcc 69780
gcgcaagcgc aaggtcgtgg agaccaacat ccgcgacatg ctcgtggaca tctccgagac 69840
ggacacgcag tacgacaagc tgcccttcac gaacggcgtg ctggacctgg ccacgggcga 69900
gttcctcacc ggcgaccgcg cgaaggcctg cgtgtgcacg gtctccaccg ggtacgcctt 69960
ctcgcgcgag gagttcgcgg ccgcggcgga ctcggaggcc atgcgccggc tggtcggcgt 70020
catcgacgac atccagccgg acacgcccga gaacgccgat aaccgcgcgc tgtacgagcg 70080
cgccatgtcc agcgcgctct gcggcgccac gaagacggtc atcgtcttct tctacggcga 70140
caccatgacc ggcaagtcca cgagcaagcg tctgctcatg tccgcgctcg gcggactctt 70200
catcgagacc gggcagaccg tgctcacgga cgtgctcgac aagggcccga accccttcgt 70260
ggccaacatg cacctgcggc gcgcggtctt ctgcagcgag ctcccggact tcgcctgcaa 70320
caacgcgcgc aagctgcgct ccgacaactt caagaagctg accgagccct gcatcgtggg 70380
ccggccctgc ttctccaaca agatccacaa ccgcaaccac gccaccttca tcatcgacac 70440
caactaccgc ccggtcttcg accgcgtgga caacgcgctc atgcgccgcg tggcgctggt 70500
gcgcttccgc acgcacttct cctcgtcggc cactcgcgcg gccgccgcgc acaacgtcga 70560
gtacagcgcg gtcaaggaga tggacgagag cctggacacc aagatccagc gcaactactt 70620
ccgctacgcc ttcctgcgcc tgctcgtgca gtggttcggc aagtaccacg tcccgcaggt 70680

ctcgctggcg cccacgcccg acgcggtccc cgacttcgcc ttccaccgcc gcgtggccga 70740

gctggtggtg gccagcaacg acgcgcaccg ccgcgcgatg gagtcgctgt ccaagctggg 70800

gtacgtgctc gtgggcggca acgtggccat gcccgcggac gccttccggc agcggctggc 70860

cgcgcacttc aacgcgcgcg tgcacggcgg cgacatagac gccttcatgt tcaagcacaa 70920

gaaggtcgtc aacgtaacgg aggagtacgt ggagtacgta ttcatcgaag atgtcgagaa 70980

taaatagacg ggtatgaact cggacgtgat aaagctgttc gtcgggcacg acgagtccgt 71040

gcccggcatc ctgccgcacc agctcgcgac cgtggacttc ctgatacgcc gcgttctaga 71100

cgacaacgtc agcgtgcttc tcttccacat catgggctct gggaagaccg tcatcgcgct 71160

gctgttcgcg atggtggcct cgcgcaccaa gaaggtgtac atcctggtgc ccaacgtgaa 71220

cgtcatgaac atattcaact acagcatggt catggtcgct aacctgttca acgcgccctt 71280

cgtggccgag aacatattcg tgtactcgac gactagtttt tattcgctaa actgcaacga 71340

cggcgtcata aactacaacg gcctcggcaa gtacgagaac tcggtcttcg tggtcgacga 71400

ggcgcacaac atcttcggga acaacaccgg cgagctcatg atggtgatca agaacaagac 71460

gcgcgtgccc ttcctgctgc tctcggcctc gccgatcacg aacacgccgc tcacgctcag 71520

cagcatcatc agcctcatgt ccgagaagga cgtggacgtc ggcgacatcg tggtgcaggg 71580

caagaaggtg ttccagatcc tgctgaacga gcacggcgtg cgcgtgatcc gcgaggtgct 71640

caaggggcgc atctcctact acgagatgcc ggacacggac atgcccgagg tgctctacca 71700

cgggcgccgc ttcctggaca cgcgcgtggt ctactgccgc atgtcgcgcc ggcaggagga 71760

cgactacctc accgtgcgcc ggctctgcaa caacgagatg ttcgagaaga acatgaacaa 71820

cgtgtccatg gcggtgctgg gcccgctgaa cctggtgaac aacctggacg tgctcttcca 71880

ggcgcaggac aaggacctgt acccgaacct gcgcatcagc aacggcgtgc tctacgggaa 71940

cgagctcacc aagctggaca tcagctgcaa gttcaagttc ttcatctcga aggtgggcgc 72000

catgcgcggg aagcacttca tctacttctc caactcgacc tacggcagcc tggtcatccg 72060

caacgtgatg ctcagcaacg ggtactcgga gttcggcggc tcgcagagca acaatccgca 72120

caccacgccc gacgggcgcg ccaagacctt cgcgatcgtg accagcaaga tgaaggcctc 72180

gctggaggag ctgctcgagg tgtacaactc cgcggagaac aacgacggtg gcaagctcat 72240

gttcctcttc tcctcgaaca tcatgtccga gtcctacacg ctcaaggagg tgcggcacat 72300

ctggttcatg accatccccg acaccttctc gcagttcaac cagatcctgg gccgcgccgt 72360

gcgcaagttc tcctacgcgg acgtggccgc gcccgtgaac gtgtacctca tggcggcggt 72420

gtactcggac ttcgacgagg acatcgtctc gctggaggac tacagcgtgg aggacatcaa 72480

cgcgctgccc ttcgacgtga agaagctctt ctacctcaag ttcaaggcca aggaaaccaa 72540

ccgcgtgtac gccatcctgc aggagctctc ggacgcgtac tccgcgcgcc cgcacccgca 72600

gctcgtggac gtggtgctgg gggagatcgt gcgccagttc ttcgcacggc actgccgcgt 72660

gcccgccgag gacgccgcgc tcgtggccgc cgtcgaggcc gttctcggca cgcgcgaggc 72720

agcggccgag tacatccgcg cgatagtgga cggacacttc ttcgtgacca acaagacctt 72780

cgggaagtgc ctgctcttcc ggcacgagcg cgacatcgtg accgtgccct tcgagctcga 72840

gcacgacccc ttcgcgtggg cgatcaactt ccgcaaggag gtcagtgtgg tgaatatata 72900

acggcaaaca taaatagaaa gactgtcctt ttgcgcgatg tcgaccttcc ggcagacggt 72960

gtacctggcg gtgacgctgc agccgcacga gctcacgctc gacttccgcg gcaacgtcgc 73020

ggaggcggtc atgcgcgagt acctctacaa ggagaagggc gggctcatgg ccaccgacat 73080

cgaggtctgc ctcggaaacg agatgccgct ggggcgcatc gtgaacaacg cggttgtggt 73140

ctcggtgccc tgcaacgtga ccttcaagta ctaccgcgtc ggcgacaccg tgagcggcac 73200

gctcaacgtc gaggacgaga ccaacgtctt cgtggactgc ggagacctca tctgccagct 73260

cggcaagagc tcgggcggcg tgaccttcaa cgagtccaag tactgcctcg tgcgcaacgg 73320

agtcgtctac gagcacggca gccgggtctc ggctgtgctg cgcgaggcgc gctccggacg 73380

cgagtccgcg ttcgtgttct ccgcagtgct gctggacggc gtccccgccg aggagaagga 73440

cgagaagaag gacgagggcg agaaggccgc ggaggaggag acgcccgcga gccccgccgc 73500

caaaaactag cattattggg ccgcgcgaac cttcgataaa tgcgcacgta cacgtcgctg 73560

ctctcgaagc tgctcaagag caaccggcgg ctcgggagca cgcgcgtctt ccgcgacccg 73620

ctgcagcaca tcagcgcgac cgcctttgtg caccggcgca tcgaccggca ccggcgcgtc 73680

tccatctgcg ccgtgctcac caccaccgac gggctcgtgg tcgcgtgccg gcgccggtac 73740

tcctttttgt cctccgagct cgcggagacg cgctcgcccg cgcggcgcgt gctgctcgca 73800

accaagcacg cggacgctct cgcgcgcctc ggcgccgcgc gcccgcgcga cgacgtcatg 73860

tttccgggcg gcgccccgct gtccggggag tcgccgctgg cgtgcgtgct gcgcgaggtc 73920

gaggaggaga ccgggctgcg cggcgaccag gtcagcgtgg acgagcggct gttcgtgcac 73980

gccttcatcg acgacctggt ctcgggccgc gacttcgacg cgatcatctt cacgggcgca 74040

gtcgcgcttt cgagcgcgga ggtggcgaag cagttccggc ccaacgacga ggtcaagggg 74100

ctggtcttcc tgcaccccga ggacgcggag ggcgtgggcg tgatggcgcg gctggcggcg 74160

ttcgcgcgct gcgcggcgcg cctgcgctgc tggggcgcgg ccgtcacgcg atagaggcgg 74220

ggtccaccac gtacacgagg cgcccgccgc tcacgcgcac ggtgggcggg tcgcccagcg 74280

cggtcaggaa gttcccgtcg tcgtcgaaga ggcgcccgcc gcgctcgagg aagcccttgc 74340

gcaccgtgac cagcgccgtg gaggtggagt accacacgct ctgcccgtcc gcgagccgcg 74400

ccgcgcgcgc gggcccgcgc gcgtccgccg ggcgcgccac cagcgcggac cagccggagt 74460

cgtcctccag cggcgcgaag tccgtgaagg cctcgcgcac ccactccagc gagcagcgct 74520

tgagcacgcg gaagagctgc gtgaactgcc gggacttgtc gcggatgagg gccagcaggt 74580

cctcgtccac ggtggcggcg ccggagtcct ggcgcgcgac cacgaagtgc acgttcacgt 74640

agcggcggtc gggcggcgtc atctcgtggc tgttcaggcg caccgcgcgg cccacgatct 74700

ggcgcagcga ggcctcgttc caggtcatgt ccaggatgaa gatgtcgttg atggagagga 74760

agctgaggcc ctcggagccg ctcagcgaga acacacagac cttgatcttc tcgccgtcgg 74820

tgttgtcgca ggcgttgaag gcgtccacga gcttggcgcg cgtgtcgcgc gtgcgcgagg 74880

agaactccac gctggagacg ccgaaggcgc ggaagtagag cagcagcatc tcgatgccgg 74940

tcacgttgac gaagggctcg aagaccagac acttgcccgg cgaggccagg atgcgcaggc 75000

agacctcggt gtacttgcag ctgcgctcgc gcagctccgc gagcagcgag acgtccgcgg 75060

aggtcatgcg gtcgccgctg acgggcgcgc cgctgcggaa gagccgcatg gccgcctccg 75120

agaagacgcg gtccttgacg gcgcgcgcga agtccaggaa gagcgcggcc acggcctcgt 75180

cgtactcctg cttggagagc acggacttgt cgggcgcgtc ctcgaaggcg aaggtggccg 75240

cgatgcgccg gtacacgcgg aagaccgcgg cgccggactt gcgctccatg gcggccgcgc 75300

ggcggtaggc ctcggtctgc ttcgcggtca tgtccacgta catcatgcgc acgcgcttgc 75360

gcgcgaaggc ggcggagccg tcgacgtcgt cgaagatgga ggcctcgttg gtgactaagt 75420

acgagcacag gccgccgagc ttgtccacga ggtcctcggg gttcgcgagc gcgccgccgt 75480

tgaagagcgg cgtctgcccg accacgccgg ggcgcagcag gttcacggcc atggagaact 75540

ccttgacgct gttcaccacc ggcgtggccg tgaggcagag cagcttcccg cggcccatgg 75600

ggatgttctt cgcgaggtag ttgtacaccg tgcgcgcggg ccgctggcgc ccgtcctcct 75660

tggtcagcga catcgagatg aagttgtgga actcgtcgat gaccacgcag acgcggctgc 75720

tcgacgaggc ggtcttcatc agcgtgaaga agcggtggtg gaagcgcggg tcgtcgtagt 75780

tgatgaaggt gcacccgggc acggcctcgg gcgcgaagcg catcatcgtc gaggtccagg 75840

gctgctccac gagcgccttc ttcacgagca cgaccaccgt ccagtccgtg aagacgtcgc 75900

gcaggtgctt gagcacgtac accgcggtca cggtcttgcc cacgcccgtc tcgtggaaga 75960

gcagcagcga gtgcatgctg tccaggccca ggaacacgcg cgccacgaag agctggtagt 76020

ccttgaggcg cacggactcc tccacgccct gcatctcgga gggcatgtgc gcggtgcgcc 76080

gcagcgcgta gtcgatgtag gccgcgtgcg cgctggtcat ggcgacggtc ggcgctcctt 76140

ttacggggtc tgtcgtctat ctattgtcgg cgcgggtctg atttaggggc agtagttaca 76200

aaaacgtttc cgctgctcgg cgcggcgttt ggaggagcgg ttgcggccgc ggcggcgcag 76260

gcgcgcgcgg cgcgtcttcg tggtgcggtg gccgaaccag cgccggtgca tgaccgggtg 76320

cgagaccgcg gccgcgcgat ccgcgctcat gcaggttgcg taggtgcggc acatgctgcg 76380

cagcacgcgc cgcgtgcgcc gctccacggc gtcgagccgc ctcgcgacga tgggaaagag 76440

ccggcgccag ccgcgcacgg cgaagagcgg gcgctcgcag accgggcgcg cgagcgcgtg 76500

gtaggcgccc agcagccgcg ggtccagcga gcgcacgtag gtctccacga agccgttgcc 76560

gaagacgatg gcctgtgcgc atagcgggtt cgtcatctcc ctcttggagg cgatggcgtc 76620

gcccacgaag gcgcgcacgc cgcagtgccg cagcaccagg cgccgccgcg ggaagtgcag 76680

gtgcgggccg agcgccgcgc gggcggcggg gatgtgcagc cgcggagaaa aacgcgcgcg 76740

tccctccatg gcatctaagc gctccgtctg ttttcagtta tatcgccgcg ggcggctact 76800

gcagcagcag cttgagcttg cgctggctct cgttctcgat gctcttggac tcggaggtca 76860

tgctctcgta gagcagcgag tgcgtgacgt agagcgcctc gtacacgcgg ctggcgaagg 76920

ccacgaagcg gtccacgaac tcgctctcta cggggtcctt gagcacgcgg aagggcacgg 76980

ccagcgcgtc gcgccaggcg gcggccttgg tgcgctcgcg cacgtgcgtc acgaaggcgg 77040

cgatggccgc gcgccgcggc tcgctggcga ccatgacggc gctgtccttg agccagctgt 77100

cgctcacgca cttgaagagg cgcacggtgc cgaagaggct gcagtacacg cgcagcgcgt 77160

gcaccacgtc ggtgccgaag agcgtgggca gcttgagcac cacgaagcgc tcctgcgtga 77220

tctcgagcag cgggcgcatc accgcgaagg tgatcgcgtg gtagtcagcc acgtagaggt 77280

tgttctcggt gaggtggttg ttggagcgga tggcgccgcg ctccttgcgg tagagccggt 77340

tgcgcgcgct gaggtcgagc acgaccgcgt cggccctgcc gcgcgctctg gagcgcacgc 77400

tggtgatgcc gtgcgcctcg agcaccttct cgacgtcgcg ctcgtcgatc atgaggtcgt 77460
gcgtgtacag actcagcatc tccgtgggca tgcggttgat gtcgttcacg cgcgagcact 77520
gcaggaagta gttggtcccg taggccaggc tgggcaggtg cccgacgccg agctgcaggt 77580
ccagcgcggg cgtcgagtcg aaggtgggca gcgtcacgct gagcccctcg cggatgctgc 77640
ggcgcacggc ctccaccgcg tccatggccg atttattgga cgcacagtct gttttcattt 77700
cgcggctact gcgcagtcac cttctcggcc acgatccccg cgtcgtagct gagccggtac 77760
acctcgttgc acaccacgac catctgccgc ggcacgtaca tgagcgggtt gtgcgcctcc 77820
atgtgcgcgg tggtcacgcg caccgccagc ttgtccttgc ccctggagac gttggagttc 77880
agcgcggtgg gcgagaagaa ggtgctgggc gtaaagttga actgcagcgt gcgcacgccg 77940
ggcgtcttgc cgaggatctc gccgaagacg cgcgagactg cgctgttctc cgagtacagc 78000
acctcgttgc cgaagcgcac gtccatgcgc gcgatgacgt cgatcttgtt cttgaagtcc 78060
acgcccttga ggaaggggtc ggccacgaag aggtccttgg cgcgcgcctc cggcgagcgg 78120
ttgtcgccgt tgtacacgtt gcgctggcag gtccacacgc ccacgggcac ggaggcgtcg 78180
ccgatgttca cggagtggat ggcggtcgtg aagcggatgc gggaggtcgc gcggctgtag 78240
gcccccgtga tggcggagaa cttcttggac atgttgtaca cgacggagtt cttcctggtg 78300
gcgaacacta ggatgttcgt gtgcaggaac acgcgcatgc ccacgggcac gtcgtcgatg 78360
cgcacgaaga cgtcggtgtc ctggatggac acgacgcccg acgggggcac ctcgactatc 78420
tccgcggtct cggggaagcc ctcggggtag cagttcgaga cgatcaccat gtcctccagc 78480
aggcgctcca cgaaggccat cacgaagtcg ccctcggact gctggaagcc ggggtacgat 78540
atgaagcggt tgttggcgtc gctgagcacg ggcttcatgt acacggacag ggaggtgcac 78600
gcgtgcacgt ccgtgatcac cgcggtggtg tggttgatct gctccacgcg ccgccgcggc 78660
atctcgatga aggccgggcg cgggcacagg ttcttgacca tgtagccgat gaagctcagc 78720
tccatggagt aggggaactc cttggcgagc ttggcggcgt cgaaggtctc gtcgtagacc 78780
atgacgcagg cgacggggtt cagcgtgacc gtgaccgtga ccttgctgtc gctgagcttg 78840
agcgtgctga aggtcttgtc cgcgtcaaag ggcgtcttga tgtaggcgtg cacgcaggcg 78900
gcctccttga tgacgtcgtt gggcgagctc ccggtggaga ggtcgttgag ctcgcgcgag 78960
aagcccgaga gctccatcac gcgctcgttg tccaggcagg agtcgaacag ctcctcgccg 79020
gaggtctccc agatggtgtc cgcggcggag ttcacggcca cgtggcggat gagcttgtac 79080

gcgatgtagg gcacgtagca catcttgccc acgcccttta tctcgggcag gtccacgctc 79140

agcacgaagt tgttcatggc cgagatgtac ttgtcgcgga tctcgaaggt cacggtgacc 79200

gcgtcgctgg tggtgtccac cacgccctgc gtggtgatgt actgcggcat gtacaccgtg 79260

ggcgcgcggt ggtccgtggc gaacacgctg gcgcgccgca cggcgtcgtc gccgcccacc 79320

aggctcacca cggagttatt catttattcc ctgggaaaac cagttaaata aggctcttca 79380

gagccatgcg caccgtccgg ccgtcgggct ccaggtagca gcgcccgtag acgccctccg 79440

tggcgcgcgt ctcgttgatg agcgcgcgca cgcggtcggg gtccgcgtac atctccagcg 79500

gcagcagctc gatcttgggc tcctcgcgca gcgcgacgag gtgccggatg gagcccgcga 79560

aggagtcgcg gcacaaccgc gagcagaact cgcccacggc gccgccgtcg agcgtctcca 79620

cggccagcgc ggccgtgccc acgcgctgac ggcagaacca gcacgtgccg tccgcggcgc 79680

gcagcgccag ccgctccgcg gacaccgtgt tgaagtactt cggcagcacg tactcgatgc 79740

ggcacgccgc cggcggcgcg caggccgacg cccgcggcgc ggatatgtcc acccgcgaga 79800

gcgcgatgcg cttcatgggc ggcggtggct gctatttatg tcgcccgcgg cttttcaaag 79860

gtcgagcgag cacgccgcga agcgcgcggg cgagaacacg tactcgtggc cgaactccgg 79920

gatctgcgcg gcgcgcttgc gcgcgcgcat gtgcgcgagg aagttctccc aggtgagctg 79980

gttgctgttg ttcttcgcgt agttcttcac ggtctgcgga cgcaggttgc gcgtcacgcc 80040

cgtgacctcg aagatcttgt ccaggaagaa ggagtagttg atggttttgg tgggcgtgat 80100

ctcctggcag aagaagacca gctgcttgaa tatctcgatg acctcgttga tcttctcggt 80160

gctgaggtcc agcttctcgt ttttgacctg gttgatgatc tcgaagacca gcttgtagtc 80220

cttcttgttg atcatttcgc tgtccttgag gaagctggag acgtagttgg cgtccacgtc 80280

ctcgggccgg atctggtgcc ggtccatcat cgcgcgcagg tcgcggatga cctcctccga 80340

gcactgcttg gagagcagcc gccggagcac gttccgcagg tggatgagct tgttcgacac 80400

gtggaagttg gacctcttct gcacgcggat gcccatggga aacacggtct cgcagaacag 80460

gcagaactcg tagtccgcgt cggacacgag cccgttgcgg cggcagccgc cgcacatgcg 80520

caggttcatg cttgctccag ccccagcacg cgcaggatct cgcggtccag cactttagtg 80580

tccagcgtgc gggttctaca gaactggagg aagcccgcga gcgcgcgcgc gcgccctggc 80640

tgcgagagca gcagcatgcg cgcgttctcg gggtcctcgt tgatgacgcg cgtgaggttc 80700

agcgagcacc gcgtgcagcg ccgcggcggg tcgagctcga ccgagtacgc cgcgaaccag 80760

acgttgtcgc ccatgtatta tttattaaca cagaacgtcg cacatgttgc gcgaggacat 80820
gtacgggtcg tactcctgcc cgtagatgag gatggtgcag taccgcgaga tcatgagcat 80880
ggcctcctcc atggtgagca ggtcgtcctc gaacatggcc ttgtgctgca tgtgctggct 80940
ctgcttggcg gccatcgcgg ccgcgccgtc gccgcgcagc cactcgttca gacactggcc 81000
gtcgccgccg gcgccgctct cctgcgcgaa ggtgttgcgc atggcgcgca tgagcctggc 81060
ctccctggcc gagcggctga gcacggtcat ggggtcgtag agccaggggc ctgcgtccgt 81120
gaacaggatg gtgcagtacc cgttggcgaa ggcgtccgcg ccgctgcagt tgtcgatgcc 81180
gtcgccgacc ctgtagcaca ccgcggagac cagccggtac atgatgccgt tgagcatcat 81240
gtcctgcgac acctcgatgg gaatgtcgct gatcacgggc cgcatgttcg tgaagcagtc 81300
gcccgtgctg gccatgcccc cgcgccggtt caccaggaac accagcacgc cgttcgtgat 81360
cacgggcgcg cggtcgcgct cgtagaggta gccgctcgcc gcgcacacgg cggacgccac 81420
gtccgtgcgc gagaccgcgc ccatgttctg ggcgggcatg tacagcactc gcccggcctc 81480
cgcggtgcac gagaagggct gctccccgcc cacgtggatg ggcgccgtcg atgtcgtgat 81540
catcttgctg gagtccacca ccaggtaggg caccgtgtgc atggccatgt cgcccatgcc 81600
cccgatcccc gttccgaacg acggccgcga cacgctcacg agcgtcggct tgaacgagac 81660
tatggagaag atggaggcca agatctgctc ctcgtcggtc atgatggagg cgcacgaggg 81720
gtggatgatc ttcattaggg cgttgtcgat ggactcgtcg ctctcgcagt agaagacgcc 81780
catgcggagg ttcaggatgc accgccgcag gttggtgtgc agcaccgcgc gctggatctc 81840
catggacacg gagtcgccga cgccgggcat cacgatgggc gtttcctcgg tgagcttgtt 81900
caccagcagc tggtagttgt tgggtcgcac gcggctgttg tggtggagct gcgccaggag 81960
cgagaggctg tccccgttga cgaacgcgga ctcgatggcc ggcagcttta cgccgaacag 82020
cgccatggcg atggggtgca cgaagcccac ggagtcggac gacttgaacg agaagagcag 82080
gtcgcccgag gagctcatgt ctttgaagtg cacggactgg aactgcgtgg cggagagcaa 82140
gttctggtag ctggacatgc tctgcaggtc gtcgatctcc ttcatctgct tgtttacgcg 82200
ggtcgagttc ctcccgtaga tgatcaccag cgggtgcgtc tggctcacgg atagccccga 82260
gtccgtcatg gcggcgcgca cgctgttcag cagatcgaac agctccgacc ggtctctgtt 82320
ctggatcccg acctttgcca tcacagacat gagctcctgg atggtcatgt tcttgtggtc 82380
tcggcgcgtg gaccgcaggt agtcggcgat catctcgccc tccttacgga tcttcatctg 82440

ccagtcgtgc atggaggtca tgcggtccac aggcatgagc acgctgtcgg aggacgactg 82500

cgcggcgctg ccggactggc gcgagccagg gcgcgcggac gacgggcgcg cggagctgcc 82560

gcggctggag gaggacctgg acctccgcga ggatcttcgc tgcgaagagc tgcgcacggg 82620

ccgctgggcg cgcgccccgg cggaaaccat gtcctcgcgg tttatgctga ggagcgagct 82680

gcagaccgcg cacgacagcg actgctttgg aatgtggatg tggtcgcact ccagggacat 82740

gcccgcattg tcgtagcccg ggaccaagtc gaactttgca ttaaaaaaat ctgatgcgca 82800

cgcgggcgat tccatttata ccggaagttt ttatgaggtg ccggtattat ccacgcgatc 82860

tcgcagtgtg ctgggagact ctagcgtagc cacggacccc gtgagcagac gacgcaagtc 82920

gttgatggcc gactgcgtga cggacttcgc cgtctcgatg tcgcgcgtaa ggctgagcga 82980

ctcggcgttg aggtcgcgca cgctgtccgc gatgtcggcc agctcctttt tgatgaaatc 83040

cttatcatta tcggcgttga tgactttgtc cggcactcta gactctagaa ccggtgacgc 83100

ggcgggcgcc ttgatcgtcg ggcagctgga cgccgggtac tggggcggcg gcaatgattg 83160

ctgtaggaag atgggcttag cggcaggcgc cggttttgtc ggcaagggcg gcgccggcgg 83220

gcactgtcgt gtagacggcg ggcacgccgg cgcggggcac gccgccggtg gcggacatgt 83280

cggcgcagtt gcgggtggac acgcgggcgc gggcgccggc gcgggacacg tcgcggcagg 83340

agccgggcac gcgggagccg gagctggagc cgggcacggc gcggcaggag ccgggcacgt 83400

cgcggcaggc gcagggcacg cgggagccgg agccgggcac gccggcgcag gaggacatgc 83460

cggcgcagcg gcaggacaga ccaccgctgt cgcggagcac gcggcaaccg gcgtggagca 83520

cgcggcgggc attacgttca gaggcgtcga ctggaccttg gcaccgcggg gcagtagcga 83580

tggcgctagg ggcgactgtc cggtggttgg acgctgcatg caggcagtcg cagatttcag 83640

acgggactgg taatacctgc ccgcttcctt caccgtgtac ttgtcgacgg agtctacctc 83700

ctcatctgga ggacatggct gctccggtgc gggaacgact gtttgaggac acttggtgaa 83760

cagactggag ctggtctccg atagcaccag tttagacttg gccaagtcgg aggcaaacct 83820

tcttctgaga tccatttaag ccttcaaaat tgaacgtgta cgccgaccgc taaatggaag 83880

aatcggtggc cgtcgagtac gcggacgagg acgaagatga gattgaggag tacgaggagg 83940

aggacgatga cgaggaggaa gagtctgccg agggcgccgc cgcctcctcg gtcagcgacg 84000

tagcgctctc tgccgccgag aagctggtgg cctcggaggt cccggacgac gcggctgccg 84060

cggacaccaa cgtgcgtcaa cgcgtcaccg cgcgcgtgga ggagcttaag gcgcgctaca 84120

cacggcggat gagtctattt gagctcaccg gaattgtagc agagagtttc aatcttctgt 84180

gtcgcgggcg gctgccgctc gtggcggacg ccgcagaccc ggcgctcgac aacgagctaa 84240

aagtggtggt tcgggagctc gaggagggcg tctgccccat cgtcatcgag aaaaacggcg 84300

agttcctctc gccgggcgac ttcgaccccg agtgcctgcg ctaccacctg acgtacatga 84360

ccgacctctg gaagtcccag gggcgcatgt agccgcggct acgccgactc ggcggcctcc 84420

gcgatttttt cttttatcat gtccagcagc tcgcgcacca cgatggggcg gccgcagtac 84480

gtgatgccgt tttcggatat cacgctctgt gcgatgtcca ccagcgagcc ctcgcgctcc 84540

cagtactcgc gcgcgagcac ctccttgtac aacgcgcggt ggttggccac gtaccgcacc 84600

agcgtctgga tgttcttcac gcccacgctc ttgaggtcct gcggcgagaa cttctcgcgc 84660

agcgacacga agacgtcccg gacgagcttg ccgatctcca cgttggtctt gaactcgttg 84720

tacagcacca cgtagagctt gcacacgacc gtagcgaact tcgcgggctt gagatccttg 84780

ttctggaaga ccagcatgct gctcatcacc ttcttcatga aggccaggta cttcgcgcgg 84840

tcgccctcga cgctcacgct cccgacctcg aggtcggaca cgcagcggat tccgtgctcc 84900

gcgctctccg cggagacgcg cagcagctcc tggtactcct tgagcttctg cttgtccgtc 84960

atcagcgagt tgtcgaatac cgccaccagc ttgagcacgt agttctcgtc cgagaagacc 85020

ttgttcagac acttcaccag gaagctgtag tggctctgca ggatcttcat gaccgcgttg 85080

gcgccgctgg ctccgcggac gtgcgatatc atctccatga tcttcttaga gtcgtcgatg 85140

atctcctcgg tgtcgttgcg catgttgcgg tacattgcgt tcagcgagac cagcgtctgc 85200

gcggccagga gcacgtcgcg gaacacgcgc gcgaactcgc gcttctcctc cgcgtcggtg 85260

atgctgttgt acacggactt cgccaccgcg ttcgacttca ggaaccagaa ggagagcgcc 85320

tggtagttga agtgcttcat cagcgccagc acgtccgcct cgctcatttc cggcgcaatg 85380

gggcacaccg agctctcgag cacgggcacc atgctgacga gcgtgtccac gtccgtgtcg 85440

aagtccaggc agtccacgca gagcccggtg ccgcggctca ggtgatcgcg gctgatgtcg 85500

tagaagcgct cgtagcaggt gcggaggcgg tccatgtcgg ctgcgtttta gagagacaca 85560

cactcttgaa ttatggctgc gggtagaact cctgcagcag cgccggcgca cgcgcggagt 85620

ccggctccac tcccagcttc agcgcgcagt tcacggacca ggtcttcatg aagcggtcgg 85680

gcgcgtccgt gaccacgtgc cggaagagct tcgcgaagtg gcggctcacg gcgttgggca 85740

cggtcgcgtt gcgcacgaag gccgtgaagc gcgaggtcag cttcggcgcg aagcgcttgc 85800

cgtccacgaa gaagcccgag gtggtgagcg agagcccgtt ctcctcgcgc accacgcgtc 85860
gcgcggcctt gtgcggaaac atgctcgcga ggcgcccgct cgcgtcctgg tctaggtgga 85920
tggcgtccgt ggccgcgtcc ttgcggatgc gcaccacgtc gtgcacgatc tcctggatga 85980
ggatgcgcgt ggccgcggtc tccgccagcc gcatggggaa gtagaccatg tccccggaga 86040
tgagcacgtt cccgctagcg tttacgtagc tcactatctc ggacacggtg cgcagacgca 86100
cgatcgcgcc ttcgcagcag tgcaccacgt agtacccagc ggtggcgcgc aggcgcttgt 86160
tgtccgcctc gaagtccgcc tccaacccct cgttgaagta cttgtcgaat atgatgggca 86220
ggaaggatag ttttgactcg gtgaccacct ttccgaagtt gaggatgtac gggttcagcg 86280
cgctgcggtc gacctcttcg tcgtacacgc aggacttgaa ggtgtcggtg tgcgcctggc 86340
tgcgcaggaa gcagcacgga atgcagatgc gctgcaggcg gtggaagatg gagaggaagc 86400
ccacgctgtt gtagcgcccg tcggggtcca tgcacgaaaa catgacgccg tttccgttta 86460
cgaagacctc gcgcgtctcg gacttgaaga agttgttgct gaccttggcc atgttcgcgt 86520
ccagcgactg cacgatcacg ggcttgcggt tcttggtctt ggtgttctgg cagatgcgcg 86580
accagtacac ggtctccacc ttggtgaagt ccgaggactg cttcacgttg ttgaacatca 86640
cgctgatggc cacgatcaag aacgtgaagt acttctcgat gttcgggatg tagttcttta 86700
ccttcacgga cacgtgcgac ttcgcgagga tgatcgagat gcgcttgtcc gtggagagca 86760
ggatgttgtt ggtcgccgtc tccacgaaga tgaagctcgt ttccatgtcc agcttcatct 86820
tagacgtgat ggtcgtgttc agcgacacct tgtaggtgat gtcgcccttg acgcggtcca 86880
tctttacgtc catgctctcg atgagcttcg tgaacagact cacgtcgttc accgtgagcg 86940
tcttcccgtc gctcgagatg accaggtcgc cttccgggcc ccacaccgag aggttcagcg 87000
gctcgtccac cagcaggaag cgagtccccg tcatcgacac gaagaagtcg tccgtcttcg 87060
agagcaggat gtcgaagtcg cccacctccg ctcgcttgtc cggcgactgc tgcgcgatcg 87120
cgcggagccc ggactcgcgc aggttcgtgc ggaagatgtt gttgaacttg gtctccacgt 87180
tcatgtttag gtcgaggttc gcgaactcgc ggatgagccg ctcctcgaac ttgaggatgg 87240
agtcgttggg ctcctcgaag gagccgaact ccggcgcgga ggtgtccgcc gcgcgcgcca 87300
cccagaccac caggaagttg cacgcgtccg cgtacgcgtt gtagaggatg ccgtccgtgc 87360
ggatgagcgt tttcttttgc gtgggcgaga acgggttgaa gatggtgttg tccacgtagc 87420
tgtactccag gttgttcttg tgcgagtaca cgatgatctc gtcctgcagg cccagcaggc 87480

tccccaagta ccccttgagc tgccgcacgc gcatggtcag caagatgtgt ctgcgcacgt 87540
gctcggggtc cttctggatg tactgcttcg cgaagaagta gatcggcgag gcctcgtcca 87600
cggagtcgta cagcgatagg tacagcacgc gctcgatctc ctggtggcgc cccaccagca 87660
ccaccagctg cggcgcgacg gtgtagagca tgttcgcgcg ggcgtattta tagccggcgt 87720
taaactgaaa taaaatacgc gggtcgcgag gcagcgccat gttccagccg gtgcccgaca 87780
tggccgccga ggccgacatc gacctcggcg acgtcagcgt ggacgcgacg cgcgcgggcg 87840
cgcgcgagaa gaccgtcttc ttcgcgcgca acaagcgcat gtacccgcac cgcagcaagg 87900
acgaggagcg caagctgtcg ctgggcttct tcttgcagcg gctggacttc ctcacgtcgc 87960
gcgaggtcaa cctgcagttc cggtcgctgg acgcgctgcg caccgagaac gtcatgaaga 88020
agaacaacgt gctcgtggcg ccgtacatcc tcatcgcgac gctcgcgggg cgcggtttcc 88080
gcatgacgga gaccatggtc gagctctact tccccgagct gtaccgcgag accagcaagc 88140
gcttccgctt ctgcgcgcag ataaaggtca tccaggactt cctggggttc gcccacgaca 88200
gctaccacac ttacgacttc gagacgtact tcgcgttcgt ggcgctggtg ctgcgcggcg 88260
cggactctgc ggccgaggcc ttcgacgtcc gcgccgagag cgggcttgtg cgcagcctca 88320
ccgagatcac gtaccggctc tacgtgatgc agctgcgctc cgacgccgcg cagtggagcg 88380
tgagcaccgg cgccgtagtc tcgcaggcgg tgaacaccgt gctgtcggtc gtcggcgacc 88440
tcgctgcgcg cgcggaggcc gagcggctca cgcccgtgtg cgacctcgcg cgcgagaacc 88500
cgctctcgct cgaggacctg cgcaagtacg gcccgcggct gcgctcgctg ctcacgacca 88560
tggcgcgcgc gcgatccttc aagacgaacc ggcgggacaa ggacgcgctg tcccggttct 88620
gccgactgac ggcgggccct agcccgtctg cgtgccgcgc gtcgccatag gcgtcggcgc 88680
gcgctcgccg ccggaacact cggggtcgct gaacatgtag atgagcgcga cgcctagcag 88740
caggtacatg atcatgctga tcacggtttt gaacacgacg gcggcgaacg tgttggaccg 88800
cagtcggtgc tcgcagaagt gcatgaacag gtgccgcatg aggtcgatgg ccccgttggc 88860
cacctggaaa agggcgaggc cgccgatgga cttgatcacc gtcacgtagc acggccgcat 88920
tccgacgacg ctatttactc actgtcaaaa gaaacggcgc catccgaccg gaggttgagg 88980
ttgcgcttca tgttgttcca gtacatctca ccgatgctcg agtagtacgc cgtcagccgc 89040
gatatttttt ctcgcaccag ctcgtaggcc ttctgcatct ccgcaacgcc gatctccgcg 89100
tcgcccacgt accggccgct gcggcgcacg atcagcagca gcgccttcag gttctccagc 89160

gcgatcatgt ccatgtacag cgacttcgag agctgcacga agaggttgta ccgctccagg 89220

atgctgttct tcacctcgtc cgcgatcggg actccgaaga tgcgctccgt ggtgtacacg 89280

gactgcgtga gctgcttgaa gagcgcggag atgcagcagg tcgcgcgctt gacggcgtcg 89340

agctgcttct cggagcgcgc gctcgcgatg ctcagcgcgc tgttcacgac gttgctcgtg 89400

tcgcgcacgt agcgcgtctt cagcgcggcg ttgatggcat ccgcgatctc gttgctgctc 89460

acgctcgagt cgtccgagct gcccgagacc tcgtccagca gccccgagat cgtgatgtcg 89520

ggcgagccgc cgacggtcac cagacggtcg agcaggttgc agggcatgga catgaggatg 89580

ccctcgctcg agaggcagcc ctcgtcgatc atgctctgca ggttgcgctt gaaggccgtg 89640

ttttcgggca tgaacccgtc cacgctcatg agctcgtcga ccgtgctcgc ggaaaagatg 89700

cccctcacgt tgatgcggtc cagcatgccc atgtcctgcg agcacagcac caccgactgg 89760

tcggccgtct cggcggcgtc cttggcgccg ccgtagatga tgcgcggaaa ccgccagctc 89820

gccggaaaag agaaggaggg aaaccggcac tgcgcgctcg ggcctcggta gccctgcgcg 89880

tcgcgcacgt tggtggccgt gaccatgaac tgcagcaggt cgtgcgcgga cgccatgatc 89940

ttctccacct cctccttgct gcagcagacc ttgcccaggc tgcgcgcgat gttcgttttg 90000

ctcactgagg gcgagaccgt gacggcggtg tgtcggcggc tgccgagcgt gtacgcgctc 90060

acgctaacgc ggtaccccat ggcgccgaag agcagcttca cgaagtccag gtagctctcc 90120

ttattgatgt agtgcggcgc gcccttgtct tccatcctca gcccggcgta ggccatgagc 90180

acttccttca tcgccgtctc ggggtccgag ttgcacacca gccgcagcat ctggaagaac 90240

tgcatgaagg cgcgctgcga gagcccgatg tggtggttgg gctgcgtcga ccggcgcggg 90300

aactccctgg gcgtcatggc gttgatgccc gagagcgtct ccatcacgag cgcgcccacg 90360

gtcttctggc ccatgacgcg cgggtaaaag cacacgcgga ggggctcctt gccggccgcg 90420

agcgcgtccg agagcagcga gcagtacgtg acgttgtcgt ggtcgaagag cgcgaaggtg 90480

tagcagacgg agctcatgaa gagcgagtcg gcggtgctca tggacttgaa ctccgtgtac 90540

gcgattccgt cccagaacag gctctttccg ggcgcgatca gcggggacgc gcggtcggcg 90600

cgcatcagca tggagagcag cgtcacgtag taacggatgt tggcggaaat gtctacgaac 90660

tgcatgccgg gcgaggccac gcgcagggtc gcgcccgagg tagtgagcac ctccaggctg 90720

tccatgagcg tcacgctggg gtgcagctgc gcaaggcgcg ccagctggct ctggtagaag 90780

atggacacgg cgaggctggc cacgctgccg cgtgccatgc gcaggttttg cccgttgaag 90840

gtgagctggc gcagcgagaa cacggagtcg aagtactgga agaaggtgag caggtacttg 90900
agcggcatgg tcgtcagctc ggtatccacc tgcggcgtct gtgcgagcac gattccgttc 90960
ttggccgcgg cgtcggggat gtcgtacatg gcgtccattc tggcgcggga ggcgtcggtg 91020
agcagcgcgc gcacgttgag cagcatgagc aggtcccgcg ctagcatggt cccgtcgacc 91080
agccgggcgc gaaagccgat ctcggcgggg ccggcgatgt tggggtagat caggttcagc 91140
aggtacgtgt tgtcgaagct cagcgagggg aaggagatgg gagacttcgc cgggaggccg 91200
gtggggtagc gcacgtagcc gccgcagatg cgcgcgtgcg cctcaaagct ggtcacgcga 91260
gtcttcagca ggttgcgggt gaagggcggc acgtccttga aggactgcgt gcagatcacg 91320
gggttggcgg tgtcggtcag cttgaggttg gtgggcttaa gctccgcgaa gttggggccc 91380
agcagcacgg ggacgaggtg cgagttggcg gcgctgtcga gcaggaagtt gatgccgaac 91440
tgcttcacgg cgacctcggt ttcctcgtcg ctggcgagct tctccgcgtc ctcgaggaag 91500
agcgcgtcca gcgggtgcac gtacgtgcgg ttgacgtcgt agctgggctt gaagtccgaa 91560
cacagcgtgg ggagcacggt ggagacgagc tggaacatgt attccgcgcc ctccacatgg 91620
tgcaaggcca tgtgcacgtt tggggccgtc atttatttag tattaaatga cggccgtacc 91680
ggtaaccgat attcctggag actacgggcc gacgtccttt tcggaggaca actacccgct 91740
gaacaagcac tacgagctca ccaaaggcca gctctcgatc ctgcgcacgg tcaacgacaa 91800
gctgctcgcg cgcaccgtgc agcactcgga cggggagagc gatgagagcg aaagcgagga 91860
ggacgacatc tccagtccgc tgccgccgga cgaggaggag ccggactcgt gcgtggcccg 91920
ggtcatgccg cgggacgcgg acctggcggc gccaaaaaag gccgacggct acatcattgc 91980
cgccgagcag cagcgccagc agcgcataaa cattctggta tccgatcgag aggccgtcgt 92040
ggagcgggag ccggttcaga cgtcgttcgc gcgcgtctcg gctatcccga tccacgggga 92100
cggcgcgcgc cgcaccaccg cctccttctc cgcgaccacg ccgtcgctgg gcgccgtgtt 92160
cgacgacgcc aagcgcgtgc ggctgctgga ggaggaggtc aaggagctcc gcagaaagtg 92220
cgcgacctct caggataacg gaaacctgga gaacttcacc aaggtgctgt tcggcaaggc 92280
gccgcgcgcg agcgagctga acaagcgcgt ggtcatcgtg aactacgcca cgctgaacaa 92340
cgtgacgctg tccatggagg acctcgagaa gtgctccgac gaggaagtgg accgcatgta 92400
ctcggtcatc cggcgctaca acgagacgcg gaagaagaag atcctggtca cgaacgtggt 92460
catcatcggg atcaccgtgc tcgagcacgt gctggtgaag cttggcttct cggaggtgcg 92520

cgggctcagc gccgacctct cgtcggagct catcgacgtg gagatcggcg aggactgcga 92580

gcacatcgcg gagcgcctgg ggttcgggaa cagcccggtg ctaaacgtgg cgctcttcgt 92640

ggtaaagctg ttcgtgcgga agctgaacct gatctgatca acacatgccg ccgtcgaggt 92700

ccatggcgtt catgaggttg gaggcgcggc ggcgcgcgcc ggtggaagcg gtggaggcgc 92760

tcgaggtcgt ggagcaggga gtgttgctgg aggaggcgcg gcggcgggag ctagaagcgg 92820

aactcgaggt tccgctggtg gtgctgcggc gactcgtgcc gctcgtgccg ctcctgctag 92880

tgccagtgcc agtgccgctg cggcgtgaag taccggtgcc ggacctgccg ctggagcttt 92940

tcttgcggcc gccgttaacg ctgtcgatgc cgagcaggtc ctcgcacacc tcgccgacgg 93000

ttccctgcac gtccaacttg ccgttcttga caaccccgta cacgatcttg ccgcagttgg 93060

acacagcctg gatggtggtc tcgtcgctgt caaaggcgtt cattccgccg cacccgccgt 93120

cgttgtttct tcgagaaggc gcgccgctgc ggcgactcct ggtgctgccg ctggaccgag 93180

ttccggagga cctggagccc gtggaccggc tgccggtcga cctggtgccg gtagtgcgct 93240

ttctggacga agaggaggag gcgcttccgc ggcgggtgga cgaactagcc tccagcgcac 93300

cggcgccgcc cacacaatcc acgtcggcgg cggcgcctcc gcgaatgacc tgctcgttgt 93360

tgagctgcgt caggagagat cgcagctgcg gcgcgatctt ctgcaaggtg ctcacgtagt 93420

cgtcgtagct gctctgcggg cgctgcgcca ttttttcgga cgccatttat tacgcggaat 93480

atctacgacg acgcagcact gaatcggttt ctcgcgacgg gagattccgc ggtcggcgcc 93540

ggtgcggggt tgtcaccggg cgacgaggta accagcgcgt ggaaggcgcg cacctggtcg 93600

tccgtcatct tgtcctcgaa cgaggacgcg cccgggggga gcaggtcctt gttgcgcgga 93660

acggcgggcg ccgagacgca cgaccggcgg tacatcatga tgacgatgta gcacacgatc 93720

gagatgacga tcacggtcag cagcgcgtcg aggagcccca tttattacct gtatatgccc 93780

gcgtttaccg ggcggtgagc tcaatgtcgg tgttgtttag ccgggcgtac gggacgctgc 93840

cggagcactt cctgtacatg ctgaacacga acagcccgag cagcagcacg gcgcccacta 93900

tgaagcaggt tacgcacagc gcgcgccaca cgtagtcggt gacgttgttg gtgttcttgc 93960

tgaaatccac gaaggcgaag acgcaggcgg ccgtcagcag cagcacgccg catatcagca 94020

ctccggagta gtaagagctc aaggtctcga atatgtccat ttatctgagg agaaatttaa 94080

attactgaat ggacgaagtg gaatagaaac cacgagaaca cgacggactg cagcacgaag 94140

atggtgctca gcttcgtctt catgggcatg cagaagttcg cggccagcgc catacagaag 94200

atgaacacga gcaccgccgg gtcgtagtcg gacaccattt acactacgct aaaaggcata 94260

tctcggcgcg cgacgtccac gagcaccagc acgcggacgc ccgcgggcgc gccggcggcg 94320

accgcggcga gctgcccggc cgtggggttc accagcagca gtgcgcgcgc ggttcgcggg 94380

acggggtctt cgtaggccat ggtcggcgtg gacccgggac gcagcggccg cccctgtctg 94440

tcgaagaggc cctcgggaaa cgaggtgccc ggaacggcca cgacgacggt gtcgctatct 94500

agaaacattt atggtcttgg tttccacgga tcgcctcgag tagaccgcca cgaagtagaa 94560

gatgacgccc gccgcgagcg ccgccaccag gaagggcggc acggcgggca ggttcgcgga 94620

cgcgttgtcg cgcacgccgg ggtccgggtc tgcgtagccc gcgcccacgg ccttgccgca 94680

gtcggcgatc atgtgcgcgc gcgagttctg catgaccagg ctgtccacgt cgatgcggca 94740

gcccacgtag cggcaccgcg agcgctgctc gtcctggctg aagaagagcc acttgcggtc 94800

gcgcgactgg tccgtgcact cgtgcgcgcg acagacgcgc gggcccaggt acttcccgag 94860

cgtggtgccc gcgacacacg cgcactccgg cgcggcgcgg tgcgcgtcgc agtagcgccg 94920

cagcgcggag tcgccgaagg cgaaggaggc gggccgcgcc acgcgcacga actccgagca 94980

gaagcgcgcg tccatgtgct tggcgcagag cgccgcgtag gtgtccagcg ccgcgtagcg 95040

gcccgtgcgc agccaggcca tgcactcggg cgcgtcaggc tccaccgcgc agcggctggc 95100

cataacgccg tcgcagtgcg cggtcttgta cccgttcgcg aacacggacg ggcacccggg 95160

ctccggattt gtgcagcagc gcgccatggc ggcgtccgtg ggcggcgccg aggcgccgat 95220

ctcgaacgcg cacatggtgc cctggcgcag gtacggcttc gcgatctcgg gaacgtagtc 95280

tgcgcgcagc agcgagcccg ggcggaagaa gagcgagtcg cagggcgggc ctcgcacgag 95340

ccgcgcgcgg ctcgccagct ctggcgagag gaagcgcccg cactgcccgg ggtccatggt 95400

cggcagcaga cagaaccgcg gccgtacggt cttcagcttc gggtcggaga aggtttctga 95460

ttcttccgcg aaggcgaagg tgtccgtggc gctcgtgtgt gtgacgcgca gcgcgtactc 95520

gcctggcgtc ggcgtgtcga gcacctccac cttggatacg gtgtccccca tttgaagacg 95580

ctatttacgc cgctgcctac tcggcgaaga ataggtcctc cgacttggcg cccgcgtaca 95640

ccgggcaggc gggcgcggcg gagcgagtgc gcacgatacc gcggccagtg aggcggaagg 95700

cgtagatggc gaacagcagg ccgagcacga tgtacatgaa agtggtggcg cccacagacc 95760

cggtcacgtg cgtcacgatg atggtgacga tggacatgat cgtgcacacg atggccatgc 95820

cggtgttgtt ggccgcgtag ggtgcatga tctgcatggc cgcacagtat ccgatgacca 95880

ggcacggcag cgggaggata agtgaggcaa tacctatcat tactagagcg agcacggggg 95940

tggacgtcaa ggccaataca aaaatcacaa tacctgttag tatgcggata tcctcgtact 96000

ggaggacgct gtaaggcgcg atattccctc cgggcactgg cctggggtta gccgggacta 96060

ggggggagtc ggcagtgccg gggtccttgg ggagaaaggc attctgctcc tccgggctga 96120

agagctcggc gtcctgaacg ccgccggcgg tgaactcgtc gttatagtaa ctaaagtagc 96180

tttccattta tatgttgaaa aatgtttgga ggcgtacagg tggacgacaa actctacgcg 96240

tacctaaaaa aactcgccgg acgcgggcgg ccgctgtgtc tgttccgcga caacggcgag 96300

ttcgtcgaag tcttcgcggg gtccgcgttc cgctttgtgc tgcccgtggg cctcttcgcg 96360

gacctgcgcg tgcgcacgcg cggcgtggcc ttcccgaaac tgcgcgactc cgcgcgcatg 96420

cgcggcgtgc gggtggacgc gcacacgctg ccctcgctgt accccaacca gcgcatcgtg 96480

gtggacgagg tgctcgcggc ccgcgaccag ttgctggccg cgggccgcgc cgtgtacgtg 96540

acgctgcatc tggcttgcgg cttcgggaag acgctgaccg cgtgccacct catcgccacg 96600

cacggccgcc gcgcggtggt gtgcgtgccc aaccgcatgc tggtgccgca gtggcgcgcg 96660

gccgtggcgg agctgcgggt gcccttcgcg gtctcctgtg acggcgcggc ctcgctgctg 96720

cgctcgggcg agctcgaccg cgccatggtg gccatcgtgg tcagccggca cttcgccaac 96780

gacgacttct gccgcgcggt gagccggcag tttgacgtgc tcgtgctcga cgagtcgcac 96840

acatacaacc tcatgaacaa caccgcggtc tcgcgcttct tgaccaagta cccgccgccc 96900

atgtgcttct tcctgaccgc gacgccgcgc acggccaacc gcatctactg caaccgcgtg 96960

gtgaacgtgt ccgtggtcag ccgcctcacc aaggtagtgc gcgtggtgga cgccttcttc 97020

gagccgtaca ccacgcccaa gatccgcacg ctcgagcgca gcctcgaggg accccagaac 97080

aagtaccacg tcttcaccga gaagatcctc ggcgaggacg tgcaccgcaa caagctcatc 97140

gtggacaccg tggtcgcggc catggccgcg ggcgaggcgc ggcgcgtgct cgtgctcacc 97200

aagctgcgcg aacacatggt cgggctgcac gccgcgctct gcgagcgcct cggtgcggag 97260

acggtctttc tcggcgacgc caagaacagg aagacgcccg aggtcacgcg cgcactgcgc 97320

gacaaggacc gcttcgtgct cgtgtccacg gtcttcttct cgggcacggg cctggacctg 97380

cccaacctgg acgcgctcgc ggtggccgcg gccgtgctca accgcatggt catggagcag 97440

atgatcggac gcgtgtgtcg cgagtcgcac gccaacacgc gcacgctgtt cgtgttcccg 97500

gactcctccg tgcgcgcgat ccgcgacacc gtgtctgcgt ttgcgcagcg gctcgtggcg 97560

ctggcggtgg acgggctggg cttcgtccgc gagcgcgccg ccgccggcgc gaagaacgag 97620
ccggcgctgt acagcgccat cagcgggcga gatctcgcag cggtgtaagc gcggacccgc 97680
acgccgcgca cgagagcgtg ctggagcagg cgagtcccag cgacagtgtg gacagcctgt 97740
ccacgtcctt gatgctcacc agccgcgagt tgcacgacga gcacacgggg tcgctactat 97800
catcgaccac cgtggtgacg cggcggcgtc tgcgcttttt gtttccagcg gcgacatcga 97860
ccacgcctcc cttagagccc cccttcgccc ccgccttagc tttcaccgcg ctcatctttt 97920
atttatcata aaaacacgtc tgcgtacgcg ttcgcgcaca cgtcccgcaa atccgcgcgc 97980
gcgccgcagc gcgtgaagcg cgcggcgtcc gcctccgcga tccgcgcgca cggcagcggc 98040
gcgcccttct cgtccgccat cacgcgcgca gagatcccgg tggcccccag cgcgtacgac 98100
accaccacgt cgccgacgca gcggtacacg ttgccggagc cggcgaggcg gtcgaacgcg 98160
gcgccctcct ggcgcagctt gtcgaatatg cgaggaacga ggatgttaaa aatgagaacg 98220
aaatagcaga tcagcaaaaa cagcgagatc atgacctccg agagcgattt atataccttg 98280
aaagagctaa tacgacttcg ggactcgctg cacctcgcca ccggcgccgc cgtcgagcgc 98340
tacaacgcgc tcgtggagtg ggccgcgcgc acgtactgga cggtcgcggt gctgccctcc 98400
gcgccgtgcg cctccatcga gaagtactac tgcgtgtgca aacccgactg cgcgctcgag 98460
cccggcgagt actccgtgag ccggctgcac ttcggactca cgcacgcctg ggtgcgcggc 98520
gccgccttca actcggccag cggcgccgag gtcgagccgc cagaggaggt gcgtagggcc 98580
tgcgaggcgc tcgacgccgc cttcgcggac ctcaccttcg tgcgcttctc ggtcttcggc 98640
cgcgagtgga cggtcgacga cgccgtcaca gaccactcct cgcgcgacga ggtgctcgcc 98700
gcgtgcgccg cctccggcgt gcgcgtcgcg cgcacgctgc gtgtgcgcgt gcgggcggga 98760
gagtccttcg cgcgcgcaga cttcgacgcg gtgcacgccg cgctgcgcgc ggagggcgac 98820
gtcgctcgcg gcaccgcggt ctgtctcgcg ctgcgcgggt catcgcgccg ctggatagcg 98880
gaccgcgcgc ctcgatgctt catgcgcgtg cgccgcgtgg agctcgagcc cgtggacgcg 98940
cggcaccact gcccggtgct gatctccgcg cgcggcgacc gggtgctctg ccgcggcgtg 99000
gggcacctcg cggacgcgcg cgcgcgcgag ggcgtcttcg tggccgtgcg caggtacccg 99060
gagtgtctgg tgctctgcga cgaggcggcc gccggcgcgg cggagtgctc gcgcgaggag 99120
gcgctgcggc tgctggtgcg ccgcttcggg cgcgacttcg ccgtcagcga ggagggctac 99180
gtcttccgcg tgcaggacat ggacttgcgc ggcgtgtccg cgcgactggg gctcgcgccc 99240

tgcgcgagcc tggaggagct gcgccgagcg gtggagcgcg accgcgcgct gatgaggcgg 99300
ctgcgcgcgg agggcgccgt gcgcctcgcg tgcgagtgcg tgggataccc gcgccagaac 99360
gcggtggagc tcataaataa tatgcgcttt caaataacgg aagaaggcgc ggtggcgaac 99420
tttgagctgg cgaacgcgag ctgtctcggc aacccgaccg cggagtccat cttcgcgagc 99480
ttcgcgcagt tcgtgccggt cttcaacgtg ctctcggcga tcgcgcgcgc gcagccatga 99540
tcgtggcggc cttcgacctt ggcacgcgca accccgcgcg caccgtgctg gaggtgctcg 99600
acggcacggt gcgcgtggtg gacgtggcca agctggactg gagccgcgac tgggagaagc 99660
gcgtgcaccg cgacgtgacc gccttccccg cgaacgtggt gctcgtggag cgccagtgca 99720
agatgtcgcc tttttctaag ttcatatact tcatacgcgg gctgctctac gacgggcggc 99780
gccgcacgcg cgtgctcgcg gtgccgccgg ccatgaccgg cagcacctac cggcagcgca 99840
agcgccgctc ggtgcgcacc ttcctcgcgc tcgcggagag cttcggcatc ctggacgccg 99900
tgcccgcgcg gaagaagctc gacgacgtcg cggacagctt caacatggcc atcaattacg 99960
tgctccgaac aaactgaaat acgactggaa cgaataagtc atgctggcgc tgttcgagtt 100020
cctgcggtcc gtggaggact gctaccggcg caccatcttc aacttccaca tcgcgcacag 100080
cgccgaggcg ggcgatgtct acggcgtgct gcgcgaccgc attttggcgg ccacgcgctt 100140
cgaggaggta gcgccgccag ggctcgcgga cgcgctggcc aaggtggtct actgcgacat 100200
aagcaccacc aagcacctgg tcaaccacgc ggccttcgcg gcgcgcgcgc ggccggcgcg 100260
gcgcggaggc agcctcgcgc agttcttcga cgtgcacgtg ggcgaggacg cggagagccg 100320
ccgcaccgcg gagatcttcg accgcgagcg ctcctcgctg gtctcgtacg tgaagaccac 100380
ggccaagcgc tgcaagatcg actacggcga gatcaagcgc accatccacg gcgggcggca 100440
gacctacttc tcggggcggc gctcggacga cttcctgagc accaccgtgc gcgcggaccc 100500
gagcaagccc tggatcaagt ccatctccaa gcagctgcgc gtggacatcc tgcaccacgc 100560
gatctgcacg cgcggcaaga gctccatcct gcagaccatc gagatcgtgc tcacgaaccg 100620
cacctgcgtg aagatattca aggactcgac catgcacata atcctctcca aggacgaccg 100680
cgagcgcggg ctcgcggacc tcgcggacaa gctcttcggg acctacgcga ccaccttccg 100740
cgtcatcgcg gccatcaccg gcaacgcctg cttcgcggcg gtggcggacg cggccgcgcg 100800
cgtggtcgcg ctcccggacg cggacgcgaa gctggcggcg gtgcgcgggc tcgcggagtg 100860
ctacggcgtg cgcaacttca aaatcggcat gttcaacctc accttcacgg gcgccatcga 100920

gcacacggtc ttcccctcgc tgatccccgc ggagagcaag atcaagttct tcaagggcaa 100980
gaagcttaac atcgtcgcgg tgcgctccac cgaggagggc cgcgagtgcg tggagcaggc 101040
gcaggcgctg ctcgcggcca tgcgcgagcg ctccgcgcgg ctcgcggccg cggacgtggc 101100
caccgcgagc gtggacttcc tcaaggagct gctggggcca tagtgaaata atactgattt 101160
cttaaatatg gagcaggcgc tcggatacaa gtttttgttg cccgacccca aggacgacgt 101220
ctactaccgc ccgctccact tccagtatga gtcatacgcc aacttcatca agcaccggct 101280
taaggacatc ctcacggtgc ggcgcacgct gctcaccttc aagaacggca ccgagtccat 101340
cgtgctcgag atcgacgacg tgaagatctc ggcgccggag ttctcgccca tcgtggccag 101400
catcaagggc cacagctacg aggcgctggt caccttcacg gtgaacatct accggcacgt 101460
gatgaccaag gacggcctca ccgttaccaa gatcaacagc tacgagggca ccgactcgca 101520
cctcgtcaag ctcccgctgc tcatcggcta cgggaacaag aacgcgctgg acccctccaa 101580
gttcgtggtc ccgaacgcca tcggcggcgt cttcatcaac aagcagtcca tcgagaagct 101640
cggcatcaac atgatcgaga agatcaccac ctggcccaag ttccgcgccg tgaaggccaa 101700
ctccttcacg ctctccttct cctcgatctc gcccgtgcac gtgatgcccg cgcggtaccg 101760
acactacaag atcctgctcg acgtgaacca gcccgacaac ttcgtgatct cctccgcgaa 101820
gaccttcatc accgtgaacg tgatcgtgat ggtgcagttc ctcgcggacg tcacgctcga 101880
gttcgtggcg cgcaacctct gcttcgacat gccgcccgag gccgcgcacc tggccaccgc 101940
gctcgtggag agcgcgaaga ccgtgcccgc gggcgcggac gtggccgagt acgtgaacgc 102000
gctcatcgcg gccgagcacg cgaagcagaa gtcgacgctg tccaaggagg agttccgcta 102060
cgagatgctc agcaacttcc tcccgcacat gcaggacagc gccaaccagc tcaagggcct 102120
gtacctgctc tcgctggtgc gcaagatggt cttctgcgtg ttcttcccga accggtaccc 102180
ggaccgcgac tcgctggtct gccaccgcgt gtacacctac gggcgctact tcgaggcgct 102240
ggccatggac gagctcgaga cctacatcgg gaacatccgc aacgacatcc tcgcgaacca 102300
caagaaccgc ggcacctgca ccgtgaacat ccacgtgctg accacgcccg gcttcaacca 102360
cgccttcgcg gcgctgctca gcggcaagtt ccgcaagtcc gacggcagct tccgcacgca 102420
cccgcactac tcctggatgc agagcatctc catcccgcgc agcgtgggct tctaccccga 102480
gcaggtcaag atctcgaaga tgttcaaggt gcgcatgtac cacccccagcc agtacggctt 102540
cttctgcgcc tcggacgtgc ccgagcgcgg gccgcaggtc gggctcatct cgcagctctc 102600

cgtgctcgcc tccatctcga acatccgcac cgcggacttc gtcgagctca ccaagcgcgt 102660

ctgcgactac gtgcgctcct accccgcgcg cgacatcagc tacttcgaga ccgggttcgc 102720

ggtcaccgtc gagaacgcgc tcgtggcctc gctgaacccc gcgatcgtgg acgcgttcgt 102780

gctcgacctg cgccggcgca agcggctcgg cttcttcggg aaccgcgaga tcggcgtcgc 102840

gctcgtgcgc gaccgcatga acgaggtgcg catcaacttc ggcgcgggcc ggctcatccg 102900

cccgctgctc gtggtcgaga acggcgtgct cgtcatggac gcggaggcgg agcggctcga 102960

gcgcgacctc gccgcgatga ccttctcgga cgtgctgcgc gagttcccgc acgtgatcga 103020

gatcgtggac gtggagcagt tcagcttcag caacgtctgc gactccgtgc agcgcttccg 103080

cacgctgccg cccgaggagc gcgcgctctt cgacttctgc gacttcccgg ccgagttccg 103140

cgacgggtac gtggcctcct cgctcgtggg catcaaccac aactccgcgc cgcgcgccat 103200

cctcggctgc gcgcaggcca agcaggccat ctcctgcctg agcgcggacc tgcgcaacaa 103260

ggtcgacaac ggcatccacc tcatgttcgc ggagcggccc atcgtggtca gcaaggcgct 103320

ggagacctcc aagatcgcgg acaactgctt cgggcaccac gtcaccatcg cgctcatgtc 103380

cttccgcggc atgaaccagg aggacggcat catcctgaag cggcagttcg cggagcgcgg 103440

cgggctcgac atcctcacct gcaagaagta ccaggtcgag atcccgctcg agaacttcaa 103500

caaccgcgag cgcgtgcgct ccgcggcgta ctccaagatc gacgtcaacg gcgtggtgcg 103560

cctgaacgcc ttcctcgagc agggcgacgc catcgcgcgg aacgtgtcct cgcgcacgct 103620

cgacgacgac ttcgtcgccg acaaccagat cagcttcgac atcgcggagc ggtactcgga 103680

catctacgcc gcgcgcgtgg agcgcgtgca ggccgacctc accgacaagg tcaaggtgcg 103740

cgcgctgacc gtgcgcgagc gccgcgccat cctcggggac aagttcacca cgcgcaccag 103800

ccagaagggc acggtcgcgt acgtggccga cgagactgag ctgccctacg acgagaacgg 103860

gatcgcgccg gacgtgatca tcaactcgac ctccatcttc tcgcggaaga cgctctccat 103920

gctcatggag gtcatcctca ccacggccta cgggcacaag cccttcgccg aggacggctc 103980

caaccgcccg atctgcttcc ccagcaccaa cgagaccgac ttcgagacct acatcgagtt 104040

cgcgcggcgc tgctacgcgc tctcgcaccc cgaggccgcc gcggacgacc ccgagttcga 104100

gcaccgcgtc ttctgcgagc gcgtgctctt cgaccccgag accgacgagc ccttcgcggc 104160

gcgcgtcttc ttcgggccgc tgtactacct gcgtctgcgg cacctcacgc tggacaaggc 104220

cacggtgcgc tgccgcgggc gcaagaccaa gctcatccgg caggccaacg agggccgccg 104280

ccgcggcggc ggcatcaaga tcggcgagat ggagcgcgac tgcatgatct cgcacggcgc 104340
ggccttcacc gtcgccgaga tcctgcgcga ctccgaggag gacgcgcagg aggtgctcgt 104400
ctgcgagaac tgcggcgaca tcgcggcgcg gctcaacggc acgcacgtct gcatccgctg 104460
ctccaagatg agcctctcgc cggtgctcac gcgcatggac tccacgcacg tgagcaaggt 104520
cttcaccacg cagatgaacg cgcgcggcat aaagatccgc gtggagttcg agaagcagga 104580
cccctgcttc tacgggactc cgaaacggtt cagcctcgcg cccgacgagt cgctgttctc 104640
gccggaggac tgaacccgcc gtcgcgaccg cgtcgcgacg actagcttat cgttcgactg 104700
atgcgaaacg cgcggcggcg ccgcgactta gcttatctcg actgatgcga acgcgcgacc 104760
tctcgcgact ttctagcttc tcagactgat gctaccatat cgcggcgtgc tggccccacc 104820
accagggctt ctcgccgtgg ctgacgcggg gctggctgcg acgcgcgctg cagtagctgc 104880
gcgcgcccca gtcgccgcgc acgtgcgccg ggggcaggct cccgtccagc gcgtgccgcg 104940
tcacctcggc gccgggccgg cggcacgtgt gcacgtccgt cttgttggag acgagcaccg 105000
cgtactgccg catggtctct atgtgatgct ccaagtgctt gcccgccatc cggttggact 105060
cgcagcacgt ttttgcttcg gctaaggttt tttctagagg ggatagtagc ttatccacgc 105120
gctcgggcag gacgcacgcg gagccgtcga accctacttt gaacggggtc accttgatgt 105180
tcccgtcgta gcggtcccac agcatcctga ggtaggttgt accgtcgggg tctgggtctg 105240
tccacactct aagcttttcg ctacagcggc cgtcgtacgt aagacggtct ctacgctcgt 105300
agtagtttct gcttatgttg ttggggtctc catgctcgta gtagtataaa tcgtacgcgc 105360
ctggcttttt taagtcgttt tcgtcgttgc tgacgtgtat cacgtcggga taataggata 105420
tcctaactgc actacaatct atagtatttg gtctagtaag ctgttcgaga tcaccttgtt 105480
catcatgatc tactgatttg tacacggcac cgtcgtgttc cgacggacgt atgaatatgt 105540
ccatggtaaa cgatgtaccc actttggaaa acgtatccca tgcagtaaag catagtccgt 105600
ccattataaa ctcaggaaca ctcataacaa atcgaaatct gtgaagtttt tcgaacacca 105660
cttttacatg gtctttgtca cgaacatcat tgccgtttac ttcagacatg aattgaagga 105720
acgctaaaga gtttcttgtt tcttcatgaa tctttccatt atacgtccat ccagtttcta 105780
gaattctata tatgcttttt gcatcgaccc cgtaccacca gtacatggga actccgaaat 105840
atatagctgg gtttgagtac caatgggcaa gagtgcccat tgcgtttaaa aagtcttgac 105900
aaaaaaatgc agtttttctg tcgataactt gacttggact acgttcgtgg acatcgtaca 105960

tgtccataat tggttcattg gtaacggtta catgacccgt cattatcttt ttaacaatca 106020
taagatacag tttgcctaaa gtcgaaatat gtataacgtt aatttttaca tgttctccta 106080
acgtaattgc gtttttactt agccattcgt cgtctacaaa aatcttacga tacataggat 106140
ttctctctac gtatcttcta aagtatagat ttaccggtct accggcgaca ttagcgccat 106200
ctatagcagg agcaagctgt atgtatcgtc gtataatgtc tcgtataagc tttctgtctt 106260
ctctgggaat acacgacacg gaacttagag actggtgcca gtgtctttca accaaagact 106320
tgaacctagc aaccaacgcg ttgtcactct ccatttataa ttaaataatt atcccaactt 106380
cgtatgttaa tccttattac cagatagcac cgctccttcc tctccaccac gtactatcta 106440
aaggatacct gtaagggtaa tgtctggata acgggcgtgt gagccaagac gtgttatggt 106500
gtcttcccca ccaacggtcc acttctctaa ctaccggagt gctagacgtt gtcgatccca 106560
ctactgttgt ttctccatta cctgtaatct ttgaagcgca acaagtgtta gtctttgcca 106620
aatcttctaa cggcttaatt aggtcgttaa gtctgtcaat atccatgcac tgaggtgtat 106680
cgttaccagt ttccccaact ttgggaggaa cttcctgttt agtgtccaaa taacccataa 106740
acctgtctct aagcattctt ctgtacgtgc ttttgtcatt gtctatgtct cccaaaaacc 106800
tgtgatttac gcatccgttg tacgtaagtc tgtcccttcg ctctcgctcg ttatttccta 106860
cttcttctat tgtactgtaa tgttgatagt ccaagtaata gccactgttt tcatgatttc 106920
ttgtaaatat aatcggtgtt ttattattga catcgtgttg cctactgtac gtatcttcca 106980
tggatctagg aacttgtcta gaaaattgag gactagaaat acgccttcca aatcctggat 107040
gataaaccaa acgcaatgca ctacagtcga catcgtcact gtctctagtt atcccatcaa 107100
caactccttc tttgtgatgc tccactgttt taaagttaac tcctctgtat atgttgagtg 107160
ttaaaaaaat atccacattg aacgcagttc catattttgt tattttatcc cacgacgtat 107220
aacacaaacc atccataatg aattccggaa ccgatactac aaaattagta tcgaacttat 107280
caaacgaaat gtttactcgc ggttgtgctt cgtatttttt gtcatacaca tctgtcatgt 107340
attgtacaaa atctatagct cctctagcat cgggcatgct aacgtctgga aaatcatttt 107400
ttaactgttc tagtacgaac tgttgatttt ggtcatctct ccaccagtac attggcaatc 107460
ctatcacaag agacttattt ttataataat ttgctacaga agctatatgc cacatgaaat 107520
tatagcaaaa ataatccatc tgtatgttta aaaccggttt actagtctgc tgagagtagc 107580
tatccatgat agtgtttccc tcgccaatac ttcctgacat tattctgtaa acaaccatta 107640

acaaaaatct tcctaccgtt gttatgttgt caaaagttct tatgttttga gcactttcga 107700

gtaaccatat gttattttca aatatacttt tgtaaactgg atttctgtcc acataactct 107760

ttaaaaatag atttactgga aggccgcttg ggttatctcc ctgtataggc ggcgcttgct 107820

ttatgtattc gcgtaacaaa tctcttacaa cttttctagt tcttctaggt atacatgacc 107880

tattattcaa aggctttgtc cagttagcgt ttataaacgt tgtaaagtca ctgactagct 107940

tctccattta taattaaata attacagacg gcaacacagc ggttatctaa tatctgctgt 108000

atcctgtctg tacatctatt tttctgttga gatcaagaag agctttacgt agactctcca 108060

agtgtctttc tagtctgtct aaccggttac ctgtttctct gcagcaatca gttatagttt 108120

tgtaactgtc taacaagctt acgaggcgct cttccacact ttctttagtt ggagctccag 108180

ccgcgtacac tccgttggtt gaattgcctg tatcatcagg ctgagtcaat aggttttctc 108240

cgtcattttc atccatattg agtccaacga acacaaacga gtaagtgttc ctctatttaa 108300

agtattgatt ttagaaaaag gcaagcctcg ctgccctgat tcggcggcaa acacgggttg 108360

aacacgcgga agtcgctcgc ggccgtgaag atctcgtccg cgcacgcctc cacgctcgcg 108420

aagcgcccag gcgagacgcc gtcgtgcgag cggaacccga actccgaggc cgccaccgcc 108480

gcgcccttga agagcacgca gcgccacttc ttgcgcacgt cgaaggcctc gtcgttgggg 108540

tcgaacacgc gccggtccac gcgcgggccg cccgccgtgc gcgcgaactc cagcgccgcg 108600

ttcgccgcgt tgaactcgcg gatgttgtcg tagttctcgt agacggccca gagctgcagc 108660

gccacgaaca tggcggccgc cgccgcgagg gccacgcaga gcgcggacac cgcgtccatc 108720

ttttatgtgc agaattattc gttggcgcgg agctcgcgca gctccgcggc gcgcagccgc 108780

gcgaaggctg ctttgagcgc gcgcagcagc tcctcggtgt ccgcgcgcag catgtcgaag 108840

cggtggtagc tgtccaggcg cgcgcggcag ccgaagaagc gtgcgacgca cgcggtgacg 108900

atgtcgttca cgtagagcac gcccgaggcc gtgcagtaca cggagcgcgg ctcgcgcggg 108960

tccggcggca cgtccacggc gaccgcgtgc gcggccacgt cctcgagcac cttgcgctcg 109020

agcacggcga ggaagtcgcg cagctggcgg cggttgtcca gccaggcgta ggtggtcgcg 109080

aagagcgtga gccgcccgcg cggcgcgatc gcggtgtagg gcgcgtaccc gcggaactcc 109140

cgggggtgca cgaccttgac gttctcgtgc tcgcgacgga aggcctcggt gtcgagcagc 109200

gccgcgagcg cgtccacgag cttgtcggag acctccacgc ccgcgccgaa ggcgatgagc 109260

tcgatcttct gctcgctctt ggggcggaag tcgtggaagg tgtgcagcag catctcgcgg 109320

agctgcggcg gcttctcgac ggcctcgagc gcgtcgccgc ggacgaggaa gtagtcgagg 109380

tcgtgcagcg agacgtgctg cccggcggcg ctctgcacaa acttgaggaa gacgcagagg 109440

cccgcgcggc gctcgagcac gtcctcgacg tgcgcgtgga atacgtgccg cgagggcatg 109500

gcctcgatcg cggagagcca ctcctcgttg acgcaggtgg tggtgttctc cagcaccacg 109560

ccctgcgtga gcgcgggcca ctgcaggtgg aaggcgaact cgtgcttgat gagcgaggcc 109620

acggccgggt ccaggtccac ggccagcgcg gcctcgccga cgaggggagc gtccgccatc 109680

acgcggagga cgcctggccc atctcctttt tcgccttttt attcaggatc attattcttt 109740

cgttgacgag gtccatgagc atcttgatgg cggcggccgc ggccgccgcg tcgccgccgc 109800

acatctgcgc gatgcgcgtg agcatgtgca gcagcgcggc ctcgtttagg tcctcctcca 109860

tttagaggcc gtgagggcgc gcgtcgtcgc gacgagggga cgcctcccgc ggcagcgtgg 109920

tgcgcacggc gaaggcgagc agcgcgccgg cgcactgcgt gagcacacac tccgcgagcg 109980

cgacgaggag ctcggagggc gcgagcacca tttagaggcg cgcgcgggtt taattgccgc 110040

cgtcagagtc ggcatcatcg cccttgtcgc cgccgtcctt gcagtcgccc ttggtctcgg 110100

cgtcgacgat gtcggcgagc cgcgtcttca tgtgcgagaa ctgcgcgagc aggatgccgg 110160

ggtcgagaca gcgcttgacg acgctctcgt cggcgaagtc gtagcagatg cgctcctggt 110220

tctggcagaa caccgagtct tcgatgatca acaccctcct ggtcccggcc gaccgcatga 110280

tggccatggc ccggatgagc ctcttcttcg atccgcgtat ggacatggac cggagcacgt 110340

tctccacgtc ggagtcggag acgttgcagc agcagaggtg cgtgatgctg gcgcgcccgt 110400

tgacggggat gtgcttgtag gtctggcaga gcagcaccag cgacacgttg atgtgccgcc 110460

cgtagttcat gaggcccaag agtgtgggcg accgcgtctg cgtgtcgccc atatcgtcga 110520

gaatgatgag gaacttctgc ttcttcgtct gcgcgtgccg ctcgatcttg cgcttggcga 110580

ccgagaggtt gtactcgagc tcctcgtgcg tggtgacctt gtggatgtgg tccggccaca 110640

cgaagccgtc gtaggcggcg ttgtagacgg gcgtgaagag caggatgtgc ttgaagcggc 110700

gcacgagcgt gcggaagagc gagagcaggt aggcggtctt gccggagccg gagccgccga 110760

cgagcgccat cctgaagggc gcctcgatga gactctcccg cttgaagcgc acctcctgca 110820

cgacatccat cgtatattta ctgtcactaa attaccggct ccgagaaata tagaaattag 110880

agcctcctag agcacaccga ggcgcatcgg caagatggca cataacacgt tcgaaaacga 110940

tagcgagtcg gcagctaaca accagtacgt ggcgtcagtc aagcgccaga aaatgattcg 111000

gcgatacatt aagatgttct tccggttcgt tacggcgata gctatcattg tcctggctat 111060
tctagttgtg attctgtcgc tatctctaga cgaatgtctg cacagagaac accctcatga 111120
ctattcacat gtacaaaatt caacatgcga cggcattact ttaggtggtg aaaagtgtct 111180
cagacttaat ttgccagcaa cgtgggaaga tgctaataga caatgtggta atcttgggtt 111240
ttacctacca tctactggcc ttgaaaagaa atttccttgg cttgtgacct atctcgacgg 111300
aacttgggga aacactcaga actccgtatt tggaccaacc ggtgacttgc agaatgtcat 111360
aggaccgaaa gaatacaaat atttttgtgt gtccgattag atgattataa tctaataaat 111420
gggttgctgt aaggtcccta accgccagtc tataaggact ttgaaaaagg cgtcctgtcc 111480
ggtcgccagt ctcgtcacca ttctctccct agtcaccagc ctcggtgcga tagtcagata 111540
caccaatttt tttctaaaag aagcatgtga cgaaggatgg atgcccataa aaaacatatg 111600
cattttaaac acgcactttg aagccaccaa tgacgatgcc cacaggatat gtgaaaacct 111660
agacggaaag ccgccggcca tccctaaccc tactctgtta aagggtgtca cagttctcac 111720
cggcgaaaag aaattttgga tgacccatca cgaagactat actactgtgt ttgagcatat 111780
agacgatagg acgactccta aaaacacaga ctatgacagt aaaaaacaca cttgtttgat 111840
gagcgaggac ggattgatac accataactg catgatgaac gtgactgtgg tatgcatgaa 111900
ggagatgcac ggataactga aaatatactg tttgaacgca aagacgccat gtcgcgactt 111960
caaatactga cctcatttgg acaaatcttc gcacccgacg aagctcggct gcgcgagatc 112020
gcgcgtgatt tgggaatatg caccataaaa cgcgcattcg gcgacatgct gtacggcttt 112080
atagacttcg acccggtgcc cctgacccaa gtaaacatgc tcatgtccaa ctgctacttc 112140
gcggtcaacg gcaacctgct tccgtgcacg gaggacttcc ggctcagact cccggcaacg 112200
gagatctctg cggcctacct gacgagaacg ggacggacga tcctgtgcgg caaagacttc 112260
aacatagtag cgccgtcggg gttcaagccg tccatgcggc tgcgcgacct cagtcacgtg 112320
tctgcgcttg tagagatcct ggaagtctac gacgagtccg gggagtacca attcgtgctc 112380
ggccccagcg cgcagttcat gctgcggctg atggagaagg agaacgtctg tctgttcggc 112440
agcgggtggt gcatagtgga cctgcgcaag ctggacgtac ccatataatc agcatccttg 112500
tttttatcct gtctttttat cagtttttta gctagttaaa acataaatag taaagctaaa 112560
aagaggagtt ctggagtctt gcaacaacca ggatgaaggc ggtgttgttg ctggcgttac 112620
tgggagcgtt caccaacgca gcgcctttgt tagaaagcca gcgttctaac agtgaggaaa 112680

aagcaaattt ctgctcgacg cataatgatg aagtgtacgc caggttcagg cttcagatgc 112740
gcgtgggtgt acgacacagt ccgctctaca ctcccagcaa catgtgcatg ctggacatag 112800
aagactccgt tgaggacata gaagagtcca cagaaaaaga atacgcgtct acggccacgg 112860
gtgaggcggc cggagtgaac gtgtcagtgg cactagtggg agaaggcgtg agcataccgt 112920
ttagttacat aggccttgga ttcaacccgt cgcttgaaga tagctacctg tacgtcaacg 112980
tctcgtcacg agctccttgg gttaaacaga cttcggacct atccgcgaac ggcggctggg 113040
gtatcaaaca ggttctagaa aaagagttac tggccatcca aatagggtgc gacaaccaaa 113100
aatttcccga agaacccaca actacacccc cctcacctgt cactacaacg ctttcctcaa 113160
caactccaga tctgaatgaa gaaaacacag aaaatacgcc gacgaccacc ggcgccagtg 113220
tagacagaaa gcgcaatcca gctgacattg acttctcgct gctcgtggac ccccgatgcg 113280
tgacctctgt agacctgcac gtcgagctca gggacgcgtg catagactac aaacaagagt 113340
cgccgttgtc gctgaagggg aaatatggag acggcgaact agtaaaaaag gagattaaag 113400
acgtgggaaa gaatcacaat atgtgcagtc ttaacctcaa ccctggcaat tgagctgttt 113460
ttattcggca atataatagg tgattattga acattaaaca aaacttatcc cacaacgccg 113520
caacaatgga agtgctggtg atcatctcta ttatcgtcgc cgtaatatgc ttgaccggag 113580
cggtaatgta cctccttatt gaactcggct tagccgccga gcgcgctaac aaacgcgcgc 113640
gcgtgaagaa aaatatgcgc aaattagcca ctcaattggg aaatggatct gtcgactccg 113700
gcataggcat aggcccgtgc ataatgtcgc gcaccatgga ctctggaccc agtcgctggg 113760
acagcgacag tgaggatgac ggggacagcc tgtccacgac gtccaccagc ggagggggga 113820
ctctcacccg agtgtgggtt gggagcgccg ggcctatgta cgaaaacttc tgcgggaacg 113880
gcacccgcca ctcccccacc aacgaccctg gctaccactc gcgggagact ctctgcagcg 113940
gacctccccg tcaggcgccg gcgctaccgc ccaccccgaa gcccgacgag gtaacggtgg 114000
acgtggggcc cggtcccgac gaccaacacg gtccgtacga ggaacctgat cccattcccc 114060
cgcaggaacc cgagccgccg gtgcagattg aggtaaccat caacgggccc ggtggagaag 114120
gcgaggcgga aggagaattt ttctacgacg agtagccgcc aaaactgaat aactatcggg 114180
cttcgtaaac gcgcagacat gccgctgttc cggaagctca tggtttcgcg ctccctggtc 114240
aaggaatgtc tgactctgga cttccggcag ggcgagcgtc tccctacgcg atgcttcctc 114300
ccggtgcccg cggggacgac attccacaga gtctgcgaca cctcgccgct gacggacgaa 114360

gtctcccggc acgtgcagga gcccgtcatg ggcaccggac gggtccagta ctactacttc 114420

gagagcggcc agggcatgat cggcgacaac gcgggcatgg cgcgcatgct cgtgtgcacg 114480

cggtcggcgt acaacggcgg cgacgtcgtc gtgcggtcca cgcggagcag agcagacaag 114540

accgtggtcg cgccctgcca gggcatggcg ctgctgctga gccccttctg cgccttcgac 114600

atcacgccgg tcgagagcgg ctccgcgata ttcgcggagg tcatcgtcac cgcgcccagc 114660

atggactacg tcgaggcggt caccggcacg ggcgaggcgg ccgtgcggat attcaactcg 114720

caccacccgc tctggccgcg acacggctcg aacgtctgct tcgcgctgcg gttgctgcga 114780

gacgtgcgca cgggcgagcg cgtggtcgag cagatgttca tggacgggcg ctggcacacc 114840

gtgctgagga cgtcctgcgg caacaaggtc tgcgtgcccg ccgacctcgt gggccagacg 114900

aacctcgagg aggtgccctt ctgcgacgtg acgcccgaga tcatgcgccg cgcgctggcg 114960

atcgacccgc cgtacgaggc cgtggcgcac ccgcaccgct gcgtgtacgg cgccatggac 115020

gtccggtgcg cgaacgagta cctcgtgtac tgcaccttca agacggagcc gacacggcgc 115080

agcacgtcct cgccgggccc ggacagcccg ctgtcgcccg cgactccgtc gacctcgcgg 115140

gccgcggccg cgcgcgtccc cacgacgccg caggaagtgg cctcgccgac cacgaggctc 115200

ctggagacct gtctgcgcga cgccctcgac ggactctgac ccgaaggacc caccgtccac 115260

tcacattcca ctgccagaca actcaagctt tttctgcatc tacctcgcta ataattgaat 115320

tgttatagga caaacaggcg cactcgagca caatggcgtg ttttatcgaa ttgttagact 115380

ccatcttcaa ccgacgccac cgtaatttcg ggccggagga catgtacagg ccctctgacg 115440

ccccgccccc caaatcgcac acgcctcgca ctccccgcac cccgcggacc cagtgtcccg 115500

gacacccgcg gcgacaaagc tcctctccca tctacggcgc ttatgtggac tccctgccga 115560

ggaacagaaa gcggttccag aatcaacaca gttgtcccgg agattacgag cggtgtcaac 115620

tctcggacac catcagcctg gatgcgacgc tactcacggt tacagtgacg tccatctcca 115680

gcatatccag ctctagtagc tcagactcta tctctctggg gcagtgcaga ctgtccatgg 115740

tgtccgcaac atcaacctcc acctccacaa ccttctcctc ctcggaatga gcgccacact 115800

tatttttgta taatagtttg tattgaacct tagagacatc cacaaatagt taggaagcat 115860

gagtagttca agtagcgaga ccacccctaa gcccaagccc atccctgctc ctcccatgac 115920

tcaggaggag tttaacaaag aagtgaagaa acgaaaagaa cagaaaaagg aaaaatctag 115980

aaccgttgaa cgtgagtcag aaaccgtaac tgtatcttcc gacggatcag agataaaaaa 116040

gacttacgag cgcgagtctg agagaacaac cgaaacagaa aagaacaaca cgtcaaccga 116100
tgataataag cagaacaccc ctgtagagaa accagaggaa accaagcctg cttctactcc 116160
tgaaggtgtg aagccagccg agactcctgc cccgactact gacccccaac ctactacaca 116220
accacccgca gaatcaaacc ctggaagtca acccgcacct gcttcagaac caaccccc gc 116280
acctgagcct gcaccggaac ccactcagcc tgcatcagta actcaacccg ctccaacacc 116340
agagccaagt ccagccccta agcctactcc ggcttctgaa ccaaccccag catctgagcc 116400
tacttctgct ccagaaccta caccatccgc agaaccaact cctcaaccaa ctgtagaaac 116460
accaccatct gctccagcac caactcccga ggcccaacca cccgccaaca gcaatcccac 116520
tactgaaact accactggta ccagcacctc ctaagtgagt acgtaagcat ttcggagtaa 116580
cgtcgtagca agcgctagtc cgccgcgagc ggttctcgca agttttttcg ggtaaaaagc 116640
gtacaccgtc gccttgtcgc ggcggtgtac gcttttttca cgccctttttt gcaaaattta 116700
aattgtaccc gcgccggctc taggaaagat ggcgtgcctc agagtgttcc tggcggtgct 116760
cgcgctgtgc gggagcgtgc actcggcgca atggatcggc gagcgcgact tctgcacggc 116820
ccacgcgcag gacgtcttcg cgcggctgca ggtgtggatg cgcattgacc ggaacgtgac 116880
cgccgcggac aacagctcgg cctgcgcgct ggcgatagag acgccgccga gcaacttcga 116940
cgcggacgtc tacgtcgccg cggccggcat aaacgtcagc gtgtccgcga tcaactgcgg 117000
cttcttcaac atgcgccagg tagagacgac gtacaacacg gcacgccggc agatgtacgt 117060
gtacatggac tcctgggacc cttgggtgat cgacgacccc cagccgctct tcagccagga 117120
gtacgaaaac gaaacgcttc cgtacctgct ggaggttctg gagctagcga ggctgtacat 117180
tcgcgtgggc tgcacggtgc ccggagagca gcccttcgag gtgatcccgg ggatcgacta 117240
cccgcacacc ggcatggagt ttctccagca cgttctacgg ccgaaccgcc ggttcgctcc 117300
ggcgaagctg cacatggacc tcgaggtgga ccaccggtgc gtgagcgccg tccacgtgaa 117360
ggcgttcctg caggacgcct gtagcgcccg caaggcgcgg acgccactct acttcgcggg 117420
gcatggctgc aaccatccag atcgccggcc aaaaaaccca gtaccgcgcc ctcagcacgt 117480
atcgtcgccg atctccagga agtgcagcat gcagacggcg cgctaagggc gctcaccgcg 117540
ctgacggcgg ccgtggtgtg cgcgatcgcc atcgcgctcg agcgcggggc ggaggccgac 117600
gccgtggacc ttatccttat aaaattttca atgatatgct agtttttatg cgaccttcct 117660
tagaaaattc ggaattcaaa aatgaaataa aacggcgttt agcacgcata ttattaatac 117720

cgaccaccat ggcaggcgtc cgcagctgcc agaagaaagt cccttctact gcgggctcca 117780
tgtcatttca acggggcaac cggagcatcc agccggcgat gtccgaggcg ttgcagaatg 117840
atttcagcta caacccgcga ccgcctccgc cgagcgcaga agagattgac ttcttctgcg 117900
tggacatgcg caaagtactg atggaaattg aggccaagcc cagcagctcc aagtaccccg 117960
atttcatcca cccggttgac agcagcccgc cgtgcacgcc ggcgcgcaag cgcaacggct 118020
tcggccgcaa ggcactgaac aaaacccagg tgccgcagca ggccaagcgt gacggctact 118080
cccgctaatg cagtccacac acttcacaca ctacatcagc actcaagctt ataatcacta 118140
cacaatgaat cagcccacca cgtgcgaagc acacacataa aatcacccac ctgtcctgat 118200
cgttcccaat actcccaatc accgtgcttt acacgcacgt taatcaccct ttccttcctt 118260
catgcgttcc tgatcgttcc tcctccttaa tcacacacac accccgtaat tttgtacttt 118320
tgtactttaa tttgtacact ttacacactg actttgtact gcctttgtac tttatttttg 118380
tactgaaatt ggacgatact tatctttgta ttcacatcca agttttgcaa attccacagc 118440
cggtagcgaa aagtgaaatc gtaccgtttt aggcttcgat ccccctcccg cgcgaagact 118500
cgccagcatg gactctcgta ggctcgctct tgccgtcgcc ttcggaggcg tcctcgccag 118560
catgacacag cgccgccgcc tggcttctct catcgccagc atcggccaac ggctgatggg 118620
cggcgacggc atgcgtcgcg tcgccgttcg gttgatcgac cagctcatgg ccggaccccc 118680
ggacatcaac gacgaggcct tccagcgcga gatccgcgtg ggcgagctct tccaggcgct 118740
ccaccgcgtg gtcgagcagg cacgccgaga gaagtacttc gaggtctgcg gcgccggcaa 118800
cgacgccgac gcgcccgtcg tcgagatgga caccgcggcc gcaccccccgc agccccagcc 118860
cgcgcccctc gtggtcacgc cgcagaacgc gttcatgttc gtgccgcaag gcagccacgt 118920
gcacgtggac gagagcgtgg acccgttctt cggcatgagc ccctccatct tcgggcgcga 118980
cctcccccct cagccgcccg aggagctgct gagcgactac gacccgctca tgagccaggc 119040
cggcgagccg ccgagcccgc ggtcgccctg cgaggccgac ctctggtgct tcgagacgct 119100
cggcgacagc gacagcgatt gagaccgcac cacaccctac ctcacccacc ccacactcca 119160
cctcacctca ccctaacacc cgacacccaa cacttcaacc ggacaatgaa ggagtcccac 119220
atttcactga aggacgcgga tgaagccgca ctcccccaca tgaaggattg gcaacggtca 119280
aacatttcac ctgcaatgaa ggacgatgcg cggtcgcatt ggcctgcgac cgacatcgca 119340
cacatgaagg acacaattgg tttgttaatc cggacaatga aggacaaatt gtttttgtta 119400

atcaggacaa ttagaacaca atcaaatttt tgtacgatca taaaatcgat atttgatgca 119460

catatattag taagtatatt agactaaatt ctccggggag gcaagcagtt ggatacggcg 119520

gggcggggca cgacgtgcac ggagagttcg ggcgggtccc ccttcccccc acccccacgg 119580

caccacgatg cgcctaatct tagcgctcgt ggcctgcttg ttggcggcgc cgatgccgtt 119640

atcgggtcgt tcgacaagca ctccaaacac gtccccctcc gcactcggct cgacgagttc 119700

ggaaccaagc tcggaagacg ctgtggcttc gagcacaacg acaagcacac tcacaagcac 119760

tacaagcaca ctcactatgt ccacaagtgt ggacaccact actacctcgg gcgctacaac 119820

gtccgcaaac agcactcctg cagcgagtgt gagctcctcc acacccgcaa ctaccgaggc 119880

atcgacggca ccaacgacgc cgtcgacgcc gacgacagtg aaggtaacga agggcaagga 119940

agacacgaag gcgtctgcct acctcgtttt actaatcacg ttcatggtca tgaccacgct 120000

cgtgatggtc gtggtcgtgg tcgtggtcgt gtacaaacag ggactctgta actgctgctg 120060

taagtttccc ctgctgcaaa gagctcaagg actacctcga cgaggaggag agcgccgggc 120120

tgtacgacgc cttgacgtgg agccactcag actccggcct ccggctcgtc gtgcgcgcgg 120180

accccagatg atgaggatcg gataagatcg gcgtgttttt cccgcccgtc gcgaacatta 120240

tgcctctaaa tgccgagaat taactgaaat tcaaacacgc tttgggactc aactccgtga 120300

cccacactca accatggctg gcttcctagg cgcattcaga ggcgtgtgct ccgacttatg 120360

gcagtcgctc cgtggacacg gacaccactc ttccagctgc ccgcgacgac gcgccaacag 120420

catggacgac cgcgaccggc gccgacaccg ccaccgcgag atccccaaca gctcggcgtc 120480

gctgaacagc gacccgatgc cgcaacgcag tgcgggtgcg cgccggcact acgactgccg 120540

cccctcggaa aagagcagac actcctccga caagcaccac tcggcggacc gacaccactc 120600

ggcggaccga caccaatcgg cggacaggga cagacaccgt cgcagtcgca agaactacga 120660

ctcgcacccg tcgcgcagga accgcaacta cgagcgggcg gactaccaga gacaccccctc 120720

acaaacccac ccagacgccc ccgcgcagac ctcgacgctc aaggtgacct ccctaagcac 120780

cagctgcagc accctgtccc aacatcacta cgagaccccc gaccacatct acgacatccc 120840

ggaagacagt cgcggggcgt cggctccccc tcgcgcggac ctcgcgctcc ccccgctcgc 120900

catgcccaaa tccaagccgc gtcgcacgcg cccggcgtcc atgaacgact gcctgatgaa 120960

gcactgcggc gccggcagac ccaacctcca agacgacata tgcacactat gtactgatat 121020

agagacacag ctgagcgcac tagagaagtc tctggagtca gagctcaact tctatcgtcg 121080

ctacatacaa gacactaaga cattgctcgc cacgcgagca gcaaacatcg gcagcaaagc 121140

tctgatctac accgacgact acaacggcag tggcgacgtc ggcgaaaagg agcactgctc 121200

ggaggagtgc tgcaaagtgg aggaagttct gtgagaaagt gcgtttttct gtaatgtgaa 121260

ataagatagc cttatgtgtg cacagacatg gcgaacaggc tcgtgttttt cgaccccgag 121320

accctagccg aggccgacgg catccccggc tatggggtgt tcgagcccgg caagaagaaa 121380

tgcatcttca caaagatccg caccagcgtc gcactcgcgt gccggtacgc cgtctcggac 121440

ggcggcctca tcgacgagtt cgtcatggct acatacggga ccagacgcgc gtgccggctc 121500

gtccggcacc tgacgatagg cgcggagggc gtgatgaccc ggcccgccag caactgcgcg 121560

ccgcacatgg tgctcatctg cctcagaggc gtggccgccg tgtccagcga ggacatgggc 121620

ttcggtcgct gcatcatgga gcgcggcacc atgttcatgg tcaagtccgc gcacagcgcc 121680

gtcgtctgcg gcaaccccgc ctgcgagctg ctcgtcctct tctacgacta cttcaccccc 121740

atcccccggc cgctctccgg agacgaggtg ctgttcaccc gcgacctcgc gcacgtggac 121800

tacgcccccg agtcggcggt cgtcttcaag atggattaca acctcgagac cgacgtggcc 121860

acgctgtttg tcggggggta catattccgc gccaagggcc tgatgatgga gacgcgcgaa 121920

caagtgggcg acgagtgcga ctgctgccgc cacagctcgc cggtgctcgt catggatcgc 121980

gagaagatga tgtcgtcgct gcgcatgatc cccagcatcg tgcccggcca gcgggagatt 122040

tggcttcgcg agcgcggctg ggccgtcctc gagacggacg cccgcggaca ctgcgagccc 122100

ggcgtcctga ggctggcgct cgccggcctg cggctgttcg caggatgcct gcgctccgtc 122160

gtggggcggc gcgagctgtc gctgttctgc tacggcgtcg ctcccaagtt cggcggggag 122220

ttcgaggacg cgccgcgccc catggagatc gacggttagt tgtttttatc cctgtacata 122280

cgccgcaaac tgaaacttta gggcaccgcg taatagtgca cgaacgccca gtggaccgct 122340

tccgcagcca tggaaaacaa cgacggcaac gaacgcaaca acgaacaccc gcacgttcga 122400

gaattcaagg aggcgtccct gtacgggttt ctggtggcgg ccgcggacgt gaccgtcgaa 122460

gacgtgcacc ggtaccttca gttcggcgcg gacgtgaact acaggggcgc gtacctgtgc 122520

acgccgctgc acgcgtacct gcagtccggc tgcgaaaagc gcctagacgt cgtggacgcg 122580

ctgctggacg ccggcgcaga catcaacgcc aaggagatct gcggtctcac gcccgtgcac 122640

ctgtacgcga gctacgcgga cgtggacgta gagttcatgc gcgggctcat cgagcgcggc 122700

gcgagcgtgt gcggcgagag ctcggtcacg ggctgcctgt actcgtacct gtacacacac 122760

agcgtggacg gcggcgcgcg cctggacgtg gtcgagctgc tcgtgcaggc gggcgcggac 122820

gtgaacgtcc gcggcgaggc gcgcaagacg ccgctgcacg tgcactgcgc gggcttcgag 122880

gtggattcgg acatcgtgga tctgctgctg cgcgcgggcg cggaccccga ggcgctcgac 122940

gaacacgggc tcacgcccgc ggacgtgctc gtgaagtccg tgggcgccaa cgtggagacg 123000

ctgcggctct tcctcgacgc gggcgtgagc gtggccacgt cgcgcgacgc gcgcggacgc 123060

acgccgctgc accaccacgc agactccttc cgggcgagtg cgggcatcgt gcgcgaactg 123120

ctcgccgccg gctgcgacgc ggcggccgcc gacgacctcg gaaacacgcc cctgcacagc 123180

ctcgccacct tctgctcgtg ccggcgctcg gtgctcgacc agctcatcgc cggcggcgcg 123240

gacatcaacg cccgcaacca ctacggccac acctgtctgt actacgcgtc catctacaac 123300

ccctccgtct gctcgcggct catcgccgcg ggtgcggacg tgaccgcgcg cacgccggac 123360

ggacgcacgc cgctctcggg catgatcatg cgcaagcaca cgcgcgccgt gcgcgccgcc 123420

ctggcgacgc ggcctcccgc ggacgccgtc gccgcgtcgc tggacgtcgc agtacagccc 123480

gagcccacgg acgccactcg cgcgtgcgtg cggtacgtgg tgctctgcgg cggcacgctc 123540

tcggcgcgcg tgcggtcgcg acacgcggac ttcgtgcgag agtgcgaaag cgaggtggtc 123600

gtgctcagaa ccaccgtggt ggggctgccc ggcacctcgc tgctggacat cgtgcgtgcg 123660

gcgcagccgc cgccggtact gctctccccg cgcgtgcacc acgtgctgca gaagctgtgc 123720

gtgtacgcgg agttggtgga cgcgcggctg cgcgagatgc ggcacaagac caacctcgtg 123780

gacgcggtgt cgcggctcgt gtgtccgtgc gcgctgccgc cggaggtggt gcgcggcatc 123840

ctcgtgcacg tgccgataga cagcctgcgg cacacgttga ccctcggcgt ggcgcaggcc 123900

tcgcgtttcc ttccctcgca taaatgaaat attatttttt gtggtagacc ggatctcccc 123960

gatggacccc gccggacaac gactgcgcgc gccagggccg tggcgcctga acccgccgac 124020

cgcggccgcg ctggaaagcg cgctgctgcg gcccgcggcg tcggcgggcg ccgaccgctg 124080

cgcgaacgcg cacgtggacc gccgcaacat gggcgtcggc gagggccgcg aggtgcccgc 124140

ggacgtcgag gggctcatga ccgagatcca cctgcggtac ggaatgacgc gcgtccaccg 124200

gaacgttcac ttcgtgcagt tctggcacgg cgagcacgtg cgccggcgcc ccgcgcgaca 124260

cgtgttcacg gtctggatct gcctcagcgg cgaggtgcgc atctacgcag agtgctgcca 124320

ggcggggcac ggcttcgtgc tctgccgaca gatggcggca gggtacatgt tcgtgaccga 124380

acccacggac tcggtcacgg tctcggtgcc gcaccgactg cgcaactcgc ggtcgccggt 124440

gtggctggcg gcggtcttcg ccacgcggca cttcgagccg ctgccgccgc ccatgtacgc 124500
cgtgcccggg cacgtggtgc tcgcgcgcag cgcctccatg ctctgcgact gctggccgtc 124560
ggacccgcgg cgccgcaacg tgatcttcta catgcggctg tcgggcgcga tggtgcgcgt 124620
ggtcgtgccg ggcgcggagc tcgagatcga gtgcacctcg ggttccggc cggaccactt 124680
ctccatcaac gacgagtgcg tgtgctgcga gcgtccgcac gtcgcgcgaa ccgcggtgtg 124740
gacgctggcg gagatttgcc gcggcgccac ggtggtgctc gcgccgccac tgccccgcga 124800
ccgcgccgcg gggctgctcg cggagatccg cctggcctcg ctgcgatggg tgcgcgtgcg 124860
tgcggtccgc agcggcagag aaagcgtggg cccgttcccc tcggtggtgt gggcggcggt 124920
cttctccgcc gttcggctct tcctggacgg aaccgtgcct gccttcccgg cgtgtgtgga 124980
gaatggacgc gcggcgtacg gcatggtgta cgtgcccccg gaggagccgc ggatggacgg 125040
gctctgtgtg ttcccgacgc ccgccgagcc ggcggcgctc ttcgtccgcg gagaccaggt 125100
gcttgaggcc ggcgcggccg ccgccataat cgcggccgct gagaagcgcg tccaggccgc 125160
caatgggtct cctgctgccg cggaggagga cataggtgcg gcggccgatg ccgccgcaga 125220
gagcgtggag caggaccagc gcgtcgagtt cgaccttggg cctgggcctg accccagcca 125280
agaagcgccc gcggacgcgc agcgtgccga ttcggacgac gacaccggct ccgagaccga 125340
gaccggcgac gagagtgtgg gcggcgagga tgacagcgac tcctcctcct cttactcggt 125400
gatgtcggac gacgaaaacg acagcggcga cgagggctgg ggcgactcta gcgactccgg 125460
catcgaggac gacgacggcg gtgtcgccag gccgccgagg aagaagagga ggaagagcgc 125520
gacgtcctcg gcgcagcggc ccagatgctc ggagactgac cggtggtgaa aacataaaaa 125580
taaactgttc aacacttgta ctccgggcac caacactact atccataccc accctccctc 125640
cacacactac aatggcaaac agagaagaga ttgacgcctc cgccgtcatg gctgcctacc 125700
tcgcgagaga gtacgcggcg gctgtagaag aacagctgac gccgcgcgag cgcgatgcgc 125760
tcgaagccct tcgcgtttcc ggcgaggagg tccggtcgcc gctgctgcaa gaactctcga 125820
acgcgggcga gcaccgcgcc aaccccgaaa actcgcacat ccccgccgcc ctcgtctccg 125880
cgcttctcga agcccccacc tcccccggcc gcatggtcac tgcgattgag ctctgcgcgc 125940
agatgggccg ggtatggacg cgcggccgcc agctcgtcga attcatgcgg gtcgtgtacg 126000
tgctcctaga ccgtctgccg cccacggccg acgaggacct cagcacctgg ctgcaggccg 126060
tcgcgcgcgt gcacggcacg cggcgccgcc tgcaccgcgt tctcggcgtc ggggccgtca 126120

tggcaggcgt cggtatgctg ctgctcggcg tgcgcgtgtt gcggcgcaca taacttttta 126180

tctcggctca aactgaaata cgacattgga ctacgaaacc tatgattttg ctcacggccg 126240

cgcgagatag gataataaat aacctttgag caactaacat ggccgatgag agagaggccg 126300

acggcgcgct gttccggtac ctggagagcg aggaccgtcc ggacgtggag cacatgcgcc 126360

ggctgctgga cgagggtgcg gacgtgaact acgcgggccc gcgcgggtac gcgccgctgc 126420

acatgctcat gcgcggcaac ccgctagacc ccgacgcggt gcgactgctg ctcgccgcgg 126480

gcgcggacgt gaacgcgaca tcgctctgcg ggttcacgcc gctgcactcc tacatgtgct 126540

tcgggaccgt gacgccagac acgctgcgtg cgctcatgcg ccacggcgcg agcgtcagcg 126600

acctcgagcg caacatcaac gcgctgatcg agtacttcaa ccgcgacggc tgcatgggcg 126660

gcgcggaggc gaccgtgatc gcactgctgg tggagcacgg cgcgcacgtg aacgccaaag 126720

acgaccttgg acgaacgccg ctgcacatct acctgtccgg cttcttcgtg tcggcaccgg 126780

tggcgctcgc gctgatcgcg ctcggcgcga acccgaacgc cacggacgcg tacgggcgca 126840

cgccactgca cgccttcctg cgctcccgcg acgtggaccc cgctgtgctg aagacgctca 126900

tcgccgcggg cgcagacccg ctcgcgcgcg acatcatccg gcgcacggcg ctgcactacc 126960

actgcgagtc cttcaagacg cgcgctagtg ttatagagac gctggtggcc gccggctgcg 127020

accccgcgag cacagacctg ctcgacaaca cggcgctgca cagcatggcc atgggcagct 127080

cctgccgcgc ctcgctgatc cgcccgctgc tggccgcggg cgtgtccgtg aacgcgcgca 127140

acgcgcggct gcagacgccg ctgcacctcg cggccgtgtt caacccgccg gcctgcgcgc 127200

ggctgctggc cgcgggcgcg gaccccgcgc tcgcggacct ggacgagaca acgccgctgc 127260

tgagcatggt gcggcacaac tgcgcacgcg cgctgcgcac ggcgctgccc ttggcgccgg 127320

acgcgctggt ggccggcgcg gtgaaccgcg tgaacgcgcg cacgccgagc gcggccacgc 127380

gagagtgcgt gatggcgctg gcgctgcgcg gcgcgctgga cctgctgagc gcggagagcg 127440

ttgccaccca cgcggccgcg atccgcgcct gcgaggcgga ggtcgcgctg ctgcggagca 127500

cgcgcctggg cgcgccgccg acgacgctct tcgcgctgct gacaggacga ccgaacacgc 127560

tggtttccgc gaaggcggcg cgacgcgcga tggcggacgt gtgtgtctac cgcgcggcgc 127620

tggccgcgcg cgtggagcgc gtgcgccgca agtcctcgct ggtcgagcgc ctcaccgcca 127680

tggtgtgtcc gtgcgctctg ccgccagagc tagtgacgcg catcctcgcg ctcctgaccg 127740

tggaggaact cgcttgcgca atgcgcaaat aataatgaac tataactagg cttattagag 127800

gcactatttg tgcagagtcg ttagttatag ttagtgtact taccattgga atgtcgaaga 127860

acaaaattct ggtgtgtttg gtaattattc ttacttatac attatacaca gatgcgtatt 127920

gtgttgagta tgaggaaagt gaggaagata aacaacagtg cggtagtagt agtaattttc 127980

ctgcgagttt accgcacatg cttagagaac tcagggcagc gttcggaaag gtaaaaactt 128040

tcttccagat gaaagaccaa ctgaacagta tgctactcac acagtcgctc ctcgacgact 128100

tcaaaggcta cctcgggtgt caggcacttt ctgagatgat acagttttac ttggaagagg 128160

tgatgccgca ggcggaaaat cacgggccgg acatcaaaga gcacgttaac tcgctgggag 128220

aaaaactcaa aacgctgcgt cttcgactgc gtcgctgcca ccgcttcctg ccgtgtgaga 128280

acaagagtaa ggccgtggag caagtcaaac gtgtgttcaa catgctgcag gaacgaggtg 128340

tttacaaggc catgagcgag ttcgacatat tcatcaacta catagaatca tacatgacta 128400

ctaaaatgta aaaatgtata caacttttag ttatcgttcg gattctcgta tcgttctgca 128460

tactatgtat ataaaatgta tattaacata gttacagtta cagttacagc tatattttta 128520

tgctcacaag atgctatata attgaaagga aattgttcac tctctgtcag ggcgccatgg 128580

actttctagg cgccgcgctt cacgactacg ttgccgatgc gcccaaggtc tgcgccgagg 128640

aggtgcggcg gctgctggcc gcaggcgcct ctgtggagta cgcgggcgag ttcgggaaga 128700

ccgcgctgca ccagtacatg ggccgttccg gcgcggaccc cgccgtcgtg cgcgcgctgc 128760

tggacgccgg cgcgcgcgtg gacctcccgg agacctgctg cggctgcacg cccgtgcacc 128820

tctgcctcat ggccgccaat atcgacgtgg aggttctccg catgctcgtc cacgagggcc 128880

gcgtcgagga ctgcggccgc gccgagctcg cctccgtggt gctcaaggag ttcgtggtga 128940

accgcgcctt cgacgagaac gtcagcgagc gagtgatgcg cgttcttgtg gccgcgggcg 129000

cggacgtgaa cgccgccagc gtggtcgacc gcacgccgct gcacgtctgc ctcacgggca 129060

tgtccacgca cccgggcacc atcgccgcgc tgctgcgctt cggcgcggac gtgaacgccg 129120

tggacctctg cggcatgtcg ccgctggcgg tgctcgtgcg ctcgcgcgcg gcgaccgcag 129180

agctggtgcg catgctgctc gacgcgggcg cagacgcaca cgctgtcgac agtcgcctgg 129240

actcgctgct gcaccagcac tttcagtccg cgcgcccgcg gccggaggtg gtgcgcgagc 129300

tcatccgcca cggctgctcg ccgcgggcgc ggaaccgaat cggcaacacg ccgctgcacg 129360

aggccgcaaa acactcctcc tgcaaacact cgctggtggg gccgctgctg gctgccggcg 129420

cgagcgtgga cgcgcgaaat aacacgggca ggacgccgct ccacttggcg gtggcgtcca 129480

acccgcgcgc gtgccgccgg ctgatcgcgc ttggggcgga cgtggtcgcg cgcagttacg 129540
cgggcgtcac gccgctggcg cagctgatcg cggacaataa ctccgcgctg gtgaccgcgg 129600
cgctgaacac gcagcccgag ccgcgggccg tggcagagtc gctgcgagcg accacgcccg 129660
tcggcgagac agcgtgctcg cggctctgtg tggcgtacgt ggtggcgcgc gcgccgagcg 129720
aggtcctcgg cgagcccgag cgcgccctgc acgcggcctt cgtggcggag tgcttagcgg 129780
aggtagcggc catacacgcc gtgcgctgcg gcacacctcc ggtctcgctg ctggagatcc 129840
tggtggccgc gcgcccgccg cggagcctgc tctcgcgccg cgcgtggcgg ctggccagcc 129900
ggacgacagt ttaccgcgcg ccgctccgtg cacgcatcgc ggccatgcgc catcgctcgc 129960
gactggtgga gcgcgcgctg cgcacgctgc gcggctgcgt gctcccgcgc gaggtgctgg 130020
agcgcgtgct gcggtgtctg tccacacagg acctgcgggc ctccggactg gccgagtagc 130080
tttttctgag ataagtgaat aaacatggtg ggattcgatc ggcgccgcca acgccacgcc 130140
atggacgccg ccgagatgga ggatctcgac atcaacgcgg agtcggcgct gtacgactac 130200
ttcatcctga acgcggacag agcccgcgtg ggcgaggtgg ttatgcttct cgcacagggc 130260
gcggaaataa actacgcgga cagcttcgac aagacgccgc tgcacctgta cttgcacacg 130320
cgacacccgc gctcggacgt gattctggcg ctgatggagg cgggcgcggt cgtggacacg 130380
ccggagcgct gctgcggcgc gaccgcggcg cacctgtaca tcctcaacgc ggccaaggtc 130440
gacctgtcgg tgctggaggc catgctgacc tggggcgtgc gccagaacga ccagcactcg 130500
gagcgggtgc tctcgagctt gttgcgcgag tacgtggtga cccgcgccta ctcggatcag 130560
accgagccga tcatggactt gctcatcggc atgggcgccg acgtggacat gccggtcggc 130620
gtgagtcgca cggggctgca cgcctgcctt acgggcctga acgcgaaccc gtgcatgatt 130680
cgcgcgctgc ttcggcgcgg cgccagcgtg accgcaaaag acacctacga gatgacgccg 130740
ctggcggtgc tgctgaagtc cgcgagcgcg acgccggagc tcgtgcgcat cctcgtggag 130800
gcaggctccg acgtgagcgc caccgacttc cgcctcaacg gcatgctgca ccagcacgcg 130860
cagtccacgc gcccgcgcgc gagcgtcatg cgcgagctca tccggctggg gtgcagccca 130920
gcggccaaaa acatgtttgg gaacacgccg atgcacatgc tggccatgga aagctcctgc 130980
cgccgctcac tgatcctccc gctgctggag gcagggcttt ccgtgaacga ggagaacccg 131040
cactacggca ccgtgcctct gcacgtggcc tcggggtacg acaacacgca gggctgcctc 131100
aagctcctcc gggatggagg agaccccacc gtcgtgtcgg ccgccggacg cacgccgctc 131160

tcgaacatgc tcgtcaaacg caaccacgtg gcggtcgccg gcgcgctgtc gacgcacccg 131220
agcgcggcag tagtcgtgca ggctctcgag caggctctcg agaacgtgct gaacgccggg 131280
cccagcgagg cctcgcggct cgccgtggcc tttgtggtgg cgcgcgccgg cgcatccgcg 131340
ctaccggagg ccgtgcgccg tctgcacgag ggctttgtcg ccgactgcga gcgcgaagtc 131400
gagctgcttt cccgcaccat gctcggcaca ccggccgtga gcgcgctggt cgtgctggtc 131460
agcaaggagg tctttggcac tgttatctcc tcgcgtgcgc tgcgcgtcgt gcgggaggtc 131520
cgcgtgtacg caaggccgct ccgcgaggcg ctcataaatc tgcgccacaa atgccgctta 131580
gtttccagcc ttaaaaggca ggtgggacct tgctcgctgc ccggcgaact ggtggagcgc 131640
gtgctcgcga ccgtgccact gaccgacttg cgccgctcgt gcggccgccg cgcgcccgag 131700
taactgcccg tcccgttgct acgcgactcg agactgcccg ctgtttttct ttccccgttt 131760
cttcttatta ggagttgttg cccgcctcca tgatcctcgc acgcgccggc gggcaacctc 131820
gcacgcccgc ggcggccgcg ggcgccgccg aggacggcga gcacagtgat cgccggaagc 131880
gcaagcgcaa gacgcccaac tgcgaagacg ccgacaactc cgacgacgag ctagcgcaga 131940
cgccgtgcga ccgcgagtgg ccggactgtc gcgcgagctc gatcacgagc tccgactcgg 132000
tctctctcgg cgacgagatc tacctgcgat acgtggcctc gcaggtggac ttcgcgcaga 132060
cctgggcccc gccagtgcgg ctgctgcgct ccttcgggaa cttctcgaag gaaacgctca 132120
accgcatgtc gcggcgcggg tacgtgaacc gctcctactt ccagatggcg cacgcgcgct 132180
tctcgcccac caacgacgac atgtaccaca tggccacggg cgggtacggc atcgtgttcc 132240
gcttcgaccg ctacgtggtt aagtacgtct tcgagcaccg caacggcatg tccgagatgg 132300
acgcctctac ggagtacacg gtgccgcggt tcctgcgcaa taacctcaag ggcgacgagc 132360
gcgagttcgt ggtctgcgcg ctgcccatgg ggctgaacta ccggctgggc ttcctgcact 132420
cgctgtaccg gcgcgtgctg cacacgctgc tgctgctcat gcgcgtggag gaaggccagc 132480
ggccctcggt ggagatgtcc aagaagccgc tgctgcgctg gttcgaggcg cgcaaggaca 132540
gcgagtcctt cgtgcgcctg atctcgtact tctacccctc ggccgtgcag agcaacgtga 132600
acctgatcaa caacttccac cacctggtgc acttcttcga gcacgagaag cgcgcgcggt 132660
acgtgttcga ccgcggggcc gtgatcgtgt tccctctggc gcgcgggtcc gcggactcga 132720
tctcgccgga ggcggcggcg gcgctgggct tcgcgccgca ctcggagttc ctcaagttcg 132780
tgttcctgca gatcgcgctg ctgtacctga agatctacga gctcccgggc tgcacgaact 132840

tcctgcacgt ggacctgaag cccgacaacg tgctcatctt cgacagtgcg cgcgcgctca 132900

gcgtgaccgc ggccggcgcg actttccgct tcgaggagcc cgtgcgcgcg gcgctgaacg 132960

acttcgactt cgcgcgcgtg gccaccatcg agaaccgcaa gatcgcgggc agcgtccgcg 133020

tgccgcagaa ctggtactac gacttccact tcttcgcgca cacgctgctg cgcgcgtacc 133080

cgcacatcgc cgcggaggac ccgggcttcc acgcgctgct ctcggagctc acggtctcgt 133140

gctcgcgcgg gacctgcgac cgcttccggc tgcgcgtgtc ctcgccgcac cccatcgagc 133200

acctcgcgcg gctggtgcgc cgcgacgtct tctcccgctg gataaatgcc gccgcggacg 133260

cccccgacgc cgccgcactc tcctgagccc acgcccgcgg cgccgggctc gctgtacgac 133320

gtcttcctcg cgcgcttcct gcgccggctg gccgcgcgcg cggcgccggc ctcggccgcc 133380

tgcgccgtgc gcgtgggtgc ggtgcgcggc cgcctgcgga actgcgagct agtggtgctg 133440

aaccgctgcc acgcggacgc ggccggcgcg ctcgcgctag cctccgcggc gctcgccgag 133500

acgctggcgg agctcccgcg cgcggacaag ctcgccgtcg cgctcgagct gggcgtggac 133560

cccgagcacc cggagctgac gccggacccc gcctgcgcag gcgagagcgc actcgcacag 133620

aacatcgaca tccagacgct ggacctgggc gactgcggag accccaaagg ccgccgactg 133680

cgcgtggcgc tggtgaacag cggccacgcg gccgcgaact gcgcgctcgc gcgcgtggcg 133740

accgcgctga cgcgccgcgt gcccgcgagc cggcacggcc tcgcggaggg cggcacgccg 133800

ccgtggacgc tgctgctggc ggtggccgcg gtgacggtgc tcggcgtggt ggcggtttca 133860

ctgctgcggc gcgcgctgcg ggtacgctac cgcttcgcgc ggccggccgc gctgcgcgcg 133920

tagccgcgca aaatgtaaat tataacgccc aacttttaag ggtgaggcgc catgaagttg 133980

ctcgtcggca tactagtagc cgtgtgcttg caccagtatc tgctgaacgc ggacagcaac 134040

acgaaaggat ggtccgaagt gctgaaaggc agcgagtgca agcctaggcc gattgttgtt 134100

cctgtaagcg agacgcaccc agagctgact tctcagcggt tcaacccgcc gtgtgtcacg 134160

ttgatgcgat gcggcgggtg ctgcaacgac gagagcttgg aatgcgtccc cacggaagaa 134220

gtaaacgtga cgatggaact cctgggggcg tcgggctccg gtagtaacgg gatgcaacgt 134280

ctgagcttcg tagagcataa gaaatgcgat tgtagaccac gattcacaac cacgccaccg 134340

acgaccacaa ggccgcccag aagacgccgc tagaactttt tatggaccgc agatccaaac 134400

gatgatgcga tcaggtcatg cggaagaagg cgccacggag caaagtgaaa aaggaccgcc 134460

tagcagtcga gaccctcccg ccgcagccgc ggacacccca cacccgcctt ccacccgcca 134520

gacgccaaca ccgcagccaa caagcatgca cccctcgccg cgcaggctgc tcggcgcgct 134580
cgcgctggtg gcgctgggct tcctcctcgg cgggctcttc cgccccgcgg cgccgccgct 134640
gccggccgcc ctcgtggagg cgggccccgt ccgcgcgaac ggctccgcct cggtgacctg 134700
cctgaccgtc ggcggcgacg ggcggcacat ggcggtggtc gcgcacggcg gcgggacgct 134760
ctcgccggtg tacccgctcg ccgccggcat gcacgcgacc ttcgcctcgc tgcgcaaggg 134820
cgcgctgctg ctgaacgtcg cgaccgtgca catctacgac gtgcgcgcgc tcgggccgga 134880
gttcgagctg acctgcgtcg cggtggcggg cggctacaac gcggcctggg cggccgcgcg 134940
gcccgcggcc gagtggcgcc gccagctggc gcggatgcac cgctcggagc tgtgaccctc 135000
tccctcccgg tctcccatcc gtttttgtaa tcggccttag tagattagac cagcatcccg 135060
cgcccttgtc cgagaacaag taacagtaac cgttacctca ctcgccactc ctcggaataa 135120
tagaacgaga gaacgagaga acgagttaac cgttgctcac tcgctcactc ggtgtgagag 135180
aacaagtaac gttgctcact cgctcactcg gtgtgagaga acaagagaac gagtagctgt 135240
tgctaactca atcacccctc ggagtaagag aacaagagca gtcaactacc cactcagtct 135300
tggatgagag gcagaggacg agttgacgag ttgaacagtt aatcctcact cactcagagc 135360
gagagagcga gagagtggag gacgagttaa caagtcaatc ctcactcaga gtgagagagt 135420
gagagagtgg aggacgagtt aatggttaac agttatcacc actcagagtg agagcggagg 135480
acgagtcaac cactcgctcg cccactccga gttagagagg gaaccagtgc gagttaacgc 135540
gcacacgagc gagagaacgt aaactcgctc gcgcgctcgc tcggctaacc gtcggcctct 135600
cccaaaactc ttcgtaaaac tttcccgatg acagttcacc ctccaaaact ttgtaaaact 135660
aaactgttcg gaggtcggtc tgctgcctct ctaactctcc gtaaaacgtt tgtaaactgt 135720
cggaggtcgg tgacccgctc aaccgtccgc gaaaactttt cgcaggcagt gtctgcctct 135780
ctcggactct ccgcaaacac tttcgcggaa cctcggaggg tggtcgacct ctctccaaac 135840
tcttgcaaaa ctttttcgcg gaaccgttgg aggccagtcc tccctccaaa ctctttgtaa 135900
aatcttttcg aggccagtcc tcctctccaa aacgttccgc aaaatctttg ggaggtcggc 135960
ctctcctctc cagaacgttc cgtaaaactc ttggaggccg cccgcggcac gcgaggcgga 136020
ggatccgagg tgtcgacctc cctcaaaaac tttgtaaaaa ctttttataa aactttccgc 136080
ggaacctcgg agagtaggtc gacctctctc aaaactctta taaaactttt ccgcggaacc 136140
gttggaaggt aggtcgacct ctctcaaaac tcttataaaa cttttccgcg gaaccgttgg 136200

aaggtaggtc gacctctctc aaaactctta taaaactttt ccgcggaacc gttggaggca 136260
ggtcggcctc tcaaactctt tgcgagaact cttcgcgaga actcttcgat aactttagga 136320
ggtcaggtcg acctcccaaa acttttgcga gaactctctg taaaacttta ggaggtcggt 136380
acctccctca aaactttta taaaactttt cgcggaacct ccggagacgg gccgccgccg 136440
cgaccgcggg agcggagagg ccgacctccc gagacgttcc gcgttaccgt cggggtaggc 136500
gtcctctcga gaacgccaaa agacttcgtg caaaaacttt tcggaggggc gcggagggcg 136560
ggtcagctcc cgcgaactcc cgcagaacct tttcgcgcga ccgcgaaggc cggccgcctc 136620
tcccacactc tcaagagctt ttcggaggag aggaagggca ggtcgccccc acctccgacg 136680
ctttgtaaaa acgtttacgc ggaacctcgg aggcaggtcg cctccctcga aaactcctcg 136740
cgaaaccttt aaaaactttt tgcgaaaact tttcggagga tgtcggaggg cgggcggctc 136800
ttccaaacct ccgcagaacc ttttcgcgca accgttggaa gacaggtcgg cctctctcga 136860
aaacttttaa aactttgtaa acgcgttggc gggaccgtcg cgggagagcg gccgcccgcg 136920
gcacgcgaga ggaggaaccg ttggaaggca gtcggcctct cccgaaaact ttttataaaa 136980
acttttccgc ggaaccgttg gaggcaggtc ggcctctctc agagtctaaa aactttttgc 137040
gggactcgga cggcgcggtc acccgaccac ctgactcctg tctcacccgt actacttgga 137100
cttctgtttc cctgactccc gactccctga cctcccgact ccctgactcc cgactccctg 137160
actcccgact ccctgactcc cgactccctg actcccgact ccctgactcc cgactccctg 137220
actcccgact ccctgactcc cgactccctg actcccgact ccctgactcc cgactccctg 137280
actcccgact ccctgactcc cgactccctg actcccgact ccctgactcc cgactccctg 137340
actcccgact ccctgactcc ctgactccag agcgaggtct cgcggctgcg gggtgccgcc 137400
tccgcggagt cgcgttcccg cggacgcccg tcctcgaaag cattcagcag ttccagcctc 137460
tgccgtagct cctcccgcag gaactcctgg tccgcgttcg tcgcggcacc gcggctcagc 137520
cgccgcggga gccggccgcc gcccgcgaag ccgcggatcc 137560

SEQUENCE LISTING

<110> Bayer AG, Leverkusen, Germany
<120> Recombinant proteins of Parapoxvirus ovis and pharmaceutical compositions therefrom.
<130> LeA 35228
<160> 1

<170> PatentIn version 3.0
<210> 1
<211> 137560
<212> DNA
<213> Parapoxvirus ovis NZ2
<220>
<221> CDS
<222> (3)...(539)
<223> ORF: L1
<220>
<221> CDS
<222> (781)...(449)
<223> ORF: L2r
<220>
<221> CDS
<222> (1933)...(1664)
<223> ORF: L3r
<220>
<221> CDS
<222> (3269)...(2790)
<223> ORF: L4r
<220>
<221> CDS
<222> (2799)...(3851)
<223> ORF: L5
<220>
<221> CDS
<222> (2962)...(3753)
<223> ORF: L6
<220>
<221> CDS
<222> (3784)...(3122)
<223> ORF: L7r
<220>
<221> CDS
<222> (4341)...(4129)
<223> ORF: L8r
<220>
<221> CDS
<222> (4904)...(4428)
<223> ORF:1ar
<220>
<221> CDS
<222> (6517)...(4970)
<223> ORF:1r
<220>
<221> CDS
<222> (8042)...(6684)
<223> ORF: 2r
<220>
<221> CDS
<222> (9989)...(8070)
<223> ORF: 3r
<220>
<221> CDS
<222> (11195)...(10062)
<223> ORF: 4r
<220>

<221> CDS
<222> (11493)...(11227)
<223> ORF: 5r
<220>
<221> CDS
<222> (11802)...(12038)
<223> ORF: 6
<220>
<221> CDS
<222> (12358)...(12080)
<223> ORF: 7r
<220>
<221> CDS
<222> (13980)...(12364)
<223> ORF: 8r
<220>
<221> CDS
<222> (14826)...(14053)
<223> ORF:9ar
<220>
<221> CDS
<222> (15080)...(15394)
<223> ORF:10
<220>
<221> CDS
<222> (16838)...(15423)
<223> ORF:11r
<220>
<221> CDS
<222> (19021)...(16847)
<223> ORF:12r
<220>
<221> CDS
<222> (19704)...(19156)
<223> ORF: 13r
<220>
<221> CDS
<222> (20314)...(19736)
<223> ORF:14r
<220>
<221> CDS
<222> (20401)...(22101)
<223> ORF: 15
<220>
<221> CDS
<222> (22125)...(22940)
<223> ORF:16
<220>
<221> CDS
<222> (23003)...(23866)
<223> ORF: 17
<220>
<221> CDS
<222> (26908)...(23873)
<223> ORF: 18r
<220>
<221> CDS
<222> (26926)...(27213)

<223> ORF:19
<220>
<221> CDS
<222> (27626)...(27216)
<223> ORF:20r
<220>
<221> CDS
<222> (29754)...(27616)
<223> ORF:21r
<220>
<221> CDS
<222> (32217)...(29800)
<223> ORF: 22r
<220>
<221> CDS
<222> (33380)...(32418)
<223> ORF: 23r
<220>
<221> CDS
<222> (33602)...(33393)
<223> ORF: 24r
<220>
<221> CDS
<222> (34466)...(33622)
<223> ORF: 25r
<220>
<221> CDS
<222> (34735)...(34502)
<223> ORF: 26r
<220>
<221> CDS
<222> (35905)...(34739)
<223> ORF: 27r
<220>
<221> CDS
<222> (37294)...(35905)
<223> ORF: 28r
<220>
<221> CDS
<222> (37200)...(39248)
<223> ORF: 29
<220>
<221> CDS
<222> (41037)...(39229)
<223> ORF:30r
<220>
<221> CDS
<222> (41374)...(42066)
<223> ORF:31
<220>
<221> CDS
<222> (42336)...(41731)
<223> ORF: 32r
<220>
<221> CDS
<222> (42407)...(41997)
<223> ORF:33r
<220>

<221> CDS
<222> (42410)...(43765)
<223> ORF: 34
<220>
<221> CDS
<222> (43770)...(43958)
<223> ORF:35
<220>
<221> CDS
<222> (43980)...(44534)
<223> ORF:35
<220>
<221> CDS
<222> (45727)...(44537)
<223> ORF: 37r
<220>
<221> CDS
<222> (45760)...(46557)
<223> ORF:38
<220>
<221> CDS
<222> (46567)...(47568)
<223> ORF: 39
<220>
<221> CDS
<222> (47572)...(48303)
<223> ORF:40
<220>
<221> CDS
<222> (48352)...(48621)
<223> ORF:41
<220>
<221> CDS
<222> (49887)...(48634)
<223> ORF:42r
<220>
<221> CDS
<222> (49917)...(50693)
<223> ORF:43
<220>
<221> CDS
<222> (50719)...(51102)
<223> ORF: 44
<220>
<221> CDS
<222> (51059)...(51511)
<223> ORF:44a
<220>
<221> CDS
<222> (51584)...(52591)
<223> ORF:45
<220>
<221> CDS
<222> (52509)...(53066)
<223> ORF:46
<220>
<221> CDS
<222> (53523)...(53023)

<223> ORF: 47r
<220>
<221> CDS
<222> (53607)...(57473)
<223> ORF:48
<220>
<221> CDS
<222> (58070)...(57528)
<223> ORF: 49r
<220>
<221> CDS
<222> (57700)...(58662)
<223> ORF:50
<220>
<221> CDS
<222> (59674)...(58673)
<223> ORF:51r
<220>
<221> CDS
<222> (62089)...(59678)
<223> ORF: 52r
<220>
<221> CDS
<222> (62198)...(62881)
<223> ORF:53
<220>
<221> CDS
<222> (62909)...(63862)
<223> ORF: 55
<220>
<221> CDS
<222> (63858)...(64271)
<223> ORF: 56
<220>
<221> CDS
<222> (64309)...(66831)
<223> ORF:57
<220>
<221> CDS
<222> (67266)...(66799)
<223> ORF:58r
<220>
<221> CDS
<222> (67803)...(67273)
<223> ORF:58ar
<220>
<221> CDS
<222> (67915)...(68607)
<223> ORF: 59
<220>
<221> CDS
<222> (68624)...(70984)
<223> ORF: 60
<220>
<221> CDS
<222> (70994)...(72898)
<223> ORF:61
<220>

<221> CDS
<222> (72938)...(73507)
<223> ORF: 62
<220>
<221> CDS
<222> (73540)...(74211)
<223> ORF:63
<220>
<221> CDS
<222> (76120)...(74207)
<223> ORF: 64r
<220>
<221> CDS
<222> (76749)...(76186)
<223> ORF: 65r
<220>
<221> CDS
<222> (77698)...(76799)
<223> ORF: 66r
<220>
<221> CDS
<222> (79343)...(77709)
<223> ORF: 67r
<220>
<221> CDS
<222> (79816)...(79367)
<223> ORF: 68r
<220>
<221> CDS
<222> (80529)...(79858)
<223> ORF: 69r
<220>
<221> CDS
<222> (80774)...(80529)
<223> ORF:70r
<220>
<221> CDS
<222> (82815)...(80788)
<223> ORF:71r
<220>
<221> CDS
<222> (83835)...(82834)
<223> ORF:72r
<220>
<221> CDS
<222> (83874)...(85583)
<223> ORF:73
<220>
<221> CDS
<222> (85535)...(84402)
<223> ORF:74r
<220>
<221> CDS
<222> (88096)...(85574)
<223> ORF:75r
<220>
<221> CDS
<222> (87759)...(88667)

<223> ORF:76
<220>
<221> CDS
<222> (88920)...(88542)
<223> ORF:77r
<220>
<221> CDS
<222> (91652)...(88938)
<223> ORF:78r
<220>
<221> CDS
<222> (91667)...(92674)
<223> ORF:79
<220>
<221> CDS
<222> (93466)...(92681)
<223> ORF:80r
<220>
<221> CDS
<222> (93761)...(93486)
<223> ORF:81r
<220>
<221> CDS
<222> (94060)...(93788)
<223> ORF:82r
<220>
<221> CDS
<222> (94238)...(94080)
<223> ORF: 83r
<220>
<221> CDS
<222> (94508)...(94242)
<223> ORF: 84r
<220>
<221> CDS
<222> (95571)...(94498)
<223> ORF: 85r
<220>
<221> CDS
<222> (96187)...(95600)
<223> ORF:86r
<220>
<221> CDS
<222> (96202)...(97665)
<223> ORF: 87
<220>
<221> CDS
<222> (97915)...(97643)
<223> ORF:88r
<220>
<221> CDS
<222> (98251)...(99537)
<223> ORF:89
<220>
<221> CDS
<222> (99537)...(99974)
<223> ORF:90
<220>

<221> CDS
<222> (100001)...(101140)
<223> ORF:91
<220>
<221> CDS
<222> (101168)...(104650)
<223> ORF: 92
<220>
<221> CDS
<222> (106354)...(104795)
<223> ORF: 93r
<220>
<221> CDS
<222> (1079A7)...(106400)
<223> ORF: 94r
<220>
<221> CDS
<222> (108256)...(107990)
<223> ORF:95r
<220>
<221> CDS
<222> (108719)...(108300)
<223> ORF: 96r
<220>
<221> CDS
<222> (109679)...(108738)
<223> ORF:97r
<220>
<221> CDS
<222> (109861)...(109682)
<223> ORF:98r
<220>
<221> CDS
<222> (110830)...(110033)
<223> ORF: 99r
<220>
<221> CDS
<222> (110208)...(110417)
<223> ORF:100
<220>
<221> CDS
<222> (110469)...(110651)
<223> ORF:100a
<220>
<221> CDS
<222> (110915)...(111397)
<223> ORF:101
<220>
<221> CDS
<222> (111419)...(111913)
<223> ORF:102
<220>
<221> CDS
<222> (111949)...(112485)
<223> ORF:103
<220>
<221> CDS
<222> (112593)...(113450)

<223> ORF:104
<220>
<221> CDS
<222> (113323)...(112967)
<223> ORF:105r
<220>
<221> CDS
<222> (113526)...(114152)
<223> ORF:106
<220>
<221> CDS
<222> (114199)...(115236)
<223> ORF:107
<220>
<221> CDS
<222> (115353)...(115787)
<223> ORF:108
<220>
<221> CDS
<222> (115859)...(116551)
<223> ORF:109
<220>
<221> CDS
<222> (116729)...(117523)
<223> ORF:110
<220>
<221> CDS
<222> (117572)...(117114)
<223> ORF:111r
<220>
<221> CDS
<222> (117423)...(118085)
<223> ORF: 112
<220>
<221> CDS
<222> (118968)...(118375)
<223> ORF: 114r
<220>
<221> CDS
<222> (118508)...(119119)
<223> ORF: 115
<220>
<221> CDS
<222> (119588)...(120202)
<223> ORF: 116
<220>
<221> CDS
<222> (120314)...(121231)
<223> ORF: 117
<220>
<221> CDS
<222> (121380)...(123920)
<223> ORF: 118
<220>
<221> CDS
<222> (121288)...(122256)
<223> ORF: 119
<220>

<221> CDS
<222> (122350)...(123924)
<223> ORF: 120
<220>
<221> CDS
<222> (123962)...(125566)
<223> ORF:121
<220>
<221> CDS
<222> (125193).,.(124591)
<223> ORF:122r
<220>
<221> CDS
<222> (125689)...(123935)
<223> ORF:123r
<220>
<221> CDS
<222> (123839)...(123297)
<223> ORF:123ar
<220>
<221> CDS
<222> (125652)...(126170)
<223> ORF:124
<220>
<221> CDS
<222> (126121)...(125699)
<223> ORF: 125r
<220>
<221> CDS
<222> (126279)...(127769)
<223> ORF: 126
<220>
<221> CDS
<222> (127851)...(128408)
<223> ORF:127
<220>
<221> CDS
<222> (128520)...(130076)
<223> ORF: 128
<220>
<221> CDS
<222> (130105)...(131700)
<223> ORF:129
<220>
<221> CDS
<222> (131790)...(133283)
<223> ORF:130
<220>
<221> CDS
<222> (133246)...(133920)
<223> ORF: 131
<220>
<221> CDS
<222> (133972)...(134370)
<223> ORF: 132
<220>
<221> CDS
<222> (134418)...(134693)

<223> ORF: 133a
<220>
<221> CDS
<222> (134402)...(134992)
<223> ORF: R1
<220>
<221> CDS
<222> (134853)...(134419)
<223> ORF: R2r
<220>
<221> CDS
<222> (135628)...(135897)
<223> ORF:R3
<220>
<221> CDS
<222> (136780)...(137112)
<223> ORF: R4
<220>
<221> CDS
<222> (137558)...(137022)
<223> ORF:R5r
<400> 1

ggatccgcgg cttcgcgggc ggcggccggc tcccgcggcg gctgagccgc ggtgccgcga 60

cgaacgcgga ccaggagttc ctgcgggagg agctacggca gaggctggaa ctgctgaatg 120

ctttcgagga cgggcgtccg cgggaacgcg actccgcgga ggcggcaccc cgcagccgcg 180

agacctcgct ctggagtcag ggagtcaggg agtcgggagt cagggagtcg ggagtcaggg 240

agtcgggagt cagggagtcg ggagtcaggg agtcgggagt cagggagtcg ggagtcaggg 300

agtcgggagt cagggagtcg ggagtcaggg agtcgggagt cagggagtcg ggagtcaggg 360

agtcgggagt cagggagtcg ggagtcaggg agtcgggagt cagggagtcg ggagtcaggg 420

agtcgggagg tcagggagtc gggagtcagg gaaacagaag tccaagtagt acgggtgaga 480

caggagtcag gtggtcgggt gaccgcgccg tccgagtccc gcaaaaagtt tttagactct 540

gagagaggcc gacctgcctc caacggttcc gcggaaaagt ttttataaaa agttttcggg 600

agaggccgac tgccttccaa cggttcctcc tctcgcgtgc cgcgggcggc cgctctcccg 660

cgacggtccc gccaacgcgt ttacaaagtt ttaaaagttt tcgagagagg ccgacctgtc 720

ttccaacggt tgcgcgaaaa ggttctgcgg aggtttggaa gagccgcccg ccctccgaca 780

tcctccgaaa agttttcgca aaaagttttt aaaggtttcg cgaggagttt tcgagggagg 840

cgacctgcct ccgaggttcc gcgtaaacgt ttttacaaag cgtcggaggt gggggcgacc 900

tgcccttcct ctcctccgaa aagctcttga gagtgtggga gaggcggccg gccttcgcgg 960

tcgcgcgaaa aggttctgcg ggagttcgcg ggagctgacc cgccctccgc gcccctccga 1020

aaagtttttg cacgaagtct tttggcgttc tcgagaggac gcctaccccg acggtaacgc 1080

ggaacgtctc gggaggtcgg cctctccgct cccgcggtcg cggcggcggc ccgtctccgg 1140

aggttccgcg aaaagtttta taaaaagttt tgagggaggt accgacctcc taaagtttta 1200

cagagagttc tcgcaaaagt tttgggaggt cgacctgacc tcctaaagtt atcgaagagt 1260

tctcgcgaag agttctcgca aagagtttga gaggccgacc tgcctccaac ggttccgcgg 1320

aaaagtttta taagagtttt gagagaggtc gacctacctt ccaacggttc cgcggaaaag 1380

ttttataaga gttttgagag aggtcgacct accttccaac ggttccgcgg aaaagtttta 1440

taagagtttt gagagaggtc gacctactct ccgaggttcc gcggaaagtt ttataaaaag 1500

tttttacaaa gtttttgagg gaggtcgaca cctcggatcc tccgcctcgc gtgccgcggg 1560

cggcctccaa gagttttacg gaacgttctg gagaggagag gccgacctcc caaagatttt 1620

gcggaacgtt ttggagagga ggactggcct cgaaaagatt ttacaaagag tttggaggga 1680

ggactggcct ccaacggttc cgcgaaaaag ttttgcaaga gtttggagag aggtcgacca 1740

ccctccgagg ttccgcgaaa gtgtttgcgg agagtccgag agaggcagac actgcctgcg 1800

aaaagttttc gcggacggtt gagcgggtca ccgacctccg acagtttaca aacgttttac 1860

ggagagttag agaggcagca gaccgacctc cgaacagttt agttttacaa agttttggag 1920

ggtgaactgt catcgggaaa gttttacgaa gagttttggg agaggccgac ggttagccga 1980

gcgagcgcgc gagcgagttt acgttctctc gctcgtgtgc gcgttaactc gcactggttc 2040

cctctctaac tcggagtggg cgagcgagtg gttgactcgt cctccgctct cactctgagt 2100

ggtgataact gttaaccatt aactcgtcct ccactctctc actctctcac tctgagtgag 2160

gattgacttg ttaactcgtc ctccactctc tcgctctctc gctctgagtg agtgaggatt 2220

aactgttcaa ctcgtcaact cgtcctctgc ctctcatcca agactgagtg ggtagttgac 2280

tgctcttgtt ctcttactcc gaggggtgat tgagttagca acagctactc gttctcttgt 2340

tctctcacac cgagtgagcg agtgagcaac gttacttgtt ctctcacacc gagtgagcga 2400

gtgagcaacg gttaactcgt tctctcgttc tctcgttcta ttattccgag gagtggcgag 2460

tgaggtaacg gttactgtta cttgttctct gttcccttcc tcggtgagcg gtgcgtcaac 2520

ttgttctcgt gagtgagtac ggtcacttgt tctctcgttc tctacctcga ggagtgagtc 2580

aacttgttct cgtgagtgag ttgaccgtaa ccgttccctt acctcaagtg agtggcggtc 2640

gcttgttctc gtgagtgggt taaccgcgtt cccttaccgg agtgagcggg cgggcgataa 2700

aaataatcaa ttgactgatt cgctcgtgag cgagcgaagg gcggcggaca agggcgcggg 2760

atgctggtct aatctactaa ggccgattac aaaaacggat gggagaccgg gagggagagg 2820

gtcacagctc cgagcggtgc atccgcgcca gctggcggcg ccactcggcc gcgggccgcg 2880

gccgcccagg ccgcgttgta gccgcccgcc accgcgacgc aggtcagctc gaactccggc 2940

ccgagcgcgc gcacgtcgta gatgtgcacg gtcgcgacgt tcagcagcag cgcgcccttg 3000

cgcagcgagg cgaaggtcgc gtgcatgccg gcggcgagcg ggtacaccgg cgagagcgtc 3060

ccgccgccgt gcgcgaccac cgccatgtgc cgcccgtcgc cgccgacggt caggcaggtc 3120

accgaggcgg agccgttcgc gcggacgggg cccgcctcca cgagggcggc cggcagcggc 3180

ggcgccgcgg ggcggaagag cccgccgagg aggaagccca gcgccaccag cgcgagcgcg 3240

ccgagcagcc tgcgcggcga ggggtgcatg cttgttggct gcggtgttgg cgtctggcgg 3300

gtggaaggcg ggtgtggggt gtccgcggct gcggcgggag ggtctcgact gctaggcggt 3360

ăcctttttcac tttgctccgt ggcgcctggt ccggggcaag ggctgcggcg ggcgcccggg 3420

cgggcgggcc gctaccccgc cgcgcggccc gcggccgcca gcgccgcggc cagccgccgc 3480

caccgcgggc ccgccgcgcg cagcagcccc gccgccgagc gccccgcgcc gccgcgggcg 3540
cgcgccgagg ccgcggcccc gcgccgcgcc agcagcagcg gcagccgcgc gtccagcggg 3600
ccgccgcggc gcagcgccgc gcgcagcagc gccgccagcg gcagccgccc cgccgcgtcc 3660
gggcccgccg cgcgcgcgcc cgccgccagc agcgcgcgca ccagcgccgg cgagggcgcc 3720
gcgcgcggac caggtgctcc acgagcaggg tggtgagcaa ggattctcga gaagtaggag 3780
tcatgtgtga cgacaaggag agacgttata ttaggcgcgt cctacttcac tttgaagatg 3840
gtgtaaagtg ttaaaacttg aacaccgttc actccaccac tgccgttacc gtgtcctgcc 3900
ccaaaagcaa ccacagtgct ttttccacca cctgttccaa atccgttcca aaagctccca 3960
tccattgttg ttagaacttt cagatgtttc tctaggttgt ttagttccac tgcaagtttc 4020
gaccattatc gttactggac atgctgttgg taatgagttt aataaccaat cataaaaata 4080
gttataattt gttataaagc taataaagta gcaaacactt taatgttata ttttgcctaa 4140
ccctccgtta acaccaccat taacaccacc acttaagctt ttactaccac cactaccacc 4200
tccaacacac attcttttct ctaaaggtcc ccaaattcca cctcctgaac ttggacgttt 4260
tacagcacct ccgggtgtac ttgcgtaccc tttagaagtt ccactgtgac tgtagatatg 4320
atactgtcct tctccaggca tgattaaagt gtgttgtaat tagtgttatc tacgcaactg 4380
tgcgagactc tcgaataaaa agaagctaca ttttacaatt ttgattagct gatgtaccac 4440
gctgtatcgc ggccaccaca agcacccgat ccagtagaac caaatccaga gtcgccgcgg 4500
tcagtgttgt ccaagcagtt aacctcttga actgctgggc acgatatgcg ttcgcatatt 4560
agctgagcta tcctgtctcc cttcttaacc tcaaagtcac tgtttccaaa gttaaacagc 4620
accactccga cgttgcctcg gtagtcttcg tcgatcacgc cagcgcccac gtcgataaag 4680
tgtttgactg caaggccaga acgtggtgct atgcgtccgt agcaaccaga agggggcttt 4740
atcagaaggt cagtaaatac tacgcgactg caatgcgaag ggatgacaca gtcgtatgca 4800
ctacataggt ctaatcctgc ggcaccagga gatcctctgg ctggtatagt ggcgttttgg 4860
ctgaggcgaa caacctgaag agtttccgtg tggcagaact ccatggctag ggtggcgagc 4920
ggccgatcga ctacggggtg tacaatttac actttctcca gaaaaatcag gggcgggtca 4980
gcatggcgcg gcgcaagtcc agcagcgagt cgtacgacag gaagcacagg atggaggtca 5040
cgatctccgg cggcagggcg cacgggcaca tgaggccggc gatctgctcg gccagcgaga 5100
cgcgcagccg catcatgcag atcttgccga agagcgccgt cccgtagatg gggaactcgg 5160

ccgcgcgctc caaaaaggcg ttctgcacga agagcgcctt cgcgtcgtcc gacgcgcgca 5220
gcacgtccaa cagcgtcgcg tccgtgtggc agcgcaccgc gcgcatgctt gcgatctcct 5280
gctcgcacgc gcggatcacg gtcgcgtagt ccgccagcgc gcgctccgcc agcatgcgcg 5340
cgccctcgcc gcgcagcgcc agctcctgca cgcacagcag cgcggcctcc gagcgtcgga 5400
acacgtggcc ccattgctcg gatgtgatca gcgcgcgcgc gagcagctcc gtcggcggcc 5460
ggcgcgcgag cacggccgcc gtcgcgcgca cgttgttgcg gcgcagcatc tccgaaaccg 5520
cgcataggcc cgaggccgac atgtgctcga gctccgcgcc catgcgcacc agccggcagc 5580
aggcgccgtg gctgaacacc gccgcgcggt gcagcgcggt ctgcaggttg ttgttgcgca 5640
ggttcaggtc cagcccgcgc tcgagcacga agtccacgac gccgcgctcg cagctcccgt 5700
aggtcgccat gtagtgcagc atggtgttcc cacacgcgtc tacggcggcc gggtccacgc 5760
ctaggcccgt gagcgtgcgc accatgccct cggagatctt ggccgtgcgc gcgaggtggt 5820
gcagcgttgt gcgcccgtac gcgtccacga cgcacgcgtc cgcgcccgcg cgcagcatca 5880
tgtccacgag cgcggcggag acgccgccgg agcacagcag cgccgccagc ggcgtcaagc 5940
cgttgcagtc gcaggcgttt gggttcgcgc cgcgctcgag cagcagccgc agcacgtcct 6000
cgcggatcca ctggttcttg gcgtacacgt gcagcggcgt tacgccgtag gtgttgccct 6060
cgttcacgcg cgcgcccgcg tccagcagca gccgcgcgac ctcgagctcg gcgccgtcgg 6120
ggccgcagaa agccaggaag gaggagagca cgctgtcgca gacaacgacg ctggcgtcgc 6180
agaccacgtc cgcgcccgcc tccagcatga gcgcgaccac ctccggccgc acgccgtcgt 6240
actgcacgta ggcgtgcagc ggcgtgcggc cgcaggagtc cttggctttc acgtccgcac 6300
cggcctccag cagcacgcgc acgatctccg cgcactgctc gtgccgcgcg aagtgcacgc 6360
agaggtgcag cggcgtgcgc ccgtgctcgc cgcggaagtt cacgtctgcg tcggtggcta 6420
cgagcgcgcg gaccgtttcg aggtccacct gcccggactc caggtagcgg aagagcaggt 6480
ccgcgtgcgg gaccacgacg gactcccgcg agagcatggc ggcgtttaca aatattgaaa 6540
tcttttttca ctcatcttta tggcgctgaa cgcgcaataa gggtgagagt aaaaaacttc 6600
tacaaaaagc gtacaaaagg tacaaaaggt aaaaaaggcg gggcggggac gggctggggt 6660
gctgcgagct gaattggcct ctacacaggg acgccctcgc cggagccggt gagccggtag 6720
ccggcgccgg cgatcatggt caagcgctgc acgagctcgt tgcgcttgac gccggcctct 6780
gaaacgcaca ccatgtggtg gatgtaccgc tcgatgcact cgcagcgcgg gagagtggag 6840

tcaagatcgg atgcgagttg cagaatgtca tcccagagct cggagaactt gctgtacagt 6900

tctcggaggt ctctctccat tcgagccgta agagagtcag gatgcggtgt tccttcggga 6960

gtctgagcga acaccgcgaa caggctggtt atgccgtgtt ctagaataga gtggttccgc 7020

gttaatgccg cagacaaggg tcgtcgtccg cgcaacgact ggcggcagag cgctgtttgt 7080

gccgcaccgc ccattcctct ggcgatcgca tccaccgacg cagtgatcat ctgcgcgccg 7140

acgtcattgt agcgcgcgtt aaactcagta atcatgatta cgagattgca gatttcatag 7200

tagcactttt ccaagtcgac gcgcagtttc acgatctggt tgacaatctt gcacgccttt 7260

cgccgcgtct ccgccacgtt ggcgactcgg acttgcgctt cctggtcgat ggacggcgga 7320

aacacttcaa acccaaggtc gcacagttca gcggtgggga ctagcgtcac gatgatgtac 7380

tccgcatcgc cacccacttg cggcaggaag aacaccgacc gcgcggcggg aacgaccaga 7440

acgtcgcctt cctgcatgtt ggtttttaga aacttagtgt tgttcacgga gatgccggcc 7500

atgccctcgt ttttgacaca tattatggtg acgtacgcgg cgaccgtggg ggccatgtgg 7560

tggcgcatgt accactcgtc gtgcttgagt ttcagaccgt gagattcgcc aacctcgaag 7620

tgcatgttgg cgtctctgac gtagcgcgag aactcgctgc gacagattcg cgcgggcgcc 7680

cggtggaacg tcgactcgaa gagactgatg gctgtccatt cgcccacatg agtgaccacc 7740

gaagaagtgt tttcgatccg agtctcgaac accgagtcca cgagcaccgg acagttggtt 7800

ccgggcaccg tcagcaccaa gggccgcgcc tccacggggg cgacggacga agccacggag 7860

tcggtgtccc cgtacccgta gtcgtcgtcg gagtcgccgc ctccgtcggc cccgtcgcgc 7920

ggcctccgca gcggcatgca gccggcggtg ggaacgcact ggtttcggcc acggccgaag 7980

cggccaaaca gtctcgccag ggctgacatc cttggacggc cacaccaaaa ccaaaaaaac 8040

atattttatc agttatttgt cgattttcac cggctcaccg agggcaggac ctcctggatc 8100

ccggacaccc ccgccaggca gcgggccgcg cgctcgcgca cccaaaagcg gtcgtagccg 8160

tgccggagca cgaaggccgc cgtggcgtgg cagtccacgc gctcgatgaa gccgtggacg 8220

gcgcggcgcg cgtagctcgc cgcgaaggcg cggaccaccg ccgagcagcg ccccgagggc 8280

gagtcgtccg tctccagcgc cagcggcatg ctcgcgatgc gcgacatcag gttggaggtc 8340

tgcgggatgt tgagctcgcg cgtggcggtc atctgcgcct cgagcccggc cttgagcacc 8400

tcgtcgcagc ggccccactc cagcgcgcag accacgcgga tctcgtaccc cttgagccgc 8460

agcgcggtct cgatgtccac ggaggtgagc accgcgctga agcgcaggcg ctcttcgtcc 8520

gcggggtcga agagcacggg gatcttaacc tccgcgctgc gcgtgacctc gcagagcgcg 8580
atcgcgagca gcccgcgcgt gagcttgctc accatgcgcg gcttgcccac ggggtacagc 8640
tggctcgcga cctcgcgcag cgggtacgcc agtcggaagc agcgcgcgtc cgcgggcacc 8700
gggctcgcgc ccgtctcctc gaggaagagc gcggcctcaa ccatgttcag cgcggagaag 8760
tgcaccgggc aggcggcgca gccgcgcgcg gcgttcgcga gcaccatctc gcgcagcccg 8820
cggaaggccg ccatgtcgca ggaggggaag atgcgcgcga gcgcggcctg gtgcgcgagc 8880
gccgcgtccg agagcgcctg cgcgccgcgg cggcgctcct cggcggcggc cgcgctctcg 8940
tccgcggaga ccacgtcttc gggcacgtcc acgcagacgc cgccccagaa ctcgcagtac 9000
tcggagaaga gcgtcgcggg cgcaaagcgc gcgaggtcca cgaaggcgac gcggttgccg 9060
agcctggaga gcagcgtgtt ctccgagatg cgcgtccagc ccttgccggc gagctccatg 9120
acctgccgcg tgtcgaagag ggagctgtag aagccgtaca cggtgatgtt ttccttgcac 9180
gtcgtcagcc acatgaggaa gtcgcgcacc accagcttcg cgcagtctcc ggagaacacg 9240
gggccggcgt tcgtcgcgat ggagttcagg cgcacggtgc cgtcactgcc gaagcggtac 9300
acgaaccagg cggccacgct gttgccagag ggcgtgtgaa cgtgtggctg cgcccaggag 9360
tcggcgctcg cggcggtgcg cacgtcgtgc gagagcacct cggtgtcggg gcgcgagtag 9420
gtgctggggt ctttgatcca gatggcgtag ctgcccacgc agcacacgtt catgaggtcg 9480
agcagcgtct gccggcgcag cggcgtgccg agccggcgca cggcgtcgtg cgagaccatg 9540
cgcaggtcgt agaggcccac gtccgagagc cactggttga gctcgtccat ggacagggcg 9600
tcgcgggggg gcgggctgtc ttcgaaggcg gcgcggagct cgggctccgt ctccgcgcgc 9660
tgccgcagga tgtccaggaa ggggctggag gagtcgggga tgtagcagtc ggggtcgtgc 9720
ctggacacta tagcgaaccg ctgcgtcgcg ggcggcgggg ctagcgcgtc ggcgcgtgcg 9780
tcgatgaagg tgcacgatat acgcacggac ttgagcgagg ggaggacgac tgcggcggcg 9840
cgcgcgccct ccgcgtcgaa gatcatcgtc tttccgtccc tcgcctttgc gagcgcgtat 9900
tctccaggca cgaggtccgt cggcggcggc tcgtcccagg cctgccggtc agggacgccg 9960
ccgcacacct ttccccagaa ccccagcatc ctccaaaata cctataagga cggccaatag 10020
cggggcttgc gggcgttcgg accttccgcg ctttaatttt aatttattgg cttgcagaac 10080
tccgagcgcc agtcccgctc gaagaccgcg gacaggtcct tgacgatgtc gcccttctcg 10140
gcgttcacgc tcacgaaggc gtggtagcgg tagtgcgtgc cgtcgaggtt ggcgaccgtg 10200

aggtgcgcga aggtgtcgtc cacgatgagc agcttagtgt tgttcgcggc gtcgtcccgg 10260

ccgggtacca cgaacttgcg cacggacatg tccacgctgc cgacgccaaa gtcgtcgagg 10320

ctgcgcgcgg ccgagaccga aagcgggtcc gcgttcttcc actcggtaat gatcacgcgc 10380

acgcgcacgc cgcggttgat ggccgcgcgc agcagcgcgt caatgatctg cggccagtac 10440

tccacggcgc tggcgtgctt gatcaccggc accatcgaga gcagcgagag gtcgatgctg 10500

ttcttggcgt tctcgatgcg gtgcagcacg aggtcctcgt cgagcgtgcg gtagaagcct 10560

aggaagcgct ccggcgagtc cgagaagaat acgccgcccc cggagtggtc gaggtggaag 10620

ttcgtggccg tgggcgtgac gatggcgcag cagagccgcg tgaacggcac cttcggctcc 10680

acgatcatgg agtagaaggt gttgtagcgg ttcatgaggt cccaggccag gtgcttgttg 10740

gtggagtaga gcccgaggtt cttgatggtg gacacggacc cgcccgtgag cgaggcgctt 10800

cccacgtacc agtgcccggc gtccgagagc cagaagctgc cgagaaggtt gccgacgccc 10860

tccttggtgg acaccttgac cttgtagtag ttgacgcccg cctcgcgcag ctcgtccgcg 10920

tccttgtcct tgctctgcac gtccacgagc agcgtgacgt ctacgccctc cttggcgagc 10980

gtgcagagct tgtccttgac gtcgacgccc tccttggtgg agctcaggtt gcagcagaag 11040

ctgcagatgt acaagaactt cttcgcggac tcggcgatag cggtgaagca gtcgagggtg 11100

ctcatgttgc cctgcgccaa agacgccacc tctgcgggca gcgtctccac gacgcggcag 11160

tcggcgccca gggggatgga ggagaacggc cacatttatt tatctcacaa aaataatagg 11220

gcttcaggga aagtctttta gcaggcgggc gagttcttcg agttcgctta ggagttcttc 11280

catttcttcg gaagtcagca actggagctc ggacttgatt tgaatatctt cgaggaaacc 11340

gtctagcatg ttcgccatgt cttccgggga gcactgcgcc acatcttcgg ggacaggatc 11400

gggtgtgggc attaggtctc cgcttacttg aacgtcgtcc atcatcctgt cgatgaggtc 11460

ttcgacttct agacggggtc cgtagatcag catatttggt gatggaggta gtttaaggtg 11520

cgagagttag tgttatacga ccgccaacgt gtgtttatcg cgcgtacatt ttcaataatt 11580

aacaaactcc ccttcctgcg cctgctcgag aagcagctcg tccagctcct cctgtcggcg 11640

cgcggccacg cgtctttccg cgaagagtac catcagctcc agccccacgc cgcacagacc 11700

caggacgccg aacaccaccg ccgccgagat cgacagaccc agcagcaccg acatcctcac 11760

gcgggcatcc ggctatttaa tcgttctgga aacgtattaa tatgggcgtc gtcatgtgcg 11820

ggtgtctgtt tgtgtgggcg ggctggatcg cgcgccgcgt gcgcggcctc tgcgcggcgc 11880

tgcgccagag ggtgtcgcgc gacaagggct acgtggccgt catccagacc tgcgacgacg 11940
actacttcac agaggaggag ttcgacgacg gcaagcaggt ggtcgcgctc ctgcgcgacg 12000
tctcgcgcgt ggttgccgcg cccgcgggcg tgacggaata agttaggata aggagtcgag 12060
gggagaaaaa cagcggtcac actataaact cgcgcgaggc cgattttgac gtgctcatgt 12120
ctggaagctc cgctttctgc agcgcggagc ggcacacgaa gcacacttcc gtgttggtgg 12180
gagttatgca gtggacgtgg tagccgtgcc cgcacaccat gacttggaac ggacacgcgc 12240
cgggacaggc cgcgtttatg catccttccg gcgagcgctt gttgcagatg tagcacacgt 12300
ctgagcacgc cagcgtgcag gacacggcca gcttccactg cttaacctta acgggcatgg 12360
ctagttgaac acgaccatgg gcgagtcgcg agcctcgagt cgggggttca gggcaaaccg 12420
tttcacgccg tcaacggttc ttctctttgc aattttctct ctgcacaggc tcgtcagcgt 12480
catctcggcc aggcgcgcgt cgttgcctag gtgccgcgcg gcgtcctcga ccgtcacgcc 12540
cgtcttgccg gcctcgtcca tgagcacaat gcataccagg tgcgcgctag agcatatgac 12600
ctcctgctcg cgcccgccgg cagcggggat ggttagctcc gcgcgcccga aggccgccag 12660
cggcgccacg tcgtaggcag tgtctgctcg ggcgagcgcc gactccacgg caccgcggag 12720
cgactccggc ggcgtcagcg cggccagcgg caccggcgtg ggcacggtgt acacgttcac 12780
gggcatgagc accatctccg ggtcgtggtg gccgctctct tcgccgtcgt gctccatggg 12840
ctgcggcggc ggcagcagcg ggagcagcag ccgtccggac atgagccggc gcacaaggtc 12900
gttgagcgcg gacgaggcca tcggcgggta cagctccatg gccagcttca gcgataggtg 12960
cttctcgagg ttgacgccgg tgtagacgct cttcacgatg cgcgcgaagg ccacgcgcgc 13020
gaaggccgcc agctcctcgc gcggcaggcg ctcgatgtag gagagcagca tgtcggtgtc 13080
gcacggcggc gccgcgacca ccgcgccgta gagcgccttg cccgagagct tttccagcgc 13140
ccttgcgtgc aggccgtggg tcttgagcac gtccacgtag ttcacgtaca ggcagagcgc 13200
gcgatcgagg ttgctctccg cgacgtgcgt ctcgatgcac tccacgatga gcgggcccat 13260
gcggtccttg atgaggtcta tgagcccgcc gtaggcgact cgcgcgctca tgaggcacgt 13320
gcggcagtac gccatcaggc cctcgaggtc cgcggctatc acgtcctcga ccacgttcgc 13380
cacgacgcgc tgccagagcc gcaccttgct cacgttctgg tgccgcacca tgtccacgag 13440
ctcgtcgtac gagccgccgg gctcgtgcgc gcgatcgacg atgcacctcg ccatggtgcg 13500
gctctggcgc atgagctcgt tcgtgaagcg cacgcacgcg tcctcggaga agagcgcgct 13560

caggcaggag tagcagcggt ccgcgacgag gtgcgggaag cggcactcca cgacgccgcg 13620

gccgatccgc agcacgcact cgccgtacat ctcgtccatg gcctcgcgca gacagtcgtc 13680

cagcacgtcc gcgttgtgcg cccactggat cacgcagagg tagggctcga tgttctcgcg 13740

cgcgttttcc acctcctgca ccatgtactc gagcacggtc atgtcctcgt ggatgtcggt 13800

gcccagcatg cgcccgggcg gcagccagct cttgcgcgcg atcgcctctc gcaggcacgc 13860

caccgccgtg aaggtgttga cgcggagctt ggtcagcagc cgccgcagtc gggagatgtg 13920

tgccacggag aggtccatct ccatggcctg ggcgatgagg cgcgtgagtt cctcctccat 13980

ggcggcggct ccgcgggcag atatacgcga acaacggtaa gccgtgctat ttcatttttg 14040

gacaaaaagc tagtcgtcga cgcgcatgtt gtcgaggttc cggcacagcg agagcacgtc 14100

gtcgcgcgcg cgcctccggc gcagttgatt gttcgcgcgc cgcgcgtccg cgagcgcctg 14160

tctgtacatc gcggagtccg cgtacccgtg cagcggcgag cgccgagcgc cgggcctcgg 14220

gctcgcgcgg cgcgggagcg gcgttggcgc gcgcctcgag cgccgcgcga agtgcgcctg 14280

catggccagc aggcaaccga acggcaccat gtatcggtcc atgaggcact ggctggccgc 14340

ggacggctcg cgcgggtgca tcacgccgcc gcccacgtcc tccatgacgt cgcgcagcac 14400

gcagcgcagc atggtctcca tgctgcgctg cgtgaaccgc accggggagg cgtcgacgta 14460

gaagccgtcg gccacgaagt agagcgcgtc cagcccgccg agtttctcgc cgagaccgac 14520

gaagagctcg tccacgtgcc agtccaccac cgaggccttg aagagcacca cgtgccggat 14580

gtcgtgcgag cgcgcgagct cccaggtgtc ctcgccgatg ttgctggcgt cgatgcggcc 14640

tgtcatgcgc acgctcacgc acggcgtcat cccgttcttg tagcagaact ggcgcgcgag 14700

ctcctcctgg cgtacgatgt cgaccatgct ctccatgaag gaggtggaga gcagcatcgc 14760

gccgcgcgcg gcgcgggtcg cgttttcgtc cacctccact tccatcccgc cgccgatcct 14820

aatcatctat cgtatttaaa ttttcggcgg agcagacacg cggctgctcg ctgcgcgatc 14880

gcttcagccg cggcggcgtc acgcacgcgt tgcggcggcc ggcacgcacg gacgaccgcc 14940

ggggctgttc gctgagcgag cgccgcggcc gcgtgacgcg acagtcgcag gtgggttgcc 15000

gggagtcgct cgcgcgcctt cttcgcattt cgccggaacg ccgcgtttat gtaggggatt 15060

atattttcaa cgtaactaaa tggacggggg cgtgcacaaa cggcctttca tcgtgaacgt 15120

ggatggcatg ggcaaggtgc tcgtgctccg gtacttgcgg atgtgcgagg tgcccgaggc 15180

caagtgcgag gggtcgcgcg cgtcctgcgt gctcaagatg gaccctcccc gctcacccag 15240

ctgcgagcgc aggccgtctc tcccgccgtc ccccccatgc cccatgcgca cgcctcccgg 15300

gtcgccgctc caggctccct tgatgcgcac gcagatgctc caggggctgt tcgacgctgc 15360

caaaaacaac ggcgagcaga tgtgccgccg ccagtaacct aggctgcgca gtacgaaagt 15420

tagtgcgtga tcacgttttt tgcaatgtcg atcacgccgt gcgtgcccgt cttgcgctcg 15480

cgctccacca cgctagtcac gggccgcgcg tccgagacta gcgaccccag catcgagcgc 15540

acggcgccct ccgcggcggg gtggcgcgtc agcagcagga acatcacgat gtgcgcggag 15600

acgccgcgcc ggctcagatc gtgcacggcg tcgccgtcca tgagcacggt gttcgagaag 15660

tacgtgaaca gagtgttgtc tcgcaccagg aaggccgagt tcgagacgct ctcgaagtcc 15720

acgatctcgt cgtcctgcac gcccatgtcc aacagcgtct gcacgagcgc gggctcgtcc 15780

aggaacacca ctgcgcgcgc gaacccgcag tccagcgcgc gcgcgtccgc ctccaacacg 15840

cgcgaggcgc cgccctccgg cggcaggaag gcgcagggca gcggcgtgcg tccgtccgcc 15900

ggcgcctccc cgagctcctc gagcgcgaag gccagcagcg tctccatgcg cgcgcgcgcc 15960

ttgtcgaagt tgtccgcgag gtcgcggatg cggtctgtct gcgagaacat cttcagcatc 16020

gccatgagct gcacgaaggg gtgcagcacg tatatgttgt ccacgagcag cgtgggcagc 16080

gcgcgcagcg tggcctgccg cacgttgaag ctgtccagga tgtgcccgcc ctcctcgtcc 16140

tgcagcacca cgtagttctt caggtagggc acgcgcagca gcacggtctg ccgccccgtg 16200

acgaagtata tcaggaaggc gaggttgatc aggaacgggc gcgcgttcgt ctgcaccatg 16260

tcgatgtcgc cgtactctat ctcggggttc agcaggtgca gggcgtacga gccgtagcac 16320

acgcaccgct tgttgtgtcg gcgcaggtgc tccttcacga gccgcttgac cacctccacc 16380

aggtccgagt gcttgtgccg cgccatcggc gcggcctcct cgggcggcgg cagcactgcg 16440

tacgagttga gcgcgcggct ggcgagcgcg cgcgcgcggg gcgcgtccac gcgccgcacc 16500

gccgcgttga ttgcaggcgt cggcgtcgtg agagagccca gcgtgcgcgt gaactcgctc 16560

acgatcacgc tctgcagctc cagcaccgtc aggatctggc ccagcttctc caggcgccgc 16620

tgcctcgaga agtactcctc gatgcgcgcg gcgatctcct tctcggagcc gcctagcttc 16680

ttgaagaagc gacgacgact ctttacaaca agagagagaa aaagcttcct atcgaagttg 16740

aggacgcggg tcatgttgcg gcgctgcgcg cgcaggagca cgcagcgctc catggagggg 16800

cgcgagccga ggtactcttc gatcacgggt ggagccatga cagctatttt ctgaacccgc 16860

gattattgta cagcgcaagc cgcgcgcaga cctgctggca cagcagcgtc gtgtttcgca 16920

tgcacacgcg cgaggactcg atcgtgcgcg cgtccggtgc ccaggcgcgc agctccatca 16980

gttcctgctc gacgaagtcc acgggctcca cgaagcgctc tgcgcagagt ccgtccgtga 17040

acgcgttgac gatctgccgc acgagcacta ccacgtccac ctgctccacg aggcgcacgc 17100

ccatggcgac gtgcacgaag aggcagcgga agagcgcgtc catggccatc tggtggtccg 17160

agcagggccc gaccgcggtc ttgcagccca gcgcgaagcg cccgatgccg cggtactgca 17220

ccatctccga gggcgagaag gagagccgct ccatcttgag cacgggcggc gggcccgccg 17280

gcagtccgcg cgcgaggtcc agcaccggcg tccacccggg cgtgaacatg tccgggatca 17340

ggaagagccc gtagctggcc atgcgcgcga tgtcgaaggc gtggtccacg accttgttca 17400

cggcgctgtc cgcgcggttt acgcgcagcg cctgcaggat cacgtttccg gaagcgtgcc 17460

gcgtgatcgc gaggtccgcg gtcgcgtacc cgcgcaggcc cggcaccgcg tacgcggtca 17520

gacacacggc caggcgcgcg ctgtgctccg aggagaagat ctcctgctcg tagccctcct 17580

cgggctcctc gcactcgcga gggcgccgca cgtcctcgac cgagcgcagc cgcatctctc 17640

cctccgacac cagacagcca agcgactccc tcaccgccgg cgcgagcacc tccgtggcgc 17700

agagcgcgtc gtgcacgcgc ttgagtgtgt tcggcttcag cgcgtagccg aagagcagcc 17760

gcgtcatccg cgagcccgag aacgcgaagc ggcgcacgta ctcctccgcg agctcggggc 17820

ggtcgttgat ccacgaggta gagaagacgt ggtcggaggc gaaggggtct gcaccaaccg 17880

cgagcagtgt ggacagaggt acggtgtcga ggaagtccac gacgtcgggg aagttctggc 17940

gcacgcaggc ctcggcgacg cgtctggtgt gcacgcacat gtcggtgacg ggcacccggt 18000

ggccggactc cacgacggac acgcagacgt cctcggtgac ggcgtccacg ggcatggtcc 18060

gcagcagctt gccgagcacg tcgccgaacc cgccctcgag cgccttgcgc cacacgaacc 18120

ccgcgtcgaa cttcccgggg aagtccgcga tcaccgaaag ctccgcgtgc gagaggttgt 18180

ccacgttgag gtaggtggcg gcgtccacga agatgggccc gaaggcgccg gtgtcggaga 18240

cgcggtctct gaggtagtcc ctggcgtagt ggaggtactc ccgcacctgg ccggcgcgga 18300

tgcgctcgag cgcgaaggcc ttcatggtct cggagcagag cacggagtgc cgcagcccgt 18360

ccagtgtgcg ccgcacgtcg tagacgccgc gcatctgcgc gagcatctcg acggcgtccg 18420

ccggcgtcgc cgccgcgagc cccgggttca ctggaggtat cctgtgttct gcgagcatgc 18480

gcttgaggaa acagaggtcc agcggccgcg tggtgtacag cgcggaggcc atctcggggc 18540

gcgtctcgac gatgtcctcg atcatctcgt ccgtgaatgc cgcgttgatg ttgtgcacgc 18600

tgcgcgcgtt cacgtgcagg aggatgtcgc ccacgttgtc ggggaatcgc tcctcgatga 18660
gccggacgtc gtcctccgtg atgttcatgt agggaataca gcggcagagc agcgcgtagt 18720
ccgcgaactg cgcgatgtag ggcgtgtgga actcgatgtg tctggcgaag agcgcgccgc 18780
agcgccgccg cgagagctct tcgagcaggt cctcgggcgt gacgtgctgc gggcggaaga 18840
ggtgcaggtg cgtggggtgc tcggcggcca cgcgcgcgta cagccgccgc gggaggtgtc 18900
tggggtgcac gccggcgagc acgagatcca tggcctctga ggtagacagt gcggcgaacg 18960
cgcgctcggt gccgcccgcg gcgacagcgg cgccgacaaa tctcttgagc agctgcagca 19020
tcgcgtgttt gggctttcgc ggaaggcgct tattttaatg ttattggcgg tggccggtgc 19080
gagataaaaa ttagaactga tgccgcagtt gttgatgata tgatgattgc gctggccggc 19140
gcgagataaa aattagaagc tgatgccgca gttgttgatg aggatggtga gtgcgctgga 19200
gcaagcggtg tggcgcgcta gcttcttgct ggccccgtcg gccacggcaa cgacctttcc 19260
ggatatcgtg atggtgcagg tgaagcgcgg acagtgatcc tctccgccag cacgcgtctc 19320
gcagaactcc agaggtctgt gtgtcatcat gcagaactcg ttgaccgcgc tgaccgggtt 19380
aagacttttg aggcgtatca cggcagactg agtcatgatg tcgatgtcgc cgccgaaaag 19440
cgtatcgcac ccagcctcgg tctccatggg ctcggtgtcg gagttttcgt cctcctcggt 19500
gggcgcggag ggcgcgcact ctacgaacca gcggggcggg tttccgtcct cacagcaaac 19560
ctcgtccgag tccagcaggc ggtacagctg gcggtttgcc tcgtgtttgg atatgccgag 19620
ctccttcgcg atttgcttgg ccggcagctt gtcgtcggat tttctgagaa gctcgaggat 19680
cagagacgcg cactcgcagg ccattgtggc gatttacggg gcgtgcgttt ttttaggatt 19740
ttggcttgcc tttcttttcg cagaacttgg gaggattgaa actcttttgg caatttttgc 19800
aggcgtactt gatcaagggc ggctcgtccg ccgagcgcgt ctggatcatc atcggcatgg 19860
tgttcttgct ctggcacgag gggcagggca ggttgaactt ctcgtcgagc acgttgaagt 19920
acccgctgta gtcgtggtcc ggcacctcct cgatgtcgta gggcacgcgc gcggccacgc 19980
acttgaccgc gaagagcagg taccgcagcg cgtcgtgctc cgcgccgctg gtcgcgcgga 20040
tctgcgcgca caggtccgcg tagtcctcgt tcgcgtccac ctccaggctg cgcttgttct 20100
tgtacgagag ccggttcttg gcgtccttcg agtactcgat gccgatgttg tgcgcggggt 20160
caaagttggt ctcgtcggtg ttcgaggtct tggtgttcac gatgttcttc agcgcgaagc 20220
gctgcgcgca gtccagcgcc catcgcgcga tgcgcgcggc ctccgccgcg tcggtgtgct 20280

tcgccgcgag gtcgcgcagc cggtcttcgt ccatcgcccg attttaggtt gggtatatta 20340

tctcaattcc gctcttccgc gggccgcggg cgcgcgcccg cggcaaatta ggcgttacaa 20400

atggacttcg tgcggcggaa gtacatgata cacgccatcg accgcaacct cgacttcatg 20460

aaggccgagg tccagcagaa ggtctccatc ttctccctcg ggcacgtgct cgcgctccac 20520

tacctggtca ccgcctttcc gcaggcggtc atcaccaagg acgtgctcgc gagcacaaac 20580

ttcttcgtgt tcgtgcacat gtcgcagcgg cacgaggtct tcgacgccgt gctcaaggcg 20640

gccttcgacg cgcctcagct ctttgtgcgg gcgctctcgc ggcacttcga ggccttcgtt 20700

gccgccatcc gggcctaccg cgcgacctgc gcggagctgc tggccgacgc gcgcttcatg 20760

gaggtggctg cgcgcgcggc cgagctcgcg gaggtcattg gcgtgaacca cgacatcgcc 20820

gcgaacccgc tcttcgcgga cggcgagccc gtgcgcgacg cggagctcat tttcgcaaag 20880

accttccgca agaccgagtt ccgcgccgtc aagcgcctcg ccgtgctgcg gctgctggtc 20940

tgggccttcc tcgtgaagaa ggaccttggc ggcgagtacg cggacaacga ccgccaggac 21000

ctgtttacgc tgctgcagaa ggccgcgggg cccgtgcgcc acagcgcgct cacagagagc 21060

atccgcgagt acctcttccc cggagacagg cccagccact gggtctggct gaacgcgcgc 21120

gtggccgacg acgcagaggt gtaccgcgac cggcccgcgc gcacgctcta cgagcgcgtg 21180

ctcagctacg cgtactcaga ggtcaagcag gggcgcgtga acgccaacac gctcaagctc 21240

gtgtaccggc tcgaggacga ccccgacatc aagggtctgc tgctgcagct catctacgac 21300

gtgcccgcgg acatcgtcgg cgtcgtggac tccgcgaacg aggagtggcg gagctacttc 21360

gtgagtctgt accgcgagaa cttcgtcgac ggacgcacct tcacctcgga cgcgcgcttc 21420

cgcgacgacc tcttccgcgt ggtcgccgcc gtcgatcccg acttcttcga gcccgagcgc 21480

atccgcgagg ccttcagcgc agacgcgcgg ctgcgagagc gcttcacgga catggacctc 21540

aacaacgcct tcatgtcgca cctcatctac gactccgtgg accccgacgt cgccgccgcc 21600

gagcgcgggc tcgcactgcg cgtgcacaac gaggactccg actacttcat ccgggagtac 21660

aacacctacc tcttcctcag cgagaaggac ccgctggtgc tggaccgcgg ggcgctcacg 21720

cggctctcgg acgtccccgc cgagcgcttc cgcgacctct tcagcgacag tgtgctgcgg 21780

tacttcctgg acgcgaagct gggcacgctc gggctggtgc tcgaggacta ccgcgaggac 21840

gtggtcgccg ccatgcttcg gcacctgcgc cgcgtcgagg acgtgtcttc cttcgtgacg 21900

tacgccgcgc gcaagaaccc cgcctgcgtt cccggcgtcg tgcgcgcggt cgtgagcaac 21960

ttcaaccccg cggtggtcgc ggccatgcgc cccttcctgc gcgagcacat gacgcgcgtg 22020

gacgcgctgc tggacggaat gccgcacctc tcggaggccg accgtcggta catccgccgc 22080

gtggtgctgc agggccgcgc ctgattcgcc gtcaataaat cgcgatggtg gacagcggca 22140

cgcacgacgt ggactccgcc gcgcaggagc gcacgcccaa ccagcagacc ttcttcacca 22200

aggggctcag tccgctgatg cgccacacct acatctacaa caactacgcc tacggctgga 22260

ttcccgagac cgcgctctgg agcagccgtc tgggcgacta ccgcgtcacg gacttctacc 22320

cgatctcgct gggcatgctc aagaagttcg agttcatgtt ctcgctgctg gcggaccccg 22380

gcggcgcctg ccccgcgtac gagcccaagc tcaacaccga gttcctgaac cgcggctcct 22440

tctcgggacg gtacgtgaac cccttccacc gcttcgcggc gctgcccgag cgcgagtaca 22500

tatccttcct gctgctgagc tcggtgccca tcttcaacat cctcttctgg tttaagggcg 22560

agaccttcga cactgccaag cacagcctgc tcggcgccgt gtacaccacg cccgagcggc 22620

acatcgagct cgcgcggtac ctgcggcgca cgggcgacta caagccgctg ttcagccgcc 22680

tgggcaacga cgacacctac tcgaagccct tctctgggtt cacgcgcatc agcaaccccca 22740

cgcccatcgg gcggctgccg ccctcggact tcgagacgct ggccaacctg agcaccattc 22800

tctactacac gcgctacgac ccggtgctct gtttcctggt cttctacgtg ccggggctct 22860

ccgcgaccac gaagatcacg cccggcgtgg agttcctcat ggagaagctc tcgctcgcgc 22920

ccgagaacgt ggtgctgctg tagcctcaaa cataaaatat aggcgccttt gatcgcactg 22980

cttcagttca gacagagcta agatggcttc ctacatcagc ggcgctagcg ccagcgcgaa 23040

caccgcccag ggcggcgatt ctcagtaccc acagtactat tatcacacac gcacctccca 23100

aggcgacatc cgcgacgaaa gcgaaggttg cttccacacc acggacgacg agcacttgga 23160

tctgtccgac gactacctcg gcgatggcgc accacactgc ggacacagcc acaaccacag 23220

tcgcagagat ggagatcggc accgccagcg cgcaccgcgg ctctatgagg acccggtgcc 23280

cgcgaacatc atggtgccca cgctcagtct agagcagctg ctggaggaaa cctcggtcgc 23340

ggggggcctt ctcggcggca ggacagagag ggacgtggaa cagctcctgg aggagttctc 23400

cgcactctgt cccggggacc agatcaccgc gctgcgctgc atggcggcct ccttttaccg 23460

cgacgcgctg ttcgcgccgt acgcctgcat gcacctcatc gccagtcgga tgcgcgtgca 23520

ctacgcgcgc gaggtcgtgc acgtggccga ggacctcgcg gacgcgatgt ctgcgaacag 23580

cggcgtctgc ttccggcggt accgaaagcg cgtgctagag gacatgctcg cggaggagat 23640

gggcgtgtac aattacctcg cgcgcgccaa cgcggacatc tgcgaggaca acctgctatc 23700
ggccgtggag acgctgctgc ggcgcttccg tcggatgggc tgctaccgct ctctgtgcat 23760
gctcaagatc ctcgcgctgc agcacgagga cctggccggc ttcatccgcc gcagcataag 23820
aaaaacctgc aacttggcac acgcgcgcac gcacacggtt tacgtgtagt taccctgtaa 23880
agacgggctt gctcccgaac aagcgctcga agaagagcgt gcacatagcc ttattgtcca 23940
gcaagttgac tatctctgta cacagcctct tgaagtacac ctcgtacatg atccgctcgt 24000
ttttatccag tctgaaggtc ttgtcgacca cgcgctcgta ggacttcacg ttcgcgatcc 24060
ggcgccgcca ggggccctcc tcgcacacgt acgcgaagaa gtagcgctcg ccgatctcga 24120
tggcctccgc gttcgccgcg ttgtaccgcg tcactagcgc cacgttgggg ttgtcggggg 24180
acttgaagtt cttgtggtgc gttcggctca gcaggaacca gtccagcggc atgctgcgcg 24240
cctcgaactc gaaggtgagc tcgtcctcca gcgagcgcag gatctccacg cccacgttcc 24300
cggagccctc ctccgccagc gcgcggcaga gcatgtcctt gtacttgcgg atcatgagct 24360
tgtggaaggg cgccacgtcg cggcgcgtct cgctggtgcc cttgctcacg cgctcgctgc 24420
cgccgccgtc gctcaccgca aacttgatcg tggtgtactt cttcttggac tgcatgatca 24480
ggttgcagta caccgcttcg aactccacct tgaagttcgc gaagagcacg tgctcgttga 24540
tcacgcgctc cagacagcgc cccacgcgcc gcgagaacgc gatgtcggag gcgcccacct 24600
ccaggaacac ggagtcggtg tcgccgtaca cgctgcggaa gcccacgcgc tccgtgcgct 24660
cgccggccac cgccgcgtcg atctctagct ccgcggcgcg gcccgcgaag gcctcgtcgc 24720
gcagcagcgg gttgtccggc gccgccgcca gcgacagccg cgtgccgcac accgacgcgc 24780
cgtctagcgt gcgctccagg tacgcgatca tggtgcgccc gatggccgtg cagctcttgg 24840
ccgaggcgta cgagaagagc gcgctgttgc ggaagcccat gagcccgtac acggagttgg 24900
ccgtgatctt gtacgtgtac tgcatcgagt tgtagatctc gcggtccacc gcggtctccg 24960
cggccttcat cagcttcttg tacttggcgc gcgcgtccag gaaggagcgc agcagcatcg 25020
ggatgatgcc cttggcctcg cggtcgaaga tggccacctc ggcgacgagc tccggcgagc 25080
gcggctcgca gggcaccgcg atgtaccgcg gcgccgggaa catccgccgg acgtccacgg 25140
ccgcgacctc cgcgtcgagc cggttgtccg agacgaccac gccgacaagc gtctccggcg 25200
acaggttcgc gtagatgcac acgttcgggt acaggctgtt gtagtcgaag atgagcacgt 25260
gcttgttgtg catcttctgc ttgggcgcca tcacgcggcc gccctcgtag aagaacttgg 25320

acttcgtgtc cgcgcgcacc atcaccgtgc ggttctccag cagcagcttc atcagcgggc 25380

ccttgatgca ggtgctcgcg cggtactcga agaccacgct ctgcggcagc aggtacgtgg 25440

acgcggcggc cgcgatcttg gtctccacgc cgtagtgcga ccagaggtag aggcagaggc 25500

aggcgtcgtg caggcagtac cgcgccatgt ccagacacac gtccagcgag tagttcgcgt 25560

acatgtccgc gaggctgacg tcgtccttgc cgaaggccag cgtgacgcgg tcgccgggcg 25620

cgcgcgccgc ggggtccgcg aggtccacgg tgaagccgtc ctcgtcgacg cgtttgtgca 25680

gcacgcggca cacgcgctcg tccacggtca cgtagttgcc ggtgctgagc acgcgagcga 25740

acacggccgc gttcccgtcg gcgtcggtgc tgcggtcgcc gcgaaagcgg accgcgtccg 25800

gccgcgcgtc ctccacgacc gcggtgcagt ggaaggcgtt cttggatatg gcgtccagct 25860

tgtaggagtc cagcttctcg gtgcgctgga tgaaggcgta caggtcgaag tagatggtcc 25920

cgttgttgtt gttgatgtgg aaggtggtgc tcgagacgcc gccgacgccc ttgtggctgg 25980

acttcgtgcg ctcgtacacg cagaagttga ctgtctcggt cccgtccggc agccggaagc 26040

ggatgtgctc gcccgtgagc agcgacagcc gcgagtccag gtaccgcagg tcgaagttgt 26100

ggccgttgaa ggtgaccacg aagtccagcg gcatctcgag caggcgcttg gccacgcgca 26160

gcagcgtcac ctcgggacac agcgtgacct ccgcgtcgaa cttcacgtcc gccgggtcca 26220

ggcagaccgg gatctcgcgc cgtgccgcct cctcgaggtc cgcgtcggag agcatatcgg 26280

agttcgtaag cgtgaatcgc cgctccgcgc cgtccttgtc caccacgcag aagctgatgt 26340

gcgagacggc gttcttgaag acggaaggaa actttttctc gaagtggcac tctatgtcga 26400

ggaagagccc cgagcgcgtc acgttgaagc gcgggatctt ctccgcgaag cacgcgccgg 26460

ggtcgtcgca gtggaagcag ttgctcccca ggtcgcgcag cagcgcgggg tccacgcggt 26520

agcacccgtc agggtcgatg tcgtgcgcca cgaagaacca ggacacgttc agaaagtcgg 26580

acatgaagac ctctggcggc gcgagcttgc gctcgctggc caccagacac agctctatct 26640

ccgagcgctg gcgctccgga atcttcgccg agcgcgctac gatctcgtcg atgctgacca 26700

cggacatggg cccgagcgcg cgcgtccacg ccagcggctg ggcgatgtcg gccaccgcgt 26760

ccgcgcgcac cacgtagtaa aagtgctgca cgaagcgcag gtacacgacg gcgttgtcgg 26820

cgcggcgcgc cttgaggaag aggaaccggc tgtcattgcc gcggttctcg aaccagttca 26880

aacatttcag ctccatttca aagagcataa taacatttca tttaaatgga gcctcgcttc 26940

tggggccgcg ccatgtgggc ggtgatcttc atcgtgctgc ggcgcttcga ggagcaccgc 27000

gacctcgagc gctgcaagcg gcagctgtac gtgatctgct ccacgctgcc ctgcatcgcg 27060
tgccgacgac acgccaccgc cgccatcgag aaaaacaacg tcctctccag cgaggacccc 27120
aactacgtgc tcttcttctt catcaagctc ttcaacaacc tcgccttcga cgacagatac 27180
aagatcgacc ccgcgaaggt gcgcccgctc gtctagagca tgccctcgta cgcgcgcgag 27240
ttgtccgagt acacggtcac cgcgatgccc tcgcgcggcg tcacgtgcac gtgcatggag 27300
tccgtgatca cgaagcccac gcccgtgacc ggctcctcgc ccgcgtcgtc cgtgaagagc 27360
agcccgtggt tgaagaggta gaactcgttc tctgcgagcg aaagccgccc gcggtcgcag 27420
aggtagtaga agatgtcgta gtcgcgcacg gccagcgtga acgtggactc gctcaccacg 27480
atgtacttgt ccagctcctc cagcacggac gcggccgcgc ggcgcgcggt ggcgcggcac 27540
tgcgtcgggc actcgcacgt gtctgcagag tacaggatgc gcgttcgccc cgaggcggtc 27600
tcgagaaaaa cgttaatcgc ctccatcgcc cagaagcgac tcgaggatcg cgagcaccgt 27660
gcgcagcacg agcccgatgg tgcagaaggg aacctctccc gactccgagc actcgcggat 27720
ctccgtctcc acgcggtcgt gcacttttat ggagggagcc gtcgttccag tggcctccat 27780
cgcgacggac accaccttgg ccacgaactc gcggatcttg ctcatgcgcc ggagcacggt 27840
cacgcggaag aagacggccg cgagcaggta ctcggccacg cagctcacgg cgatgagcgc 27900
ctgcgcgtgc ctggtgttga gcacgtgcgc gtcgggcacg aagtccgaga tcttcaggtg 27960
cgagagccgc acgagcgcgt tggtgtcctt gacgccgtcg caggaaatgt tcgcgaagat 28020
gagcttctcg tagtcgagcg cctcgaccac gcgctgcgcg tccatgtgcc gctcgcccga 28080
gagcgcgcgg ctcaaagggc cccagcacac cgagcccgcg aagcaggggt ccaccacgcc 28140
gtgcatcgcc agcagcgtca cgtcggccac cgccgcgagg atggccgcgt cgtcgagctc 28200
gttcgcgggc gtcgcgcccg tcagggacgc gctgcgcagc gcggacccgc ccggcgccgc 28260
gtgccgcgcg cagaactcgc acccgcagcc cggcggcagc ggcggcttcg agagcagact 28320
tatgagcccg cccacgtgcc cgtgctcggt gatcagaagc gccaggatgc tgtcggtgct 28380
gccctctatg gcggctgtgg ccgcgctaga aggctctccc gtgacctgcc atccgcagac 28440
aaccttgagc atcttgcgct tgagctcgtt gggcgcctcc gaggccagca tcgtgcgcgg 28500
gaacgtcgcg ttgaggcgga agtcctgcag cagcttttcg agcgtcgcgg tcttggcgtg 28560
ccgccctttg cgccactcct cccccaggtg ccacatgagc tcctcggccg tgtccagcgt 28620
cggcgagacg cgggccttgg gcacgcgcgc cgcgctgcgc atcagcggct ccgaggcgcg 28680

gaagctgccg cgcgcgtgca ggcgcatgga cgcagacgag gaccgcatcg agggtctctg 28740

gtggaagctc gtcatcgtga agcgccgcgt gagcggacct acctcgtcgc gcgactcgtc 28800

gaagtccggg tcagggtccg tcgtgtcggt ctcggcgctg tgcgtgctgg gcgcgctgct 28860

gcgggcgctg cgcgtgctgc gcgtgctgcg ggtgctctgc gtgctgaagc tgcgcgagca 28920

ggagtccgcc gtgtccgctt cctcgtagtg gaagtccacg tgctcctccg gcagccggcg 28980

cgagcgcgac ttggagatgc cgaccatctt gtccgcgccg accgggcgca cgcagagcgc 29040

catcgcgccc tcgcgcaccg cctgcgcgaa ctcgcggctg gcaaccatgc tggggttgag 29100

gaacttgatg atgttgaagt atggccactc gcagacgagc cgcgcgcagg tcgcgacgtc 29160

gtcgacgctt ctgagggccc tgttcagcgg ggggatgttg ctgccgtcgg ccagtgcgac 29220

gaggtcctcg ggggagatct cgagcttggg aatcatctcg ttcacgagcg cggggtcgat 29280

ggcgtggcag acggtctcta cctcggtgcg cgagagcttg agctgcgcgc aggccgccca 29340

cggcgcgcac gtgagcgcga acaccgcgga gactcggccc ttgccgacca tcgccacaac 29400

ctcgggctcg tagaccaggc cggccttgag cagagcctcg agcacctcta tgtcgtcggc 29460

ggcgagcagc gcgcgcctgt ggaagcacac cgcgggcatc atcttcttgc agatgccgcg 29520

ggtgctcttg agggccgtga taaggtcggg gagcctgacg tgctgcggct ggaagaacat 29580

gacgttggcg gggctcgcgg ccaccgcctc gtcgtacacc gacatcggca ggtcgccgtg 29640

gagcccgcac ggcagcaggc tcagcagctg cgcgcgcgag agcttctcgg cgcagaagtt 29700

cttcttggcc agggcggccg ccgcgtcgcg ggccttcttg gggtataaca acatggcggg 29760

ctttaaacac gaaacaaaaa tccgggttgt aacatttcaa ttttgcatgt tctgggcctc 29820

ctcgcagagt ttctccaggc cgccggccac gatggcgtcg acgaagaggt cggcctcggt 29880

gaagcggtgg ttgccgcgca ccgccaccag cgcgttctcg gtgaccacca ccgacagctg 29940

ctgcgcagcc gtgcgcaggt cgaagtgtcg gcacgcgagc ccgtggccca gagtctggtc 30000

cagcgccgcg agcacctcct ccaggctctc ctcgcggtgg ttgtagaacc acatcagcac 30060

gaagtaggcc acgtaggtgt agaggtagtg cgcgaccgcg cgggcgcgca ccagcggctg 30120

gttgcagcgc gcgaaggcca ttccgctggc gatgaggtcg tcgtccagcg tggcgtagtc 30180

gccccagttg agcgcgctga actgcacgct gtagaccgcg cgcacgaacc cggactcgag 30240

gatgtcgatc tgcgacggcg cgccccaggt caccagccgg tacacgatgc cgcgctgcat 30300

cagccgcatg aggtcgccgc cgacctcgcg caggcggtgc gtcagcgagg cgaaggccag 30360

cttccgctgc gtgtactgca ggcccacggc cacgcacccg tccgactcca ccagcacgag 30420
cttgtggtcc gtgcagccgt cgctgcgcag gccgccctcg cgcgccatca tgtcggtgac 30480
gaacatgtac ttgcgcgcgc gcggcccgac caggatgcgc ttcttgcctt ccatgcgcag 30540
caccacgtcc tctagcagcc gcgtgctgtc cacgcgctcc acctgcgggt ggatggtggg 30600
ctggtagttg tacagcagcc gcggggacca gttgtaggcg aacgcgaaga ggtgcgtgtc 30660
caggagagag atggggaaga cgccgccttc ggtggcgcgc cggaggatgg cgagcttggt 30720
ctccgcgggc agcaggctcc cggtcaccgc gccgaagaac atggggtggt tgcggaactt 30780
gagcgcgtac gcgctcagca cctcctctcg cgagaatatc gagtccgcgg ggttggagct 30840
cgcgggcagg agcacgtcct ccacgctcag cacctcgtcg atgaagggca gcaccatgtc 30900
cacgttggag gcggtgaacc actccatgtc cacggcgttg cgctccacga tcacccggat 30960
cgtctcctcg gatatgttct cggcgaaggc gctctgcagc tcgatcaggt tctcggtggc 31020
gtcgatgctg agcccgaggc gcatgccctg ttgcagcacg tcgttgatgg ggttcacgta 31080
catggccacc gccatgcacc cggccggctg cacgcgccag aggttggagt acgcggacac 31140
gtccgtgatg cgcagcgtct ccagcacgtt gtacatcgcc gcgcgcattt ccagcgtgtc 31200
cacgtacacc tccgcgtgct cgaagatgat gtcgagctcg aacgcggaca gcggcagact 31260
cacgtagatc tggcaggcgt caaagagttc ctgcgcgtac tcagagaagc acgcgtacgc 31320
gatcacgccc gcgcggttga cgatgtagtc cgccttgaac atcttcgtga ggtaggcgta 31380
cagcgaccgg caggccacgt gcggccttag ctcgtcgagc acggcgtcca gcacctcgcg 31440
gtgctgcagc acggaggggt gcaggaactg cgtgaagtcc accgcggtct catccatgaa 31500
gtcggggatg gtctcgacca ccaagcggtg gttccgcacc gcgcggaagc cggtgttcat 31560
cggcgcgatg ctgtgctggt cgtggacctc cagcaggatc tcgtagccga tctctcggca 31620
gctcagcgcc gcgcgcgcct ccgtcacgct cgcgttccgg aagaactgcc gcaggtgctc 31680
gtccgtgcgg cagagcgtga cggattgggg gatgcgggaa aggtcgcaaa gagggctgtg 31740
gacggagatg cgccgcagcg cgtggtctac gaactcaaaa ccgcgcctcg acatcgtgaa 31800
gtcgcggagg gcgtacatgt tgtaggaaca gaggcggaag aggcagtcta tgtctagcat 31860
gttggaaacg cagtacgcgt gtctgtggag gtgcgggagc atggcgggca cgacctcggt 31920
cgtgttctgg agcatggtgc atacgaggtc gggcgctgtg aggtcggggg tgacgatgag 31980
gatgcaggag ctggagaact tgctgagctc ggaggccagc cggtgaaggt tgtagtcgtg 32040

gcgctggctg aagttgctgt ttatcgtgcc cgtgaagccc atgagcgccg ccagcgtcag 32100

gtcctcgcgc tcgatggccc agatgtctag gccggaggct atcatgcagc gcaccagcgc 32160

ggcggcgcgg tcgctcgtgg ggtagctcat ggtgctgtcg gtcgtgcgaa tcagcatggg 32220

ataatgcttc atttttacgg tcgggggttg cggactgtgg ggcgcacagg gcctgcgggc 32280

ggctcgtgcc ggtccgcggc gttcgccgaa cgcaggaacg ggcccatgcg cgcccaggcc 32340

atccacagcc ccgccgtcag cgccagcagc cagacgaata ccacgatcat cttttatgta 32400

gctggaactc gcgctcactc cccgccgcac ggcgacgggg agagcccaga gccgagctcc 32460

atgcgcgtgc tctgcacggt gagcgactcc acgagcttgg acacgttcat gcgcgtgttg 32520

tcgggcacca ggtgcgcgag gcgcgcgtac acgtcatcgt gcatgcgctt gctgcagcgg 32580

tccaggctcg cggagagcgc ggagactagc gcggtgtgct tcgcgtacac gaagtcgccg 32640

agacacacgg tctcgagccc gagcacgtcg gcgctctccg cgtccttgag cgccacgaat 32700

atcttctgct cctcgcgcgt catcgagcgc atgaggtagt cgtgcagccg cgagcgcgag 32760

atgagcccct gagagatctg cgggctgcgc atgaagcgcc ggcgcatcgc gcacagcagc 32820

tcctcgtcga cgacgtacat gctgtccttg atggagctct tctcgtcgat cacgagcaga 32880

ccgtcgttgg cgaccacgtt gatgaaatcg tccacgtgcc gcgcgtctat gtcgtagcgc 32940

gtgccgccgc actcgatgtg cgagggcggc gacttgagcc ggttcgcgag cgccttcacg 33000

tcggagacgt cgatgtacag cgaggactcg cgcgggacgc agccgaggat gcgcgtctcg 33060

agcggcgtga ggatgagcac gcgctcggcg ccgtcgacga gcttgccgtc gtcgggggaa 33120

aagaagttgt tctccacgat gctcgagacg aggctggcga gcacgccgtc gcggtaggcg 33180

ccgagcgcaa actcctgcac aaagggcgcg tgcagcaagt ccacgggaat gcgcatctcc 33240

acgcggcgcg cgaccgactt cttcttctgc aggtgccgcc ggtccaccat ctcgtccacg 33300

atgtccgata tgcgggagca gaggtacgcc ttgaggacgt tggcgtttac cttgttgaag 33360

atgagccgtt cgtcctccat ttaagctgct cagacgagct ttaaatagtg gaaacacagc 33420

agcacgccga tcgccgccgc tatcaggccg attagaaaaa cggtggtcca ggggacgccc 33480

ttgggcctat cgcacgccgg cttttcggtc attacggtgc gcacgatgtt taggaactcc 33540

tcgaagtcct cgtccgagtt ggagaggaag gagccgaaga cgccggtgta cagtttgtcc 33600

atttactact agatattaaa cggcgcttcc aactcctcgt cctcgaagcc cgcgccaggc 33660

tcgacgacgc ccaggccgcg cacgtcctgc tcctcgttga acgtggtctg agtctcgctc 33720

atgcgcacac acgtctgctc gccctcgaga ccgagcacgg tcagcgagca ctcgcgcggc 33780
atggtgatct tcttaaccgc gaaggtgact ttgccctcgc cgcccgagcg gtagaacacc 33840
accggcgcca ggatgagcgt cgccatctgc gcgtcgcggg ttgcgaggtt ttctatctct 33900
cgcgtcagcg gacatatctg cggctgggtg tcgtcgtcgc tggtgaactc caggagagcg 33960
ccggtcaggc ggttgagata cagacatccg gacttaaagg tgttgtcgat ggccgtttcg 34020
gtgttgaagt tgcgcagcga cggcggaacg cgagcgccgg ttttgatgtt gtcgtatatg 34080
ttctccagca gctggtagag cagcggactg gcgctcacgg gcttgaggcg accgaagtac 34140
ccgctgtcgt tctgccgctg catgtcggtc ttcttgctcg ggtagatctt aaactcgccc 34200
ttcacgacga tgagcggcga gacgagcttg gatgctagac tttcgacgag acacacgttg 34260
atgttggagc agctcgggta ctgcgacgga gttagagtca cggcctcgat gaccttggtt 34320
tggctcgacg agagcgactt agcgaagttg atcgcgtcgg tgcacgacat cgcctggttc 34380
tcgccgaacc gcctggcgga cgctgcatcc tcctgctgag gagcgcggtt agacgcgacg 34440
gtggttttgg atacagcgcg tttcattatt gcggcgattt taaagtacgt gtatactttc 34500
agttttgtcg ccgagcgttc agcgcctgca tgcagaggaa gtacaggatg atggtgcacg 34560
ggatcgtggt cagcagcgat acgaagtcca tcactgtgag gacgcgcagc gccccgcgcg 34620
agcggatgcc cagcgagggc gcgccgcggc gcgcgatggt ggccccgttc gtcaccacta 34680
ccagcagcat taggatggtc gcgcccacgg cgacgcccag gtcccgcgac tccatttata 34740
gtacagtata gagcgaccgc gtcacgaact ctcggctggc caacacgcgt ccgtcgggcg 34800
ggtgtccgcc ggccttcccg cggaactccg ggacctcgaa gctggacttc gtcacgcggt 34860
acgtgtactt gccgcgccag accaggtttt ccttctggaa gacgccgtcc atggtcacgc 34920
ccgccatgaa ggcgtccttg acgatgacca gcaccgcgtc tagcttgcgc ccgttgatgt 34980
gcgtgacgaa gtccgtgccg ctgcggctcg cgcagcggat gtccacgccc gagggcaggt 35040
ccaccacgaa cacgaagcgc ttcggcgcgt agagcaccag gtccgaggac ggcgacgccg 35100
aaggcgccga ggggaactgc cggtggtcaa aagggtgcac cacgcccacg atggacgtga 35160
cgcggtcgtc cgggaactgc gtcgcggcgc cgccgccgcg gtgccgcgtg accgtgcttc 35220
tgcccacgtc gtcgcagacc acgtgcagct ccgacacgat cggcagcagc gtggccagca 35280
tgcggtcggt ctctgtgcgc gtcgcgcagc ggtacgcgat cccgcagtgc gcgtcctgcg 35340
tgcgcccgaa gaagagcacc agcacgctcg cgtcctggtc gaagggacac acggccatca 35400

cgcccaccgg cggcggcccg tggcctgcgt acgcggagga gaactcctgc acctcgacca 35460

cggcgtcctc gcgcgcctcg ccgggcacca tcgccgccgc cggccgcagc gcccgcacgg 35520

tctgcttaac cgcacgcgcg gcggaggccg cgctcggcgc gactacgcgc acggccgcgt 35580

gcgcgcccgg cggcggcgcg gttccggcca tccagcccac cggcgagaag aacacgtcgc 35640

agacgtgcac gcccgcggcc tgcagcgcgc gcgcgagcgc gcgcacggcc tcccactcct 35700

cgcgaaaggc gctcgcgacc gcgagcgcct tcagcaccgt gtccacggag ttgacgggct 35760

tctggaagag gttctcgttg ttgtagatga actcggggag ctccacgggc actgtgaaca 35820

gcctaatctc gtgcgcgccg ctgggcgtga gccgcgtcgc gggcttgcgc acgccggcgc 35880

cgatctgctt gaagaagtgg ttcatggcgc cgccggcttc tcgggctccg gcgggagcag 35940

actatttatt cgggaggtta tcctttccga aagcacctgc acggacttcc gcgtccagcg 36000

ctccatcttc atgtactcct tcatgccgtc gctgagcacc tcgacggcct ccagcttggg 36060

cgctgtcggg tcgaagagga tgctcttgag cagcgtcatc ttcttgtccg cgaggaagcg 36120

gaagtaagtg tagatgcagc gcagcgcgcg gaagttctcc gggtgcttga tggtgcacag 36180

gatcatgaag atgcaggtga acatgccgca ctcggactcc atgagctggt tgacctcgag 36240

gttgatgcag ccgcgccgcg ccttgaagtt gtccacgaag aagcgcatga gcacgtccac 36300

gtcgcagttg cggttgtcca ggtccgcggt ctcggcgttc acgttgaagc cgtccgagaa 36360

ggagtagaag tagaagtact tgcaggggtg gaactccgag gggctgttgc cgccggagtc 36420

gtagaaggac acgagccgcg agacggtgtc gaagatgcag cacttccagt ggaacatgta 36480

gcagaagccg aacatcacgt agcgccgccc ggcgcgctcg atcttgtcct tgagcgtgag 36540

gctgaccatg ttgcagcgga agcggtccgc cttttcgtgg atggccgcgc cgttgaggaa 36600

gttcaggttg aactggccca ggtacgcgac ctcggtgccg aacgcgaagg gcgccaccag 36660

actctggatg ctcacgttgc tcatccaggc gctgcggtcg ggctttatgg cgatgggcac 36720

taccttggtg ttcacgcccg tgctgacgcc cgcgcgcgcg aggtcgtcca cgttcagcgg 36780

catctgcgag aagtccacgg cctcggacac cttctcgcgc aacgagggct tgaagaagaa 36840

gcccagcggg accttccact ccagcgcgat cgcctcgcgg aagccgtagc gacccttgag 36900

gctggccagc aacgcggtct tctgcgcgac ctcgtccttc tcggtgtccg gcggcgcggc 36960

gtcgatgagc ccgcgcttcg cgaagtccag cagcgccgcc agcgggatgc acgagacgcg 37020

accggccgtc gcggattcgt cgaagcgccg caccacgtac ccgttgcagt tggtcttgaa 37080

gttggacacg tccaggtgcg cgctgagccc caccaccgag tagatgtggc acagaaggtt 37140

ggtgaacccc agctccggga ttttgctcac cactaaatcc gtgtacttgt ccatttatca 37200

tggagaatca tctgccggac atgctgatgt ttcccaactg cgtttctgtg tttccctttg 37260

agtactcgct ggaggacgtg ttccgcctcc ccgaggagcg acggcgcgcg ttcgccatgg 37320

ccgtgttccc gctctccaag caccgctgga ggggcgcgcg gctccagcgc gacgagcgaa 37380

gcgtgtggct cagcgtcgag gaggaccgcg ggcgcgcgct ggacgagcgg aactgctcct 37440

ggctctcgga cgtggccgcg cgcatggtcg acgacgaggg ccgcgcggtc acgcccgagg 37500

cgtacgcctt catgcgcgcc gcgcccggcg cgcgcgtcgc cgagctcgcc gcggacgcgg 37560

gcgtgctagc gggccttgtc gccggcggca acgcgctgcg cgtcttctcc tcggagtcca 37620

cgcaggcgcg cgagggctgg aaggcgcgca gcgtgggcgt gctcggcaac gcggcgccgc 37680

tggcgcccgt gccgctggca tcgctgcgtc cggaagtgca gcgcgagctc ttcgccgcct 37740

ggatcggccg ccgccccgtg gtgctcacgg gcggcacggg cgtggggaag acctcgcagg 37800

ttcccaagct gctgatgtgg ttcaactacc tcttcggcgg cttcgagcgc ctggacgccg 37860

tccgcgagtt cgcggagcgc ccgctcgtgc tctcgctgcc gcgcgtcacg ctggtgcgcg 37920

cgcacaccgc gacctacctc gcctcgctgg gcttcggctc ggccgacggc tccccggtct 37980

cgccgcggta cggcgccatc ccggacgccg agcggaacac ggccccgcgc gcctacgggc 38040

tcgtggtggc cactcaccgg ctcacactga ctgccatccg ccgctacgac acggtcgtag 38100

tggacgagat ccacgagcac gaccagatgg gcgacatcgt ggtcgcggtc gcgcggaaac 38160

tgggctcgaa catgcgatcg ctggtgctta tgacggccac gctcgaggac gaccgcgcgc 38220

gcctggagga gttcctggac cggcccgcct ttgtgcacat agagggcgac acgctcttcc 38280

ccatccgcga ggtctacgtg aagaacacgc aacagccgcc gctctcgcgc aagtacgcgg 38340

aggcggagct gcagaacgtg gcgcaggcgc tgggcacctt cgtccccgag cagggaaagt 38400

gcggcatcct cttcgtagcc acggtggcgc agtgcgcgct cttcgcggag accatcgagg 38460

ccaagcaccc cgggctgctg gtgcgcgtgg tgcacggaaa ggtgccctcc gtggccgcgg 38520

tgctcgagga ggtatacgcc gcggaccggc ccgcggtgct ggtttccacg ccgtacctgg 38580

agtccagcgt gaccgtgcgc accgccacgc acgtctacga cactgggcgc gtgtacgtgc 38640

ccgagccctt cggcggccgc gagaccttcg tctccaagtc catgtacacg cagcgcaagg 38700

gccgcgtggg ccgcgtggcg cccggcacct acgtgcgctt cttcgacacg cggctcgcgc 38760

tgccgctgaa gcgcatcgac tccgagttcc tgcacccgta cgtgctttac gcgcgcatct 38820

tcgggctaac gctgcccgac gacctgctcg tgcagcccag cgacctcgcg ctgctgcgcc 38880

gcaccgagga gtacgtcgac ggcttcggca tcagcctctc gcgctggacg cagctgctgg 38940

accggcacta catgcacatg gtcgagtacg cgaaggtgta cgtgcgcggc gggcgcctcg 39000

ccgccgcgct ggacgccttc gagcgcaccg gcgtgatgac gcacgaggcc accgaggcca 39060

tccgcgccgt ggacatgctc gcggccgtcc taaacgtgcg caagtccaag gaccgctacc 39120

gcgcggagtg caaggtgctc ttcgggccct tcgcgggcaa aaagttcgtg gtcgccgggc 39180

ggcgtccgcc cggctcgcac gtgctcatgg tcacagaccg cgtcttcatc gaggccgagc 39240

ccccattctg aggaccacct tcttggagac gcccgagaag tcgtcggcga cgccgcggcg 39300

cgccaccaca aggcagtacg aggtcacgtg cgggcagcgc gcgatgcagc ggaaggcttc 39360

ctcctgcgac agcgagaagg cgaacacgta gaaggtgtgc ggggacttca gcggcgtgtg 39420

gtccatcgag tagatgacac cgagcttctt catgcgccac ataagcgcgt tgatgtggtc 39480

ggcgcgcagc gcgcggccct tgagcacgcc gcagacgaag ctcgagcagg ccacgacgtc 39540

gtagcgcgtg ttcctgccga agaccaggtg cggcgcgccg gcggcgcgcc gcgcggccgc 39600

gcgattctcc acgatgtcct ctatggagcg ctcgctcgca aagaagtcca ggaacatgta 39660

ctggtaggcc acggccgggc gcgacttgct gaacttcatg aaggcgtccg agtccatgat 39720

ggcgtccatg tcctcagcgg cgagccggtg ctgcagccgg atgccctcga aggtgtggaa 39780

gagccgcgcg tccgcgtgca tggacagcgc gagagtgacg aagttgagaa ggtccgcgtc 39840

gccaaagcgc acgagcacgt taccgggcgt gcgcgtcttg cgcatgagcc gcgcgggcgc 39900

gccgtcgttg tggctgcggc ggcgcatttt gtcgccgggg gactcgggcg gcaggtcgat 39960

catgaccagc cggtgccgct gcgcgtcctc ggcgttgaag atcgaggacg tgaagcccgg 40020

gtacagcacc acgcagtcgc gctccgagat ggcgtgcagc acgtcgcgct tgagcccggc 40080

caccagccgc tccgcgttct cgacgaagta gttctcgtag tccaggatgt cgtgcgccat 40140

ccaggggaag ttcaggtacg cgttcatggc gtagtcctcg gcgtcgaagc agatgcgcgt 40200

gtctggcgtc gccgcgatcg gaaggtcctt gatgccgcgg agcagcccgt cgtagtcgga 40260

ctcgtccacg aaggagagca ccacaaagag gtcctcgccc acggtttcgt agtcgaagag 40320

gtggtaaagc tctcttagcg ccagcacggc gagcgcgttg tccagcgagg cgtgcacgcg 40380

cgccaggatg ctgtagaagg gcgtggccat catcacggcc ttgccgccct cgcaggcaac 40440

ggcgcgcggg aaaatgacct ccggcgtgcg cggcagccgc ccgaacgtcg cgttcagcag 40500

cgcgaccgtg gccgcgtcgc tctggcgcag gaacactacc accgaggggc ccgagatgct 40560

gagcatgcgc tcgcgcatgc gcgctggcag gtccggcgtg gtcacgaggt ccgcgaagcg 40620

gccgccgttg tagaggtcgc cgccgccgag gaaggtgagc acgtcgaagc agtgcagcac 40680

ctcgttgcgg aagtagtact cgttctcgag ctccttggcg tacgcgcgta tgtccacgtt 40740

ctcgaagttt gttcgcagac cgccgccgtc gaagaaccag gacgcgagct cgcggacggc 40800

gtccgcgggc ctgttgcggc ggctcttgca ccagaagctc atgtagttgc gcgaggtgga 40860

ggcgttcgcc aggaagaagc ggtggtcgaa ggagatgagc acatgctcga gcaggtgcgc 40920

gagccccagg accgcgccca cgtcgcgccc aaaaccgaag tttgatatcc ccaggtagac 40980

gtcccgtttc atagacggcc tcaggaacac cctgacgccg ttttccaaca ctatcattct 41040

ccggtattta cttacccaaa agtagtatgg ggagaagtgt ttgaacgtcc cctcgccttt 41100

ttaaatcaaa agtagacttc tcgcgcccgt gcgccaccgt cacgcgcgcg cggcgcgagt 41160

ccataccggc gatcaccgcg ctgctctgcg gtgcgtccgg ccgcgggaag agcacggtct 41220

cggagatccc gtccagctgc gcgtcggtgc gctgccgcca cgcgtgcgcg tccgcgagct 41280

cgcgcacggc cagctgcatc ttgttcgtcg gcaggaacgt gaacacgtac gccgccgcca 41340

ggaaggctgc gaagagcacg aactcaaccg cccatgacat ttagggagct gattttgttc 41400

cacgcggcga cgcacgtcgt gacgggcgac cccgaggcgc cgcggcgcgc ggcctcgctg 41460

tgccgcggct tcggcgtgga cttccgcgcg attcacgcgg agttcgcgcg gcggtacccg 41520

cgcaccgcgg ccgccgtgga gcgcgcgcag ccgctgcccg aagtcgatgc cgcctttccg 41580

ccggacgcgc gccggcaagt cgtgcggctg cgcctcgagg ctgcggcgct ggtcgtcaag 41640

gagtcgcgtg cgctatcggc ctccatgcgc ggcgtggcgg tggtcgacgg ctgctgcgtg 41700

cgcgtgtgcc gcgccaacga cgagctgcta gagttcctcg cgcggcgcta cgaccccgcg 41760

gtctaccgct acgcggaggt gccctcgccg agcgtgcgcc cgggctcgaa agtcttcgcg 41820

tgcgcgggcc gcagcgtcac ctttgcggcc gcgcaccgga gccgcatcac ggccaaccgc 41880

ccgctgcgcg tggtcgtgac cgaggcctgt gtggacggcg tgctcgcgcg cggcgccgcg 41940

gaggtcttcg accgcggctc cggcgtgctg ccccgcgcgc tgcgcgagat cttctaccgc 42000

ctcgacgagg acggctgtcc cacgggccag acgccaggct tcgcggacag tatggcgtcg 42060

cgcagctgat ctatgtccac ctttttctcg tcgatctgcg ccacgaccac gaaactgcga 42120

atgtccacag cggccatggt cttggccacc gggtcgtact tgaggagcag cacgtactcg 42180

ttgccgaagt gctcggtgac ctcggtgatg agccggtaca cgcccatgcc gagcacgttc 42240

accgcaccgt ccttggcgaa gagcgagagg atgttcacgc acttcagctc catctcgccc 42300

tcgaggcgcg cgagcatgcg ccgggtgacc tcgcatactg aacaaagagg cttacctagt 42360

aagataagcg ttagcttagc cgcggtcggt gacgcgtcgg aggccattta tggggatcaa 42420

aaacttaaag gcgttgctgc tcagccacgg cgcgctgacc ccgcacgagc cgggcggcga 42480

cgagcgcttc cctgccgtgt tcgtggacgg cttcagcgtc atgatgacca tggcgtactc 42540

gtgcgcggac gaagacgagt tccgcgcggc cgtcgaggag cgcgtgcagc actggatgag 42600

cgtgtccgag agcgggcgga tcgtggtctt cctcgaccgc ggcgagattc cgatcaagca 42660

gccgctgcgc gaccagcgcc gcaaagccac gcgcgaccgc gccgcgcgcc accgcgagtt 42720

catcgccgcc gcggaggcag acgcggcggc agaggccgtt ggcgcccgcg aggacaaaca 42780

ggaggacgag cacgcggagt tcgccgagga gatccgcgct gagaagcagc taaagctgca 42840

gcgcatccgc ttccagctca gcatcgccaa ccacgaggtc gttaagtcgc tgatagagtc 42900

cacgctcgcg cgcgctggcg atgccgtgga gatcgtcttc tgcgacggcg tcgacgcgga 42960

gatggtcatg tgcgcgcgcg gacgcgccga ggccgagcgt cgcgggcgct ggccgctgct 43020

cgtgaccacg gaccaggacg cgcttttgtt cacgtccacc gatcgcgacg agaagatagt 43080

gagcaccgtc tccgcctgct acgcgttcag gcccaccgag acgaccgagt acctgtgcaa 43140

acttgcggcg ctggccaacg gctgcgactt cttccccggg ctcggcggca tatgcgtgag 43200

tgtggagtcg ctgcgccgcg ccacgctttt cccggaattc tccgtgcgca acgccgccgt 43260

gagtctgtgc acgcggccca tgcggctgtc cacgcaggac gcgctggagc cagaggccgc 43320

cgccgaggtc gtggaattca tcaggcggta cgccgccggc gacgagcgca tctaccgcga 43380

ggtgccgccc ggcgcgtgct gcggacgcgc gtttgtgcgc ggagcgctcg cggccgagtg 43440

ggccgacgcg ctgccggcgg ccacgggtct gagcgtggtc gcggacatga tcgcgtgtct 43500

gcccgcgcgg cgggaccccg cgcccgagga ggtagagcgg ctgctggcgc tggaggcgcg 43560

cgcgcgaggc gcgcgcgtca cggatgcgat gctcgcgcag actgcgcagc tgctgggtta 43620

cggcgcgagt gcgggcgccg acggcgcctc cgccttcgcg gtctcgggcg ccaagggcct 43680

gatgtgtcgc ctgcgcggca cggccatgtt cttcaacgcg gagtacgtgg aaattgaaag 43740

cgaacccaga ctgttaaagc tgcggtagca tggtgttccc gatcgtgtgc tcaacgtgcg 43800

gccgcgacct gtcgcacgag cggtttctgc tcatcgtgcg acagcggccg ctaaaggttg 43860

ttttgcggac ggtgcgcaac gtctgctgcc gtataaagtt gtctacacaa atagagccgc 43920

accggaacct gacggtgctg cccatgctcg acataagctg atttttcttt tccgctcgta 43980

tgcgcgagtt cggactcgcg gcgcgcatgg cccgcgccat cgaggacgtg tgtccgcgcg 44040

gcgcggtgat attcgtatcc agcgccgcgt ccatgaccga ctgcctgaac ccgtcggtgt 44100

tcaagcacgc ggcgatatac gcggggcgcg tggaccgcgc gccgctgccg ccgccctcgc 44160

cggtcccggc ggaggccgtg acggagccct gtgcgataga cgccatagcg ccttacggcg 44220

cgcgcgtggt cctgctctcg gagctgctgc ggagctgcgt ggccgttcag gcctaccgcc 44280

tggcagtccc cggcgccctc gcgctcatga acctcgcggc cgacgcggcc ttcgagctcg 44340

tgggcacgcc ctacggcttt aacagcgacc gaacgtactg cttcaagctc gttgccgact 44400

gctttgctag cgtgggcgtg acaacgaaga ccaggcgcat catgggtcgc gacgtcgtgc 44460

tcagccagga cttcctggag agcggcatgt ggaccaaggt gctggactcc gccgcggagc 44520

cgccgtggct ggtctagaac agcggcggcg cgcgggtccc gagcacgggc cgcgccacct 44580

gcagccgctg ctgcagcgcg cggcactgcg cctcggcgtc ggccgtctcg gcggggtcga 44640

cgggcgtcgg agttgcggag gtggtcctga acggctgcgt gttcaccgag acgcggatgc 44700

gctccttgca ggagcgctgc tcgatgcagt tggccagcat cttcatcacg tgcaggtact 44760

ccagcaacac gaacttttcg agggtgatgc cgtcgaaggg cgacgacccc accacgccca 44820

gcgggctgga caccgcgccg tcgagcacct cgccgcggga ctccttgcgc gcgcgctcga 44880

gcaggtcctc tgtccgagcc accacactgc cgaagtcggc ggccgcgggg gcgggaacag 44940

gcgcagcagc gctgtccgcg tccgccggca tctcctcgat cttgagaccg gccgcgaact 45000

ccgaggccgc gtgcacgggc gaggcgccgc gccgcaccat gaagtcgcac agacgcgata 45060

gcgcggagga gcgcaccggc atgtcgagca ggcgctcggc ctccatctcg gcgaccgagt 45120

cggcgcacgc gtccggcgcg cccgcccgca cgagctcgtc gcagcacccc gcctccttca 45180

tgagcgcggg catgagcttg tactgcgcca tgttcaccag cccgtacttg agctcgagca 45240

ggtccgcgag ctcggaggcc atgggtcggt ttttggtgta gatgacgcgc tccacggcct 45300

ccgccatgtc cacggcctgc atgagctcgc cgacgagcac gctggccacg agcgtggcca 45360

gcgtgacgcg cacggtgggc acgcagaccg cgaagaagga ggtggagtgg gtgaagcgca 45420

tgagcgcgcc gtgcagacgc gcgaggtccg cgctgttgcc cgcgtgcacg aagcgccggc 45480

gcagccgcgc cagcgcctcc acaaggtcct ctcgcgtggt cacgcgcacg ttcgcgatgc 45540

acaggtcgtg gatcgcgttg gcgatctgcg cgcggcgctg cggcgagctg ccgggcagca 45600

gccgcgcctt ggcctcgacg tcgacggtgc tcgagagaca gccgcaggcg gcgccgcgga 45660

cgacgaactt caacaacgac tcgaacacgc gcgcgcccgc gcggggcgct tgcttggacg 45720

actccattta ctttaaataa tttacgagat caaaataaaa tgactctgcg catcaaactc 45780

gagaagctca agcagatcgt aacttacttc tcggagttca gcgaggaggt ctcggtgaac 45840

gtggacgtcg gcgatggcct catgtacata ttcgcggcgc tgggcgggtc cgtgaacatc 45900

tggaccatcg tgccgctcag cgcgagcgtg gtatacgacg gcgatgtcag ccgcgtgttc 45960

aacctgcccg tgctcaaggt gaaggcctgt ctgtgcagct tccaccccga ctcggtggtg 46020

agcctggagc ccgacctcga ggacaacgtg gtgcggctct cgagccacca cgtggtcagc 46080

gtggactgcg acaacgagcc cgtggcgcac cgcacgaaca ccgccatctg cctgggcatt 46140

aaccagcgca agtcctacgt gttcaacttc cggcgctacg aggagaagtg ctgcggccgc 46200

accatcgtca acctggacct gctgctgggg ttcatcaagt gcatccacca gtaccagtac 46260

atcacggtct gcttccgcga caagaagatg gtgctgcaca cgcccgggaa ggtggacaac 46320

ttcttccgcg agtactccat gaccgagtgg gcgcccgacc tcgagcgctt ctcgttcaag 46380

atccccatct cctccgtgaa caaactccgc ggcttcaaga agcgcgtggt catgttcgag 46440

tcgcgcgtgg tcatggacgc cgacgacaac atcatcggca tgctcttcac cgaccgcgtg 46500

ggcatgtacc gcgtgaacgt gttcatgtcc tttcaggacc ggtctctttc atgcgactaa 46560

atactcatgg gcgggtcggt gagcctgccc tcgcgggacc tgccgccgcc ggtgcgcacg 46620

ccggagatga acatcgtgcc cgagcgcgac ctcgcggaca cgatggcgcg cctctccacc 46680

gcagacccgc cgcagccgct gggcgtcggc gacgacgcgc gcatggccgt gctgaagacg 46740

accttccccg agttcgcgat atcgcggccc gcgacgggca tgctcgccgc gcagcgaatc 46800

aggtacgacg gcgacccgcg cgtctgctgc ggcgggttcg ggatctcgca ttactgggag 46860

aagggggcgc gccgatcgaa cgtcgcgttc gagggcgcgg cgctgcgcac ctgcgacccc 46920

acgcgcttcg acgcgggcgc gtgcgacgcg ctgctcttcc gcgagtgcgc cgccggcggc 46980

gtcgacgcgg acttctgcgc gcactggatc aacgcggccg tgacgcggcg cacggaccga 47040

cagtcgcgcg cgcggctgaa cgacatgttc gtgcgcgatt gccaaaacga cgccgcccgg 47100

cctcactgcg tggcctggat ccgcgcgatg cgaagcgcgc gcgcgacggc ggacgacggt 47160

ctaatagacg ccgtgctctc ggtgcagagt cccgagttca agggcaagca catgcgctgc 47220
agctacccct cgccggccac tctcgccatg gccgcgaacg tggacgagcc gcgcgagtgc 47280
tgggaccccg agtgcgtggc cgggaacgtg gacttcatgc taagcgataa ctacacgaac 47340
ctgggcttgt gtcggctctc gcgctgctcc atcggcgtca cacacctgcg gattgacgcg 47400
cgttcgcggc tgcgcatgcg gtgcgccggc gcgcttgccg ggctcacgaa ggcgcccgtg 47460
aaccagactg tcgtcgtcgg cgacaacctc gcgcgcgcct tcgagccgcg cgtggaaacg 47520
ctcagcgtgt tggcgctgtg cgtggtgtat ctgctaattg tctggctcta aatgggggcc 47580
gccgccagca ttcagaccac cgtgaccacc gtcagcgagc gcatccgcaa cgagctcgag 47640
cagagcgcga gcgctagcgc gaccgccgac tgcgacgtca ccatcgggag tctgattatc 47700
cgcaagaacc taggatgcag cgtttccgtc cggaacatgt gctcggccaa cgccggcgcg 47760
cagctggacg ccgtcatgaa ggccgtgagc agcaccttca acgacctctc gtcggaccag 47820
aaggcctacg tgcccgggct gctcacggcc gcgctcaaca tccagaccac ggtgaacacc 47880
gccgtcaagg acttcgagac gtacatgaag cagacctgca cggcggacgc ggtcgttcac 47940
aacaaaatca agatccaaaa catcgtcatg gaagagtgcg cctctctgcc agggagtccg 48000
gccacgcacc tggatttcgt gaacaccggc acggccgtgg gcaactgcgg cgtgaaggcc 48060
gtgatggacg tgctcgcgaa ggccagcacc accgtgcgca acgaccagga ggccggcaag 48120
ggctaccaga ccatcatcat cgcgatcgtg gtcgccatcc tggcggccat cttcgcctgg 48180
tacgcgcggc acatgctatt catgtccacc tccgacaaaa tcaagctcga gctcgccaag 48240
aagcccgtgg tgcactggac cacctacctg gacaccttct ttacggaatt tccgccgtcc 48300
gtctagatac gcgcaacatt gaaacattat atccacctct caaacggcgg tatggtccga 48360
cgcgtcctcc tcgagcgcgt ggacggcatc gtcgagcact cgcgcgcaga ccgacgctac 48420
ttggaggcca ttcagcgaca cctcgagggg tctacgcccg ggctgcggca gatgtggcgc 48480
ttcctctacg acctgctgct gacggtgttc gtcgtcatgt acatcgtctt ccgcctaatc 48540
gtgcgcaacc ccggcatctg cgccatcctc gcgctcgcgg ccgcggtgta ctacctgttt 48600
ttgtgtctct ttagcatgga ctgatggcga tcacagacag accatcgccc gcgcgcgcgt 48660
gaccagctcc ggcgccgcga agacgtcctg caccgggaag tcgtcgatct cgaacacgga 48720
gccgtccgcg gaccagatca cgcgcacgtt gtcgctcacc gagacctcgg tcagcgtcac 48780
gcccagcaca accgcgtcgt tggtgctcac cagcaccagc gcgccgggct ccgcgcgccg 48840

gtgcagcggc ggccccgaga ctgagcgccg ctgcacgcgg aacatgtccg cgaactgctt 48900

cgagagcaag tccaggtggt tgcggatgat ccactcgaag aagtacgcgc aaccgccgcc 48960

gccgcacagg aagcgcgaac ccgcgggcat cagcagccgc acaacgtcca tgtagcaggc 49020

ctgcggcagg ctcgcgcggt acagccgcgt cttcggcgag agcaccacca ggctggaggt 49080

gctcatctgg aagaccagct ggctaacgga gacggtgagc gtgcacgcgg gcacggaaac 49140

cacgtccagg cagatgtcgt ccagaaagat gctccgctgg tagaggtggt acaggatggc 49200

cacgatctga aaggccgtgg cgtcgctgat ggcgcagggg cggtcggcgc agcgcatctg 49260

cgcgcaggac cagccccga aggactcgaa gcagacggtg agcatgcccg tgctcggaca 49320

gtgtggcgag cgccgacaca ccggaaagcc cacggccttg cggcagcgca ccatggtcga 49380

gagctctatc cagcagcctg cctcctcctc gcccatgccc atggctaccg gcgtgaaggc 49440

cgtgacgtcg tcgcagatgc gccgctccag aaaccccacg cccgaggagg ggtgcgcggc 49500

cggcggcgag gtgatgcgcg ccgggacgcg gctcggagcg ggctcgggag gcgagctgcg 49560

ctcgacccgg gcagtcgccg ccggccgcga tgccctgcgc gcgggcgcgc gctcgcgcaa 49620

cttgtttgac ttgctggcct cgtcgctagc gtcatcgaag cggtcgttcc tgtcgccgcg 49680

gacgtccgcc tcgtcgcccg tcggctgcgc ggcgggcgac gtgccgtccc gcgtacggcc 49740

cgcgttcggc gcgaatgtca cgcgccggtg cacgtacggc tccgtagagc ccgtgggggc 49800

gccgcgcccg cgcccgcggc ggaaggcctg ccgggacgcg ccgaagcggg cgaactcccc 49860

cttcgcccgg cccctttttt cttccatgat atttatcaca aaaaaaactt ctctaaatga 49920

ccaatctgct ttcgttggtc gacccggagg acctggcctt ctgcgccggg ttcccgtcct 49980

tcgacgagac catgctcgtg atcgcggggg cgcgagtgcg cttcccacgc tcgctgctct 50040

cgctcttcaa cgtggtgccg cgcaccatga cgcgctacga aaccgagctc gtgggcaccg 50100

agatggtggt gggcgccgtg ttcaccaccg cgtacaacgt ccgccgcaac ctaggcctcg 50160

gcgaggagcc cgtgaccatg cgcgacatcg agaagtactt cctggactcc gagaacgagg 50220

tgctcacgct catcgtgcac aacaccgact tttccgccat gagcggcgtg cgccggcgcg 50280

gcggccggcg catcgccaac cccgtcatct tccgcagcgg gtccacgccg ctgctcatcg 50340

tgatggagtc gcgcaagaag accaacatct accgcgagcg caccgcggag caggccaacg 50400

cctcctacag ggaggtcggc tcctcgctcg cgctggtcac tcggtacgcg ggtctgcagc 50460

tggttgacgt gcacacgccc agctccgtgc taacggtctc cgccgtctac ggcttcaccg 50520

aggacaaggg gctcaagaag ctgggctccg acaaggagct cgcggactac cagtccacgc 50580

cgctcaccga ccccatccgg ctcagcgact tctccaatat attcgacggc gtcaagaaga 50640

gcatccagct cacgaacgtg cccgtgccct ccaccggcgc cgaggccgcg ccgtaggctt 50700

tcatgcgcga taaatcggat ggcggcgccg acgacgcccg cggtgcacct cacgccggtg 50760

ttcgtggagc ctacgatcgc gcactcgctg ctgcgcgcag agtcctacct cgcgatcgcg 50820

gtccttgagc tcgtgctcgc gctcgcgctc gcgctcgtct tcttccgcga cgagctaggc 50880

tcgctattcc gctgcgcgcc gcgagcgcct tcgccgctgg acgcgtacct gcaggcgagc 50940

ctcgtctgcg acggcgacgc gctgctgatc gagctgcccg agggccgggt gccggcgctc 51000

gcgctggacg ggcggcccgt cgcgttcccg gggtgcgaga gccttttgta ccgcataaat 51060

ggaccacgaa aagtacgtct tgtcgatgtt cttggaggaa gataactcct tcttctcgtt 51120

cgtcgccgcg ctgtccgatg acgaggcgct cggcgccgtg cagtccgctg ccgccctcct 51180

ggacttcctg ctctccgtgg tggtccgcgg caaggagaag ctcgccgccg cggggcacca 51240

ctacgactcc atcgcggacg gacgcgcgcg cgccgcgttc gagttccgag acctgcgcga 51300

gctggcgcag ctcttcgacc ggcggccctg cggcgtccag gaccgcgtgc gtgtgcgcga 51360

cgggcccgcg cgcgccttcg tggacgcggc actggggctc atgcgcgagc gaggcttcga 51420

cggcacgcag gccgcggagc gcgcgcgcta catcgcgccg aacgatctgc ccgcgctggg 51480

ggcaatatcg gccacgctct cgccgggtct ataacgtaaa aaatattagt aaaattctga 51540

aggtccgtgt gtttcgcggg cggccaacaa accagtcgct taaatggagg gggtggaaat 51600

ggacaagccg ctcctctact tcgacgagat cgcgggcgcg cgcgactacg acgcggcctt 51660

cgcggagaag cacgagccgc ccaagatccc cggccgcgga cagatgaagc tgctggtctg 51720

cgagctcgtg tttctcaacc ggctgcacct gcacggcatg ctcgacggca gcgtcatcgt 51780

gtacgtgggc tccgcgcccg gacggcacat ctgctgcctg cactcgcact tccaggagct 51840

cggcgtctcg cttaagtggg tgctcattga cgggcgcaag cacgacccct gtctctcggg 51900

gctgcggaac gtgaccacgg tgacgcgatt cgcggacgag gcctacctcc gcgagctgcg 51960

cggcgagctg cggcgcgcca agatcgtgct catttcggac atccgctcca accgcgtgga 52020

cacagagccc accaccgcgg acctgctgcg cgactacgcg ctccagaaca ccatggtgag 52080

cgtgctcaag cccgtggcct ccagcctgaa gtggcgctgc cccttcccgg actcctggga 52140

gaaggacttc tacgtgccct gcggcaagga gatgctgcag ccgttcgcgc cgccgttctc 52200

cgcggagatg cggctgctca ccgtgtactc ggagacgcgc ccgaagctgc gtctgatcac 52260

gctcagcgac gcggtcaact atgaaaagag gatgttctac ctcaatagcg tggtccgcca 52320

gcgcgtaatt ctgaactttg actatcccaa ccaggagtac gacttctttc acatgttctg 52380

tctgctctcg tcggtggtgt gctcgtgcga atttaaatcg cccaaagaga aggtgctgag 52440

cctgcagaac cgcttcttcc gcttcctgcg catcccgccc tccatcacgc tcgggctgcg 52500

ccggcacgat gaaccgccac aacacgcggt acctggccaa gatcctctgc ctaaaggccg 52560

cggtaagaag cgaccccttc gcggtggtaa gtagggacac cgtgcgcatg tacgacatcg 52620

aggtcgagta cggcgacctc gtgacggtgg tcaccgtcac gcacaaactc gagaccagcc 52680

gcaccgtctt ccaggtcttc aacgagacct ctgtcgcgta ctcgccgctg ccggacgact 52740

acggcgagcc catcgtgctc accacgtaca tgcagcgcga gcacaccaag ttcccgctct 52800

ccatgctcta catcgacgtg gtcgcctcgg acatgttccc cacgtttaag cgccccaccg 52860

aggaggaggc cgcggtggtc gcggccatgc agcgcgtggg cgggcgccgc gagcccgtgc 52920

tcaagctccc gcgcatgctg gacaccgagc tcgtgtgcaa gatactgcac ctgcccgagc 52980

acccgctgcg cgtggtgcgc ttcctgcgcc gaaacatgtt cacgggcgtg gaggtcgccg 53040

accgctcggt gtccgtggtc ctcgactgac gaagggcagc acggtcagcg aggccgccgc 53100

caccaagcac agcggcagcc acgcgcgcgg gtccgccacg ggcacgaaga cgtgctggtt 53160

caggtacttc gcctggaagc gctccgcggt ggagtccacc ttggacccgc aggcgttggt 53220

gaggcgcacg accgcgtccg cgacgcgcac gtccccgagc gatatcacgc agtcagagac 53280

gttgcacccg gcgatgtttt tcttcagcgc gcgcggcagc agcgcgtccg cgcgcttgca 53340

gggcgcgtac cagcagtagt agggcaggcg cgtgtcgcgg ccggtgtcga ccacggcctg 53400

gctgggcttg aggcacgcgc agcgctcgtc gtccgggtgc gcgtcgcaga aggcgtaaat 53460

ctcctcgtcg ggcgcgtccg gcccgggcgc ggtcggcggc gccgcgcgac ggcggaagaa 53520

catctctgaa aaaatacttc gaccagaaaa cgaccaccga tcttatttca aagataaaaa 53580

tactattaat acgcactcgg agaatcatgt cggtggtggc gcgcgtgtcg tacagcctgt 53640

actcgcagag cgagataagc gccacggacg tggtcatcag ccagttgaag aacgacgagg 53700

acctgggcac ggtgaaggac ccgcgcctgg gcgcctcgga cgggtccata tgccgcacct 53760

gcgggctcac ggagatggag tgtttcgggc actggggcaa ggtgcgcatc tacgagtcct 53820

acatcgtgcg ccccgagtac atccccgagg tggtgcggct gctcaaccac ctctgcgtgc 53880

gctgcgggct gctgcgctcg cgcgacccgt acacgacgga cttggccgcg ctcagcgtgc 53940

acgagatgcg caagatgaag gaccggatga tgtccaagaa gaaggcctgc tggaacagca 54000

agtgtctgca gccgtaccag aagatcgtct tctccaagaa gaagatctgc ttcgtgaaca 54060

aggtggacga gatacccgtc cccaacgcgc tcatctacca gaagctgacc tccatccacc 54120

gcaagttctg gccgctgctg gaggtgttcc aggaccccgc gaacctgttc tacaaggagt 54180

acatgcccgt cccgccgctg ctcatccggc cggcgatcag cttctggata gacaacatcc 54240

ccaaggagac caacgagctc acctacctgc tgggcatgat cgtgaagtac tgctccatga 54300

acgccgagga gcaggtcatc cagcgcgccg tgatcgagta cgacaacatc aagatcatct 54360

cctcgaactc gagcagcatc aacctctcct acatcatcgc gggcaagagc aacatgctgc 54420

gcagcttcgt ggtcgcgcgg cgcaaggacc agaccgcgcg ctcggtcatc gggcccgact 54480

ccgcgctctc ggtgtgcgag gtcggcatcc ccgactacat ccggaacacg ctcacgcaga 54540

aggtgttcgt gaactacctc accagcaagc gcgtgcgcgc gctgttcgag gaccgcgcgg 54600

tcaagttcta cttcaacaag cggctgcgcc agctcacgcg catcaaggag ggcaagttca 54660

tcaaggacaa gatccacctg ctgcccggcg actgggtgga gatccccatg tccgagggca 54720

cgaacgtgat attcggccgc cagccctcgc tgcaccgaca caacgtcata tcctcgaccg 54780

cgcgcgcctc gcccggctac accatcaaga tcccgcccgg gatcgcgaac tcgcagaacg 54840

cggacttcga cggcgacgag gagtgggccg tgctcgagca gaaccccaag tccgtgatcg 54900

agcagagcgt gctcatgtac ccggtgacta tcttcaagca cgacgcgcac ggcgcgccgg 54960

tgtacgggtc catccaggac gagatcgtgg ccgcgttctc gctgttccgg caccagaacc 55020

tctcgctgga cgaggtgctg aacctgctcg ggcgctacgg gcgagacttc gcgccggagc 55080

ctggccagaa gaccttctcg ggcgccgacg tcttccgatt catgataggc gcggacataa 55140

acttcaaggg cgtgctcgag aacgggcgcg tggtggcgcc gaacgtcgac agcgacctcg 55200

tggtggccat gcgcgcaacc tcgctagcgg ggctgatcgc ggactacgcc acgaacgtgg 55260

agggcgtgcg cttcgtggac atggcctcct acgtgtacaa gcggtacctg gccatctacg 55320

gcttcggcgt gaccttccgc gacctgcgcc cggacccgag tttggttcgc cggctgcacg 55380

cgctgaacac cgagaagata gagcagatca aggacgcgta ctcgcggtac ctgcaggacg 55440

tcgcggacgg gaagctggtg ccgatggcgc ccgcggacga ggccgacgcg ctggactcgc 55500

tgctggccaa cctgaccaac ctcaacgtgc gcgagatcaa cgagtacatg cgcgagacgc 55560

tggagcgcaa ccccgataac agcctgctaa agatggcgcg cgccgggtac aaggtcaacc 55620

ccacagagct catgtacctg ctgggcacct acgggcagca gcgcgtgaac ggcgccgtcg 55680

ccgagaccaa gatatacggg cgcgtgctcc cgtacgcgtt ccccgactcc gcggacccgg 55740

aggcgcgcgg ctacatcatc aactcgctca tgaacggtct ctccggctcg cagttctact 55800

tcgcgatgct ggtggcgcgc tcgcagtcca cggacatcgt ctgcgagacc tcgcgcacgg 55860

gcacgctcgc gcgcaaggtc atcaagaaga tggaggacac ggtcgtggac gggtacggac 55920

agatcgtgag cggctcggta ctgctcaagt acgcggccaa ctacgcgaag atcccggggt 55980

ccaccaccaa gcccgtggag ctgctcttcc cgcacgagag catgacctgg ttcctggaga 56040

taagcgcgct ctggacgaag atccggcacg ggttcgtgcg catgcaccgg cagcgcctgg 56100

ccaccaagat cctggcgccg ttcaacttcc tggtcttcgt gaaaccggcg ccctcggagg 56160

cggaggcgct ctccgcgcgg gacctgtacc acatgatcca gcgcgtgatg aacgacgtgc 56220

gcgagaagta cttcttctcg ctggcgaacg tggacttcat ggagtacgtc ttcctcacgc 56280

acctgaaccc ctcgcgcgtg cgcatcacgc gcgcgaccgc cgagctcatc ttccgcaagc 56340

tgtaccagaa gctgaacgcg ctgctcggcg gcggcacgcc cgtgggcatc atgtccgcgc 56400

aggtgctctg cgagaagttc acgcagcagg cgctctcgag cttccacacc accgagaaga 56460

gcggcgccgc gaaggtgaag ctgggcttca acgagttcag caacctcatc agcatgagcc 56520

gcaaccacac cgagatagtg gcgctgaccg cgccgagcgc ggacaagctg atgccgctga 56580

aggtaaactt cgagttcgtg tgtctgggcg agctcgtgcc cgagatcgag acccggccct 56640

cgggacggcc ctccgtgcac cgcgtggaca tcacggtgca ccgcctgcgc atcaagcgcg 56700

cgcacctgac cgaggtcctg gtggacacca tcatcgagcg cttcgtgtcc ttcaacgtgc 56760

tcgtgaagga gtggggcagc gacatgaccg tggagggcga ccgcgtcacg tacacgctgc 56820

tgctgcgctt cgtggagccg gagcagctca acttccacaa gttcatgctg gtgctgcccg 56880

gcgccgcgaa caagggcaag gtgagcaggt tcaagatccc gatcaccgag accacggtct 56940

acgacgactt cgacgccgcg cgcaaggcct accgcatgaa catcgagctc atgagtctga 57000

aggagctggg gatattcgac ctcgaggacg tgaacgtggt ccccggcatg tggaacacct 57060

tcgacatatt cggcatcgag gccgcgcgcg ggcacctctg cgagagcatg ctggacacct 57120

acggcacggg cttcgactac ctgtttccct cctgcgacct gctcgcgagc ctgctctgct 57180

ccgggtacga gcccgagtcc gtaaacaagt tcaagttctg gaacgcgagc gcgctgaaga 57240

aggccacctt cggcgacggc cgcgcgctgc tgaacgcggc gctgcacaac cgcaccgacg 57300

cggtcgcgga caacagcagc tgccacttct tcagcaagac gccctgcgtg ggcacgggct 57360

actacaagta cttcgtgaac gtggagatgt tcatgcgcat ggagcgcgag atccaggcgc 57420

gcgtggcggc gcgcaagatg gaggagatcg aggaggccgc cgaggaggag ttctaggcgc 57480

gacggcgcct tactttgcga ccgtgtcacg acgacacgac acggttagga cggcgagtcg 57540

cagacgaaca tttttatgag ctggtagcgg aagttggcgt tttccaggaa ggcgccgcgg 57600

aggtcccgga tctcgtagta ggttttgagg aagtacacga agcgcgcggg ctgcgtcata 57660

gtcgggttct ccgcaagccg cttgtgcatc acgtacccca tggcggcggc gccgctgcgg 57720

ttgacgccgg ccacgcagtg cacgagcgtg ggcttctgct cggcctcgag gcgcgccagc 57780

agcttcacga gcgcgggcat gatggaagcg atgttcgtcg tgtcgtcgtc tctcagcgga 57840

atgtggtacg ccgttatccc cgcgggcgtc gagtacttgg acatggtcat gttaaccaga 57900

cacttgaagt cgacgccgga gtccccccgc agcacggcgc gcgcgtcctc ggcgctgccc 57960

aagtacacgt ggtccgtgag ccgcgtcatg cccgagggca gggccagcgg cggccccgcg 58020

cgcgtgcacc gcagcaggag cctggcgtac cactcgctct tatcgcccat atttatttat 58080

atgatacaaa tggcagacgt cacaacactg acggccaacg gtctgaccct ggagttcgcg 58140

cgcgagcgcg ctctgcgcag tctgcgcgcc gcgcgcacct ccacgctggt gttcttcacg 58200

ctcacgctcg cggcctcgct gttcgtgctc tggctgcagc taaccgagtt tcccgtcttc 58260

gaggagctcg gcaagtacgc gcgcatcaag agcgcggtgc ggtcctggcg cccgctggtg 58320

gaggctaaga cagagatcga gtccgacctc ggccggcaga agaccgccga ccggcccgag 58380

ctcttcgagt tcaggtgcgt ggacttcggc aagttctacc tgccggtgag gtacagcccc 58440

acgaccttcc tgccgcaagc cgtgcgccgc ggcgcgggcg atggctggat ggtgcacaag 58500

gcggcggccg tggacctcgc cgcgcagcag ttctgcgagt ccgtgctgcg gcaccgcgcc 58560

aacaacgtca tcacatgcgg gtcagagatg atgcggctgg tgggctacag cggctacttc 58620

gaggacgacc actggtgcgc cgcgacgtcc ggcgtgctga cgtgaacgat cacacgatgg 58680

ccgtgaccag cagcccggcg atgaaccaca gcagccgcga gttcggcagc agcagcacga 58740

gcaccagcag gtacgccagg atgaagatgt cgaccacgtc cacgtcgaag agccccatga 58800

aggagaagag cggcgtggtg aggaagtaga tggcgccggg ccagaagcgc gccagccacg 58860

tggcgagcag cgaccacagg gagggcgcgc cgctgagccg cgtcttcacc tgtatgtagt 58920

actcggggta gaccacctgc tcggcgccgg agagcaccac gcgcgccaga gagagccgct 58980

tctccagcgt gaacacctcg gtgagcaggc cgctgcgcag ccctccctcc ttgatgatcg 59040

cgtcgtagag cttcttcatg ccgccgacgc tgatgatgta ggcgtctagc gagacgtcgt 59100

acccgccggg gtagaccatg agctcggggt cgccggtgcc ggggacgttg gtggccagcg 59160

cgccggtcat gtaggtctcc ttgagctgcg tcatgtacca gccgttcgcc ttcatcgcct 59220

cgatgagcgg cttcaccatc tcgggcttgc ggaaggtcat gtcgttgtcg accaccagga 59280

tgaagtcatc gtcggagtac ttggtgggga cagtgccggc cgatatgctc tcccagaggt 59340

tgaggtggtg cgctgcgcgg cgctgcatct ccttcggaca cgtggacttg cacatgtccg 59400

tgaagaagtg cgggtagtct ttggagtcca cgtctttcca ttccaccgcc ttgagcacgt 59460

ggtcgccctt ggggtgcggc gcgggaggag atggcttggg tgccggcgcg ggggccggtg 59520

cgggggctgg ggcaggagag ggagcaggcg cgggttgagg cttgggcggg tcgtcggcga 59580

ggcccaccag gtacggcagc gtggggaaca cctccttggt cccgcggcct tcggcaaccc 59640

cgattatgta ggccgtgatt tcgggtggat ccatttagtt attaaaatta atcatataca 59700

actcttttat ggcggctatg gattcggcta tccagtcctt gaccgagccc acgatgcccg 59760

ccaggaacag gaagaaggcg aactccaggt ccacgcggtt cagagagtcg ctgaagtaca 59820

cgaagacgtc gctgtccggg aagaagctgc gccggaacat gttgtacccg ttgaccttgt 59880

gcgcgacgtg ctccgcgctc agcagcgtct cgtcgaaggg gtacgggtcg ctgaagcgga 59940

acacgtacat ggccgggttt gcgtagtagt acttcatggt gtttgtgacg aagaggctcg 60000

ccagcgagat gatgattttt ttcttctcga tctcgatctt gatgtggtcc tcgaagcgct 60060

tcatgttgta ggcgttggtg tcgtgcacgc ggatgagcac gcgcgagtcc gacatgatgt 60120

cctggaactc cgcgcgcgcg tcggggctct cggcgggcgt ctccgcgggc cgcgccacct 60180

ccgcgcacac cgtcggccta gcgcgcggcg gcgtgcgcat gggccgcgcc cccacgcgct 60240

gcgaagcgaa aaactccacg gcgcgagcct cgcccgcgtc cgcgtacgac tccaccaggt 60300

agttgcggct gcgcgtggtg cggccgatgg tgttcagccg gtgcagctcc gcgaccagcc 60360

ggcggtagtg cgcctccagc tcctcgggca tgatggaggt gtacacctcg gtgagcagca 60420

tcacggtgtc gaagtcctcc ttgccgcaga cgcgcgtctt cacgaggaag tggtgcacag 60480

ccgtcgcgat agagagccgc agcgtggact cggtgacctc gacgctggcg tccttggtct 60540

tcttcgcgct ccgcgaggcc atgaacgaga cgaggaagtc cgcgctgctg ttgagcacga 60600

tgaccagcgc gacgatgaag ttgaggttca gcgtcttcgc ggactggaac agctcggtgg 60660

ccgacgcgtg cacgtcgagc aggttcgcgg agagccgcag gaagaacacg ccgcgcttga 60720

tctcggccgc gaagcgacgt tcgtactcct gccggcgcgc gttgatcgcg atgaggaagt 60780

tcaggatgag ccggttgatg ttgtacttca cggcccaggt ctgcgtcttc atgatggtgt 60840

cgaaggacat cacgatgttg aagatgaagc gctggctgtg cgagaagtag ctgtagggct 60900

cgctgaggaa gatggacttg ttggtcgcgg gcaccaccac gcccgcgcgc gcgccggacg 60960

cgtcggtgtt caggtccggg atgttcatgc cgcagatgcg gcagtaggcc atgccgtcct 61020

caaagtacac gaactcctcc acgaactcgt tgatcttggc gaagtagtcc acgtccacgc 61080

gcatcgcgac cgcgagccgg atctggtgct cgcagggcgg cgactcgaag cgcacccccct 61140

cgccccagcc cggcggctcg cgcacgacca gcgcggtgcg cgaggccggg cggaacttgg 61200

cgtcgcgcgc gttgagcagc gccgggaaga ggtcgcagag gtgccggctc gagaggaaca 61260

cgtacttgta cagcagccgg cgcgcgtccg cggccatggc gtccacgaag gcgcggcccc 61320

actccgcgac cgcgggctgc tcctccgcaa agttgttcgg gtagaccttg tccgtggccg 61380

cgaggaacac cttcttcacg tcgaggaagt cgcggatcac gatggggacg cgcgcgccgt 61440

cgagctcgta catgaacacg tagcgcaggt tgagcttgcg ccgcgagacc gggatgccga 61500

tgtgccgaca caggtacgcg aactcgaggt acttcttcga gaagcggatg cggtccaggt 61560

tcttggagac gtactgcagc atgttgcgca tgttgaaggg gatctcgcgc acggcgggct 61620

ccgcggcgtc gtcgaaggcg gtgcgcagat cgctggtgcg ctgtacgacc acggcttcgc 61680

cggtggcgtc gtcgtgcacc agcacgttaa cgcgccgctg ccggatgacc atgtcgaagg 61740

tgttgaagaa catctcgtac atgctgtgcc gagtgtcgtc cgcgatgcgc tcgcccaccg 61800

agaggctcgc ggtggcgtcg tcacgcacct gcttctcgaa cttgtacccg atgtaggaga 61860

atatcgagat cagcgtggcg tcgtcggcgt cggggttctg ctccatggtc gcgaagagca 61920

ggcggatgtc gtcctccgtg atcgcgtcca cgttgtacag gttgaccacg aagatggact 61980

tgttctcggc gatgaagtcc gtgtaggact tggtggccgt gttcgggtcg cgcatgtacg 62040

cgcggatctt cggcacgatg ctcgcgagga tggactccct ggaatccatt taaggacggc 62100

aagggcgcgc gagaccgtct caaaactgaa atcgtataaa ctcttaaaaa atcggtattg 62160

aaagtacgca ccaccaaata aagcgtcgag gtcgggcatg tcttcgtggc gactcaaaat 62220

gagcaagtgt tcaggttcca gcagcgtcca gactctcgag gatctgcgta atcgtcttcg 62280

ctccgaggcc ttgggcaacg attgccaaga gccccgcgac gacctcttcc ccagcggcga 62340

ggagtgtctg gacatcgacg ggccctgccc ttgcgatgag gcggagcagg agatcgacca 62400

ggagcagttg cccgtgcccg aaaccgtgcc cgaaccgccg gccaagactc ctaagcgccg 62460

accagtgaag aaggataagg cagataaggc agataaggac aagtcgacca gaggcgcaaa 62520

gaaaccgtgc ccttcggacg acaaggatga cgagctcaag agcaacgacg tcgacaacaa 62580

cgaagagtcc ggcgacacag acggcggcgc gagcgcccga agccccagcg acatcgacaa 62640

cgtggacgaa atggacgact ccgacctcat ggtggcgttc tccaccatcc tcgcagactt 62700

caaggacctt acccaacgag tgaaagctct ttcgtccgtg ctcacggacg tgcaggcggc 62760

cggcatacgc aggagcttct cgacgctcgg caaggctctg acggaggcgg cccacatcgc 62820

caacaccgga tctaagccag tcactgcgcc tcgcaagaag aaggccgccg cctgcaagaa 62880

gtaggcgcac taaatagcga ggctcggtat gcgggcgctg cacctgtcag acggcaaact 62940

tttttttgac aaggagctga cgcagccggt ccccgacgac aaccccgcgt acgctgtcct 63000

tgcgaagatc cggatcccac cgcacctctc ggatgtggtc gtgtacgagc aggacctcga 63060

gtctgcgcag cagggcctca tcttcgtcgg gcgcgacgcc aagggccgaa agcagtactt 63120

ctacgggcgc ggacacgtgg agcggcgcac ggccgtccgc aacgccgtgt tcgtgcgcgt 63180

gcaccgcgtc atgaacaaga taaacgcctt catcgacgac cacctcgcct ccggcagcga 63240

ggccgaggcg cagatggccg ccttcctgct catggagacg agcttcttca tccgcgtcgg 63300

caagacgcgc tacgagcgcg agagcggcac cgtgggcatg ctcacgctgc gcaacaagca 63360

cctcgccgag gccgagggcg gtgaggagat ccgcgtcgcc ttcgtgggca aggaccgagt 63420

cgcgcacgag tttgccgtgc gcgaggggca gcggctcttc gcggcgctgc gtcggctctg 63480

ggacccgggc gcgcccgaca ggctgctgtt cgaccggctg agcgagcgcc gcgtgtacac 63540

cttcatgcga cgcttcggca tccgcgtcaa ggacctgcgc acctacggcg tgaactacac 63600

cttcctgtac aacttctggt ccaacgtgcg ctcgctggag ccgcgtccct ccgtgaagtc 63660

gctcatctgc acctccgtgc ggcagaccgc cgagacggtg gggcacacgc cctcgatctc 63720

gcgcagcgcc tacatggcca ccgcggtgct cgagctcgtc agggacggcg cgttcctgga 63780

cagagtcgcc gccaccgaca cgctcgacga cttcgtggac atcgtcgtgg actatgtaaa 63840

taactctgag caggtaaatg gatgaggcgc tgcgcgtggc ggcgcgcgtc gtggacgggc 63900

tccggccgct ggacgtggcc gtgtgtctcg cgcagctgcg cggagccgcg cccgagcgcc 63960

gcttcccggc gctcgacgag tgctccggcg aggccttcct ggactttgag ttcgccggcg 64020
gggacgtggc gtcgcggtac ctctccgcgc acacgcgcga gctccgtgcg gcggagcggc 64080
gcgagcacat ggccgcgatc gcgcgctgcg tcaccgaggc cgacctggcg ctcgcagacc 64140
gcccccgggg caaggcgcgc gcggcgctgc gcgtgtgccg caaccgcgag aaagtcgcgc 64200
gcttggcgag gctgctgcgc gacgccgaga gcagcggcgc ggacttcgcc ttcatacgcg 64260
cggccgtggc gtagcaaaac gtaaaaacaa cacattccct aaatcgccat ggacgcgcca 64320
agtctcgact gcatgctcgc cgcactcgcg gcgaaggcgg cctcggtgga ccgaggcgct 64380
cccgaggacg aggtgcacca cgaagtggag ctcgtgctcg tggacccgcc gctgtccacc 64440
ctggccgcca cgctgcgcct ggcctcggag acggagtcct tcatcctctt cacggtgacc 64500
gcgctcgcca aggaggaggg caagctgcgc gcgcgcgtgc ccatgtcgcg cgtcgtcggc 64560
ctggacgtga agaacgtgca gctggtcaac gccatcgaca gcatcgtctg ggagcgcaag 64620
gcgctcgtgg aggagaccgc gctgcaggaa ggctgtctgc tgcgccactc caccgagcgg 64680
cggcacctct tcgtggacta caagaagtac ctctcggcca tccgcgtgga gctggtaaac 64740
cgcgtgcgcg tgcgctccaa agaagtcgtc gcggacttca agttcaagta ctttctgggg 64800
tccggcgcgc aggccaagag ctcgctgctg cacgcactca accaccccaa ggtgcggccc 64860
tcgcccacgc tggagttcga ggtcgtcccc gcgggcgagg ccgtggacga ggccgccgtg 64920
ctcgcggagc tgcgcgccgt ggcgaaggcg ctcttcatgg cgcccaccga cgccgtcttc 64980
ctggcgccgc cggccgagat gccggtgcgc acgctcatgc tgcagaagca ggagatcccc 65040
gcgctagacc tcgacggcct tttcgcggtc tccaagacgg acggcgtctc cgcgagcgtg 65100
tgcgtggacg aggacggcgt cttctgcgcg ttctcgcacc tcgcgtacac catccggtac 65160
ccgctcgcgc gcgaagtgca gggccggtac cggctctggt gcgaggccgt gcggcccgtg 65220
ggcgagcgcg tgtggtccat gttcgtgctg gtcgtggagg agcctgcggg cgatgaccgc 65280
gtcgcggccg tggccggcgc cgtggaggcg ctgcgcggcg tgtgtgcacg cgtcgagttc 65340
aaacctaagc gcgtggacgg gcccttctcg gcgacctccg agctggtgga gcacatcaag 65400
agcgcgctgc agacggagcc agagggcgtg gtgctcttct acgcgcgcgg agagaagtcc 65460
aagcgcgacc tcaaggtcaa gcgcgacaac acggtggacc agaccacgaa cgtgatgttc 65520
cggtacatgt ccagcgagcc catcgtcttc ggcgagggct ccaccttcct ggagttcaag 65580
cggtacagca acgaccgcgg gttccccaag gagtacggcg cggggcgcat cttcctgcgc 65640

gaggacgtgg tctaccacaa caacatctac tgcatcgagt tcacgaagac gcacctggag 65700

gtgggcctcc gcagcgtggt cgtgcccgtg aagttcatcg gcgagttctc gcaggagggg 65760

tacctgctgc ggccgcgcct ggccaaaacg gagtgctact tccgcaaccc ctcattctac 65820

gggaaccagc actcggtggt gctcgagcac actcgcgacc agctgctctc ggtgggggac 65880

gtgttcgacg agagccgcat ggccgccgtc gggcagacgc tggccaacga cgccttccgc 65940

ctgaacccgg acacgcccta cttcaccaac cgacgcacgc gcgggccgct gggcgtgctc 66000

tccaactacg tgaagacgct catgatatcg ctgtactgct cgaagacctt cctgaacaac 66060

gccgagcgac gcaaggtgct ggccgtggac ttcggcaacg gcgcggacct ggagaagtac 66120

ttcttcggcg agatcgcgtc catggtggcc acggacccgg acgcgcgcgc gatcgagcgc 66180

gccatggagc gctacaaccg cctcaacgcg gggctgaagt cgcgctacta caagtttaac 66240

tacatccagg agaccatccg atccgagacc tacgtggaga gcatccgcca ggtcatgtac 66300

ttcgggcgct tcaacatcgt ggactggcag atggccatcc actactcctt ccacccgcgg 66360

cacttcgcca cggtgatgcg caacctgcgc gagctcaccg cgcccggctg caaggtgctc 66420

atcaccacca tggacgggga cttcctgtcg acgctctccg agaagaccag cttcgtgatc 66480

aaccgcaacc tgcaggagag cgaaaacttc atgtcgatcg agcgcgtggc cgatgaccag 66540

gtcatggtct acgcgccctc gaccatggcg cagcccatga cggagtacat cgtgcgccgc 66600

gcggacatcg tcaagctctt cgcggacaac ggcttcgacc tcgtggacca cgcgaacttc 66660

gagaccgtga tccggcgcag ccgccgcttc gtcgagggcg tctcgcggct ggagacgcgg 66720

ccctccacca agaacttctt cgagctcaac cgcaacgcgc tcacggagat ggacagcacc 66780

gacgtggccg cgctgctaaa gatctacgtg ctgtacgtct tcagcaagcg gtaggcagaa 66840

ccagggcgtc gattccgcgc ccgcgccggc gcggaaggcg ttgaacagct ccgccagcca 66900

ggctgcggtc tcgcgcgcgt cgatcgggcc gccgtcgtcc ggcggcggct cgcgcgccgc 66960

gcgcaacacc agcgtctccg cgggcggcag aggctccaga gcctcgaaga ccgcgcggct 67020

cgggaacagc gcgcgcatca tgcgcgcgcg gtggccgaac accgccttga ccgcgcgcag 67080

tgccgagcgg ttgtccagcc gcagcgctcg gtcaaaacga tgcacgcgcg cgggcgcgcc 67140

gcggtggtcg cgctccacga gcacgtgccg ccacgccagc gccgcgccga cgcggtccag 67200

gctgggcgcg agcgccacca ggcttttcag cgcatgtaaa tctccgcgca tggccgacgg 67260

ctccatttac tactgcggag gaacgcacgt ggtcgcggcc gcgccgggcg ccgcgcttgt 67320

ggtgctggac gcgcccggtg cggcggcggc ggccgcgccc gcggggcagc gcgtcttctt 67380

cgccgagtac ggcctcgaga agcgggccgg cggcccgatc acggcgcggc tgcgccgctc 67440

cgggttccgc ggcgccgcga acgcctgggc ctccgtggcg gacttcgagg ccggcggccg 67500

tccctccgcg tggacgctgc gcgcggagga ggcttcgcgc gtgccgctgc cgacggacgc 67560

ggcgctggtc ctggcctggg gcgcgcgcga ggagccgctg cgggcgtgcg tgctggcgcg 67620

cgcggcagac gcggaggcgc cggtgggcgc cgcgctcaaa gaagccgcct tcgacgcgcg 67680

ggcgccggcg gccgcgctgt tcgcggcgct gggcgcgccc gcgctcgcgc ccccgctgcg 67740

ggcgcggcta gtggcgccgc cgggcgcgcc gccgcggacg cggctctgcg agaacccggc 67800

catgctgcgc gcgttcgcgg tgggctggtt cggcgcgcag ctgggcgagg cctccgaaaa 67860

tgaaaaggta tttgccgcct ttgataaggc gaggtcgtgt ttggacgacc gctgatggcg 67920

acgcccgcga acgcgcccgc gctgctcgtc gcggcgctgc gacaccgccc gtaccgcgtg 67980

gagtaccacc cggactggga gccggtcatc gagacgctgg tggacgagta cgacgcggtc 68040

gcgccctggc tgctgcgcga cgcgacgagc cccgagcccg agcgcttctt cgcgcagctg 68100

gcgaagccgc tggcggacaa gcgagtgtgc gtgtgcggca tcgacccgta cccgcgcggc 68160

ggcaccggcg tgcccttcca gtccccggac ttcagcaaga agaccatccg cgcgatcgcg 68220

agctcggtcg cgcgcacgac cggcacgcag ggctacgcga actacgacct ggacgcggtt 68280

ccgggcgtgc tgccctggaa ctactacctc tcctgccgcg agggcgagac caagagccac 68340

gcgatgtact gggagcgcat ctcgcggctg ctgctgcagc acgtggccaa gcacgtgagc 68400

gtgctctact gcatggggcg cacggacttc cagaacgtgc gcgcgcgcct ggacgtgccg 68460

gtgacgctgg tggtgggctt ccaccccgcg gcgcgcgacg ggcagttcgc gcgcgagcgg 68520

gccttcgagg tcatcaacgc cttattggag ctcaacggga agtctcaagt ggactgggcg 68580

cgaggatttt ctttttatag tgaaaattaa tccgtggtcc taaatggcgg cgcccatatg 68640

cgataactct cacgtgttcc tcctcaagcg cctgggcgtg ccgtcttcct gccggcgctc 68700

ggaggacccg cgcttcgtgg agatcctgac tcccttcgag ctctcaaact acatcgagcg 68760

gcacccggga tgctgcctct tcgagacgct gcgcgacgag gaggactgct ccgtcgtgcg 68820

cgtcttcgcg gacgtggaca tggacagcgt gctcgaggag gaggacttcg tcgcggcgct 68880

ggaggacctc atcgtggagc tcgcggcctt cttcgaccgc ttcgcgagcg gctcctgcgg 68940

caccgtgccc ggcgaggtca agcgcgccat gctcgcgaac ttctcggtca cgcgatccac 69000

ggccgagcac aagaccagct tccacctgat cttcacggag acgtacacca cgctggacac 69060

gctggtggcg gcgaagcgcc cgctgctgga cctgtgccgg cgctcggaca acgtgctgct 69120

gcgcgcgctg gacacggccg tgtaccgccg cggcgcgacg ctgcgcgtgg tgggcacgcg 69180

caagacgccg gagtcgagcg cggtccaccg catgcagtcg cccgacgacg acatcaagga 69240

ctacctgttc acgttcgtgg agctctcgga cgcgagcgtg tacttcgagc tcgcggagcg 69300

cgagcagcac acgctgagca ccgtctgctg ggagacctcc tacatcccct tcggcgacgc 69360

gatgcggcgc gtgtgccagg cggtggtcaa cgacatcgtg aacctccgcg acatcaccga 69420

ggacaacttc ctcgacacgc cgctggtcat cgactacgcg acgcgctgcg cgctgtgcaa 69480

gaagcccaag cacaagcacg cgcaccacat caccatgggc aacggctgcc tgcgcctggt 69540

caagggcggg aacgcgcaca gctgcaaggt caagatcatc cagctcgagg gcaaccggct 69600

cttcacggcc gcgcagatca tcatcgcgtc cgaggtcgtg aagctcaccg agcgcaacga 69660

ctacatcgtg tggctgaaca actcctggcg cttcagcgcg gaggagtcgc tcatcaccaa 69720

gctcatcctg gacgtgcggc actcgctgcc cgcggactac gccaacgaca tgctgtgtcc 69780

gcgcaagcgc aaggtcgtgg agaccaacat ccgcgacatg ctcgtggaca tctccgagac 69840

ggacacgcag tacgacaagc tgcccttcac gaacggcgtg ctggacctgg ccacgggcga 69900

gttcctcacc ggcgaccgcg cgaaggcctg cgtgtgcacg gtctccaccg ggtacgcctt 69960

ctcgcgcgag gagttcgcgg ccgcggcgga ctcggaggcc atgcgccggc tggtcggcgt 70020

catcgacgac atccagccgg acacgcccga gaacgccgat aaccgcgcgc tgtacgagcg 70080

cgccatgtcc agcgcgctct gcggcgccac gaagacggtc atcgtcttct tctacggcga 70140

caccatgacc ggcaagtcca cgagcaagcg tctgctcatg tccgcgctcg gcggactctt 70200

catcgagacc gggcagaccg tgctcacgga cgtgctcgac aagggcccga accccttcgt 70260

ggccaacatg cacctgcggc gcgcggtctt ctgcagcgag ctcccggact tcgcctgcaa 70320

caacgcgcgc aagctgcgct ccgacaactt caagaagctg accgagccct gcatcgtggg 70380

ccggccctgc ttctccaaca agatccacaa ccgcaaccac gccaccttca tcatcgacac 70440

caactaccgc ccggtcttcg accgcgtgga caacgcgctc atgcgccgcg tggcgctggt 70500

gcgcttccgc acgcacttct cctcgtcggc cactcgcgcg gccgccgcgc acaacgtcga 70560

gtacagcgcg gtcaaggaga tggacgagag cctggacacc aagatccagc gcaactactt 70620

ccgctacgcc ttcctgcgcc tgctcgtgca gtggttcggc aagtaccacg tcccgcaggt 70680

ctcgctggcg cccacgcccg acgcggtccc cgacttcgcc ttccaccgcc gcgtggccga 70740
gctggtggtg gccagcaacg acgcgcaccg ccgcgcgatg gagtcgctgt ccaagctggg 70800
gtacgtgctc gtgggcggca acgtggccat gcccgcggac gccttccggc agcggctggc 70860
cgcgcacttc aacgcgcgcg tgcacggcgg cgacatagac gccttcatgt tcaagcacaa 70920
gaaggtcgtc aacgtaacgg aggagtacgt ggagtacgta ttcatcgaag atgtcgagaa 70980
taaatagacg ggtatgaact cggacgtgat aaagctgttc gtcgggcacg acgagtccgt 71040
gcccggcatc ctgccgcacc agctcgcgac cgtggacttc ctgatacgcc gcgttctaga 71100
cgacaacgtc agcgtgcttc tcttccacat catgggctct gggaagaccg tcatcgcgct 71160
gctgttcgcg atggtggcct cgcgcaccaa gaaggtgtac atcctggtgc ccaacgtgaa 71220
cgtcatgaac atattcaact acagcatggt catggtcgct aacctgttca acgcgccctt 71280
cgtggccgag aacatattcg tgtactcgac gactagtttt tattcgctaa actgcaacga 71340
cggcgtcata aactacaacg gcctcggcaa gtacgagaac tcggtcttcg tggtcgacga 71400
ggcgcacaac atcttcggga acaacaccgg cgagctcatg atggtgatca agaacaagac 71460
gcgcgtgccc ttcctgctgc tctcggcctc gccgatcacg aacacgccgc tcacgctcag 71520
cagcatcatc agcctcatgt ccgagaagga cgtggacgtc ggcgacatcg tggtgcaggg 71580
caagaaggtg ttccagatcc tgctgaacga gcacggcgtg cgcgtgatcc gcgaggtgct 71640
caaggggcgc atctcctact acgagatgcc ggacacggac atgcccgagg tgctctacca 71700
cgggcgccgc ttcctggaca cgcgcgtggt ctactgccgc atgtcgcgcc ggcaggagga 71760
cgactacctc accgtgcgcc ggctctgcaa caacgagatg ttcgagaaga acatgaacaa 71820
cgtgtccatg gcggtgctgg gcccgctgaa cctggtgaac aacctggacg tgctcttcca 71880
ggcgcaggac aaggacctgt acccgaacct gcgcatcagc aacggcgtgc tctacgggaa 71940
cgagctcacc aagctggaca tcagctgcaa gttcaagttc ttcatctcga aggtgggcgc 72000
catgcgcggg aagcacttca tctacttctc caactcgacc tacggcagcc tggtcatccg 72060
caacgtgatg ctcagcaacg ggtactcgga gttcggcggc tcgcagagca acaatccgca 72120
caccacgccc gacgggcgcg ccaagacctt cgcgatcgtg accagcaaga tgaaggcctc 72180
gctggaggag ctgctcgagg tgtacaactc cgcggagaac aacgacggtg gcaagctcat 72240
gttcctcttc tcctcgaaca tcatgtccga gtcctacacg ctcaaggagg tgcggcacat 72300
ctggttcatg accatccccg acaccttctc gcagttcaac cagatcctgg gccgcgccgt 72360

gcgcaagttc tcctacgcgg acgtggccgc gcccgtgaac gtgtacctca tggcggcggt 72420
gtactcggac ttcgacgagg acatcgtctc gctggaggac tacagcgtgg aggacatcaa 72480
cgcgctgccc ttcgacgtga agaagctctt ctacctcaag ttcaaggcca aggaaaccaa 72540
ccgcgtgtac gccatcctgc aggagctctc ggacgcgtac tccgcgcgcc cgcacccgca 72600
gctcgtggac gtggtgctgg gggagatcgt gcgccagttc ttcgcacggc actgccgcgt 72660
gcccgccgag gacgccgcgc tcgtggccgc cgtcgaggcc gttctcggca cgcgcgaggc 72720
agcggccgag tacatccgcg cgatagtgga cggacacttc ttcgtgacca acaagacctt 72780
cgggaagtgc ctgctcttcc ggcacgagcg cgacatcgtg accgtgccct tcgagctcga 72840
gcacgacccc ttcgcgtggg cgatcaactt ccgcaaggag gtcagtgtgg tgaatatata 72900
acggcaaaca taaatagaaa gactgtcctt ttgcgcgatg tcgaccttcc ggcagacggt 72960
gtacctggcg gtgacgctgc agccgcacga gctcacgctc gacttccgcg gcaacgtcgc 73020
ggaggcggtc atgcgcgagt acctctacaa ggagaagggc gggctcatgg ccaccgacat 73080
cgaggtctgc ctcggaaacg agatgccgct ggggcgcatc gtgaacaacg cggttgtggt 73140
ctcggtgccc tgcaacgtga ccttcaagta ctaccgcgtc ggcgacaccg tgagcggcac 73200
gctcaacgtc gaggacgaga ccaacgtctt cgtggactgc ggagacctca tctgccagct 73260
cggcaagagc tcgggcggcg tgaccttcaa cgagtccaag tactgcctcg tgcgcaacgg 73320
agtcgtctac gagcacggca gccgggtctc ggctgtgctg cgcgaggcgc gctccggacg 73380
cgagtccgcg ttcgtgttct ccgcagtgct gctggacggc gtccccgccg aggagaagga 73440
cgagaagaag gacgagggcg agaaggccgc ggaggaggag acgcccgcga gccccgccgc 73500
caaaaactag cattattggg ccgcgcgaac cttcgataaa tgcgcacgta cacgtcgctg 73560
ctctcgaagc tgctcaagag caaccggcgg ctcgggagca cgcgcgtctt ccgcgacccg 73620
ctgcagcaca tcagcgcgac cgcctttgtg caccggcgca tcgaccggca ccggcgcgtc 73680
tccatctgcg ccgtgctcac caccaccgac gggctcgtgg tcgcgtgccg gcgccggtac 73740
tcctttttgt cctccgagct cgcggagacg cgctcgcccg cgcggcgcgt gctgctcgca 73800
accaagcacg cggacgctct cgcgcgcctc ggcgccgcgc gcccgcgcga cgacgtcatg 73860
tttccgggcg gcgccccgct gtccggggag tcgccgctgg cgtgcgtgct gcgcgaggtc 73920
gaggaggaga ccgggctgcg cggcgaccag gtcagcgtgg acgagcggct gttcgtgcac 73980
gccttcatcg acgacctggt ctcgggccgc gacttcgacg cgatcatctt cacgggcgca 74040

gtcgcgcttt cgagcgcgga ggtggcgaag cagttccggc ccaacgacga ggtcaagggg 74100

ctggtcttcc tgcaccccga ggacgcggag ggcgtgggcg tgatggcgcg gctggcggcg 74160

ttcgcgcgct gcgcggcgcg cctgcgctgc tggggcgcgg ccgtcacgcg atagaggcgg 74220

ggtccaccac gtacacgagg cgcccgccgc tcacgcgcac ggtgggcggg tcgcccagcg 74280

cggtcaggaa gttcccgtcg tcgtcgaaga ggcgcccgcc gcgctcgagg aagcccttgc 74340

gcaccgtgac cagcgccgtg gaggtggagt accacacgct ctgcccgtcc gcgagccgcg 74400

ccgcgcgcgc gggcccgcgc gcgtccgccg ggcgcgccac cagcgcggac cagccggagt 74460

cgtcctccag cggcgcgaag tccgtgaagg cctcgcgcac ccactccagc gagcagcgct 74520

tgagcacgcg gaagagctgc gtgaactgcc gggacttgtc gcggatgagg gccagcaggt 74580

cctcgtccac ggtggcggcg ccggagtcct ggcgcgcgac cacgaagtgc acgttcacgt 74640

agcggcggtc gggcggcgtc atctcgtggc tgttcaggcg caccgcgcgg cccacgatct 74700

ggcgcagcga ggcctcgttc caggtcatgt ccaggatgaa gatgtcgttg atggagagga 74760

agctgaggcc ctcggagccg ctcagcgaga acacacagac cttgatcttc tcgccgtcgg 74820

tgttgtcgca ggcgttgaag gcgtccacga gcttggcgcg cgtgtcgcgc gtgcgcgagg 74880

agaactccac gctggagacg ccgaaggcgc ggaagtagag cagcagcatc tcgatgccgg 74940

tcacgttgac gaagggctcg aagaccagac acttgcccgg cgaggccagg atgcgcaggc 75000

agacctcggt gtacttgcag ctgcgctcgc gcagctccgc gagcagcgag acgtccgcgg 75060

aggtcatgcg gtcgccgctg acgggcgcgc cgctgcggaa gagccgcatg gccgcctccg 75120

agaagacgcg gtccttgacg gcgcgcgcga agtccaggaa gagcgcggcc acggcctcgt 75180

cgtactcctg cttggagagc acggacttgt cgggcgcgtc ctcgaaggcg aaggtggccg 75240

cgatgcgccg gtacacgcgg aagaccgcgg cgccggactt gcgctccatg gcggccgcgc 75300

ggcggtaggc ctcggtctgc ttcgcggtca tgtccacgta catcatgcgc acgcgcttgc 75360

gcgcgaaggc ggcggagccg tcgacgtcgt cgaagatgga ggcctcgttg gtgactaagt 75420

acgagcacag gccgccgagc ttgtccacga ggtcctcggg gttcgcgagc gcgccgccgt 75480

tgaagagcgg cgtctgcccg accacgccgg ggcgcagcag gttcacggcc atggagaact 75540

ccttgacgct gttcaccacc ggcgtggccg tgaggcagag cagcttcccg cggcccatgg 75600

ggatgttctt cgcgaggtag ttgtacaccg tgcgcgcggg ccgctggcgc ccgtcctcct 75660

tggtcagcga catcgagatg aagttgtgga actcgtcgat gaccacgcag acgcggctgc 75720

tcgacgaggc ggtcttcatc agcgtgaaga agcggtggtg gaagcgcggg tcgtcgtagt 75780
tgatgaaggt gcacccgggc acggcctcgg gcgcgaagcg catcatcgtc gaggtccagg 75840
gctgctccac gagcgccttc ttcacgagca cgaccaccgt ccagtccgtg aagacgtcgc 75900
gcaggtgctt gagcacgtac accgcggtca cggtcttgcc cacgcccgtc tcgtggaaga 75960
gcagcagcga gtgcatgctg tccaggccca ggaacacgcg cgccacgaag agctggtagt 76020
ccttgaggcg cacggactcc tccacgccct gcatctcgga gggcatgtgc gcggtgcgcc 76080
gcagcgcgta gtcgatgtag gccgcgtgcg cgctggtcat ggcgacggtc ggcgctcctt 76140
ttacggggtc tgtcgtctat ctattgtcgg cgcgggtctg atttaggggc agtagttaca 76200
aaaacgtttc cgctgctcgg cgcggcgttt ggaggagcgg ttgcggccgc ggcggcgcag 76260
gcgcgcgcgg cgcgtcttcg tggtgcggtg gccgaaccag cgccggtgca tgaccgggtg 76320
cgagaccgcg gccgcgcgat ccgcgctcat gcaggttgcg taggtgcggc acatgctgcg 76380
cagcacgcgc cgcgtgcgcc gctccacggc gtcgagccgc ctcgcgacga tgggaaagag 76440
ccggcgccag ccgcgcacgg cgaagagcgg gcgctcgcag accgggcgcg cgagcgcgtg 76500
gtaggcgccc agcagccgcg ggtccagcga gcgcacgtag gtctccacga agccgttgcc 76560
gaagacgatg gcctgtgcgc atagcgggtt cgtcatctcc ctcttggagg cgatggcgtc 76620
gcccacgaag gcgcgcacgc cgcagtgccg cagcaccagg cgccgccgcg ggaagtgcag 76680
gtgcgggccg agcgccgcgc gggcggcggg gatgtgcagc cgcggagaaa aacgcgcgcg 76740
tccctccatg gcatctaagc gctccgtctg ttttcagtta tatcgccgcg ggcggctact 76800
gcagcagcag cttgagcttg cgctggctct cgttctcgat gctcttggac tcggaggtca 76860
tgctctcgta gagcagcgag tgcgtgacgt agagcgcctc gtacacgcgg ctggcgaagg 76920
ccacgaagcg gtccacgaac tcgctctcta cggggtcctt gagcacgcgg aagggcacgg 76980
ccagcgcgtc gcgccaggcg gcggccttgg tgcgctcgcg cacgtgcgtc acgaaggcgg 77040
cgatggccgc gcgccgcggc tcgctggcga ccatgacggc gctgtccttg agccagctgt 77100
cgctcacgca cttgaagagg cgcacggtgc cgaagaggct gcagtacacg cgcagcgcgt 77160
gcaccacgtc ggtgccgaag agcgtgggca gcttgagcac cacgaagcgc tcctgcgtga 77220
tctcgagcag cgggcgcatc accgcgaagg tgatcgcgtg gtagtcagcc acgtagaggt 77280
tgttctcggt gaggtggttg ttggagcgga tggcgccgcg ctccttgcgg tagagccggt 77340
tgcgcgcgct gaggtcgagc acgaccgcgt cggccctgcc gcgcgctctg gagcgcacgc 77400

tggtgatgcc gtgcgcctcg agcaccttct cgacgtcgcg ctcgtcgatc atgaggtcgt 77460
gcgtgtacag actcagcatc tccgtgggca tgcggttgat gtcgttcacg cgcgagcact 77520
gcaggaagta gttggtcccg taggccaggc tgggcaggtg cccgacgccg agctgcaggt 77580
ccagcgcggg cgtcgagtcg aaggtgggca gcgtcacgct gagcccctcg cggatgctgc 77640
ggcgcacggc ctccaccgcg tccatggccg atttattgga cgcacagtct gttttcattt 77700
cgcggctact gcgcagtcac cttctcggcc acgatccccg cgtcgtagct gagccggtac 77760
acctcgttgc acaccacgac catctgccgc ggcacgtaca tgagcgggtt gtgcgcctcc 77820
atgtgcgcgg tggtcacgcg caccgccagc ttgtccttgc ccctggagac gttggagttc 77880
agcgcggtgg gcgagaagaa ggtgctgggc gtaaagttga actgcagcgt gcgcacgccg 77940
ggcgtcttgc cgaggatctc gccgaagacg cgcgagactg cgctgttctc cgagtacagc 78000
acctcgttgc cgaagcgcac gtccatgcgc gcgatgacgt cgatcttgtt cttgaagtcc 78060
acgcccttga ggaaggggtc ggccacgaag aggtccttgg cgcgcgcctc cggcgagcgg 78120
ttgtcgccgt tgtacacgtt gcgctggcag gtccacacgc ccacgggcac ggaggcgtcg 78180
ccgatgttca cggagtggat ggcggtcgtg aagcggatgc gggaggtcgc gcggctgtag 78240
gcccccgtga tggcggagaa cttcttggac atgttgtaca cgacggagtt cttcctggtg 78300
gcgaacacta ggatgttcgt gtgcaggaac acgcgcatgc ccacgggcac gtcgtcgatg 78360
cgcacgaaga cgtcggtgtc ctggatggac acgacgcccg acgggggcac ctcgactatc 78420
tccgcggtct cggggaagcc ctcggggtag cagttcgaga cgatcaccat gtcctccagc 78480
aggcgctcca cgaaggccat cacgaagtcg ccctcggact gctggaagcc ggggtacgat 78540
atgaagcggt tgttggcgtc gctgagcacg ggcttcatgt acacggacag ggaggtgcac 78600
gcgtgcacgt ccgtgatcac cgcggtggtg tggttgatct gctccacgcg ccgccgcggc 78660
atctcgatga aggccgggcg cgggcacagg ttcttgacca tgtagccgat gaagctcagc 78720
tccatggagt aggggaactc cttggcgagc ttggcggcgt cgaaggtctc gtcgtagacc 78780
atgacgcagg cgacggggtt cagcgtgacc gtgaccgtga ccttgctgtc gctgagcttg 78840
agcgtgctga aggtcttgtc cgcgtcaaag ggcgtcttga tgtaggcgtg cacgcaggcg 78900
gcctccttga tgacgtcgtt gggcgagctc ccggtggaga ggtcgttgag ctcgcgcgag 78960
aagcccgaga gctccatcac gcgctcgttg tccaggcagg agtcgaacag ctcctcgccg 79020
gaggtctccc agatggtgtc cgcggcggag ttcacggcca cgtggcggat gagcttgtac 79080

gcgatgtagg gcacgtagca catcttgccc acgcccttta tctcgggcag gtccacgctc 79140

agcacgaagt tgttcatggc cgagatgtac ttgtcgcgga tctcgaaggt cacggtgacc 79200

gcgtcgctgg tggtgtccac cacgccctgc gtggtgatgt actgcggcat gtacaccgtg 79260

ggcgcgcggt ggtccgtggc gaacacgctg gcgcgccgca cggcgtcgtc gccgcccacc 79320

aggctcacca cggagttatt catttattcc ctgggaaaac cagttaaata aggctcttca 79380

gagccatgcg caccgtccgg ccgtcgggct ccaggtagca gcgcccgtag acgccctccg 79440

tggcgcgcgt ctcgttgatg agcgcgcgca cgcggtcggg gtccgcgtac atctccagcg 79500

gcagcagctc gatcttgggc tcctcgcgca gcgcgacgag gtgccggatg gagcccgcga 79560

aggagtcgcg gcacaaccgc gagcagaact cgcccacggc gccgccgtcg agcgtctcca 79620

cggccagcgc ggccgtgccc acgcgctgac ggcagaacca gcacgtgccg tccgcggcgc 79680

gcagcgccag ccgctccgcg gacaccgtgt tgaagtactt cggcagcacg tactcgatgc 79740

ggcacgccgc cggcggcgcg caggccgacg cccgcggcgc ggatatgtcc acccgcgaga 79800

gcgcgatgcg cttcatgggc ggcggtggct gctatttatg tcgcccgcgg cttttcaaag 79860

gtcgagcgag cacgccgcga agcgcgcggg cgagaacacg tactcgtggc cgaactccgg 79920

gatctgcgcg gcgcgcttgc gcgcgcgcat gtgcgcgagg aagttctccc aggtgagctg 79980

gttgctgttg ttcttcgcgt agttcttcac ggtctgcgga cgcaggttgc gcgtcacgcc 80040

cgtgacctcg aagatcttgt ccaggaagaa ggagtagttg atggttttgg tgggcgtgat 80100

ctcctggcag aagaagacca gctgcttgaa tatctcgatg acctcgttga tcttctcggt 80160

gctgaggtcc agcttctcgt ttttgacctg gttgatgatc tcgaagacca gcttgtagtc 80220

cttcttgttg atcatttcgc tgtccttgag gaagctggag acgtagttgg cgtccacgtc 80280

ctcgggccgg atctggtgcc ggtccatcat cgcgcgcagg tcgcggatga cctcctccga 80340

gcactgcttg gagagcagcc gccggagcac gttccgcagg tggatgagct tgttcgacac 80400

gtggaagttg gacctcttct gcacgcggat gcccatggga aacacggtct cgcagaacag 80460

gcagaactcg tagtccgcgt cggacacgag cccgttgcgg cggcagccgc cgcacatgcg 80520

caggttcatg cttgctccag ccccagcacg cgcaggatct cgcggtccag cactttagtg 80580

tccagcgtgc gggttctaca gaactggagg aagcccgcga gcgcgcgcgc gcgccctggc 80640

tgcgagagca gcagcatgcg cgcgttctcg gggtcctcgt tgatgacgcg cgtgaggttc 80700

agcgagcacc gcgtgcagcg ccgcggcggg tcgagctcga ccgagtacgc cgcgaaccag 80760

acgttgtcgc ccatgtatta tttattaaca cagaacgtcg cacatgttgc gcgaggacat 80820
gtacgggtcg tactcctgcc cgtagatgag gatggtgcag taccgcgaga tcatgagcat 80880
ggcctcctcc atggtgagca ggtcgtcctc gaacatggcc ttgtgctgca tgtgctggct 80940
ctgcttggcg gccatcgcgg ccgcgccgtc gccgcgcagc cactcgttca gacactggcc 81000
gtcgccgccg gcgccgctct cctgcgcgaa ggtgttgcgc atggcgcgca tgagcctggc 81060
ctccctggcc gagcggctga gcacggtcat ggggtcgtag agccaggggc ctgcgtccgt 81120
gaacaggatg gtgcagtacc cgttggcgaa ggcgtccgcg ccgctgcagt tgtcgatgcc 81180
gtcgccgacc ctgtagcaca ccgcggagac cagccggtac atgatgccgt tgagcatcat 81240
gtcctgcgac acctcgatgg gaatgtcgct gatcacgggc cgcatgttcg tgaagcagtc 81300
gcccgtgctg gccatgcccc cgcgccggtt caccaggaac accagcacgc cgttcgtgat 81360
cacgggcgcg cggtcgcgct cgtagaggta gccgctcgcc gcgcacacgg cggacgccac 81420
gtccgtgcgc gagaccgcgc ccatgttctg ggcgggcatg tacagcactc gcccggcctc 81480
cgcggtgcac gagaagggct gctccccgcc cacgtggatg ggcgccgtcg atgtcgtgat 81540
catcttgctg gagtccacca ccaggtaggg caccgtgtgc atggccatgt cgcccatgcc 81600
cccgatcccc gttccgaacg acggccgcga cacgctcacg agcgtcggct tgaacgagac 81660
tatggagaag atggaggcca agatctgctc ctcgtcggtc atgatggagg cgcacgaggg 81720
gtggatgatc ttcattaggg cgttgtcgat ggactcgtcg ctctcgcagt agaagacgcc 81780
catgcggagg ttcaggatgc accgccgcag gttggtgtgc agcaccgcgc gctggatctc 81840
catggacacg gagtcgccga cgccgggcat cacgatgggc gtttcctcgg tgagcttgtt 81900
caccagcagc tggtagttgt tgggtcgcac gcggctgttg tggtggagct gcgccaggag 81960
cgagaggctg tccccgttga cgaacgcgga ctcgatggcc ggcagcttta cgccgaacag 82020
cgccatggcg atggggtgca cgaagcccac ggagtcggac gacttgaacg agaagagcag 82080
gtcgcccgag gagctcatgt ctttgaagtg cacggactgg aactgcgtgg cggagagcaa 82140
gttctggtag ctggacatgc tctgcaggtc gtcgatctcc ttcatctgct tgtttacgcg 82200
ggtcgagttc ctcccgtaga tgatcaccag cgggtgcgtc tggctcacgg atagccccga 82260
gtccgtcatg gcggcgcgca cgctgttcag cagatcgaac agctccgacc ggtctctgtt 82320
ctggatcccg acctttgcca tcacagacat gagctcctgg atggtcatgt tcttgtggtc 82380
tcggcgcgtg gaccgcaggt agtcggcgat catctcgccc tccttacgga tcttcatctg 82440

ccagtcgtgc atggaggtca tgcggtccac aggcatgagc acgctgtcgg aggacgactg 82500

cgcggcgctg ccggactggc gcgagccagg gcgcgcggac gacgggcgcg cggagctgcc 82560

gcggctggag gaggacctgg acctccgcga ggatcttcgc tgcgaagagc tgcgcacggg 82620

ccgctgggcg cgcgccccgg cggaaaccat gtcctcgcgg tttatgctga ggagcgagct 82680

gcagaccgcg cacgacagcg actgctttgg aatgtggatg tggtcgcact ccagggacat 82740

gcccgcattg tcgtagcccg ggaccaagtc gaactttgca ttaaaaaaat ctgatgcgca 82800

cgcgggcgat tccatttata ccggaagttt ttatgaggtg ccggtattat ccacgcgatc 82860

tcgcagtgtg ctgggagact ctagcgtagc cacggacccc gtgagcagac gacgcaagtc 82920

gttgatggcc gactgcgtga cggacttcgc cgtctcgatg tcgcgcgtaa ggctgagcga 82980

ctcggcgttg aggtcgcgca cgctgtccgc gatgtcggcc agctcctttt tgatgaaatc 83040

cttatcatta tcggcgttga tgactttgtc cggcactcta gactctagaa ccggtgacgc 83100

ggcgggcgcc ttgatcgtcg ggcagctgga cgccgggtac tggggcggcg gcaatgattg 83160

ctgtaggaag atgggcttag cggcaggcgc cggttttgtc ggcaagggcg gcgccggcgg 83220

gcactgtcgt gtagacggcg ggcacgccgg cgcggggcac gccgccggtg gcggacatgt 83280

cggcgcagtt gcgggtggac acgcgggcgc gggcgccggc gcgggacacg tcgcggcagg 83340

agccgggcac gcgggagccg gagctggagc cgggcacggc gcggcaggag ccgggcacgt 83400

cgcggcaggc gcagggcacg cgggagccgg agccgggcac gccggcgcag gaggacatgc 83460

cggcgcagcg gcaggacaga ccaccgctgt cgcggagcac gcggcaaccg gcgtggagca 83520

cgcggcgggc attacgttca gaggcgtcga ctggaccttg gcaccgcggg gcagtagcga 83580

tggcgctagg ggcgactgtc cggtggttgg acgctgcatg caggcagtcg cagatttcag 83640

acgggactgg taatacctgc ccgcttcctt caccgtgtac ttgtcgacgg agtctacctc 83700

ctcatctgga ggacatggct gctccggtgc gggaacgact gtttgaggac acttggtgaa 83760

cagactggag ctggtctccg atagcaccag tttagacttg gccaagtcgg aggcaaacct 83820

tcttctgaga tccatttaag ccttcaaaat tgaacgtgta cgccgaccgc taaatggaag 83880

aatcggtggc cgtcgagtac gcggacgagg acgaagatga gattgaggag tacgaggagg 83940

aggacgatga cgaggaggaa gagtctgccg agggcgccgc cgcctcctcg gtcagcgacg 84000

tagcgctctc tgccgccgag aagctggtgg cctcggaggt cccggacgac gcggctgccg 84060

cggacaccaa cgtgcgtcaa cgcgtcaccg cgcgcgtgga ggagcttaag gcgcgctaca 84120

cacggcggat gagtctattt gagctcaccg gaattgtagc agagagtttc aatcttctgt 84180

gtcgcgggcg gctgccgctc gtggcggacg ccgcagaccc ggcgctcgac aacgagctaa 84240

aagtggtggt tcgggagctc gaggagggcg tctgccccat cgtcatcgag aaaaacggcg 84300

agttcctctc gccgggcgac ttcgaccccg agtgcctgcg ctaccacctg acgtacatga 84360

ccgacctctg gaagtcccag gggcgcatgt agccgcggct acgccgactc ggcggcctcc 84420

gcgatttttt cttttatcat gtccagcagc tcgcgcacca cgatggggcg gccgcagtac 84480

gtgatgccgt tttcggatat cacgctctgt gcgatgtcca ccagcgagcc ctcgcgctcc 84540

cagtactcgc gcgcgagcac ctccttgtac aacgcgcggt ggttggccac gtaccgcacc 84600

agcgtctgga tgttcttcac gcccacgctc ttgaggtcct gcggcgagaa cttctcgcgc 84660

agcgacacga agacgtcccg gacgagcttg ccgatctcca cgttggtctt gaactcgttg 84720

tacagcacca cgtagagctt gcacacgacc gtagcgaact tcgcgggctt gagatccttg 84780

ttctggaaga ccagcatgct gctcatcacc ttcttcatga aggccaggta cttcgcgcgg 84840

tcgccctcga cgctcacgct cccgacctcg aggtcggaca cgcagcggat tccgtgctcc 84900

gcgctctccg cggagacgcg cagcagctcc tggtactcct tgagcttctg cttgtccgtc 84960

atcagcgagt tgtcgaatac cgccaccagc ttgagcacgt agttctcgtc cgagaagacc 85020

ttgttcagac acttcaccag gaagctgtag tggctctgca ggatcttcat gaccgcgttg 85080

gcgccgctgg ctccgcggac gtgcgatatc atctccatga tcttcttaga gtcgtcgatg 85140

atctcctcgg tgtcgttgcg catgttgcgg tacattgcgt tcagcgagac cagcgtctgc 85200

gcggccagga gcacgtcgcg gaacacgcgc gcgaactcgc gcttctcctc cgcgtcggtg 85260

atgctgttgt acacggactt cgccaccgcg ttcgacttca ggaaccagaa ggagagcgcc 85320

tggtagttga agtgcttcat cagcgccagc acgtccgcct cgctcatttc cggcgcaatg 85380

gggcacaccg agctctcgag cacgggcacc atgctgacga gcgtgtccac gtccgtgtcg 85440

aagtccaggc agtccacgca gagcccggtg ccgcggctca ggtgatcgcg gctgatgtcg 85500

tagaagcgct cgtagcaggt gcggaggcgg tccatgtcgg ctgcgtttta gagagacaca 85560

cactcttgaa ttatggctgc gggtagaact cctgcagcag cgccggcgca cgcgcggagt 85620

ccggctccac tcccagcttc agcgcgcagt tcacggacca ggtcttcatg aagcggtcgg 85680

gcgcgtccgt gaccacgtgc cggaagagct tcgcgaagtg gcggctcacg gcgttgggca 85740

cggtcgcgtt gcgcacgaag gccgtgaagc gcgaggtcag cttcggcgcg aagcgcttgc 85800

cgtccacgaa gaagcccgag gtggtgagcg agagcccgtt ctcctcgcgc accacgcgtc 85860

gcgcggcctt gtgcggaaac atgctcgcga ggcgcccgct cgcgtcctgg tctaggtgga 85920

tggcgtccgt ggccgcgtcc ttgcggatgc gcaccacgtc gtgcacgatc tcctggatga 85980

ggatgcgcgt ggccgcggtc tccgccagcc gcatggggaa gtagaccatg tccccggaga 86040

tgagcacgtt cccgctagcg tttacgtagc tcactatctc ggacacggtg cgcagacgca 86100

cgatcgcgcc ttcgcagcag tgcaccacgt agtacccagc ggtggcgcgc aggcgcttgt 86160

tgtccgcctc gaagtccgcc tccaacccct cgttgaagta cttgtcgaat atgatgggca 86220

ggaaggatag ttttgactcg gtgaccacct ttccgaagtt gaggatgtac gggttcagcg 86280

cgctgcggtc gacctcttcg tcgtacacgc aggacttgaa ggtgtcggtg tgcgcctggc 86340

tgcgcaggaa gcagcacgga atgcagatgc gctgcaggcg gtggaagatg gagaggaagc 86400

ccacgctgtt gtagcgcccg tcggggtcca tgcacgaaaa catgacgccg tttccgttta 86460

cgaagacctc gcgcgtctcg gacttgaaga agttgttgct gaccttggcc atgttcgcgt 86520

ccagcgactg cacgatcacg ggcttgcggt tcttggtctt ggtgttctgg cagatgcgcg 86580

accagtacac ggtctccacc ttggtgaagt ccgaggactg cttcacgttg ttgaacatca 86640

cgctgatggc cacgatcaag aacgtgaagt acttctcgat gttcgggatg tagttcttta 86700

ccttcacgga cacgtgcgac ttcgcgagga tgatcgagat gcgcttgtcc gtggagagca 86760

ggatgttgtt ggtcgccgtc tccacgaaga tgaagctcgt ttccatgtcc agcttcatct 86820

tagacgtgat ggtcgtgttc agcgacacct tgtaggtgat gtcgcccttg acgcggtcca 86880

tctttacgtc catgctctcg atgagcttcg tgaacagact cacgtcgttc accgtgagcg 86940

tcttcccgtc gctcgagatg accaggtcgc cttccgggcc ccacaccgag aggttcagcg 87000

gctcgtccac cagcaggaag cgagtccccg tcatcgacac gaagaagtcg tccgtcttcg 87060

agagcaggat gtcgaagtcg cccacctccg ctcgcttgtc cggcgactgc tgcgcgatcg 87120

cgcggagccc ggactcgcgc aggttcgtgc ggaagatgtt gttgaacttg gtctccacgt 87180

tcatgtttag gtcgaggttc gcgaactcgc ggatgagccg ctcctcgaac ttgaggatgg 87240

agtcgttggg ctcctcgaag gagccgaact ccggcgcgga ggtgtccgcc gcgcgcgcca 87300

cccagaccac caggaagttg cacgcgtccg cgtacgcgtt gtagaggatg ccgtccgtgc 87360

ggatgagcgt tttcttttgc gtgggcgaga acgggttgaa gatggtgttg tccacgtagc 87420

tgtactccag gttgttcttg tgcgagtaca cgatgatctc gtcctgcagg cccagcaggc 87480

tccccaagta ccccttgagc tgccgcacgc gcatggtcag caagatgtgt ctgcgcacgt 87540

gctcggggtc cttctggatg tactgcttcg cgaagaagta gatcggcgag gcctcgtcca 87600

cggagtcgta cagcgatagg tacagcacgc gctcgatctc ctggtggcgc cccaccagca 87660

ccaccagctg cggcgcgacg gtgtagagca tgttcgcgcg ggcgtattta tagccggcgt 87720

taaactgaaa taaaatacgc gggtcgcgag gcagcgccat gttccagccg gtgcccgaca 87780

tggccgccga ggccgacatc gacctcggcg acgtcagcgt ggacgcgacg cgcgcgggcg 87840

cgcgcgagaa gaccgtcttc ttcgcgcgca acaagcgcat gtacccgcac cgcagcaagg 87900

acgaggagcg caagctgtcg ctgggcttct tcttgcagcg gctggacttc ctcacgtcgc 87960

gcgaggtcaa cctgcagttc cggtcgctgg acgcgctgcg caccgagaac gtcatgaaga 88020

agaacaacgt gctcgtggcg ccgtacatcc tcatcgcgac gctcgcgggg cgcggtttcc 88080

gcatgacgga gaccatggtc gagctctact tccccgagct gtaccgcgag accagcaagc 88140

gcttccgctt ctgcgcgcag ataaaggtca tccaggactt cctggggttc gcccacgaca 88200

gctaccacac ttacgacttc gagacgtact tcgcgttcgt ggcgctggtg ctgcgcggcg 88260

cggactctgc ggccgaggcc ttcgacgtcc gcgccgagag cgggcttgtg cgcagcctca 88320

ccgagatcac gtaccggctc tacgtgatgc agctgcgctc cgacgccgcg cagtggagcg 88380

tgagcaccgg cgccgtagtc tcgcaggcgg tgaacaccgt gctgtcggtc gtcggcgacc 88440

tcgctgcgcg cgcggaggcc gagcggctca cgcccgtgtg cgacctcgcg cgcgagaacc 88500

cgctctcgct cgaggacctg cgcaagtacg gcccgcggct gcgctcgctg ctcacgacca 88560

tggcgcgcgc gcgatccttc aagacgaacc ggcgggacaa ggacgcgctg tcccggttct 88620

gccgactgac ggcgggccct agcccgtctg cgtgccgcgc gtcgccatag gcgtcggcgc 88680

gcgctcgccg ccggaacact cggggtcgct gaacatgtag atgagcgcga cgcctagcag 88740

caggtacatg atcatgctga tcacggtttt gaacacgacg gcggcgaacg tgttggaccg 88800

cagtcggtgc tcgcagaagt gcatgaacag gtgccgcatg aggtcgatgg ccccgttggc 88860

cacctggaaa agggcgaggc cgccgatgga cttgatcacc gtcacgtagc acggccgcat 88920

tccgacgacg ctatttactc actgtcaaaa gaaacggcgc catccgaccg gaggttgagg 88980

ttgcgcttca tgttgttcca gtacatctca ccgatgctcg agtagtacgc cgtcagccgc 89040

gatatttttt ctcgcaccag ctcgtaggcc ttctgcatct ccgcaacgcc gatctccgcg 89100

tcgcccacgt accggccgct gcggcgcacg atcagcagca gcgccttcag gttctccagc 89160

gcgatcatgt ccatgtacag cgacttcgag agctgcacga agaggttgta ccgctccagg 89220
atgctgttct tcacctcgtc cgcgatcggg actccgaaga tgcgctccgt ggtgtacacg 89280
gactgcgtga gctgcttgaa gagcgcggag atgcagcagg tcgcgcgctt gacggcgtcg 89340
agctgcttct cggagcgcgc gctcgcgatg ctcagcgcgc tgttcacgac gttgctcgtg 89400
tcgcgcacgt agcgcgtctt cagcgcggcg ttgatggcat ccgcgatctc gttgctgctc 89460
acgctcgagt cgtccgagct gcccgagacc tcgtccagca gccccgagat cgtgatgtcg 89520
ggcgagccgc cgacggtcac cagacggtcg agcaggttgc agggcatgga catgaggatg 89580
ccctcgctcg agaggcagcc ctcgtcgatc atgctctgca ggttgcgctt gaaggccgtg 89640
ttttcgggca tgaacccgtc cacgctcatg agctcgtcga ccgtgctcgc ggaaaagatg 89700
cccctcacgt tgatgcggtc cagcatgccc atgtcctgcg agcacagcac caccgactgg 89760
tcggccgtct cggcggcgtc cttggcgccg ccgtagatga tgcgcggaaa ccgccagctc 89820
gccggaaaag agaaggaggg aaaccggcac tgcgcgctcg ggcctcggta gccctgcgcg 89880
tcgcgcacgt tggtggccgt gaccatgaac tgcagcaggt cgtgcgcgga cgccatgatc 89940
ttctccacct cctccttgct gcagcagacc ttgcccaggc tgcgcgcgat gttcgttttg 90000
ctcactgagg gcgagaccgt gacggcggtg tgtcggcggc tgccgagcgt gtacgcgctc 90060
acgctaacgc ggtaccccat ggcgccgaag agcagcttca cgaagtccag gtagctctcc 90120
ttattgatgt agtgcggcgc gcccttgtct tccatcctca gcccggcgta ggccatgagc 90180
acttccttca tcgccgtctc ggggtccgag ttgcacacca gccgcagcat ctggaagaac 90240
tgcatgaagg cgcgctgcga gagcccgatg tggtggttgg gctgcgtcga ccggcgcggg 90300
aactccctgg gcgtcatggc gttgatgccc gagagcgtct ccatcacgag cgcgcccacg 90360
gtcttctggc ccatgacgcg cgggtaaaag cacacgcgga ggggctcctt gccggccgcg 90420
agcgcgtccg agagcagcga gcagtacgtg acgttgtcgt ggtcgaagag cgcgaaggtg 90480
tagcagacgg agctcatgaa gagcgagtcg gcggtgctca tggacttgaa ctccgtgtac 90540
gcgattccgt cccagaacag gctctttccg ggcgcgatca gcggggacgc gcggtcggcg 90600
cgcatcagca tggagagcag cgtcacgtag taacggatgt tggcggaaat gtctacgaac 90660
tgcatgccgg gcgaggccac gcgcagggtc gcgcccgagg tagtgagcac ctccaggctg 90720
tccatgagcg tcacgctggg gtgcagctgc gcaaggcgcg ccagctggct ctggtagaag 90780
atggacacgg cgaggctggc cacgctgccg cgtgccatgc gcaggttttg cccgttgaag 90840

gtgagctggc gcagcgagaa cacggagtcg aagtactgga agaaggtgag caggtacttg 90900

agcggcatgg tcgtcagctc ggtatccacc tgcggcgtct gtgcgagcac gattccgttc 90960

ttggccgcgg cgtcggggat gtcgtacatg gcgtccattc tggcgcggga ggcgtcggtg 91020

agcagcgcgc gcacgttgag cagcatgagc aggtcccgcg ctagcatggt cccgtcgacc 91080

agccgggcgc gaaagccgat ctcggcgggg ccggcgatgt tggggtagat caggttcagc 91140

aggtacgtgt tgtcgaagct cagcgagggg aaggagatgg gagacttcgc cgggaggccg 91200

gtggggtagc gcacgtagcc gccgcagatg cgcgcgtgcg cctcaaagct ggtcacgcga 91260

gtcttcagca ggttgcgggt gaagggcggc acgtccttga aggactgcgt gcagatcacg 91320

gggttggcgg tgtcggtcag cttgaggttg gtgggcttaa gctccgcgaa gttggggccc 91380

agcagcacgg ggacgaggtg cgagttggcg gcgctgtcga gcaggaagtt gatgccgaac 91440

tgcttcacgg cgacctcggt ttcctcgtcg ctggcgagct tctccgcgtc ctcgaggaag 91500

agcgcgtcca gcgggtgcac gtacgtgcgg ttgacgtcgt agctgggctt gaagtccgaa 91560

cacagcgtgg ggagcacggt ggagacgagc tggaacatgt attccgcgcc ctccacatgg 91620

tgcaaggcca tgtgcacgtt tggggccgtc atttatttag tattaaatga cggccgtacc 91680

ggtaaccgat attcctggag actacgggcc gacgtccttt tcggaggaca actacccgct 91740

gaacaagcac tacgagctca ccaaaggcca gctctcgatc ctgcgcacgg tcaacgacaa 91800

gctgctcgcg cgcaccgtgc agcactcgga cggggagagc gatgagagcg aaagcgagga 91860

ggacgacatc tccagtccgc tgccgccgga cgaggaggag ccggactcgt gcgtggcccg 91920

ggtcatgccg cgggacgcgg acctggcggc gccaaaaaag gccgacggct acatcattgc 91980

cgccgagcag cagcgccagc agcgcataaa cattctggta tccgatcgag aggccgtcgt 92040

ggagcgggag ccggttcaga cgtcgttcgc gcgcgtctcg gctatcccga tccacgggga 92100

cggcgcgcgc cgcaccaccg cctccttctc cgcgaccacg ccgtcgctgg gcgccgtgtt 92160

cgacgacgcc aagcgcgtgc ggctgctgga ggaggaggtc aaggagctcc gcagaaagtg 92220

cgcgacctct caggataacg gaaacctgga gaacttcacc aaggtgctgt tcggcaaggc 92280

gccgcgcgcg agcgagctga acaagcgcgt ggtcatcgtg aactacgcca cgctgaacaa 92340

cgtgacgctg tccatggagg acctcgagaa gtgctccgac gaggaagtgg accgcatgta 92400

ctcggtcatc cggcgctaca acgagacgcg gaagaagaag atcctggtca cgaacgtggt 92460

catcatcggg atcaccgtgc tcgagcacgt gctggtgaag cttggcttct cggaggtgcg 92520

cgggctcagc gccgacctct cgtcggagct catcgacgtg gagatcggcg aggactgcga 92580

gcacatcgcg gagcgcctgg ggttcgggaa cagcccggtg ctaaacgtgg cgctcttcgt 92640

ggtaaagctg ttcgtgcgga agctgaacct gatctgatca acacatgccg ccgtcgaggt 92700

ccatggcgtt catgaggttg gaggcgcggc ggcgcgcgcc ggtggaagcg gtggaggcgc 92760

tcgaggtcgt ggagcaggga gtgttgctgg aggaggcgcg gcggcgggag ctagaagcgg 92820

aactcgaggt tccgctggtg gtgctgcggc gactcgtgcc gctcgtgccg ctcctgctag 92880

tgccagtgcc agtgccgctg cggcgtgaag taccggtgcc ggacctgccg ctggagcttt 92940

tcttgcggcc gccgttaacg ctgtcgatgc cgagcaggtc ctcgcacacc tcgccgacgg 93000

ttccctgcac gtccaacttg ccgttcttga caaccccgta cacgatcttg ccgcagttgg 93060

acacagcctg gatggtggtc tcgtcgctgt caaaggcgtt cattccgccg cacccgccgt 93120

cgttgtttct tcgagaaggc gcgccgctgc ggcgactcct ggtgctgccg ctggaccgag 93180

ttccggagga cctggagccc gtggaccggc tgccggtcga cctggtgccg gtagtgcgct 93240

ttctggacga agaggaggag gcgcttccgc ggcgggtgga cgaactagcc tccagcgcac 93300

cggcgccgcc cacacaatcc acgtcggcgg cggcgcctcc gcgaatgacc tgctcgttgt 93360

tgagctgcgt caggagagat cgcagctgcg gcgcgatctt ctgcaaggtg ctcacgtagt 93420

cgtcgtagct gctctgcggg cgctgcgcca ttttttcgga cgccatttat tacgcggaat 93480

atctacgacg acgcagcact gaatcggttt ctcgcgacgg gagattccgc ggtcggcgcc 93540

ggtgcggggt tgtcaccggg cgacgaggta accagcgcgt ggaaggcgcg cacctggtcg 93600

tccgtcatct tgtcctcgaa cgaggacgcg cccgggggga gcaggtcctt gttgcgcgga 93660

acggcgggcg ccgagacgca cgaccggcgg tacatcatga tgacgatgta gcacacgatc 93720

gagatgacga tcacggtcag cagcgcgtcg aggagcccca tttattacct gtatatgccc 93780

gcgtttaccg ggcggtgagc tcaatgtcgg tgttgtttag ccgggcgtac gggacgctgc 93840

cggagcactt cctgtacatg ctgaacacga acagcccgag cagcagcacg gcgcccacta 93900

tgaagcaggt tacgcacagc gcgcgccaca cgtagtcggt gacgttgttg gtgttcttgc 93960

tgaaatccac gaaggcgaag acgcaggcgg ccgtcagcag cagcacgccg catatcagca 94020

ctccggagta gtaagagctc aaggtctcga atatgtccat ttatctgagg agaaatttaa 94080

attactgaat ggacgaagtg gaatagaaac cacgagaaca cgacggactg cagcacgaag 94140

atggtgctca gcttcgtctt catgggcatg cagaagttcg cggccagcgc catacagaag 94200

atgaacacga gcaccgccgg gtcgtagtcg gacaccattt acactacgct aaaaggcata 94260

tctcggcgcg cgacgtccac gagcaccagc acgcggacgc ccgcgggcgc gccggcggcg 94320

accgcggcga gctgcccggc cgtggggttc accagcagca gtgcgcgcgc ggttcgcggg 94380

acggggtctt cgtaggccat ggtcggcgtg gacccgggac gcagcggccg cccctgtctg 94440

tcgaagaggc cctcgggaaa cgaggtgccc ggaacggcca cgacgacggt gtcgctatct 94500

agaaacattt atggtcttgg tttccacgga tcgcctcgag tagaccgcca cgaagtagaa 94560

gatgacgccc gccgcgagcg ccgccaccag gaagggcggc acggcgggca ggttcgcgga 94620

cgcgttgtcg cgcacgccgg ggtccgggtc tgcgtagccc gcgcccacgg ccttgccgca 94680

gtcggcgatc atgtgcgcgc gcgagttctg catgaccagg ctgtccacgt cgatgcggca 94740

gcccacgtag cggcaccgcg agcgctgctc gtcctggctg aagaagagcc acttgcggtc 94800

gcgcgactgg tccgtgcact cgtgcgcgcg acagacgcgc gggcccaggt acttcccgag 94860

cgtggtgccc gcgacacacg cgcactccgg cgcggcgcgg tgcgcgtcgc agtagcgccg 94920

cagcgcggag tcgccgaagg cgaaggaggc gggccgcgcc acgcgcacga actccgagca 94980

gaagcgcgcg tccatgtgct tggcgcagag cgccgcgtag gtgtccagcg ccgcgtagcg 95040

gcccgtgcgc agccaggcca tgcactcggg cgcgtcaggc tccaccgcgc agcggctggc 95100

cataacgccg tcgcagtgcg cggtcttgta cccgttcgcg aacacggacg ggcacccggg 95160

ctccggattt gtgcagcagc gcgccatggc ggcgtccgtg ggcggcgccg aggcgccgat 95220

ctcgaacgcg cacatggtgc cctggcgcag gtacggcttc gcgatctcgg gaacgtagtc 95280

tgcgcgcagc agcgagcccg ggcggaagaa gagcgagtcg cagggcgggc ctcgcacgag 95340

ccgcgcgcgg ctcgccagct ctggcgagag gaagcgcccg cactgcccgg ggtccatggt 95400

cggcagcaga cagaaccgcg gccgtacggt cttcagcttc gggtcggaga aggtttctga 95460

ttcttccgcg aaggcgaagg tgtccgtggc gctcgtgtgt gtgacgcgca gcgcgtactc 95520

gcctggcgtc ggcgtgtcga gcacctccac cttggatacg gtgtccccca tttgaagacg 95580

ctatttacgc cgctgcctac tcggcgaaga ataggtcctc cgacttggcg cccgcgtaca 95640

ccgggcaggc gggcgcggcg gagcgagtgc gcacgatacc gcggccagtg aggcggaagg 95700

cgtagatggc gaacagcagg ccgagcacga tgtacatgaa agtggtggcg cccacagacc 95760

cggtcacgtg cgtcacgatg atggtgacga tggacatgat cgtgcacacg atggccatgc 95820

cggtgttgtt ggccgcgtag ggtgcatga tctgcatggc cgcacagtat ccgatgacca 95880

ggcacggcag cgggaggata agtgaggcaa tacctatcat tactagagcg agcacggggg 95940
tggacgtcaa ggccaataca aaaatcacaa tacctgttag tatgcggata tcctcgtact 96000
ggaggacgct gtaaggcgcg atattccctc cgggcactgg cctggggtta gccgggacta 96060
ggggggagtc ggcagtgccg gggtccttgg ggagaaaggc attctgctcc tccgggctga 96120
agagctcggc gtcctgaacg ccgccggcgg tgaactcgtc gttatagtaa ctaaagtagc 96180
tttccattta tatgttgaaa aatgtttgga ggcgtacagg tggacgacaa actctacgcg 96240
tacctaaaaa aactcgccgg acgcgggcgg ccgctgtgtc tgttccgcga caacggcgag 96300
ttcgtcgaag tcttcgcggg gtccgcgttc cgctttgtgc tgcccgtggg cctcttcgcg 96360
gacctgcgcg tgcgcacgcg cggcgtggcc ttcccgaaac tgcgcgactc cgcgcgcatg 96420
cgcggcgtgc gggtggacgc gcacacgctg ccctcgctgt accccaacca gcgcatcgtg 96480
gtggacgagg tgctcgcggc ccgcgaccag ttgctggccg cgggccgcgc cgtgtacgtg 96540
acgctgcatc tggcttgcgg cttcgggaag acgctgaccg cgtgccacct catcgccacg 96600
cacggccgcc gcgcggtggt gtgcgtgccc aaccgcatgc tggtgccgca gtggcgcgcg 96660
gccgtggcgg agctgcgggt gcccttcgcg gtctcctgtg acggcgcggc ctcgctgctg 96720
cgctcgggcg agctcgaccg cgccatggtg gccatcgtgg tcagccggca cttcgccaac 96780
gacgacttct gccgcgcggt gagccggcag tttgacgtgc tcgtgctcga cgagtcgcac 96840
acatacaacc tcatgaacaa caccgcggtc tcgcgcttct tgaccaagta cccgccgccc 96900
atgtgcttct tcctgaccgc gacgccgcgc acggccaacc gcatctactg caaccgcgtg 96960
gtgaacgtgt ccgtggtcag ccgcctcacc aaggtagtgc gcgtggtgga cgccttcttc 97020
gagccgtaca ccacgcccaa gatccgcacg ctcgagcgca gcctcgaggg accccagaac 97080
aagtaccacg tcttcaccga gaagatcctc ggcgaggacg tgcaccgcaa caagctcatc 97140
gtggacaccg tggtcgcggc catggccgcg ggcgaggcgc ggcgcgtgct cgtgctcacc 97200
aagctgcgcg aacacatggt cgggctgcac gccgcgctct gcgagcgcct cggtgcggag 97260
acggtctttc tcggcgacgc caagaacagg aagacgcccg aggtcacgcg cgcactgcgc 97320
gacaaggacc gcttcgtgct cgtgtccacg gtcttcttct cgggcacggg cctggacctg 97380
cccaacctgg acgcgctcgc ggtggccgcg gccgtgctca accgcatggt catggagcag 97440
atgatcggac gcgtgtgtcg cgagtcgcac gccaacacgc gcacgctgtt cgtgttcccg 97500
gactcctccg tgcgcgcgat ccgcgacacc gtgtctgcgt ttgcgcagcg gctcgtggcg 97560

ctggcggtgg acgggctggg cttcgtccgc gagcgcgccg ccgccggcgc gaagaacgag 97620
ccggcgctgt acagcgccat cagcgggcga gatctcgcag cggtgtaagc gcggacccgc 97680
acgccgcgca cgagagcgtg ctggagcagg cgagtcccag cgacagtgtg gacagcctgt 97740
ccacgtcctt gatgctcacc agccgcgagt tgcacgacga gcacacgggg tcgctactat 97800
catcgaccac cgtggtgacg cggcggcgtc tgcgcttttt gtttccagcg gcgacatcga 97860
ccacgcctcc cttagagccc cccttcgccc ccgccttagc tttcaccgcg ctcatctttt 97920
atttatcata aaaacacgtc tgcgtacgcg ttcgcgcaca cgtcccgcaa atccgcgcgc 97980
gcgccgcagc gcgtgaagcg cgcggcgtcc gcctccgcga tccgcgcgca cggcagcggc 98040
gcgcccttct cgtccgccat cacgcgcgca gagatcccgg tggcccccag cgcgtacgac 98100
accaccacgt cgccgacgca gcggtacacg ttgccggagc cggcgaggcg gtcgaacgcg 98160
gcgccctcct ggcgcagctt gtcgaatatg cgaggaacga ggatgttaaa aatgagaacg 98220
aaatagcaga tcagcaaaaa cagcgagatc atgacctccg agagcgattt atataccttg 98280
aaagagctaa tacgacttcg ggactcgctg cacctcgcca ccggcgccgc cgtcgagcgc 98340
tacaacgcgc tcgtggagtg ggccgcgcgc acgtactgga cggtcgcggt gctgccctcc 98400
gcgccgtgcg cctccatcga gaagtactac tgcgtgtgca aacccgactg cgcgctcgag 98460
cccggcgagt actccgtgag ccggctgcac ttcggactca cgcacgcctg ggtgcgcggc 98520
gccgccttca actcggccag cggcgccgag gtcgagccgc cagaggaggt gcgtagggcc 98580
tgcgaggcgc tcgacgccgc cttcgcggac ctcaccttcg tgcgcttctc ggtcttcggc 98640
cgcgagtgga cggtcgacga cgccgtcaca gaccactcct cgcgcgacga ggtgctcgcc 98700
gcgtgcgccg cctccggcgt gcgcgtcgcg cgcacgctgc gtgtgcgcgt gcgggcggga 98760
gagtccttcg cgcgcgcaga cttcgacgcg gtgcacgccg cgctgcgcgc ggagggcgac 98820
gtcgctcgcg gcaccgcggt ctgtctcgcg ctgcgcgggt catcgcgccg ctggatagcg 98880
gaccgcgcgc ctcgatgctt catgcgcgtg cgccgcgtgg agctcgagcc cgtggacgcg 98940
cggcaccact gcccggtgct gatctccgcg cgcggcgacc gggtgctctg ccgcggcgtg 99000
gggcacctcg cggacgcgcg cgcgcgcgag ggcgtcttcg tggccgtgcg caggtacccg 99060
gagtgtctgg tgctctgcga cgaggcggcc gccggcgcgg cggagtgctc gcgcgaggag 99120
gcgctgcggc tgctggtgcg ccgcttcggg cgcgacttcg ccgtcagcga ggagggctac 99180
gtcttccgcg tgcaggacat ggacttgcgc ggcgtgtccg cgcgactggg gctcgcgccc 99240

tgcgcgagcc tggaggagct gcgccgagcg gtggagcgcg accgcgcgct gatgaggcgg 99300
ctgcgcgcgg agggcgccgt gcgcctcgcg tgcgagtgcg tgggataccc gcgccagaac 99360
gcggtggagc tcataaataa tatgcgcttt caaataacgg aagaaggcgc ggtggcgaac 99420
tttgagctgg cgaacgcgag ctgtctcggc aacccgaccg cggagtccat cttcgcgagc 99480
ttcgcgcagt tcgtgccggt cttcaacgtg ctctcggcga tcgcgcgcgc gcagccatga 99540
tcgtggcggc cttcgacctt ggcacgcgca accccgcgcg caccgtgctg gaggtgctcg 99600
acggcacggt gcgcgtggtg gacgtggcca agctggactg gagccgcgac tgggagaagc 99660
gcgtgcaccg cgacgtgacc gccttccccg cgaacgtggt gctcgtggag cgccagtgca 99720
agatgtcgcc tttttctaag ttcatatact tcatacgcgg gctgctctac gacgggcggc 99780
gccgcacgcg cgtgctcgcg gtgccgccgg ccatgaccgg cagcacctac cggcagcgca 99840
agcgccgctc ggtgcgcacc ttcctcgcgc tcgcggagag cttcggcatc ctggacgccg 99900
tgcccgcgcg gaagaagctc gacgacgtcg cggacagctt caacatggcc atcaattacg 99960
tgctccgaac aaactgaaat acgactggaa cgaataagtc atgctggcgc tgttcgagtt 100020
cctgcggtcc gtggaggact gctaccggcg caccatcttc aacttccaca tcgcgcacag 100080
cgccgaggcg ggcgatgtct acggcgtgct gcgcgaccgc attttggcgg ccacgcgctt 100140
cgaggaggta gcgccgccag ggctcgcgga cgcgctggcc aaggtggtct actgcgacat 100200
aagcaccacc aagcacctgg tcaaccacgc ggccttcgcg gcgcgcgcgc ggccggcgcg 100260
gcgcggaggc agcctcgcgc agttcttcga cgtgcacgtg ggcgaggacg cggagagccg 100320
ccgcaccgcg gagatcttcg accgcgagcg ctcctcgctg gtctcgtacg tgaagaccac 100380
ggccaagcgc tgcaagatcg actacggcga gatcaagcgc accatccacg gcgggcggca 100440
gacctacttc tcggggcggc gctcggacga cttcctgagc accaccgtgc gcgcggaccc 100500
gagcaagccc tggatcaagt ccatctccaa gcagctgcgc gtggacatcc tgcaccacgc 100560
gatctgcacg cgcggcaaga gctccatcct gcagaccatc gagatcgtgc tcacgaaccg 100620
cacctgcgtg aagatattca aggactcgac catgcacata atcctctcca aggacgaccg 100680
cgagcgcggg ctcgcggacc tcgcggacaa gctcttcggg acctacgcga ccaccttccg 100740
cgtcatcgcg gccatcaccg gcaacgcctg cttcgcggcg gtggcggacg cggccgcgcg 100800
cgtggtcgcg ctcccggacg cggacgcgaa gctggcggcg gtgcgcgggc tcgcggagtg 100860
ctacggcgtg cgcaacttca aaatcggcat gttcaacctc accttcacgg gcgccatcga 100920

gcacacggtc ttcccctcgc tgatccccgc ggagagcaag atcaagttct tcaagggcaa 100980

gaagcttaac atcgtcgcgg tgcgctccac cgaggagggc cgcgagtgcg tggagcaggc 101040

gcaggcgctg ctcgcggcca tgcgcgagcg ctccgcgcgg ctcgcggccg cggacgtggc 101100

caccgcgagc gtggacttcc tcaaggagct gctggggcca tagtgaaata atactgattt 101160

cttaaatatg gagcaggcgc tcggatacaa gtttttgttg cccgacccca aggacgacgt 101220

ctactaccgc ccgctccact tccagtatga gtcatacgcc aacttcatca agcaccggct 101280

taaggacatc ctcacggtgc ggcgcacgct gctcaccttc aagaacggca ccgagtccat 101340

cgtgctcgag atcgacgacg tgaagatctc ggcgccggag ttctcgccca tcgtggccag 101400

catcaagggc cacagctacg aggcgctggt caccttcacg gtgaacatct accggcacgt 101460

gatgaccaag gacggcctca ccgttaccaa gatcaacagc tacgagggca ccgactcgca 101520

cctcgtcaag ctcccgctgc tcatcggcta cgggaacaag aacgcgctgg acccctccaa 101580

gttcgtggtc ccgaacgcca tcggcggcgt cttcatcaac aagcagtcca tcgagaagct 101640

cggcatcaac atgatcgaga agatcaccac ctggcccaag ttccgcgccg tgaaggccaa 101700

ctccttcacg ctctccttct cctcgatctc gcccgtgcac gtgatgcccg cgcggtaccg 101760

acactacaag atcctgctcg acgtgaacca gcccgacaac ttcgtgatct cctccgcgaa 101820

gaccttcatc accgtgaacg tgatcgtgat ggtgcagttc ctcgcggacg tcacgctcga 101880

gttcgtggcg cgcaacctct gcttcgacat gccgcccgag gccgcgcacc tggccaccgc 101940

gctcgtggag agcgcgaaga ccgtgcccgc gggcgcggac gtggccgagt acgtgaacgc 102000

gctcatcgcg gccgagcacg cgaagcagaa gtcgacgctg tccaaggagg agttccgcta 102060

cgagatgctc agcaacttcc tcccgcacat gcaggacagc gccaaccagc tcaagggcct 102120

gtacctgctc tcgctggtgc gcaagatggt cttctgcgtg ttcttcccga accggtaccc 102180

ggaccgcgac tcgctggtct gccaccgcgt gtacacctac gggcgctact tcgaggcgct 102240

ggccatggac gagctcgaga cctacatcgg gaacatccgc aacgacatcc tcgcgaacca 102300

caagaaccgc ggcacctgca ccgtgaacat ccacgtgctg accacgcccg gcttcaacca 102360

cgccttcgcg gcgctgctca gcggcaagtt ccgcaagtcc gacggcagct tccgcacgca 102420

cccgcactac tcctggatgc agagcatctc catcccgcgc agcgtgggct tctaccccga 102480

gcaggtcaag atctcgaaga tgttcaaggt gcgcatgtac caccccagcc agtacggctt 102540

cttctgcgcc tcggacgtgc ccgagcgcgg gccgcaggtc gggctcatct cgcagctctc 102600

cgtgctcgcc tccatctcga acatccgcac cgcggacttc gtcgagctca ccaagcgcgt 102660

ctgcgactac gtgcgctcct accccgcgcg cgacatcagc tacttcgaga ccgggttcgc 102720

ggtcaccgtc gagaacgcgc tcgtggcctc gctgaacccc gcgatcgtgg acgcgttcgt 102780

gctcgacctg cgccggcgca agcggctcgg cttcttcggg aaccgcgaga tcggcgtcgc 102840

gctcgtgcgc gaccgcatga acgaggtgcg catcaacttc ggcgcgggcc ggctcatccg 102900

cccgctgctc gtggtcgaga acggcgtgct cgtcatggac gcggaggcgg agcggctcga 102960

gcgcgacctc gccgcgatga ccttctcgga cgtgctgcgc gagttcccgc acgtgatcga 103020

gatcgtggac gtggagcagt tcagcttcag caacgtctgc gactccgtgc agcgcttccg 103080

cacgctgccg cccgaggagc gcgcgctctt cgacttctgc gacttcccgg ccgagttccg 103140

cgacgggtac gtggcctcct cgctcgtggg catcaaccac aactccgcgc cgcgcgccat 103200

cctcggctgc gcgcaggcca agcaggccat ctcctgcctg agcgcggacc tgcgcaacaa 103260

ggtcgacaac ggcatccacc tcatgttcgc ggagcggccc atcgtggtca gcaaggcgct 103320

ggagacctcc aagatcgcgg acaactgctt cgggcaccac gtcaccatcg cgctcatgtc 103380

cttccgcggc atgaaccagg aggacggcat catcctgaag cggcagttcg cggagcgcgg 103440

cgggctcgac atcctcacct gcaagaagta ccaggtcgag atcccgctcg agaacttcaa 103500

caaccgcgag cgcgtgcgct ccgcggcgta ctccaagatc gacgtcaacg gcgtggtgcg 103560

cctgaacgcc ttcctcgagc agggcgacgc catcgcgcgg aacgtgtcct cgcgcacgct 103620

cgacgacgac ttcgtcgccg acaaccagat cagcttcgac atcgcggagc ggtactcgga 103680

catctacgcc gcgcgcgtgg agcgcgtgca ggccgacctc accgacaagg tcaaggtgcg 103740

cgcgctgacc gtgcgcgagc gccgcgccat cctcggggac aagttcacca cgcgcaccag 103800

ccagaagggc acggtcgcgt acgtggccga cgagactgag ctgccctacg acgagaacgg 103860

gatcgcgccg gacgtgatca tcaactcgac ctccatcttc tcgcggaaga cgctctccat 103920

gctcatggag gtcatcctca ccacggccta cgggcacaag cccttcgccg aggacggctc 103980

caaccgcccg atctgcttcc ccagcaccaa cgagaccgac ttcgagacct acatcgagtt 104040

cgcgcggcgc tgctacgcgc tctcgcaccc cgaggccgcc gcggacgacc ccgagttcga 104100

gcaccgcgtc ttctgcgagc gcgtgctctt cgaccccgag accgacgagc ccttcgcggc 104160

gcgcgtcttc ttcgggccgc tgtactacct gcgtctgcgg cacctcacgc tggacaaggc 104220

cacggtgcgc tgccgcgggc gcaagaccaa gctcatccgg caggccaacg agggccgccg 104280

ccgcggcggc ggcatcaaga tcggcgagat ggagcgcgac tgcatgatct cgcacggcgc 104340

ggccttcacc gtcgccgaga tcctgcgcga ctccgaggag gacgcgcagg aggtgctcgt 104400

ctgcgagaac tgcggcgaca tcgcggcgcg gctcaacggc acgcacgtct gcatccgctg 104460

ctccaagatg agcctctcgc cggtgctcac gcgcatggac tccacgcacg tgagcaaggt 104520

cttcaccacg cagatgaacg cgcgcggcat aaagatccgc gtggagttcg agaagcagga 104580

cccctgcttc tacgggactc cgaaacggtt cagcctcgcg cccgacgagt cgctgttctc 104640

gccggaggac tgaacccgcc gtcgcgaccg cgtcgcgacg actagcttat cgttcgactg 104700

atgcgaaacg cgcggcggcg ccgcgactta gcttatctcg actgatgcga acgcgcgacc 104760

tctcgcgact ttctagcttc tcagactgat gctaccatat cgcggcgtgc tggccccacc 104820

accagggctt ctcgccgtgg ctgacgcggg gctggctgcg acgcgcgctg cagtagctgc 104880

gcgcgcccca gtcgccgcgc acgtgcgccg ggggcaggct cccgtccagc gcgtgccgcg 104940

tcacctcggc gccgggccgg cggcacgtgt gcacgtccgt cttgttggag acgagcaccg 105000

cgtactgccg catggtctct atgtgatgct ccaagtgctt gcccgccatc cggttggact 105060

cgcagcacgt ttttgcttcg gctaaggttt tttctagagg ggatagtagc ttatccacgc 105120

gctcgggcag gacgcacgcg gagccgtcga accctacttt gaacggggtc accttgatgt 105180

tcccgtcgta gcggtcccac agcatcctga ggtaggttgt accgtcgggg tctgggtctg 105240

tccacactct aagcttttcg ctacagcggc cgtcgtacgt aagacggtct ctacgctcgt 105300

agtagtttct gcttatgttg ttggggtctc catgctcgta gtagtataaa tcgtacgcgc 105360

ctggcttttt taagtcgttt tcgtcgttgc tgacgtgtat cacgtcggga taataggata 105420

tcctaactgc actacaatct atagtatttg gtctagtaag ctgttcgaga tcaccttgtt 105480

catcatgatc tactgatttg tacacggcac cgtcgtgttc cgacggacgt atgaatatgt 105540

ccatggtaaa cgatgtaccc actttggaaa acgtatccca tgcagtaaag catagtccgt 105600

ccattataaa ctcaggaaca ctcataacaa atcgaaatct gtgaagtttt tcgaacacca 105660

cttttacatg gtctttgtca cgaacatcat tgccgtttac ttcagacatg aattgaagga 105720

acgctaaaga gtttcttgtt tcttcatgaa tctttccatt atacgtccat ccagtttcta 105780

gaattctata tatgcttttt gcatcgaccc cgtaccacca gtacatggga actccgaaat 105840

atatagctgg gtttgagtac caatgggcaa gagtgcccat tgcgtttaaa aagtcttgac 105900

aaaaaaatgc agtttttctg tcgataactt gacttggact acgttcgtgg acatcgtaca 105960

tgtccataat tggttcattg gtaacggtta catgacccgt cattatcttt ttaacaatca 106020

taagatacag tttgcctaaa gtcgaaatat gtataacgtt aatttttaca tgttctccta 106080

acgtaattgc gtttttactt agccattcgt cgtctacaaa aatcttacga tacataggat 106140

ttctctctac gtatcttcta aagtatagat ttaccggtct accggcgaca ttagcgccat 106200

ctatagcagg agcaagctgt atgtatcgtc gtataatgtc tcgtataagc tttctgtctt 106260

ctctgggaat acacgacacg gaacttagag actggtgcca gtgtctttca accaaagact 106320

tgaacctagc aaccaacgcg ttgtcactct ccatttataa ttaaataatt atcccaactt 106380

cgtatgttaa tccttattac cagatagcac cgctccttcc tctccaccac gtactatcta 106440

aaggatacct gtaagggtaa tgtctggata acgggcgtgt gagccaagac gtgttatggt 106500

gtcttcccca ccaacggtcc acttctctaa ctaccggagt gctagacgtt gtcgatccca 106560

ctactgttgt ttctccatta cctgtaatct ttgaagcgca acaagtgtta gtctttgcca 106620

aatcttctaa cggcttaatt aggtcgttaa gtctgtcaat atccatgcac tgaggtgtat 106680

cgttaccagt ttccccaact ttgggaggaa cttcctgttt agtgtccaaa taacccataa 106740

acctgtctct aagcattctt ctgtacgtgc ttttgtcatt gtctatgtct cccaaaaacc 106800

tgtgatttac gcatccgttg tacgtaagtc tgtcccttcg ctctcgctcg ttatttccta 106860

cttcttctat tgtactgtaa tgttgatagt ccaagtaata gccactgttt tcatgatttc 106920

ttgtaaatat aatcggtgtt ttattattga catcgtgttg cctactgtac gtatcttcca 106980

tggatctagg aacttgtcta gaaaattgag gactagaaat acgccttcca aatcctggat 107040

gataaaccaa acgcaatgca ctacagtcga catcgtcact gtctctagtt atcccatcaa 107100

caactccttc tttgtgatgc tccactgttt taaagttaac tcctctgtat atgttgagtg 107160

ttaaaaaaat atccacattg aacgcagttc catattttgt tattttatcc cacgacgtat 107220

aacacaaacc atccataatg aattccggaa ccgatactac aaaattagta tcgaacttat 107280

caaacgaaat gtttactcgc ggttgtgctt cgtatttttt gtcatacaca tctgtcatgt 107340

attgtacaaa atctatagct cctctagcat cgggcatgct aacgtctgga aaatcatttt 107400

ttaactgttc tagtacgaac tgttgatttt ggtcatctct ccaccagtac attggcaatc 107460

ctatcacaag agacttattt ttataataat ttgctacaga agctatatgc cacatgaaat 107520

tatagcaaaa ataatccatc tgtatgttta aaaccggttt actagtctgc tgagagtagc 107580

tatccatgat agtgtttccc tcgccaatac ttcctgacat tattctgtaa acaaccatta 107640

acaaaaatct tcctaccgtt gttatgttgt caaaagttct tatgttttga gcactttcga 107700
gtaaccatat gttattttca aatatacttt tgtaaactgg atttctgtcc acataactct 107760
ttaaaaatag atttactgga aggccgcttg ggttatctcc ctgtataggc ggcgcttgct 107820
ttatgtattc gcgtaacaaa tctcttacaa cttttctagt tcttctaggt atacatgacc 107880
tattattcaa aggctttgtc cagttagcgt ttataaacgt tgtaaagtca ctgactagct 107940
tctccattta taattaaata attacagacg gcaacacagc ggttatctaa tatctgctgt 108000
atcctgtctg tacatctatt tttctgttga gatcaagaag agctttacgt agactctcca 108060
agtgtctttc tagtctgtct aaccggttac ctgtttctct gcagcaatca gttatagttt 108120
tgtaactgtc taacaagctt acgaggcgct cttccacact ttctttagtt ggagctccag 108180
ccgcgtacac tccgttggtt gaattgcctg tatcatcagg ctgagtcaat aggttttctc 108240
cgtcattttc atccatattg agtccaacga acacaaacga gtaagtgttc ctctatttaa 108300
agtattgatt ttagaaaaag gcaagcctcg ctgccctgat tcggcggcaa acacgggttg 108360
aacacgcgga agtcgctcgc ggccgtgaag atctcgtccg cgcacgcctc cacgctcgcg 108420
aagcgcccag gcgagacgcc gtcgtgcgag cggaacccga actccgaggc cgccaccgcc 108480
gcgcccttga agagcacgca gcgccacttc ttgcgcacgt cgaaggcctc gtcgttgggg 108540
tcgaacacgc gccggtccac gcgcgggccg cccgccgtgc gcgcgaactc cagcgccgcg 108600
ttcgccgcgt tgaactcgcg gatgttgtcg tagttctcgt agacggccca gagctgcagc 108660
gccacgaaca tggcggccgc cgccgcgagg gccacgcaga gcgcggacac cgcgtccatc 108720
ttttatgtgc agaattattc gttggcgcgg agctcgcgca gctccgcggc gcgcagccgc 108780
gcgaaggctg ctttgagcgc gcgcagcagc tcctcggtgt ccgcgcgcag catgtcgaag 108840
cggtggtagc tgtccaggcg cgcgcggcag ccgaagaagc gtgcgacgca cgcggtgacg 108900
atgtcgttca cgtagagcac gcccgaggcc gtgcagtaca cggagcgcgg ctcgcgcggg 108960
tccggcggca cgtccacggc gaccgcgtgc gcggccacgt cctcgagcac cttgcgctcg 109020
agcacggcga ggaagtcgcg cagctggcgg cggttgtcca gccaggcgta ggtggtcgcg 109080
aagagcgtga gccgcccgcg cggcgcgatc gcggtgtagg gcgcgtaccc gcggaactcc 109140
cgggggtgca cgaccttgac gttctcgtgc tcgcgacgga aggcctcggt gtcgagcagc 109200
gccgcgagcg cgtccacgag cttgtcggag acctccacgc ccgcgccgaa ggcgatgagc 109260
tcgatcttct gctcgctctt ggggcggaag tcgtggaagg tgtgcagcag catctcgcgg 109320

agctgcggcg gcttctcgac ggcctcgagc gcgtcgccgc ggacgaggaa gtagtcgagg 109380

tcgtgcagcg agacgtgctg cccggcggcg ctctgcacaa acttgaggaa gacgcagagg 109440

cccgcgcggc gctcgagcac gtcctcgacg tgcgcgtgga atacgtgccg cgagggcatg 109500

gcctcgatcg cggagagcca ctcctcgttg acgcaggtgg tggtgttctc cagcaccacg 109560

ccctgcgtga gcgcgggcca ctgcaggtgg aaggcgaact cgtgcttgat gagcgaggcc 109620

acggccgggt ccaggtccac ggccagcgcg gcctcgccga cgaggggagc gtccgccatc 109680

acgcggagga cgcctggccc atctcctttt tcgccttttt attcaggatc attattcttt 109740

cgttgacgag gtccatgagc atcttgatgg cggcggccgc ggccgccgcg tcgccgccgc 109800

acatctgcgc gatgcgcgtg agcatgtgca gcagcgcggc ctcgtttagg tcctcctcca 109860

tttagaggcc gtgagggcgc gcgtcgtcgc gacgagggga cgcctcccgc ggcagcgtgg 109920

tgcgcacggc gaaggcgagc agcgcgccgg cgcactgcgt gagcacacac tccgcgagcg 109980

cgacgaggag ctcggagggc gcgagcacca tttagaggcg cgcgcgggtt taattgccgc 110040

cgtcagagtc ggcatcatcg cccttgtcgc cgccgtcctt gcagtcgccc ttggtctcgg 110100

cgtcgacgat gtcggcgagc cgcgtcttca tgtgcgagaa ctgcgcgagc aggatgccgg 110160

ggtcgagaca gcgcttgacg acgctctcgt cggcgaagtc gtagcagatg cgctcctggt 110220

tctggcagaa caccgagtct tcgatgatca acaccctcct ggtcccggcc gaccgcatga 110280

tggccatggc ccggatgagc ctcttcttcg atccgcgtat ggacatggac cggagcacgt 110340

tctccacgtc ggagtcggag acgttgcagc agcagaggtg cgtgatgctg gcgcgcccgt 110400

tgacggggat gtgcttgtag gtctggcaga gcagcaccag cgacacgttg atgtgccgcc 110460

cgtagttcat gaggcccaag agtgtgggcg accgcgtctg cgtgtcgccc atatcgtcga 110520

gaatgatgag gaacttctgc ttcttcgtct gcgcgtgccg ctcgatcttg cgcttggcga 110580

ccgagaggtt gtactcgagc tcctcgtgcg tggtgacctt gtggatgtgg tccggccaca 110640

cgaagccgtc gtaggcggcg ttgtagacgg gcgtgaagag caggatgtgc ttgaagcggc 110700

gcacgagcgt gcggaagagc gagagcaggt aggcggtctt gccggagccg gagccgccga 110760

cgagcgccat cctgaagggc gcctcgatga gactctcccg cttgaagcgc acctcctgca 110820

cgacatccat cgtatattta ctgtcactaa attaccggct ccgagaaata tagaaattag 110880

agcctcctag agcacaccga ggcgcatcgg caagatggca cataacacgt tcgaaaacga 110940

tagcgagtcg gcagctaaca accagtacgt ggcgtcagtc aagcgccaga aaatgattcg 111000

gcgatacatt aagatgttct tccggttcgt tacggcgata gctatcattg tcctggctat 111060
tctagttgtg attctgtcgc tatctctaga cgaatgtctg cacagagaac accctcatga 111120
ctattcacat gtacaaaatt caacatgcga cggcattact ttaggtggtg aaaagtgtct 111180
cagacttaat ttgccagcaa cgtgggaaga tgctaataga caatgtggta atcttgggtt 111240
ttacctacca tctactggcc ttgaaaagaa atttccttgg cttgtgacct atctcgacgg 111300
aacttgggga aacactcaga actccgtatt tggaccaacc ggtgacttgc agaatgtcat 111360
aggaccgaaa gaatacaaat atttttgtgt gtccgattag atgattataa tctaataaat 111420
gggttgctgt aaggtcccta accgccagtc tataaggact ttgaaaaagg cgtcctgtcc 111480
ggtcgccagt ctcgtcacca ttctctccct agtcaccagc ctcggtgcga tagtcagata 111540
caccaatttt tttctaaaag aagcatgtga cgaaggatgg atgcccataa aaaacatatg 111600
cattttaaac acgcactttg aagccaccaa tgacgatgcc cacaggatat gtgaaaacct 111660
agacggaaag ccgccggcca tccctaaccc tactctgtta aagggtgtca cagttctcac 111720
cggcgaaaag aaattttgga tgacccatca cgaagactat actactgtgt ttgagcatat 111780
agacgatagg acgactccta aaaacacaga ctatgacagt aaaaaacaca cttgtttgat 111840
gagcgaggac ggattgatac accataactg catgatgaac gtgactgtgg tatgcatgaa 111900
ggagatgcac ggataactga aaatatactg tttgaacgca aagacgccat gtcgcgactt 111960
caaatactga cctcatttgg acaaatcttc gcacccgacg aagctcggct gcgcgagatc 112020
gcgcgtgatt tgggaatatg caccataaaa cgcgcattcg gcgacatgct gtacggcttt 112080
atagacttcg acccggtgcc cctgacccaa gtaaacatgc tcatgtccaa ctgctacttc 112140
gcggtcaacg gcaacctgct tccgtgcacg gaggacttcc ggctcagact cccggcaacg 112200
gagatctctg cggcctacct gacgagaacg ggacggacga tcctgtgcgg caaagacttc 112260
aacatagtag cgccgtcggg gttcaagccg tccatgcggc tgcgcgacct cagtcacgtg 112320
tctgcgcttg tagagatcct ggaagtctac gacgagtccg gggagtacca attcgtgctc 112380
ggccccagcg cgcagttcat gctgcggctg atggagaagg agaacgtctg tctgttcggc 112440
agcgggtggt gcatagtgga cctgcgcaag ctggacgtac ccatataatc agcatccttg 112500
tttttatcct gtctttttat cagtttttta gctagttaaa acataaatag taaagctaaa 112560
aagaggagtt ctggagtctt gcaacaacca ggatgaaggc ggtgttgttg ctggcgttac 112620
tgggagcgtt caccaacgca gcgcctttgt tagaaagcca gcgttctaac agtgaggaaa 112680

aagcaaattt ctgctcgacg cataatgatg aagtgtacgc caggttcagg cttcagatgc 112740
gcgtgggtgt acgacacagt ccgctctaca ctcccagcaa catgtgcatg ctggacatag 112800
aagactccgt tgaggacata gaagagtcca cagaaaaaga atacgcgtct acggccacgg 112860
gtgaggcggc cggagtgaac gtgtcagtgg cactagtggg agaaggcgtg agcataccgt 112920
ttagttacat aggccttgga ttcaacccgt cgcttgaaga tagctacctg tacgtcaacg 112980
tctcgtcacg agctccttgg gttaaacaga cttcggacct atccgcgaac ggcggctggg 113040
gtatcaaaca ggttctagaa aaagagttac tggccatcca aatagggtgc gacaaccaaa 113100
aatttcccga agaacccaca actacacccc cctcacctgt cactacaacg ctttcctcaa 113160
caactccaga tctgaatgaa gaaaacacag aaaatacgcc gacgaccacc ggcgccagtg 113220
tagacagaaa gcgcaatcca gctgacattg acttctcgct gctcgtggac ccccgatgcg 113280
tgacctctgt agacctgcac gtcgagctca gggacgcgtg catagactac aaacaagagt 113340
cgccgttgtc gctgaagggg aaatatggag acggcgaact agtaaaaaag gagattaaag 113400
acgtgggaaa gaatcacaat atgtgcagtc ttaacctcaa ccctggcaat tgagctgttt 113460
ttattcggca atataatagg tgattattga acattaaaca aaacttatcc cacaacgccg 113520
caacaatgga agtgctggtg atcatctcta ttatcgtcgc cgtaatatgc ttgaccggag 113580
cggtaatgta cctccttatt gaactcggct tagccgccga gcgcgctaac aaacgcgcgc 113640
gcgtgaagaa aaatatgcgc aaattagcca ctcaattggg aaatggatct gtcgactccg 113700
gcataggcat aggcccgtgc ataatgtcgc gcaccatgga ctctggaccc agtcgctggg 113760
acagcgacag tgaggatgac ggggacagcc tgtccacgac gtccaccagc ggagggggga 113820
ctctcacccg agtgtgggtt gggagcgccg ggcctatgta cgaaaacttc tgcgggaacg 113880
gcacccgcca ctcccccacc aacgaccctg gctaccactc gcgggagact ctctgcagcg 113940
gacctccccg tcaggcgccg gcgctaccgc ccaccccgaa gcccgacgag gtaacggtgg 114000
acgtggggcc cggtcccgac gaccaacacg gtccgtacga ggaacctgat cccattcccc 114060
cgcaggaacc cgagccgccg gtgcagattg aggtaaccat caacgggccc ggtggagaag 114120
gcgaggcgga aggagaattt ttctacgacg agtagccgcc aaaactgaat aactatcggg 114180
cttcgtaaac gcgcagacat gccgctgttc cggaagctca tggtttcgcg ctccctggtc 114240
aaggaatgtc tgactctgga cttccggcag ggcgagcgtc tccctacgcg atgcttcctc 114300
ccggtgcccg cggggacgac attccacaga gtctgcgaca cctcgccgct gacggacgaa 114360

gtctcccggc acgtgcagga gcccgtcatg ggcaccggac gggtccagta ctactacttc 114420
gagagcggcc agggcatgat cggcgacaac gcgggcatgg cgcgcatgct cgtgtgcacg 114480
cggtcggcgt acaacggcgg cgacgtcgtc gtgcggtcca cgcggagcag agcagacaag 114540
accgtggtcg cgccctgcca gggcatggcg ctgctgctga gccccttctg cgccttcgac 114600
atcacgccgg tcgagagcgg ctccgcgata ttcgcggagg tcatcgtcac cgcgcccagc 114660
atggactacg tcgaggcggt caccggcacg ggcgaggcgg ccgtgcggat attcaactcg 114720
caccacccgc tctggccgcg acacggctcg aacgtctgct tcgcgctgcg gttgctgcga 114780
gacgtgcgca cgggcgagcg cgtggtcgag cagatgttca tggacgggcg ctggcacacc 114840
gtgctgagga cgtcctgcgg caacaaggtc tgcgtgcccg ccgacctcgt gggccagacg 114900
aacctcgagg aggtgccctt ctgcgacgtg acgcccgaga tcatgcgccg cgcgctggcg 114960
atcgacccgc cgtacgaggc cgtggcgcac ccgcaccgct gcgtgtacgg cgccatggac 115020
gtccggtgcg cgaacgagta cctcgtgtac tgcaccttca agacggagcc gacacggcgc 115080
agcacgtcct cgccgggccc ggacagcccg ctgtcgcccg cgactccgtc gacctcgcgg 115140
gccgcggccg cgcgcgtccc cacgacgccg caggaagtgg cctcgccgac cacgaggctc 115200
ctggagacct gtctgcgcga cgccctcgac ggactctgac ccgaaggacc caccgtccac 115260
tcacattcca ctgccagaca actcaagctt tttctgcatc tacctcgcta ataattgaat 115320
tgttatagga caaacaggcg cactcgagca caatggcgtg ttttatcgaa ttgttagact 115380
ccatcttcaa ccgacgccac cgtaatttcg ggccggagga catgtacagg ccctctgacg 115440
ccccgccccc caaatcgcac acgcctcgca ctccccgcac cccgcggacc cagtgtcccg 115500
gacacccgcg gcgacaaagc tcctctccca tctacggcgc ttatgtggac tccctgccga 115560
ggaacagaaa gcggttccag aatcaacaca gttgtcccgg agattacgag cggtgtcaac 115620
tctcggacac catcagcctg gatgcgacgc tactcacggt tacagtgacg tccatctcca 115680
gcatatccag ctctagtagc tcagactcta tctctctggg gcagtgcaga ctgtccatgg 115740
tgtccgcaac atcaacctcc acctccacaa ccttctcctc ctcggaatga gcgccacact 115800
tatttttgta taatagtttg tattgaacct tagagacatc cacaaatagt taggaagcat 115860
gagtagttca agtagcgaga ccacccctaa gcccaagccc atccctgctc ctcccatgac 115920
tcaggaggag tttaacaaag aagtgaagaa acgaaaagaa cagaaaaagg aaaaatctag 115980
aaccgttgaa cgtgagtcag aaaccgtaac tgtatcttcc gacggatcag agataaaaaa 116040

gacttacgag cgcgagtctg agagaacaac cgaaacagaa aagaacaaca cgtcaaccga 116100
tgataataag cagaacaccc ctgtagagaa accagaggaa accaagcctg cttctactcc 116160
tgaaggtgtg aagccagccg agactcctgc cccgactact gacccccaac ctactacaca 116220
accacccgca gaatcaaacc ctggaagtca acccgcacct gcttcagaac caaccccgc 116280
acctgagcct gcaccggaac ccactcagcc tgcatcagta actcaacccg ctccaacacc 116340
agagccaagt ccagccccta agcctactcc ggcttctgaa ccaaccccag catctgagcc 116400
tacttctgct ccagaaccta caccatccgc agaaccaact cctcaaccaa ctgtagaaac 116460
accaccatct gctccagcac caactcccga ggcccaacca cccgccaaca gcaatcccac 116520
tactgaaact accactggta ccagcacctc ctaagtgagt acgtaagcat ttcggagtaa 116580
cgtcgtagca agcgctagtc cgccgcgagc ggttctcgca agttttttcg ggtaaaaagc 116640
gtacaccgtc gccttgtcgc ggcggtgtac gcttttttca cgcccttttt gcaaaattta 116700
aattgtaccc gcgccggctc taggaaagat ggcgtgcctc agagtgttcc tggcggtgct 116760
cgcgctgtgc gggagcgtgc actcggcgca atggatcggc gagcgcgact tctgcacggc 116820
ccacgcgcag gacgtcttcg cgcggctgca ggtgtggatg cgcattgacc ggaacgtgac 116880
cgccgcggac aacagctcgg cctgcgcgct ggcgatagag acgccgccga gcaacttcga 116940
cgcggacgtc tacgtcgccg cggccggcat aaacgtcagc gtgtccgcga tcaactgcgg 117000
cttcttcaac atgcgccagg tagagacgac gtacaacacg gcacgccggc agatgtacgt 117060
gtacatggac tcctgggacc cttgggtgat cgacgacccc cagccgctct tcagccagga 117120
gtacgaaaac gaaacgcttc cgtacctgct ggaggttctg gagctagcga ggctgtacat 117180
tcgcgtgggc tgcacggtgc ccggagagca gcccttcgag gtgatcccgg ggatcgacta 117240
cccgcacacc ggcatggagt ttctccagca cgttctacgg ccgaaccgcc ggttcgctcc 117300
ggcgaagctg cacatggacc tcgaggtgga ccaccggtgc gtgagcgccg tccacgtgaa 117360
ggcgttcctg caggacgcct gtagcgcccg caaggcgcgg acgccactct acttcgcggg 117420
gcatggctgc aaccatccag atcgccggcc aaaaaaccca gtaccgcgcc ctcagcacgt 117480
atcgtcgccg atctccagga agtgcagcat gcagacggcg cgctaagggc gctcaccgcg 117540
ctgacggcgg ccgtggtgtg cgcgatcgcc atcgcgctcg agcgcggggc ggaggccgac 117600
gccgtggacc ttatccttat aaaattttca atgatatgct agtttttatg cgaccttcct 117660
tagaaaattc ggaattcaaa aatgaaataa aacggcgttt agcacgcata ttattaatac 117720

cgaccaccat ggcaggcgtc cgcagctgcc agaagaaagt cccttctact gcgggctcca 117780

tgtcatttca acggggcaac cggagcatcc agccggcgat gtccgaggcg ttgcagaatg 117840

atttcagcta caacccgcga ccgcctccgc cgagcgcaga agagattgac ttcttctgcg 117900

tggacatgcg caaagtactg atggaaattg aggccaagcc cagcagctcc aagtaccccg 117960

atttcatcca cccggttgac agcagcccgc cgtgcacgcc ggcgcgcaag cgcaacggct 118020

tcggccgcaa ggcactgaac aaaacccagg tgccgcagca ggccaagcgt gacggctact 118080

cccgctaatg cagtccacac acttcacaca ctacatcagc actcaagctt ataatcacta 118140

cacaatgaat cagcccacca cgtgcgaagc acacacataa aatcacccac ctgtcctgat 118200

cgttcccaat actcccaatc accgtgcttt acacgcacgt taatcaccct ttccttcctt 118260

catgcgttcc tgatcgttcc tcctccttaa tcacacacac accccgtaat tttgtacttt 118320

tgtactttaa tttgtacact ttacacactg actttgtact gcctttgtac tttatttttg 118380

tactgaaatt ggacgatact tatctttgta ttcacatcca agttttgcaa attccacagc 118440

cggtagcgaa aagtgaaatc gtaccgtttt aggcttcgat ccccctcccg cgcgaagact 118500

cgccagcatg gactctcgta ggctcgctct tgccgtcgcc ttcggaggcg tcctcgccag 118560

catgacacag cgccgccgcc tggcttctct catcgccagc atcggccaac ggctgatggg 118620

cggcgacggc atgcgtcgcg tcgccgttcg gttgatcgac cagctcatgg ccggaccccc 118680

ggacatcaac gacgaggcct tccagcgcga gatccgcgtg ggcgagctct tccaggcgct 118740

ccaccgcgtg gtcgagcagg cacgccgaga gaagtacttc gaggtctgcg gcgccggcaa 118800

cgacgccgac gcgcccgtcg tcgagatgga caccgcggcc gcaccccccgc agccccagcc 118860

cgcgcccctc gtggtcacgc cgcagaacgc gttcatgttc gtgccgcaag gcagccacgt 118920

gcacgtggac gagagcgtgg acccgttctt cggcatgagc ccctccatct tcgggcgcga 118980

cctccccccct cagccgcccg aggagctgct gagcgactac gacccgctca tgagccaggc 119040

cggcgagccg ccgagcccgc ggtcgccctg cgaggccgac ctctggtgct tcgagacgct 119100

cggcgacagc gacagcgatt gagaccgcac cacaccctac ctcacccacc ccacactcca 119160

cctcacctca ccctaacacc cgacacccaa cacttcaacc ggacaatgaa ggagtcccac 119220

atttcactga aggacgcgga tgaagccgca ctccccccaca tgaaggattg gcaacggtca 119280

aacatttcac ctgcaatgaa ggacgatgcg cggtcgcatt ggcctgcgac cgacatcgca 119340

cacatgaagg acacaattgg tttgttaatc cggacaatga aggacaaatt gtttttgtta 119400

atcaggacaa ttagaacaca atcaaatttt tgtacgatca taaaatcgat atttgatgca 119460

catatattag taagtatatt agactaaatt ctccggggag gcaagcagtt ggatacggcg 119520

gggcggggca cgacgtgcac ggagagttcg ggcgggtccc ccttcccccc acccccacgg 119580

caccacgatg cgcctaatct tagcgctcgt ggcctgcttg ttggcggcgc cgatgccgtt 119640

atcgggtcgt tcgacaagca ctccaaacac gtccccctcc gcactcggct cgacgagttc 119700

ggaaccaagc tcggaagacg ctgtggcttc gagcacaacg acaagcacac tcacaagcac 119760

tacaagcaca ctcactatgt ccacaagtgt ggacaccact actacctcgg gcgctacaac 119820

gtccgcaaac agcactcctg cagcgagtgt gagctcctcc acacccgcaa ctaccgaggc 119880

atcgacggca ccaacgacgc cgtcgacgcc gacgacagtg aaggtaacga agggcaagga 119940

agacacgaag gcgtctgcct acctcgtttt actaatcacg ttcatggtca tgaccacgct 120000

cgtgatggtc gtggtcgtgg tcgtggtcgt gtacaaacag ggactctgta actgctgctg 120060

taagtttccc ctgctgcaaa gagctcaagg actacctcga cgaggaggag agcgccgggc 120120

tgtacgacgc cttgacgtgg agccactcag actccggcct ccggctcgtc gtgcgcgcgg 120180

accccagatg atgaggatcg gataagatcg gcgtgttttt cccgcccgtc gcgaacatta 120240

tgcctctaaa tgccgagaat taactgaaat tcaaacacgc tttgggactc aactccgtga 120300

cccacactca accatggctg gcttcctagg cgcattcaga ggcgtgtgct ccgacttatg 120360

gcagtcgctc cgtggacacg gacaccactc ttccagctgc ccgcgacgac gcgccaacag 120420

catggacgac cgcgaccggc gccgacaccg ccaccgcgag atccccaaca gctcggcgtc 120480

gctgaacagc gacccgatgc cgcaacgcag tgcgggtgcg cgccggcact acgactgccg 120540

cccctcggaa aagagcagac actcctccga caagcaccac tcggcggacc gacaccactc 120600

ggcggaccga caccaatcgg cggacaggga cagacaccgt cgcagtcgca agaactacga 120660

ctcgcacccg tcgcgcagga accgcaacta cgagcgggcg gactaccaga gacacccctc 120720

acaaacccac ccagacgccc ccgcgcagac ctcgacgctc aaggtgacct ccctaagcac 120780

cagctgcagc accctgtccc aacatcacta cgagaccccc gaccacatct acgacatccc 120840

ggaagacagt cgcggggcgt cggctccccc tcgcgcggac ctcgcgctcc ccccgctcgc 120900

catgcccaaa tccaagccgc gtcgcacgcg cccggcgtcc atgaacgact gcctgatgaa 120960

gcactgcggc gccggcagac ccaacctcca agacgacata tgcacactat gtactgatat 121020

agagacacag ctgagcgcac tagagaagtc tctggagtca gagctcaact tctatcgtcg 121080

ctacatacaa gacactaaga cattgctcgc cacgcgagca gcaaacatcg gcagcaaagc 121140

tctgatctac accgacgact acaacggcag tggcgacgtc ggcgaaaagg agcactgctc 121200

ggaggagtgc tgcaaagtgg aggaagttct gtgagaaagt gcgtttttct gtaatgtgaa 121260

ataagatagc cttatgtgtg cacagacatg gcgaacaggc tcgtgttttt cgaccccgag 121320

accctagccg aggccgacgg catccccggc tatggggtgt tcgagcccgg caagaagaaa 121380

tgcatcttca caaagatccg caccagcgtc gcactcgcgt gccggtacgc cgtctcggac 121440

ggcggcctca tcgacgagtt cgtcatggct acatacggga ccagacgcgc gtgccggctc 121500

gtccggcacc tgacgatagg cgcggagggc gtgatgaccc ggcccgccag caactgcgcg 121560

ccgcacatgg tgctcatctg cctcagaggc gtggccgccg tgtccagcga ggacatgggc 121620

ttcggtcgct gcatcatgga gcgcggcacc atgttcatgg tcaagtccgc gcacagcgcc 121680

gtcgtctgcg gcaaccccgc ctgcgagctg ctcgtcctct tctacgacta cttcaccccc 121740

atcccccggc cgctctccgg agacgaggtg ctgttcaccc gcgacctcgc gcacgtggac 121800

tacgcccccg agtcggcggt cgtcttcaag atggattaca acctcgagac cgacgtggcc 121860

acgctgtttg tcggggggta catattccgc gccaagggcc tgatgatgga gacgcgcgaa 121920

caagtgggcg acgagtgcga ctgctgccgc cacagctcgc cggtgctcgt catggatcgc 121980

gagaagatga tgtcgtcgct gcgcatgatc cccagcatcg tgcccggcca gcgggagatt 122040

tggcttcgcg agcgcggctg ggccgtcctc gagacggacg cccgcggaca ctgcgagccc 122100

ggcgtcctga ggctggcgct cgccggcctg cggctgttcg caggatgcct gcgctccgtc 122160

gtggggcggc gcgagctgtc gctgttctgc tacggcgtcg ctcccaagtt cggcggggag 122220

ttcgaggacg cgccgcgccc catggagatc gacggttagt tgtttttatc cctgtacata 122280

cgccgcaaac tgaaacttta gggcaccgcg taatagtgca cgaacgccca gtggaccgct 122340

tccgcagcca tggaaaacaa cgacggcaac gaacgcaaca acgaacaccc gcacgttcga 122400

gaattcaagg aggcgtccct gtacgggttt ctggtggcgg ccgcggacgt gaccgtcgaa 122460

gacgtgcacc ggtaccttca gttcggcgcg gacgtgaact acaggggcgc gtacctgtgc 122520

acgccgctgc acgcgtacct gcagtccggc tgcgaaaagc gcctagacgt cgtggacgcg 122580

ctgctggacg ccggcgcaga catcaacgcc aaggagatct gcggtctcac gcccgtgcac 122640

ctgtacgcga gctacgcgga cgtggacgta gagttcatgc gcgggctcat cgagcgcggc 122700

gcgagcgtgt gcggcgagag ctcggtcacg ggctgcctgt actcgtacct gtacacacac 122760

agcgtggacg gcggcgcgcg cctggacgtg gtcgagctgc tcgtgcaggc gggcgcggac 122820
gtgaacgtcc gcggcgaggc gcgcaagacg ccgctgcacg tgcactgcgc gggcttcgag 122880
gtggattcgg acatcgtgga tctgctgctg cgcgcgggcg cggaccccga ggcgctcgac 122940
gaacacgggc tcacgcccgc ggacgtgctc gtgaagtccg tgggcgccaa cgtggagacg 123000
ctgcggctct tcctcgacgc gggcgtgagc gtggccacgt cgcgcgacgc gcgcggacgc 123060
acgccgctgc accaccacgc agactccttc cgggcgagtg cgggcatcgt gcgcgaactg 123120
ctcgccgccg gctgcgacgc ggcggccgcc gacgacctcg gaaacacgcc cctgcacagc 123180
ctcgccacct tctgctcgtg ccggcgctcg gtgctcgacc agctcatcgc cggcggcgcg 123240
gacatcaacg cccgcaacca ctacggccac acctgtctgt actacgcgtc catctacaac 123300
ccctccgtct gctcgcggct catcgccgcg ggtgcggacg tgaccgcgcg cacgccggac 123360
ggacgcacgc cgctctcggg catgatcatg cgcaagcaca cgcgcgccgt gcgcgccgcc 123420
ctggcgacgc ggcctcccgc ggacgccgtc gccgcgtcgc tggacgtcgc agtacagccc 123480
gagcccacgg acgccactcg cgcgtgcgtg cggtacgtgg tgctctgcgg cggcacgctc 123540
tcggcgcgcg tgcggtcgcg acacgcggac ttcgtgcgag agtgcgaaag cgaggtggtc 123600
gtgctcagaa ccaccgtggt ggggctgccc ggcacctcgc tgctggacat cgtgcgtgcg 123660
gcgcagccgc cgccggtact gctctccccg cgcgtgcacc acgtgctgca gaagctgtgc 123720
gtgtacgcgg agttggtgga cgcgcggctg cgcgagatgc ggcacaagac caacctcgtg 123780
gacgcggtgt cgcggctcgt gtgtccgtgc gcgctgccgc cggaggtggt gcgcggcatc 123840
ctcgtgcacg tgccgataga cagcctgcgg cacacgttga ccctcggcgt ggcgcaggcc 123900
tcgcgtttcc ttccctcgca taaatgaaat attatttttt gtggtagacc ggatctcccc 123960
gatggacccc gccggacaac gactgcgcgc gccagggccg tggcgcctga acccgccgac 124020
cgcggccgcg ctggaaagcg cgctgctgcg gcccgcggcg tcggcgggcg ccgaccgctg 124080
cgcgaacgcg cacgtggacc gccgcaacat gggcgtcggc gagggccgcg aggtgcccgc 124140
ggacgtcgag gggctcatga ccgagatcca cctgcggtac ggaatgacgc gcgtccaccg 124200
gaacgttcac ttcgtgcagt tctggcacgg cgagcacgtg cgccggcgcc ccgcgcgaca 124260
cgtgttcacg gtctggatct gcctcagcgg cgaggtgcgc atctacgcag agtgctgcca 124320
ggcggggcac ggcttcgtgc tctgccgaca gatggcggca gggtacatgt tcgtgaccga 124380
acccacggac tcggtcacgg tctcggtgcc gcaccgactg cgcaactcgc ggtcgccggt 124440

gtggctggcg gcggtcttcg ccacgcggca cttcgagccg ctgccgccgc ccatgtacgc 124500

cgtgcccggg cacgtggtgc tcgcgcgcag cgcctccatg ctctgcgact gctggccgtc 124560

ggacccgcgg cgccgcaacg tgatcttcta catgcggctg tcgggcgcga tggtgcgcgt 124620

ggtcgtgccg ggcgcggagc tcgagatcga gtgcacctcg gggttccggc cggaccactt 124680

ctccatcaac gacgagtgcg tgtgctgcga gcgtccgcac gtcgcgcgaa ccgcggtgtg 124740

gacgctggcg gagatttgcc gcggcgccac ggtggtgctc gcgccgccac tgccccgcga 124800

ccgcgccgcg gggctgctcg cggagatccg cctggcctcg ctgcgatggg tgcgcgtgcg 124860

tgcggtccgc agcggcagag aaagcgtggg cccgttcccc tcggtggtgt gggcggcggt 124920

cttctccgcc gttcggctct tcctggacgg aaccgtgcct gccttcccgg cgtgtgtgga 124980

gaatggacgc gcggcgtacg gcatggtgta cgtgcccccg gaggagccgc ggatggacgg 125040

gctctgtgtg ttcccgacgc ccgccgagcc ggcggcgctc ttcgtccgcg gagaccaggt 125100

gcttgaggcc ggcgcggccg ccgccataat cgcggccgct gagaagcgcg tccaggccgc 125160

caatgggtct cctgctgccg cggaggagga cataggtgcg gcggccgatg ccgccgcaga 125220

gagcgtggag caggaccagc gcgtcgagtt cgaccttggg cctgggcctg accccagcca 125280

agaagcgccc gcggacgcgc agcgtgccga ttcggacgac gacaccggct ccgagaccga 125340

gaccggcgac gagagtgtgg gcggcgagga tgacagcgac tcctcctcct cttactcggt 125400

gatgtcggac gacgaaaacg acagcggcga cgagggctgg ggcgactcta gcgactccgg 125460

catcgaggac gacgacggcg gtgtcgccag gccgccgagg aagaagagga ggaagagcgc 125520

gacgtcctcg gcgcagcggc ccagatgctc ggagactgac cggtggtgaa aacataaaaa 125580

taaactgttc aacacttgta ctccgggcac caacactact atccataccc accctccctc 125640

cacacactac aatggcaaac agagaagaga ttgacgcctc cgccgtcatg gctgcctacc 125700

tcgcgagaga gtacgcggcg gctgtagaag aacagctgac gccgcgcgag cgcgatgcgc 125760

tcgaagccct tcgcgtttcc ggcgaggagg tccggtcgcc gctgctgcaa gaactctcga 125820

acgcgggcga gcaccgcgcc aaccccgaaa actcgcacat ccccgccgcc ctcgtctccg 125880

cgcttctcga agccccccacc tcccccggcc gcatggtcac tgcgattgag ctctgcgcgc 125940

agatgggccg ggtatggacg cgcggccgcc agctcgtcga attcatgcgg gtcgtgtacg 126000

tgctcctaga ccgtctgccg cccacggccg acgaggacct cagcacctgg ctgcaggccg 126060

tcgcgcgcgt gcacggcacg cggcgccgcc tgcaccgcgt tctcggcgtc ggggccgtca 126120

tggcaggcgt cggtatgctg ctgctcggcg tgcgcgtgtt gcggcgcaca taacttttta 126180
tctcggctca aactgaaata cgacattgga ctacgaaacc tatgattttg ctcacggccg 126240
cgcgagatag gataataaat aacctttgag caactaacat ggccgatgag agagaggccg 126300
acggcgcgct gttccggtac ctggagagcg aggaccgtcc ggacgtggag cacatgcgcc 126360
ggctgctgga cgagggtgcg gacgtgaact acgcgggccc gcgcgggtac gcgccgctgc 126420
acatgctcat gcgcggcaac ccgctagacc ccgacgcggt gcgactgctg ctcgccgcgg 126480
gcgcggacgt gaacgcgaca tcgctctgcg ggttcacgcc gctgcactcc tacatgtgct 126540
tcgggaccgt gacgccagac acgctgcgtg cgctcatgcg ccacggcgcg agcgtcagcg 126600
acctcgagcg caacatcaac gcgctgatcg agtacttcaa ccgcgacggc tgcatgggcg 126660
gcgcggaggc gaccgtgatc gcactgctgg tggagcacgg cgcgcacgtg aacgccaaag 126720
acgaccttgg acgaacgccg ctgcacatct acctgtccgg cttcttcgtg tcggcaccgg 126780
tggcgctcgc gctgatcgcg ctcggcgcga acccgaacgc cacggacgcg tacgggcgca 126840
cgccactgca cgccttcctg cgctcccgcg acgtggaccc cgctgtgctg aagacgctca 126900
tcgccgcggg cgcagacccg ctcgcgcgcg acatcatccg gcgcacggcg ctgcactacc 126960
actgcgagtc cttcaagacg cgcgctagtg ttatagagac gctggtggcc gccggctgcg 127020
accccgcgag cacagacctg ctcgacaaca cggcgctgca cagcatggcc atgggcagct 127080
cctgccgcgc ctcgctgatc cgcccgctgc tggccgcggg cgtgtccgtg aacgcgcgca 127140
acgcgcggct gcagacgccg ctgcacctcg cggccgtgtt caacccgccg gcctgcgcgc 127200
ggctgctggc cgcgggcgcg gaccccgcgc tcgcggacct ggacgagaca acgccgctgc 127260
tgagcatggt gcggcacaac tgcgcacgcg cgctgcgcac ggcgctgccc ttggcgccgg 127320
acgcgctggt ggccggcgcg gtgaaccgcg tgaacgcgcg cacgccgagc gcggccacgc 127380
gagagtgcgt gatggcgctg gcgctgcgcg gcgcgctgga cctgctgagc gcggagagcg 127440
ttgccaccca cgcggccgcg atccgcgcct gcgaggcgga ggtcgcgctg ctgcggagca 127500
cgcgcctggg cgcgccgccg acgacgctct tcgcgctgct gacaggacga ccgaacacgc 127560
tggtttccgc gaaggcggcg cgacgcgcga tggcggacgt gtgtgtctac cgcgcggcgc 127620
tggccgcgcg cgtggagcgc gtgcgccgca agtcctcgct ggtcgagcgc ctcaccgcca 127680
tggtgtgtcc gtgcgctctg ccgccagagc tagtgacgcg catcctcgcg ctcctgaccg 127740
tggaggaact cgcttgcgca atgcgcaaat aataatgaac tataactagg cttattagag 127800

gcactatttg tgcagagtcg ttagttatag ttagtgtact taccattgga atgtcgaaga 127860

acaaaattct ggtgtgtttg gtaattattc ttacttatac attatacaca gatgcgtatt 127920

gtgttgagta tgaggaaagt gaggaagata aacaacagtg cggtagtagt agtaattttc 127980

ctgcgagttt accgcacatg cttagagaac tcagggcagc gttcggaaag gtaaaaactt 128040

tcttccagat gaaagaccaa ctgaacagta tgctactcac acagtcgctc ctcgacgact 128100

tcaaaggcta cctcgggtgt caggcacttt ctgagatgat acagttttac ttggaagagg 128160

tgatgccgca ggcggaaaat cacgggccgg acatcaaaga gcacgttaac tcgctgggag 128220

aaaaactcaa aacgctgcgt cttcgactgc gtcgctgcca ccgcttcctg ccgtgtgaga 128280

acaagagtaa ggccgtggag caagtcaaac gtgtgttcaa catgctgcag gaacgaggtg 128340

tttacaaggc catgagcgag ttcgacatat tcatcaacta catagaatca tacatgacta 128400

ctaaaatgta aaaatgtata caacttttag ttatcgttcg gattctcgta tcgttctgca 128460

tactatgtat ataaaatgta tattaacata gttacagtta cagttacagc tatattttta 128520

tgctcacaag atgctatata attgaaagga aattgttcac tctctgtcag ggcgccatgg 128580

actttctagg cgccgcgctt cacgactacg ttgccgatgc gcccaaggtc tgcgccgagg 128640

aggtgcggcg gctgctggcc gcaggcgcct ctgtggagta cgcgggcgag ttcgggaaga 128700

ccgcgctgca ccagtacatg ggccgttccg gcgcggaccc cgccgtcgtg cgcgcgctgc 128760

tggacgccgg cgcgcgcgtg gacctcccgg agacctgctg cggctgcacg cccgtgcacc 128820

tctgcctcat ggccgccaat atcgacgtgg aggttctccg catgctcgtc cacgagggcc 128880

gcgtcgagga ctgcggccgc gccgagctcg cctccgtggt gctcaaggag ttcgtggtga 128940

accgcgcctt cgacgagaac gtcagcgagc gagtgatgcg cgttcttgtg gccgcgggcg 129000

cggacgtgaa cgccgccagc gtggtcgacc gcacgccgct gcacgtctgc ctcacgggca 129060

tgtccacgca cccgggcacc atcgccgcgc tgctgcgctt cggcgcggac gtgaacgccg 129120

tggacctctg cggcatgtcg ccgctggcgg tgctcgtgcg ctcgcgcgcg gcgaccgcag 129180

agctggtgcg catgctgctc gacgcgggcg cagacgcaca cgctgtcgac agtcgcctgg 129240

actcgctgct gcaccagcac tttcagtccg cgcgcccgcg gccggaggtg gtgcgcgagc 129300

tcatccgcca cggctgctcg ccgcgggcgc ggaaccgaat cggcaacacg ccgctgcacg 129360

aggccgcaaa acactcctcc tgcaaacact cgctggtggg gccgctgctg gctgccggcg 129420

cgagcgtgga cgcgcgaaat aacacgggca ggacgccgct ccacttggcg gtggcgtcca 129480

acccgcgcgc gtgccgccgg ctgatcgcgc ttggggcgga cgtggtcgcg cgcagttacg 129540
cgggcgtcac gccgctggcg cagctgatcg cggacaataa ctccgcgctg gtgaccgcgg 129600
cgctgaacac gcagcccgag ccgcgggccg tggcagagtc gctgcgagcg accacgcccg 129660
tcggcgagac agcgtgctcg cggctctgtg tggcgtacgt ggtggcgcgc gcgccgagcg 129720
aggtcctcgg cgagcccgag cgcgccctgc acgcggcctt cgtggcggag tgcttagcgg 129780
aggtagcggc catacacgcc gtgcgctgcg gcacacctcc ggtctcgctg ctggagatcc 129840
tggtggccgc gcgcccgccg cggagcctgc tctcgcgccg cgcgtggcgg ctggccagcc 129900
ggacgacagt ttaccgcgcg ccgctccgtg cacgcatcgc ggccatgcgc catcgctcgc 129960
gactggtgga gcgcgcgctg cgcacgctgc gcggctgcgt gctcccgcgc gaggtgctgg 130020
agcgcgtgct gcggtgtctg tccacacagg acctgcgggc ctccggactg gccgagtagc 130080
tttttctgag ataagtgaat aaacatggtg ggattcgatc ggcgccgcca acgccacgcc 130140
atggacgccg ccgagatgga ggatctcgac atcaacgcgg agtcggcgct gtacgactac 130200
ttcatcctga acgcggacag agcccgcgtg ggcgaggtgg ttatgcttct cgcacagggc 130260
gcggaaataa actacgcgga cagcttcgac aagacgccgc tgcacctgta cttgcacacg 130320
cgacacccgc gctcggacgt gattctggcg ctgatggagg cgggcgcggt cgtggacacg 130380
ccggagcgct gctgcggcgc gaccgcggcg cacctgtaca tcctcaacgc ggccaaggtc 130440
gacctgtcgg tgctggaggc catgctgacc tggggcgtgc gccagaacga ccagcactcg 130500
gagcgggtgc tctcgagctt gttgcgcgag tacgtggtga cccgcgccta ctcggatcag 130560
accgagccga tcatggactt gctcatcggc atgggcgccg acgtggacat gccggtcggc 130620
gtgagtcgca cggggctgca cgcctgcctt acgggcctga acgcgaaccc gtgcatgatt 130680
cgcgcgctgc ttcggcgcgg cgccagcgtg accgcaaaag acacctacga gatgacgccg 130740
ctggcggtgc tgctgaagtc cgcgagcgcg acgccggagc tcgtgcgcat cctcgtggag 130800
gcaggctccg acgtgagcgc caccgacttc cgcctcaacg gcatgctgca ccagcacgcg 130860
cagtccacgc gcccgcgcgc gagcgtcatg cgcgagctca tccggctggg gtgcagccca 130920
gcggccaaaa acatgtttgg gaacacgccg atgcacatgc tggccatgga aagctcctgc 130980
cgccgctcac tgatcctccc gctgctggag gcagggcttt ccgtgaacga ggagaacccg 131040
cactacggca ccgtgcctct gcacgtggcc tcggggtacg acaacacgca gggctgcctc 131100
aagctcctcc gggatggagg agaccccacc gtcgtgtcgg ccgccggacg cacgccgctc 131160

tcgaacatgc tcgtcaaacg caaccacgtg gcggtcgccg gcgcgctgtc gacgcacccg 131220

agcgcggcag tagtcgtgca ggctctcgag caggctctcg agaacgtgct gaacgccggg 131280

cccagcgagg cctcgcggct cgccgtggcc tttgtggtgg cgcgcgccgg cgcatccgcg 131340

ctaccggagg ccgtgcgccg tctgcacgag ggctttgtcg ccgactgcga gcgcgaagtc 131400

gagctgcttt cccgcaccat gctcggcaca ccggccgtga gcgcgctggt cgtgctggtc 131460

agcaaggagg tctttggcac tgttatctcc tcgcgtgcgc tgcgcgtcgt gcgggaggtc 131520

cgcgtgtacg caaggccgct ccgcgaggcg ctcataaatc tgcgccacaa atgccgctta 131580

gtttccagcc ttaaaaggca ggtgggacct tgctcgctgc ccggcgaact ggtggagcgc 131640

gtgctcgcga ccgtgccact gaccgacttg cgccgctcgt gcggccgccg cgcgcccgag 131700

taactgcccg tcccgttgct acgcgactcg agactgcccg ctgtttttct ttccccgttt 131760

cttcttatta ggagttgttg cccgcctcca tgatcctcgc acgcgccggc gggcaacctc 131820

gcacgcccgc ggcggccgcg ggcgccgccg aggacggcga gcacagtgat cgccggaagc 131880

gcaagcgcaa gacgcccaac tgcgaagacg ccgacaactc cgacgacgag ctagcgcaga 131940

cgccgtgcga ccgcgagtgg ccggactgtc gcgcgagctc gatcacgagc tccgactcgg 132000

tctctctcgg cgacgagatc tacctgcgat acgtggcctc gcaggtggac ttcgcgcaga 132060

cctgggcccc gccagtgcgg ctgctgcgct ccttcgggaa cttctcgaag gaaacgctca 132120

accgcatgtc gcggcgcggg tacgtgaacc gctcctactt ccagatggcg cacgcgcgct 132180

tctcgcccac caacgacgac atgtaccaca tggccacggg cgggtacggc atcgtgttcc 132240

gcttcgaccg ctacgtggtt aagtacgtct tcgagcaccg caacggcatg tccgagatgg 132300

acgcctctac ggagtacacg gtgccgcggt tcctgcgcaa taacctcaag ggcgacgagc 132360

gcgagttcgt ggtctgcgcg ctgcccatgg ggctgaacta ccggctgggc ttcctgcact 132420

cgctgtaccg gcgcgtgctg cacacgctgc tgctgctcat gcgcgtggag gaaggccagc 132480

ggccctcggt ggagatgtcc aagaagccgc tgctgcgctg gttcgaggcg cgcaaggaca 132540

gcgagtcctt cgtgcgcctg atctcgtact tctacccctc ggccgtgcag agcaacgtga 132600

acctgatcaa caacttccac cacctggtgc acttcttcga gcacgagaag cgcgcgcggt 132660

acgtgttcga ccgcggggcc gtgatcgtgt tccctctggc gcgcgggtcc gcggactcga 132720

tctcgccgga ggcggcggcg gcgctgggct tcgcgccgca ctcggagttc ctcaagttcg 132780

tgttcctgca gatcgcgctg ctgtacctga agatctacga gctcccgggc tgcacgaact 132840

tcctgcacgt ggacctgaag cccgacaacg tgctcatctt cgacagtgcg cgcgcgctca 132900
gcgtgaccgc ggccggcgcg actttccgct tcgaggagcc cgtgcgcgcg gcgctgaacg 132960
acttcgactt cgcgcgcgtg gccaccatcg agaaccgcaa gatcgcgggc agcgtccgcg 133020
tgccgcagaa ctggtactac gacttccact tcttcgcgca cacgctgctg cgcgcgtacc 133080
cgcacatcgc cgcggaggac ccgggcttcc acgcgctgct ctcggagctc acggtctcgt 133140
gctcgcgcgg gacctgcgac cgcttccggc tgcgcgtgtc ctcgccgcac cccatcgagc 133200
acctcgcgcg gctggtgcgc cgcgacgtct tctcccgctg gataaatgcc gccgcggacg 133260
cccccgacgc cgccgcactc tcctgagccc acgcccgcgg cgccgggctc gctgtacgac 133320
gtcttcctcg cgcgcttcct gcgccggctg gccgcgcgcg cggcgccggc ctcggccgcc 133380
tgcgccgtgc gcgtgggtgc ggtgcgcggc cgcctgcgga actgcgagct agtggtgctg 133440
aaccgctgcc acgcggacgc ggccggcgcg ctcgcgctag cctccgcggc gctcgccgag 133500
acgctggcgg agctcccgcg cgcggacaag ctcgccgtcg cgctcgagct gggcgtggac 133560
cccgagcacc cggagctgac gccggacccc gcctgcgcag gcgagagcgc actcgcacag 133620
aacatcgaca tccagacgct ggacctgggc gactgcggag accccaaagg ccgccgactg 133680
cgcgtggcgc tggtgaacag cggccacgcg gccgcgaact gcgcgctcgc gcgcgtggcg 133740
accgcgctga cgcgccgcgt gcccgcgagc cggcacggcc tcgcggaggg cggcacgccg 133800
ccgtggacgc tgctgctggc ggtggccgcg gtgacggtgc tcggcgtggt ggcggtttca 133860
ctgctgcggc gcgcgctgcg ggtacgctac cgcttcgcgc ggccggccgc gctgcgcgcg 133920
tagccgcgca aaatgtaaat tataacgccc aacttttaag ggtgaggcgc catgaagttg 133980
ctcgtcggca tactagtagc cgtgtgcttg caccagtatc tgctgaacgc ggacagcaac 134040
acgaaaggat ggtccgaagt gctgaaaggc agcgagtgca agcctaggcc gattgttgtt 134100
cctgtaagcg agacgcaccc agagctgact tctcagcggt tcaacccgcc gtgtgtcacg 134160
ttgatgcgat gcggcgggtg ctgcaacgac gagagcttgg aatgcgtccc cacggaagaa 134220
gtaaacgtga cgatggaact cctgggggcg tcgggctccg gtagtaacgg gatgcaacgt 134280
ctgagcttcg tagagcataa gaaatgcgat tgtagaccac gattcacaac cacgccaccg 134340
acgaccacaa ggccgcccag aagacgccgc tagaactttt tatggaccgc agatccaaac 134400
gatgatgcga tcaggtcatg cggaagaagg cgccacggag caaagtgaaa aaggaccgcc 134460
tagcagtcga gaccctcccg ccgcagccgc ggacacccca cacccgcctt ccacccgcca 134520

gacgccaaca ccgcagccaa caagcatgca cccctcgccg cgcaggctgc tcggcgcgct 134580

cgcgctggtg gcgctgggct tcctcctcgg cgggctcttc cgccccgcgg cgccgccgct 134640

gccggccgcc ctcgtggagg cgggccccgt ccgcgcgaac ggctccgcct cggtgacctg 134700

cctgaccgtc ggcggcgacg ggcggcacat ggcggtggtc gcgcacggcg gcgggacgct 134760

ctcgccggtg tacccgctcg ccgccggcat gcacgcgacc ttcgcctcgc tgcgcaaggg 134820

cgcgctgctg ctgaacgtcg cgaccgtgca catctacgac gtgcgcgcgc tcgggccgga 134880

gttcgagctg acctgcgtcg cggtggcggg cggctacaac gcggcctggg cggccgcgcg 134940

gcccgcggcc gagtggcgcc gccagctggc gcggatgcac cgctcggagc tgtgaccctc 135000

tccctcccgg tctcccatcc gtttttgtaa tcggccttag tagattagac cagcatcccg 135060

cgcccttgtc cgagaacaag taacagtaac cgttacctca ctcgccactc ctcggaataa 135120

tagaacgaga gaacgagaga acgagttaac cgttgctcac tcgctcactc ggtgtgagag 135180

aacaagtaac gttgctcact cgctcactcg gtgtgagaga acaagagaac gagtagctgt 135240

tgctaactca atcacccctc ggagtaagag aacaagagca gtcaactacc cactcagtct 135300

tggatgagag gcagaggacg agttgacgag ttgaacagtt aatcctcact cactcagagc 135360

gagagagcga gagagtggag gacgagttaa caagtcaatc ctcactcaga gtgagagagt 135420

gagagagtgg aggacgagtt aatggttaac agttatcacc actcagagtg agagcggagg 135480

acgagtcaac cactcgctcg cccactccga gttagagagg gaaccagtgc gagttaacgc 135540

gcacacgagc gagagaacgt aaactcgctc gcgcgctcgc tcggctaacc gtcggcctct 135600

cccaaaactc ttcgtaaaac tttcccgatg acagttcacc ctccaaaact ttgtaaaact 135660

aaactgttcg gaggtcggtc tgctgcctct ctaactctcc gtaaaacgtt tgtaaactgt 135720

cggaggtcgg tgacccgctc aaccgtccgc gaaaactttt cgcaggcagt gtctgcctct 135780

ctcggactct ccgcaaacac tttcgcggaa cctcggaggg tggtcgacct ctctccaaac 135840

tcttgcaaaa ctttttcgcg gaaccgttgg aggccagtcc tccctccaaa ctctttgtaa 135900

aatcttttcg aggccagtcc tcctctccaa aacgttccgc aaaatctttg ggaggtcggc 135960

ctctcctctc cagaacgttc cgtaaaactc ttggaggccg cccgcggcac gcgaggcgga 136020

ggatccgagg tgtcgacctc cctcaaaaac tttgtaaaaa ctttttataa aactttccgc 136080

ggaacctcgg agagtaggtc gacctctctc aaaactctta taaaactttt ccgcggaacc 136140

gttggaaggt aggtcgacct ctctcaaaac tcttataaaa cttttccgcg gaaccgttgg 136200

aaggtaggtc gacctctctc aaaactctta taaaactttt ccgcggaacc gttggaggca 136260

ggtcggcctc tcaaactctt tgcgagaact cttcgcgaga actcttcgat aactttagga 136320

ggtcaggtcg acctcccaaa acttttgcga gaactctctg taaaacttta ggaggtcggt 136380

acctccctca aaactttta taaaactttt cgcggaacct ccggagacgg gccgccgccg 136440

cgaccgcggg agcggagagg ccgacctccc gagacgttcc gcgttaccgt cggggtaggc 136500

gtcctctcga gaacgccaaa agacttcgtg caaaaacttt tcggaggggc gcggagggcg 136560

ggtcagctcc cgcgaactcc cgcagaacct tttcgcgcga ccgcgaaggc cggccgcctc 136620

tcccacactc tcaagagctt ttcggaggag aggaagggca ggtcgccccc acctccgacg 136680

ctttgtaaaa acgtttacgc ggaacctcgg aggcaggtcg cctccctcga aaactcctcg 136740

cgaaaccttt aaaaactttt tgcgaaaact tttcggagga tgtcggaggg cgggcggctc 136800

ttccaaacct ccgcagaacc ttttcgcgca accgttggaa gacaggtcgg cctctctcga 136860

aaacttttaa aactttgtaa acgcgttggc gggaccgtcg cgggagagcg gccgcccgcg 136920

gcacgcgaga ggaggaaccg ttggaaggca gtcggcctct cccgaaaact ttttataaaa 136980

acttttccgc ggaaccgttg gaggcaggtc ggcctctctc agagtctaaa aactttttgc 137040

gggactcgga cggcgcggtc acccgaccac ctgactcctg tctcacccgt actacttgga 137100

cttctgtttc cctgactccc gactccctga cctcccgact ccctgactcc cgactccctg 137160

actcccgact ccctgactcc cgactccctg actcccgact ccctgactcc cgactccctg 137220

actcccgact ccctgactcc cgactccctg actcccgact ccctgactcc cgactccctg 137280

actcccgact ccctgactcc cgactccctg actcccgact ccctgactcc cgactccctg 137340

actcccgact ccctgactcc ctgactccag agcgaggtct cgcggctgcg gggtgccgcc 137400

tccgcggagt cgcgttcccg cggacgcccg tcctcgaaag cattcagcag ttccagcctc 137460

tgccgtagct cctcccgcag gaactcctgg tccgcgttcg tcgcggcacc gcggctcagc 137520

cgccgcggga gccggccgcc gcccgcgaag ccgcggatcc 137560

## Claims

1. A pharmaceutical composition comprising a purified and isolated polynucleotide with a sequence consisting of the nucleotides number 122616 to 136025 of SEQ ID 01 (PPVO insert of VVOV 82) for use in the treatment of viral hepatitis, wherein said PPVO insert of VVOV 82 down-modulates the MHC-I cross-presentation.

## Patentansprüche

1. Pharmazeutische Zusammensetzung, umfassend ein gereinigtes und isoliertes Polynukleotid mit einer Sequenz, die aus den Nukleotiden der Nummern 126616 bis 136025 der SEQ ID NO: 1 (PPVO-Insertion des VVOV82) besteht, zur Verwendung in der Behandlung von Virushepatitis, wobei die PPVO-Insertion des VVOV82 die Kreuzpräsentation durch MHC-I heruntermoduliert.

## Revendications

1. Composition pharmaceutique comportant und polynucléotide purifié et isolé avec une séquence constituée de nucléotides allant du numéro 122616 au 136025 de la SEQ ID N°01 (insert PPVO de VVOV 82), destinée à être utilisée dans le traitement de l'hépatite virale, ledit insert PPVO de VVOV 82 modulant à la baisse la présentation croisée MHC-I.

**FIG. 1**

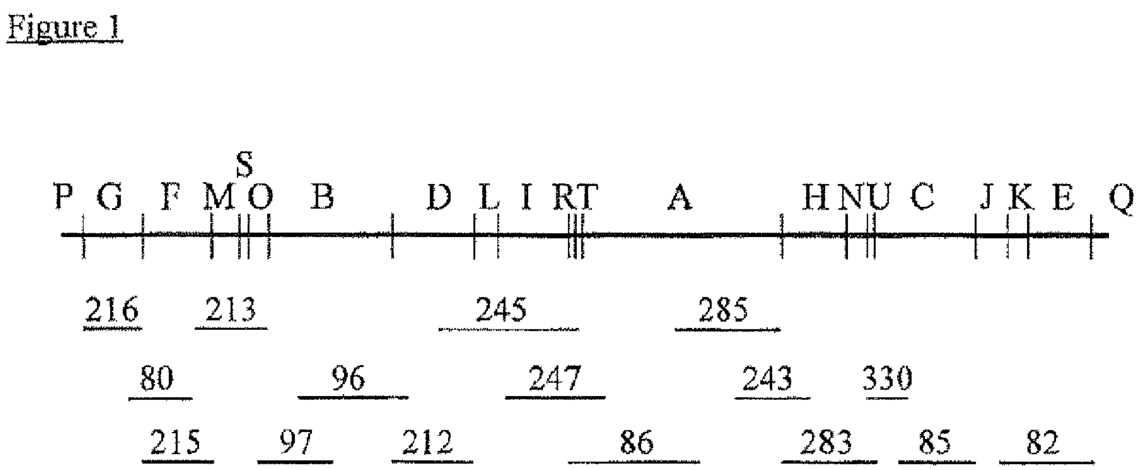
Figure 1
S
P G F M O B D L I RT A H NU C J K E Q
216 213 245 285
80 96 247 243 330
215 97 212 86 283 85 82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 3504940


### Non-patent literature cited in the description


- **CASAL.** *Biotechnol. Genet. Eng. Rev.,* 2001, vol. 18, 73-87
- **ELLIS.** *Curr. Opin. Biotechnol.,* 1996, vol. 7 (6), 646-52
- **ROY.** *Intervirology,* 1996, vol. 39 (1-2), 62-71
- **GAUDIN et al.** *Gen. Virol.,* 1995, vol. 76, 1541-56
- **HUGHSON.** *Curr. Biol.,* 1995, vol. 5 (3), 365-74
- **UHLEN et al.** *Curr. Opin. Biotechnol.,* 1992, vol. 3 (4), 363-369
- *Biomaterials,* 1993, vol. 14 (1), 5-15
- **CLELAND.** *Pharm Biotechnol.,* 1997, vol. 10, 1-43
- **HANES et al.** *Pharm. Biotechnol.,* 1995, vol. 6, 389-412
- **JANES et al.** *Adv. Drug Deliv. Rev.,* 2001, vol. 47 (1), 83-97
- **MERCER et al.** *Virology,* 1997, vol. 229, 193-200
- **MERCER et al.** *Virology,* 1995, vol. 212, 689-704
- **VILCEK et al.** *J. Clin. Microbiol.,* 1994, vol. 32, 2225-2231